(19) Europäisches Patentamt
European Patent Office
Office européen des brevets

(11) EP 0 489 423 B1

(12) **EUROPÄISCHE PATENTSCHRIFT**

(45) Veröffentlichungstag und Bekanntmachung des Hinweises auf die Patenterteilung:
**06.11.1996 Patentblatt 1996/45**

(51) Int Cl.[6]: **C07J 9/00**, A61K 31/575, C07J 41/00, C07J 43/00 // C07J31/00

(21) Anmeldenummer: **91120841.1**

(22) Anmeldetag: **04.12.1991**

(54) **Gallensäurederivate, Verfahren zu ihrer Herstellung und Verwendung dieser Verbindung als Arzneimittel**

Bile-acid derivatives, a process for their preparation and their use as medicines

Acides-billiaires, un procédé pour leur préparation et leur utilisation comme médicaments

(84) Benannte Vertragsstaaten:
**AT BE CH DE DK ES FR GB GR IT LI LU NL SE**

(30) Priorität: **06.12.1990 DE 4038833**

(43) Veröffentlichungstag der Anmeldung:
**10.06.1992 Patentblatt 1992/24**

(73) Patentinhaber: **HOECHST AKTIENGESELLSCHAFT**
**65926 Frankfurt am Main (DE)**

(72) Erfinder:
- **Kramer, Werner, Dr. Dr.**
  **W-6500 Mainz (DE)**
- **Wess, Günther, Dr.**
  **W-6455 Erlensee (DE)**
- **Müllner, Stefan, Dr.**
  **W-6203 Hochhe (DE)**
- **Neubauer, Horst, Dr.**
  **W-6240 Königstein/Ts. (DE)**

(56) Entgegenhaltungen:
**EP-A- 0 092 073** **EP-A- 0 202 703**
**DE-A- 3 742 798** **US-A- 4 418 059**

- **JOURNAL OF THE CHEMICAL SOCIETY, PERKIN TRANSACTIONS 2. Nr. 10, 1978, LETCHWORTH GB Seiten 1007 - 1010; A. GRAHAM CAIRNS-SMITH ET AL: 'Reactions of p-Nitrophenyl Dodecanoate in Normal and Modified Cholic Acid Micelles'**
- **JOURNAL OF INCLUSION PHENOMENA AND MOLECULAR RECOGNITION IN CHEMISTRY Bd. 7, Nr. 2, 1989, US Seiten 155 - 168; J. F. KINNEARY ET AL: 'Progress Toward Artificial Metalloenzymes: New Ligands for Transition Metal Ions and Neutral Molecules'**
- **CHEMICAL ABSTRACTS, vol. 108, no. 7, 15. Februar 1988, Columbus, Ohio, US; abstract no. 56445, C. BURROWS ET AL: 'Synthesis and Conformational Studies of a New Host System Based on Cholic Acid' Seite 762 ;Spalte 2 ;**
- **CHEMICAL ABSTRACTS, vol. 63, no. 13, 20. Dezember 1965, Columbus, Ohio, US; abstract no. 18614H, G. B. CRIPPA ET AL: 'New Radioprotective Agents; Substituted Amides of Cholic Acid'**
- **CHEMICAL ABSTRACTS, vol. 60, no. 5, 2. März 1964, Columbus, Ohio, US; abstract no. 5591A, A.M. BELLINI ET AL: 'Substituted Cholic Acid Amides' Seite 5591 ;Spalte 1 ;**
- **CHEMICAL ABSTRACTS, vol. 64, no. 5, 28. Februar 1966, Columbus, Ohio, US; abstract no. 6718A, AKIRA TAKAMIZAWA ET AL: 'Synthesis of O,S- Dicholanoylthiamine Derivatives' Seite 6718 ;Spalte 2 ;**
- **CHEMICAL ABSTRACTS, vol. 62, no. 6, 15. März 1965, Columbus, Ohio, US; abstract no. 6542D, AKIRA TAKAMIZAWA ET AL: 'Thiamine Derivatives' Seite 6542 ;Spalte 2**
- **LIPIDS Bd. 12, Nr. 11, November 1977, US Seiten 922 - 929; P. P. NAIR ET AL: 'Lithocholic Acid in Human Liver: Identification of epsilon-Lithocholyl Lysine in Tissue Protein'**

EP 0 489 423 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gallensäuren haben eine wichtige physiologische Funktion bei der Fettverdauung, z.B. als Cofaktoren der pankreatischen Lipasen und als natürliche Detergentien zur Solubilisierung von Fetten und fettlöslichen Vitaminen. Als Endprodukt des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enterohepatischen Kreislauf wieder zurückgewonnen. Sie gelangen über die Mesenterialvenen des Dünndarms und das Pfortadersystem wieder zur Leber zurück. Bei der Rückresorption im Darm spielen sowohl aktive als auch passive Transportprozesse eine Rolle. Die Hauptmenge der Gallensäuren wird am Ende des Dünndarms, dem terminalen Ileum, durch ein spezifisches $Na^+$-abhängiges Transportsystem rückresorbiert und gelangen mit dem Mesenterialvenenblut über die Pfortader zur Leber zurück, um von den Leberzellen erneut in die Zelle sezerniert zu werden. Im enterohepatischen Kreislauf treten die Gallensäuren sowohl als freie Säuren, aber auch in Form von Glycerin- und Taurinkonjugaten in Erscheinung.

Nichtresorbierbare, unlösliche, basische, vernetzte Polymere werden seit geraumer Zeit zur Bindung von Gallensäuren verwendet und aufgrund dieser Eigenschaften therapeutisch genutzt. Als Therapieobjekt werden alle Erkrankungen, bei denen eine Hemmung der Gallensäurerückresorption im Darm, insbesondere im Dünndarm, wünschenswert erscheint, angesehen. Beispielsweise werden die chologene Diarrhoe nach Ileumresektion, oder auch erhöhte Cholesterin-Blutspiegel auf diese Weise behandelt.

Im Falle des erhöhten Cholesterin-Blutspiegels kann durch den Eingriff in den enterohepatischen Kreislauf eine Senkung dieses Spiegels erreicht werden. Durch Senkung des im enterohepatischen Kreislauf befindlichen Gallensäurepools wird die entsprechende Neusynthese von Gallensäuren aus Cholesterin in der Leber erzwungen. Zur Dekkung des Cholesterinbedarfs in der Leber, wird auf das im Blutkreislauf befindliche LDL-Cholesterin (Low Density Lipoprotein) zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus. Bislang stellten diese polymeren, unlöslichen Ionenaustauscher-Harze (im folgenden bezeichnet als "Resins") die einzige Möglichkeit dar, den enterohepatischen Kreislauf hinsichtlich erhöhter Gallensäureausscheidung und daraus folgender Senkung des Cholesterinspiegels zu beeinflussen.

Dabei ist für die als Arzneimittel in der Verwendung befindlichen "Resins", z.B. Colestyramin (enthält quartäre Ammoniumgruppen) oder Colestipol (enthält sekundäre bzw. tertiäre Aminogruppen) die zweckmäßige Tagesdosis sehr hoch. Sie beträgt z.B. für Colestyramin 12-24 g, Tageshöchstdosis 32 g, 15-30 g ist die empfohlene Colestipol-Dosis. Neben der hohen Dosierung erschweren Geschmack und Geruch die Patienten Compliance.

Die bekannten Nebenwirkungen der "Resins" gehen auf mangelnde Selektivität (z.B. Avitaminosen) zurück, die auch bei der Dosierung simultan gegebener Medikamente berücksichtigt werden müssen, aber auch auf Gallensäureverarmung, die verschiedene gastrointestinale Störungen (Obstipation, Steatorrhoe) unterschiedlichen Grades hervorrufen. Für beide Präparate wurde eine therapeutische Bedeutung durch Kombination mit anderen hypolipidämisch wirkenden Pharmaka wie Fibraten, HMG-CoA-Reduktase-Inhibitoren, Probucol (vgl. z.B. M.N.Cayen, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Deshalb erscheint es bedeutungsvoll, bei dem gegebenen Wirkungsprinzip geeignete Stoffe ohne die Nachteile der gegenwärtig verwendeten Präparate zu finden.

Folgende Merkmale der genannten Präparate und insbesondere von Colestipol sind als verbesserungswürdig anzusehen:

- Die hohen Tagesdosen, die zurückzuführen sind auf eine relativ geringe Bindungsrate bei neutralem pH in isotonen Medium und der (teilweisen) Wiederfreisetzung der adsorbierten Gallensäuren.
- Die qualitative Verschiebung der Gallensäurezusammensetzung der Galle mit abnehmender Tendenz für Chenodesoxycholsäure und die damit verbundene zunehmende Gefahr für Cholelithiasis.
- Das Fehlen einer dämpfenden Wirkung auf den Cholesterinstoffwechsel der Darmbakterien.
- Die zu hohe Bindungsrate von Vitaminen und Pharmaka macht einen Substitutionsbedarf an diesen Stoffen und Blutspiegelkontrollen eventuell notwendig.
- Durch die Bindung der Gallensäuren an die "Resins" bereits im Zwölffingerdarm und oberem Dünndarm stehen Gallensäuren für die Verdauung von Fetten in ungenügender Menge zur Verfügung, so daß Fettverdauungsstörungen auftreten.

- Die Darreichungsform ist bislang als unzureichend anzusehen.

Es bestand deshalb die Aufgabe, ein Arzneimittel zu finden, das in der Lage ist, den atherogenen Cholesterinanteil im Blut herabzusetzen bzw. den enteropatischen Kreislauf hinsichtlich erhöhter Gallensäureausscheidung und daraus folgender Senkung des Cholesterinspiegels zu beeinflussen, welches aber nicht die Nachteile des bisher verwendeten "Resins" aufweist.

EP-A-0202703 beschreibt Anhydride von Gallensäuren. A.G. Cairns-Smith et al., J. Chem. Soc. Perkin Trans. 10, 1007 (1978), J.F. Kinneary et al., J. Incl. Phenomena and Molecular, Recogn. in Chem. 7, 155 (1989) und Chem. Abstr. 108, 56445q (1988) beschreiben dimere Gallensäuren.

Chem. Abstr. 63, 18614h (1965) beschreibt Gallensäuren, die eine Disulfidbindung beinhalten.

Chem. Abstr. 60, 5591a (1964) beschreibt Gallensäurederivate.

Chem. Abstr. 64, 6718a (1966) beschreibt mit Thiamin veresterte Gallensäurederivate.

US-A-4418059 beschreibt Magnesiumsalze von Chenodeoxy- bzw. Ursodeoxygallensäuren.

Überraschenderweise wurde nun gefunden, daß dimere Gallensäurederivate der allgemeinen Formel I eine Alternative zu den bekannten Resins darstellen:

G1-X-G2 (I)

in der G1 eine Verbindung der allgemeinen Formel II

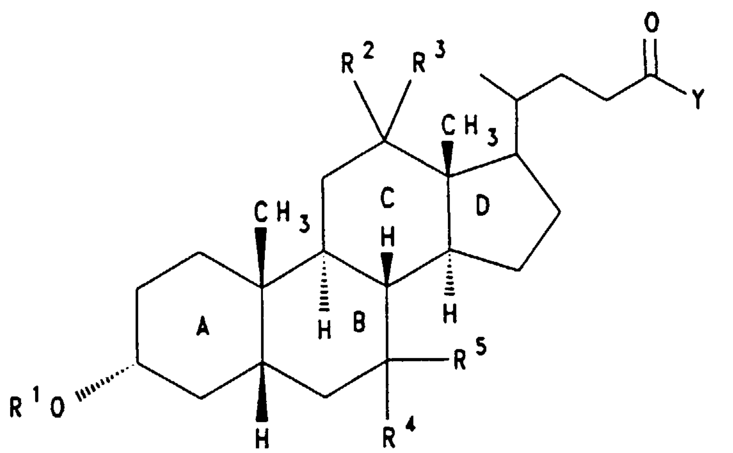

bedeutet,
worin

Y eine freie Valenz zur Bindung der Gruppe X oder die folgenden Bedeutungen hat -OL, -NHL, $-NL_2$, eine über die Aminogruppe gebundene Aminosäure oder Aminosulfonsäure, wie z. B.

$-NHCH_2-CO_2H$, $-NH-CH_2CH_2-SO_3H$, $-N(CH_3)-CH_2CH_2-SO_3H$,

$-N(CH_3)-CH_2CO_2H$, $-NH-CH(R^6)CO_2H$

und deren ($C_1$-$C_4$)-Alkylester und Alkali- und Erdalkalisalze, -OKa, wobei Ka ein Kation bedeutet wie z.B. ein Alkali oder Erdalkaliion oder auch ein quartäres Ammonium und,
wobei L

H, einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, ($C_1$-$C_4$)-Alkyl oder ($C_1$-$C_4$)-Alkoxy,
einen Benzylrest, der unsubstituiert oder 1-3fach substituiert ist mit F, Cl, Br, ($C_1$-$C_4$)-Alkyl oder ($C_1$-$C_4$)-Alkoxy,
und $R^6$
Methyl, Isopropyl, Isobutyl, 2-Butyl, Benzyl, 4-Hydroxybenzyl, Hydroxymethyl, 1-Hydroxyethyl, $H_3CSCH_2CH_2$-, $HO_2CCH_2$-, $HO_2CCH_2CH_2$-
bedeutet,

$R^1$ eine freie Valenz zur Bindung der Gruppe X oder H,

einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, ($C_1$-$C_4$)-Alkyl oder ($C_1$-$C_4$)-Alkoxy, $-N^{\oplus}H_3$, $-OPO_3^{\ominus}$,
einen Benzylrest, der im Kern unsubstituiert oder 1-3fach substituiert ist mit F, Cl, Br, ($C_1$-$C_4$)-Alkyl, ($C_1$-$C_4$)-Alkoxy, $-N^{\oplus}H_3$, $-OPO_3^{\ominus}$ oder Phenyl, das seinerseits 1-3fach substituiert sein kann mit F, Cl, Br, ($C_1$-$C_4$)-Alkyl oder ($C_1$-$C_4$)-Alkoxy, $-N^{\oplus}H_3$, $-OPO_3^{\ominus}$,
einen Biphenylmethylrest, der unsubstituiert oder 1-3fach substituiert ist mit F, Cl, Br, ($C_1$-$C_4$)-Alkyl oder ($C_1$-$C_4$)-Alkoxy, $-N^{\oplus}H_3$, $-OPO_3^{\ominus}$,
einen Triphenylmethylrest, der unsubstituiert oder 1-3fach substituiert ist mit F, Cl, Br, ($C_1$-$C_4$)-Alkyl oder ($C_1$-$C_4$)-Alkoxy, $-N^{\oplus}H_3$, $-OPO_3^{\ominus}$ ,
einen 1- oder 2-Naphthylmethylrest, der unsubstituiert oder 1-3fach substituiert ist mit F, Cl, Br, ($C_1$-$C_4$)-Alkyl oder ($C_1$-$C_4$)-Alkoxy, $-N^{\oplus}H_3$, $-OPO_3^{\ominus}$ ,
einen 9-Fluorenylrest, der unsubstituiert oder 1-3fach substituiert ist mit F, Cl, Br, ($C_1$-$C_4$)-Alkyl oder ($C_1$-$C_4$)-Alkoxy, $-N^{\oplus} H_3$, $-OPO_3^{\ominus}$,
einen 2-, 3- oder 4-Pyridylrest,
einen

Rest 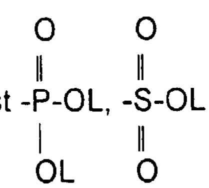 oder 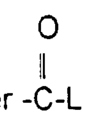

wobei L die oben angegebene Bedeutung hat

$R^2$ bis $R^5$, wobei $R^2$ und $R^3$ bzw. $R^4$ und $R^5$ jeweils gemeinsam der Sauerstoff einer Carbonylgruppe, oder einzeln und jeweils unabhängig voneinander

H, OT, -ST, -NHT, 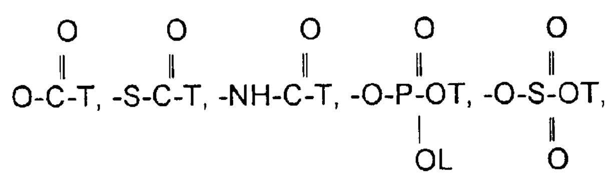 , -T

wobei L die obengenannte Bedeutung hat und T die Bedeutung von L aufweist oder eine freie Valenz zur Bindung der Gruppe X,

mit der Beschränkung, daß insgesamt nur eine freie Valenz zur Bindung der Gruppe X ausgeht,

X eine Einfachbindung oder eine Gruppe der allgemeinen Formel III

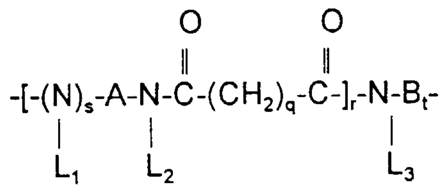 (III)

wobei

A eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

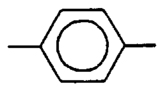 , 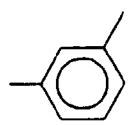 oder 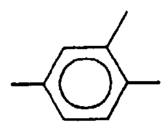

erfolgt und die Kette insgesamt 2 bis 12, vorzugsweise 2 bis 6 Kettenglieder p umfaßt.

B eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

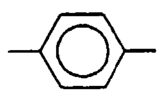 , 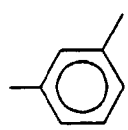 oder 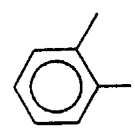

erfolgt und

die Kette insgesamt 2 bis 18, vorzugsweise 2 bis 12 Kettenglieder n enthält,

| | |
|---|---|
| $L_1$, $L_2$, $L_3$ | gleich oder verschieden sind und die Bedeutung von L haben, sowie |
| q | 0 bis 5, |
| r | 0 oder 1 und |
| s | 0 oder 1 |
| t | 0 oder 1 |

bedeuten,

G2 eine Verbindung der allgemeinen Formel IV bedeutet

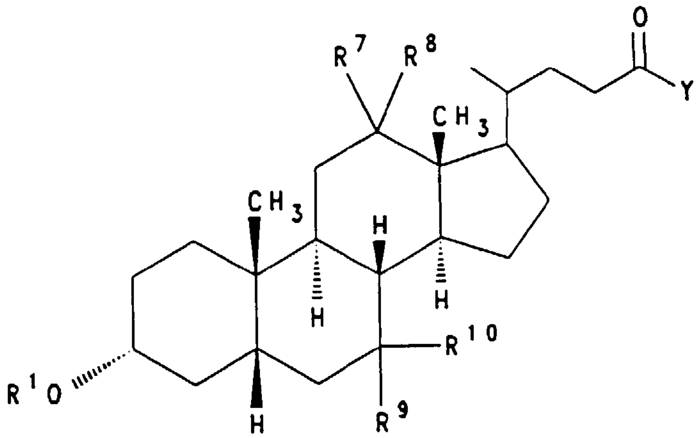

wobei

Y und $R^1$ die unter G1 angegebene Bedeutung haben sowie

$R^7$ bis $R^{10}$, wobei $R^7$ und $R^8$ bzw. $R^9$ und $R^{10}$ jeweils gemeinsam der Sauerstoff einer Carbonylgruppe oder einzeln und jeweils unabhängig voneinander H, OT, -ST, -NHT,

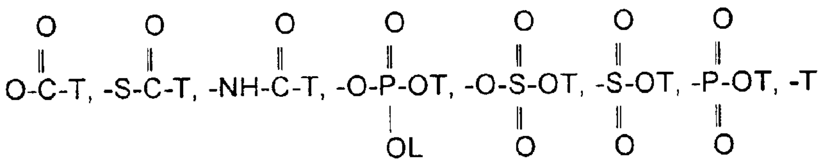

bedeutet, wobei L und T die unter G1 angegebene Bedeutung haben,
ebenfalls mit der Beschränkung, daß insgesamt von G2 nur eine freie Valenz zur Bindung der Gruppe X ausgeht,
wobei Gallensäurederivate der folgenden Formeln ausgeschlossen sind:

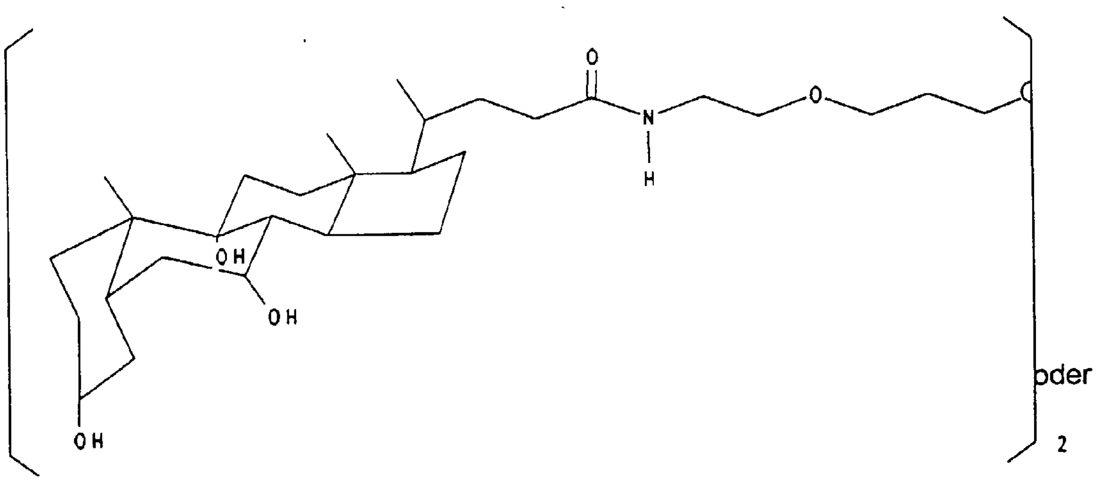

oder

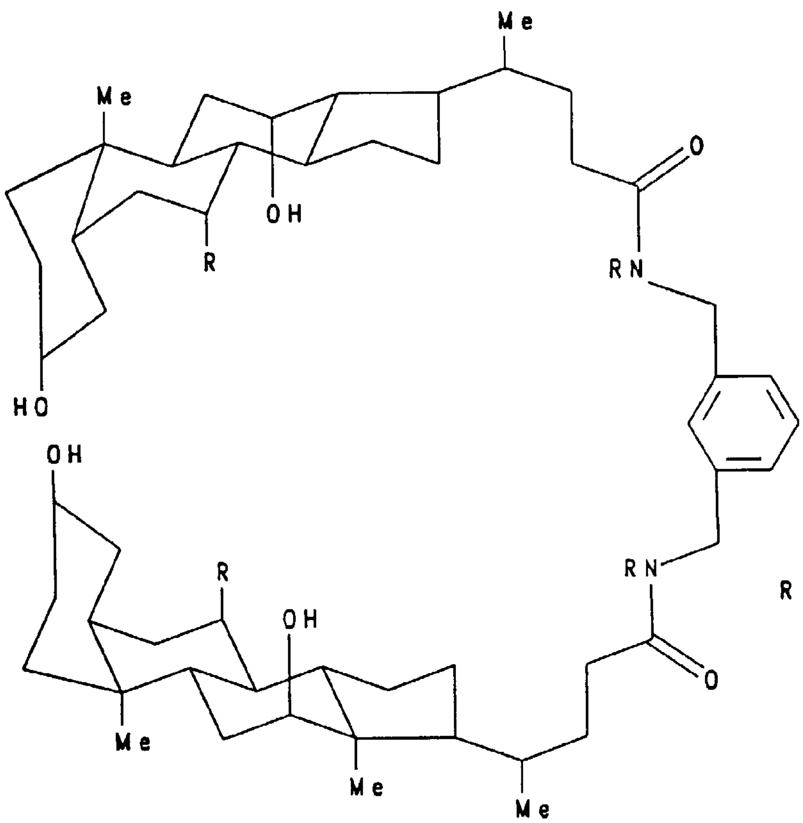

R = H oder OH

Die erfindungsgemäßen Verbindungen gemäß der allgemeinen Formel I besitzen eine hohe Affinität zum spezifischen Gallensäuretransportsystem des Dünndarms und hemmen die Gallensäureresorption in konzentrationsabhängiger und kompetitiver Weise.

Weiterhin werden die erfindungsgemäßen Verbindungen selbst nicht resorbiert und gelangen somit nicht in den Blutkreislauf. Durch Anwendung dieses neuen Wirkstoffprinzips kann nun sehr viel spezifischer und effizienter der enterophepatische Kreislauf der Gallensäuren unterbrochen werden und mit höherer Effizienz in den enterohepatischen Kreislauf eingegriffen werden, als es die "Resins" bislang erlaubten.

Durch die Verwendung der erfindungsgemäßen Verbindungen, die nach dem beschriebenen neuen Wirkprinzip arbeiten, können die aufgelisteten Mängel der am Markt befindlichen, in den enterohepatischen Kreislauf eingreifenden "Resins", völlig umgangen werden. Durch reversible Inhibition der Gallensäurerückresorption im Dünndarm wird auf wesentlich effektivere Weise die im enterohepatischen, Kreislauf befindliche Gallensäurekonzentration vermindert, so daß eine Senkung des Cholesterinspiegels im Serum erfolgt. Avitaminosen sind bei Anwendung der erfindungsgemäßen Verbindungen danach ebensowenig zu erwarten, wie die Beeinflussung der Resorption anderer Arzneimittel oder auch die negative Wirkung auf die Darmflora. Weiterhin werden die bekannten Nebenwirkungen (Obstipation, Steratorrhoe) nicht beobachtet, d.h. die Fettverdauung wird nicht negativ beeinflußt. Wegen der hohen Affinität der erfindungsgemäßen Verbindungen zum hochspezifischen Gallensäuretransportsystems im Dünndarm kommt man im Gegensatz zu den "Resins" mit sehr viel geringeren Tagesdosen aus, so daß die Akzeptanz solcher Arzneimittel bei Arzt und Patient und die Compliance der Patienten sehr hoch ist.

Besonders bevorzugt sind Verbindungen der allgemeinen Formel I, in denen die Verknüpfung, durch die Gruppe X nicht über identische Ringe der Verbindungen G1 und G2 erfolgt.

Insbesondere bevorzugt sind Verbindungen der allgemeinen Formel I, in denen die Verknüpfung durch die Gruppe X unsymmetrisch über die Ringe A bzw. D der Verbindungen G1 und G2 erfolgt.

Die Erfindung betrifft ferner ein Verfahren zur Herstellung von Verbindungen der allgemeinen Formel I, das dadurch gekennzeicht ist, ,daß man

a) im Falle von x = Einfachbindung geeignete reaktionsfähige Formen von G1 und G2 nach im Prinzip bekannten Verfahren miteinander zur Reaktion bringt oder

b) im Falle von x = Brückengruppe

α) reaktionsfähige Formen von G1-X mit G2 bzw.
β) reaktionsfähige Formen von G2-X mit G1

nach im Prinzip bekannten Verfahren miteinander zur Reaktion bringt oder

c) aus G1-X1 nach bekannten oder soweit nicht bekannt, nach den unten näher beschriebenen Verfahren, Verbindungen der allgemeinen Formel I (G1-X-G2) herstellt, wobei X aus X1 und X2 entsteht durch Ausbildung einer kovalenten Bindung, insbesondere innerhalb einer Kondensations- oder Substitutionsreaktion.

a) X = Einfachbindung

Die Gallensäuren G1 werden entweder in freier Form oder in geschützter Form eingesetzt. Nach der Verknüpfung mit G2, die ebenfalls in freier oder geschützter Form vorliegt, erfolgt gegebenenfalls die Abspaltung der Schutzgruppen und die Umwandlung der C-24 Carboxylfunktion in ein obengenanntes Derivat. Als Schutzgruppen für die Alkoholgruppen eignen sich zweckmäßigerweise Formyl, Acetyl, Tetrahydropyranyl oder t- Butyldimethylsilyl. Als Schutzgruppen für die C-24 Carboxylgruppe kommen verschiedene Alkyl oder Benzylester in Frage, aber auch z.B. Orthoester.

Beispielsweise reagiert Gallensäure bevorzugt an Position 3, aber auch an Position 7 mit aktivierten Formen von Carbonsäuren, wie Säurechloriden oder gemischten Anhydriden unter Zusatz von Basen wie Trialkylamin, Pyridin, aber auch NaOH bei Zimmertemperatur in geeigneten Lösungsmitteln wie Tetrahydrofuran, Methylenchlorid oder Essigester, aber auch Dimethylformamid (DMF) oder Dimethoxyethan (DME).

Die verschiedenen Isomeren können z.B. chromatographisch getrennt werden.

Durch die Verwendung von geeigneten Schutzgruppen läßt sich die Reaktion selektiv durchführen. Analog lassen sich entsprechende Aminogallensäuren in entsprechende Amide überführen. Auch hier kann die Reaktion entweder mit geschützten oder freien Gallensäuren durchgeführt werden.

Analog lassen sich weitere erfindungsgemäße Verbindungen nach bekannten Standardverfahren verknüpfen.

b) X = eine Brückengruppe

Die unter a) angegebenen Verfahren werden auch angewendet, um die Verknüpfung G1-X mit G2 bzw. G1 mit X- G2 durchzuführen. Zweckmäßigerweise setzt man auch hier den Gallensäureteil entweder geschützt oder ungeschützt ein.

Ein bevorzugtes Herstellungsverfahren besteht darin, reaktionsfähige Formen von G1 mit reaktionsfähigen Formen X-G2 umzusetzen. Gegebenenfalls schließen sich nach der Verknüpfung die Abspaltung von Schutzgruppen und die Umwandlung von C- 24 Carboxyl in Derivate an.

Die Herstellung von reaktionsfähigen
Gallensäurebausteinen X-G ist im Formelschema 1-4 am Beispiel der Cholsäure angegeben (z.B. r=o).

Herstellung von reaktionsfähigen Gallensäurebausteinen X-G2 am Beispiel der Cholsäure, Schema 1-4

Schema 1: X-G2 ohne Schutzgruppe

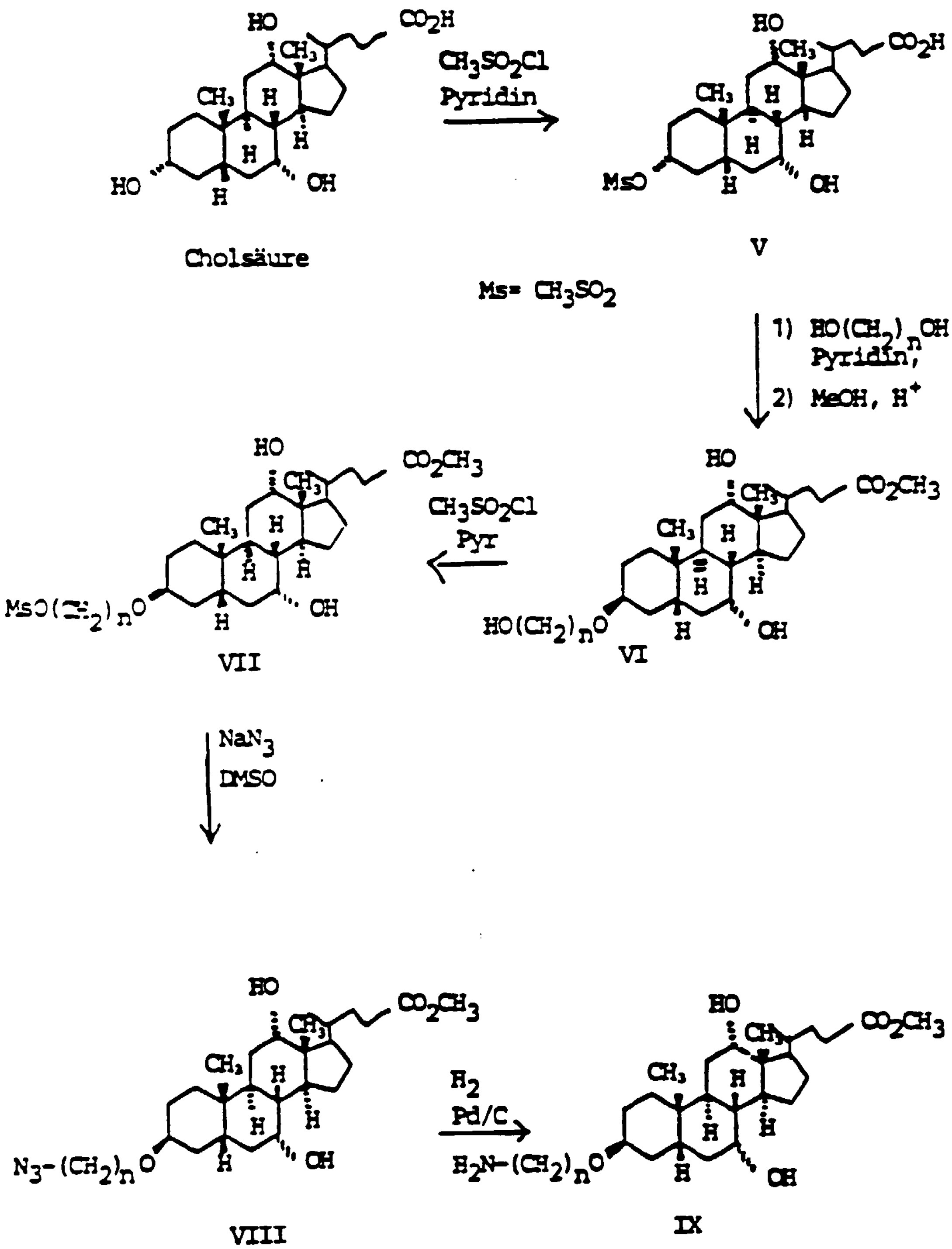


Cholsäure

V

VI

VII

VIII

IX

Schema 2: X-G2 mit THP Schutzgruppe (THP = Tetrahydropyranyl)

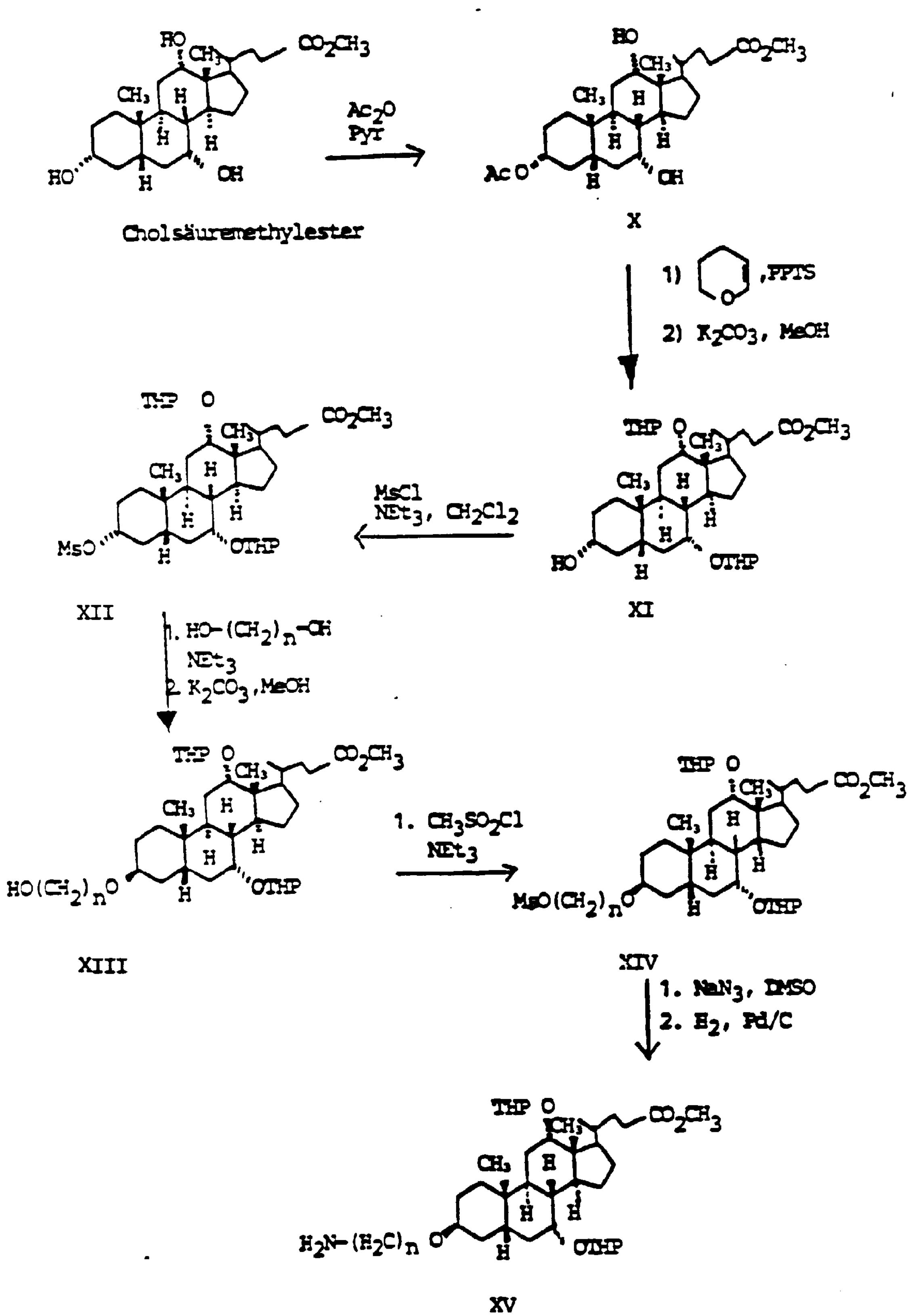


Cholsäuremethylester

X

XI

XII

XIII

XIV

XV

Schema 3: X-G2 mit t-Bu$Me_2$Si Schutzgruppe

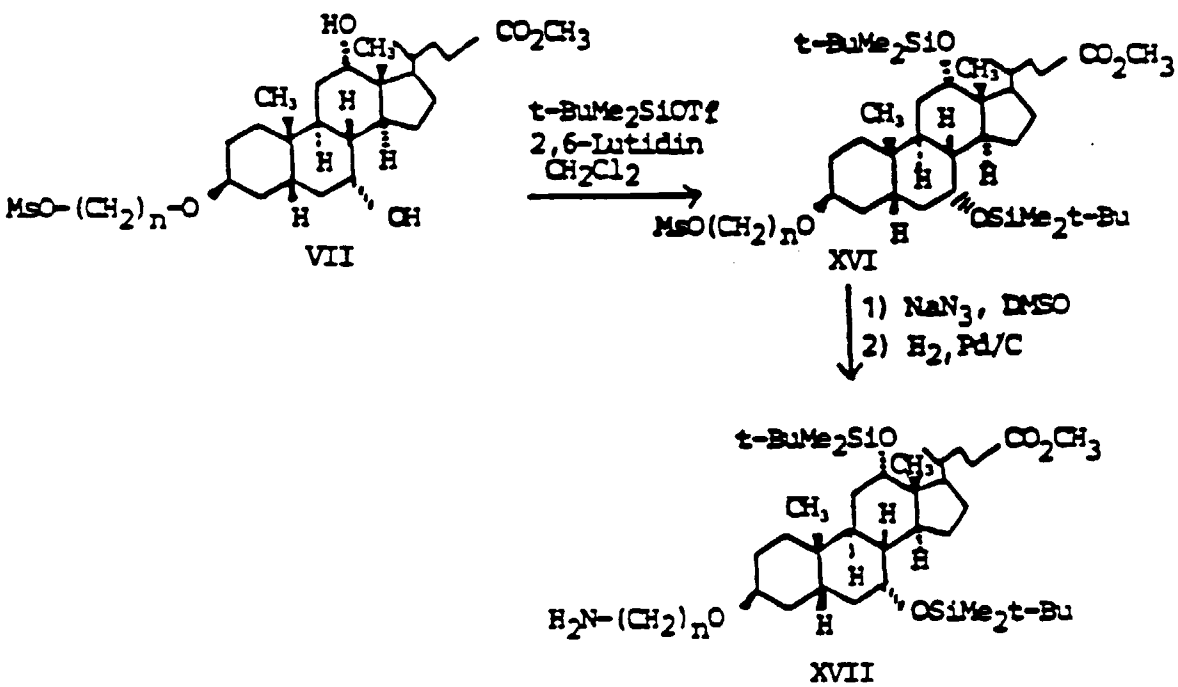


VII

XVI

XVII

Schema 4: X-G2 mit α-Konfiguration an 3-C

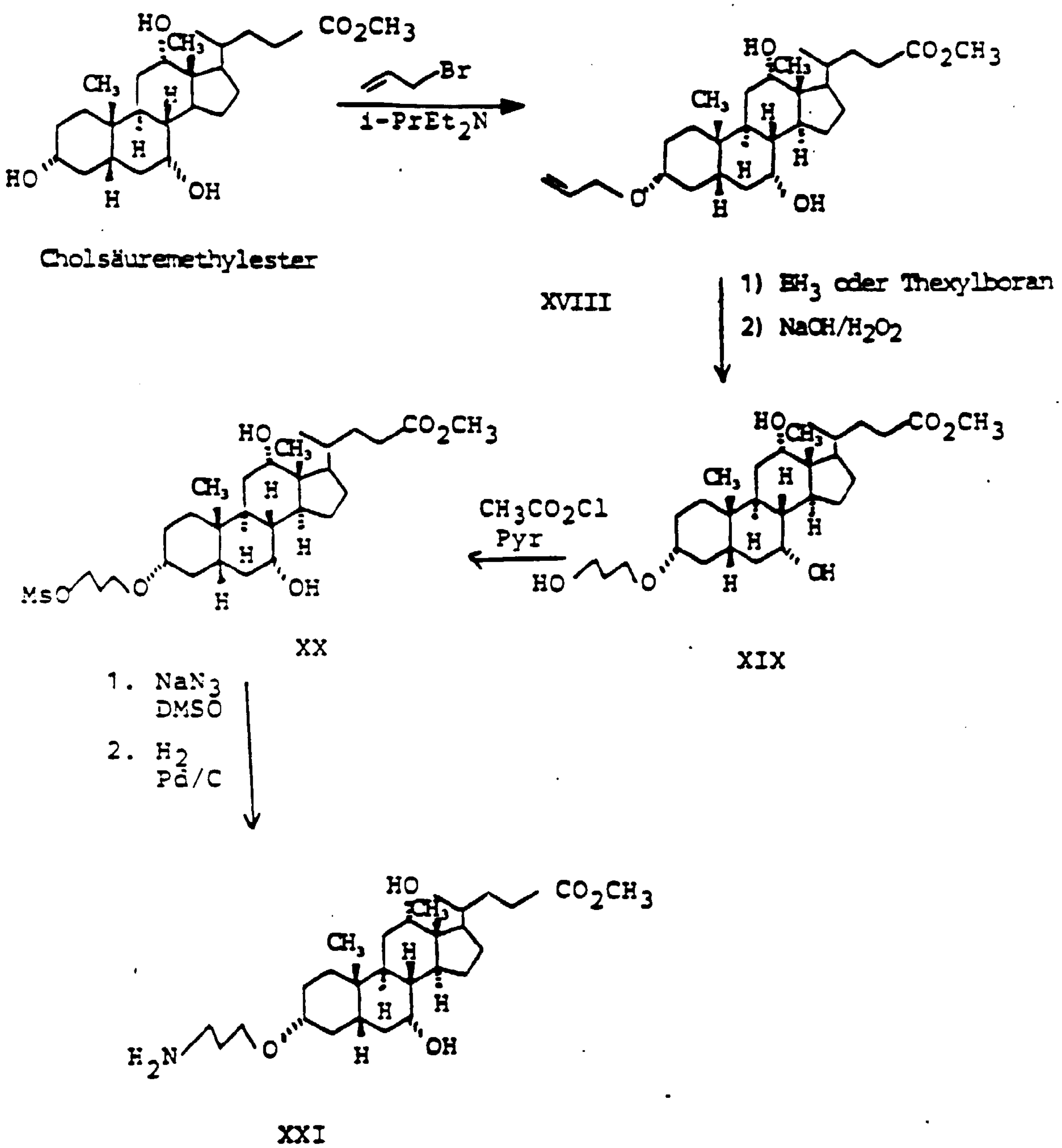


Cholsäuremethylester

XVIII

XIX

XX

XXI

Der Austausch der 3 OH-Gruppen durch Diole $HO(CH_2)_nOH$ gelingt durch Umsetzung der entsprechenden Mesylate mit den entsprechenden Diolen, die man bevorzugt im Überschuß einsetzt, unter Zusatz von Basen wie Pyridin, Lutidin, aber auch Triethylamin.

Die primären OH-Gruppen der Verbindungen VI und XIII lassen sich nach Standardverfahren weiter umsetzen. So kann z.B. XIII mit Oxidationsmitteln in die entsprechende Carbonsäure XXII [mit R(11) gleich THP] überführt werden, bevorzugt mit Chrom- VI-Reagenzien oder verschiedenen Kaliumpermanganat-Systemen. Entsprechend eignen sich auch andere Schutzgruppen.

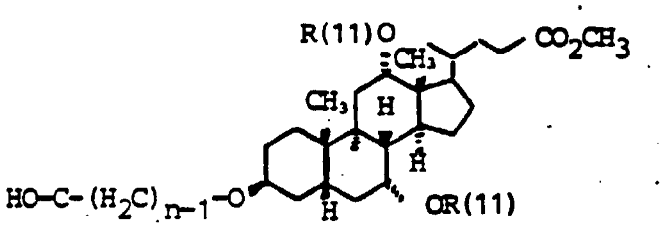

R(11)= THP, Benzyl
t-BuMe$_2$Si,
Benzyloxycarbonyl (Z),
Acetyl

Die Verbindungen XG2 (IX, XV, XVII oder XXI) können nur direkt mit G1 bzw. dessen Derivate umgesetzt werden oder nach Überführung in X2- G2 mit einem entsprechend modifizierten G1-X1 in eine Verbindung gemäß der allgemeinen Formel I (G1-X-G2).

Letzterer Fall wird in Teil c) beschrieben.

c) Zur Herstellung einer Verbindung des Typs X2-G2 wird, wie hier am Beispiel einer der Verbindungen IX, XV, XVII oder XXI, mit reaktionsfähigen Formen von Carbonsäuren, z.B. gemischten Anhydriden, Säurechloriden oder z.B. im Falle von g=2 mit Bernsteinsäureanhydrid in einem geeigneten Lösungsmittel, wie z.B. Dichlormethan, Toluol oder Pyridin und in Gegenwart von Triethylamin bei -20 bis Raumtemperatur zu Carbonsäuren XXIII umgesetzt.

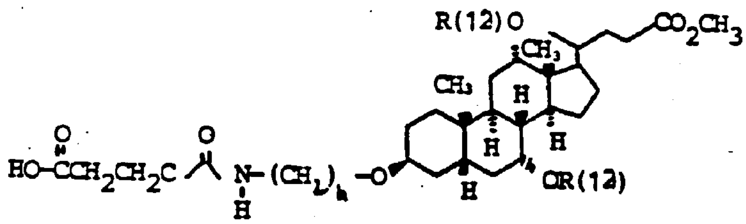

XXIII

R(12) = H, THP, t-BuMe$_2$Si Acetyl, Benzyl, Benzyloxycarbonyl (Z)

Die Verbindung (XXIII) kann nun ihrerseits wiederum mit Verbindungen IX, XV, XVII oder XXI (hier: Typ G1-X1) zu Verbindungen der allgemeinen Formel I umgesetzt werden, in denen S=0 gilt (hier Verbindung XXIV).

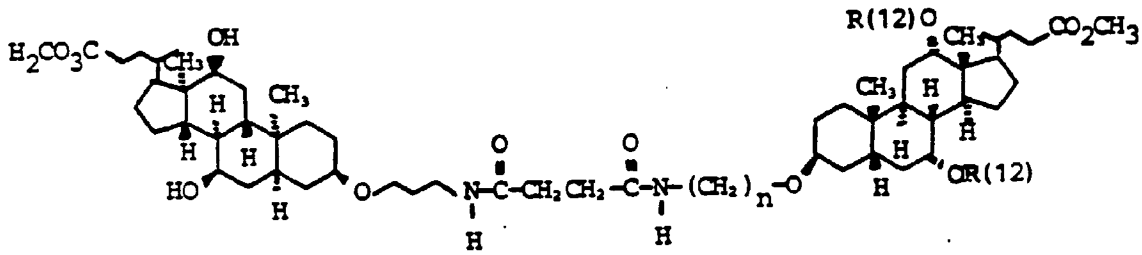

XXIV

Sollen Verbindungen der allgemeinen Formel I hergestellt werden, in denen s=1 gilt, so werden z.B. Verbindungen der allgemeinen Formel II mit Verbindungen

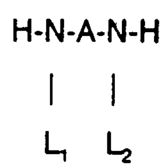

umsetzt, wobei A, $L_1$ und $L_2$ die oben angegebene Bedeutung hat. Im Falle der Ester (d.h.

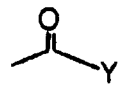

ist eine Esterfunktion) wird dieser direkt mit

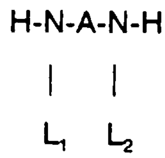

umgesetzt, im Falle der freien Säure

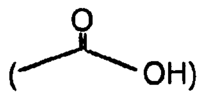

müssen diese noch in reaktionsfähige Säurederivate, wie z.B. Gemischte Anhydride, Säurechloride umgesetzt werden. Die in dieser Weise entstandenen Verbindungen vom Typ G1-X1 werden nun mit Verbindungen vom Typ G2-X2 in Verbindungen vom Typ G1-X-G2 überführt.

Die obengenannten Ausführungen zur Herstellung der Verbindungen vom Typ G1-X-G2, in denen die Verknüpfung der beiden Gallensäuren G1 und G2 über ihre jeweiligen A-Ringe erfolgt ist, gelten auch in entsprechend dem Fachmann bekannten, abgewandelten Methoden für Verknüpfungen A-D, A-B, A-G, sowie D-D, B-B,, C-C, B-D, B-C, C-D.

Die Substituenten der Gallensäure G1 bzw. G2, $R^1$ bis $R^{10}$, können sowohl vor der Verknüpfung von G1 mit G2 über X oder aber danach eingeführt werden. Eine nach der Verknüpfung von G1 mit G2 erfolgte Einführung der Substituenten ist nur dann möglich, wenn die entsprechenden Substituenten nicht selbst an der Brückenbildung zwischen G1 bzw. G2 und X beteiligt sind. Vorzugsweise werden deshalb diese Substitutionen vor den eigentlichen Verknüpfungsreaktionen von G1 und G2 über X vorgenommen.

Die Erfindung betrifft weiterhin die Verwendung der erfindungsgemäßen Verbindungen zur Herstellung eines Heilmittels.

Hierzu werden die Verbindungen der allgemeinen Formel I gelöst oder suspendiert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl, Lebertran, Ethern, wie z.B. Diethylenglykoldimethylether oder auch Polyethern wie z. B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffen, gegeben werden.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10-1000 mg.

Die pharmakologischen Daten umfassen eine Testserie, in der die Interaktion der erfindungsgemäßen Verbindungen mit dem intestinalen Gallensäuretransportsystem im terminalen Dünndarm untersucht wurde:

1. Präparation von Bürstensaummembranvesikeln aus dem Ileum von Kaninchen

Die Präparation von Bürstensaummembranvesikeln aus den Darmzellen des Dünndarms erfolgte mit der sog. $Mg^{2+}$-Präzipitationsmethode. Männliche Neuseeland-Kaninchen (2-2,5 kg Körpergewicht) wurden durch intravenöse Injektion von 0,5 ml einer wäßrigen Lösung von 2,5 mg Tetracain HCl, 100 T 61$^R$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d.h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCI-Puffer (pH 7,1)/300 mM Mannit, 5 mM EGTA/10 mg/l Phenylmethylsulfonylfluorid/1 mg/l Trypsin Inhibitor v. Sojabohnen (32 (U/mg)/0,5 mg/l Trypsin Inhibitor v. Rinderlunge (193 U/mg)/5 mg~l Bacitracin suspendiert. Nach dem Verdünnen auf 300 ml mit eiskaltem destilliertem Wasser wurde mit einem Oltraturrax (18-Stab, 5KA Werk Staufen, BRD) 3 Minuten bei -75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3 000 x g (5000 rpm, SS-34-Rotor) wird der Niederschlag verworfen und der Überstand, der die Bürstensaummembranen enthält, 30 Minuten bei 267000 x g (15000 rpm, SS-34-Rotor) zentrifugiert. Der Überstand wurde verworfen, der Niederschlag in 60 ml 12 mM Tris/HCI-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 g (20000 rpm, SS-34- Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei - 196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in Bürstensaummembranvesikel wurde mittels der sog. Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 -µCi [$^3$H(G)). Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 µl der betreffenden Inhibitorlösung, Je nach Experiment in Na-T-Puffer bzw. K-T-Puffer gelöst. Das Inkubationsmedium wurde durch ein Polyvinylidenfluorid-Membranfilter (STHV L0 4NS, 0,45 µm, 4 mm 0, Millipore, ,Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 min) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, BRD) abgesaugt. Der Filter wurde mit 5 ml eiskalter - Stoplösung nachgewaschen.

Zur Messung der Aufnahme des radioaktiv markierten Taurocholats wurde das Membranfilter mit 4 ml des Szintillators Ouickszint 361 (Zinsser Analytik GmbH, Frankfurt, BRD) aufgelöst und die Radioaktivität durch Flüssigkeits-Szintillationsmessung in einem Meßgerät TriCarb 2500 -(Canberra, Packard GmbH Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle gehemmt war; entsprechendes gilt für die Angaben der $IC_{25}$ und $IC_{75}$-Werte. Die Ergebnisse sind in Tabelle 60 zusammengefaßt.

Die folgenden Beispiele beschreiben die Erfindung, ohne aber auf diese beschränkend zu wirken.

Beispiel 1

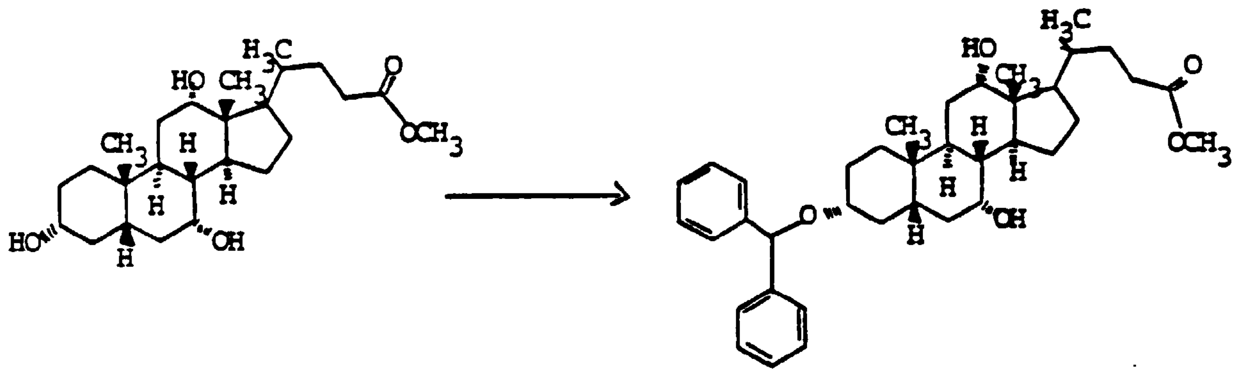

16.9 g (40 mmol) Cholsäuremethylester wurden in 120 ml N-Ethyldiisopropylamin vorgelegt, mit 11,9 g (48 mmol) Diphenylmethylbromid versetzt und 4 Stunden bei 100°C gerührt. Zur Aufarbeitung wurden 250 g Eis/20 ml Schwefelsäure nach dem Abkühlen zugegeben und mit Ethylacetat extrahiert (3x). Die vereinten organischen Phasen wurden mit Magnesiumsulfat getrocknet und eingedampft. Chromatographie auf Kieselgel (n-Heptan/Ethylacetat = 3/1) ergab 11,8 g (20 mmol, 50 %) "Beispiel 1" $C_{38}H_{52}O_5$(588)MS(FAB,3-NBA/LiCl: 595 ($mLi^+$)

In Analogie zu Beispiel 1 wurden die Beispiele der Tabellen 1 - 4 erhalten.

Tabelle 1

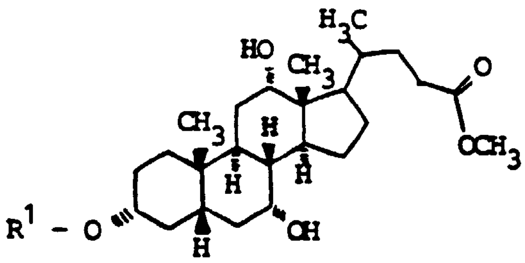

| Bsp. | $R^1$ | MS (FAB,3-NBA/LiCl oder LiJ) |
|---|---|---|
| 2 | 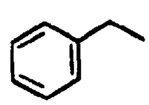 | $C_{32}H_{48}O_5$(512); 519 (M+Li$^+$) |
| 3 | 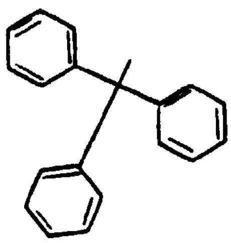 | $C_{44}H_{56}O_5$(664); 671 (M+Li$^+$) |
| 4 | 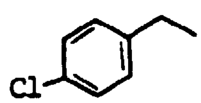 | $C_{32}H_{47}ClO_5$(546); 553 (M+Li$^+$) |
| 5 | 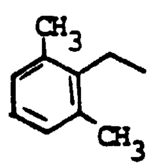 | $C_{34}H_{52}O_5$(540); 547 (M+Li$^+$) |
| 6 | 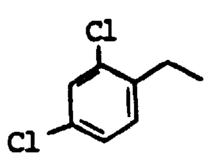 | $C_{32}H_{46}Cl_2O_5$(580); 587 (M+Li$^+$) |

Fortsetzung Tabelle 1

| Bsp. | $R^1$ | MS (FAB,3-NBA/LiCl oder LiJ) |
|---|---|---|
| 7 | 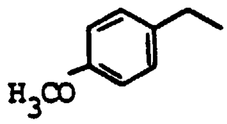 | $C_{33}H_{50}O_6$(542); 549 $(M+Li^+)$ |
| 8 | 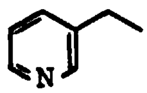 | $C_{31}H_{47}NO_5$(513); 520 $(M+Li^+)$ |
| 9 | 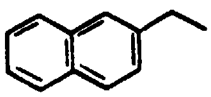 | $C_{36}H_{50}O_5$(562); 569 $(M+Li^+)$ |

Tabelle 2

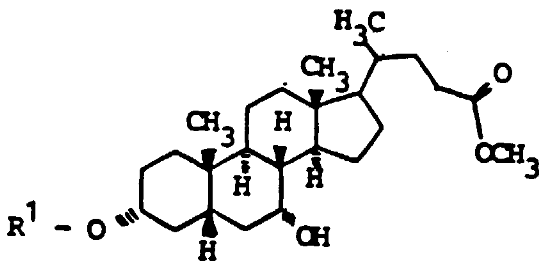

| Bsp. | $R^1$ | MS (FAB,3-NBA/LiCl oder LiJ) |
|---|---|---|
| 10 | 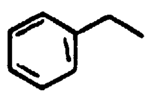 | $C_{32}H_{48}O_4$(496); 503 $(M+Li^+)$ |
| 11 | 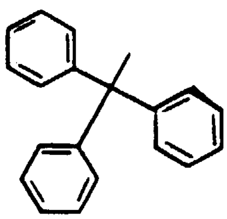 | $C_{44}H_{56}O_4$(648); 655 $(M+Li^+)$ |
| 12 | 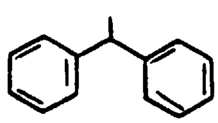 | $C_{38}H_{52}O_4$(572); 579 $(M+Li^+)$ |
| 13 | 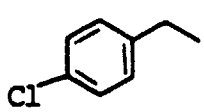 | $C_{32}H_{47}ClO_4$(530); 537 $(M+Li^+)$ |
| 14 | 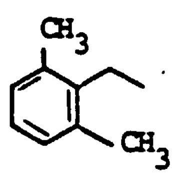 | $C_{34}H_{52}O_4$(524); 531 $(M+Li^+)$ |

Fortsetzung Tabelle 2

| Bsp. | $R^1$ | MS (FAB,3-NBA/LiCl oder LiJ) |
|---|---|---|
| 15 | 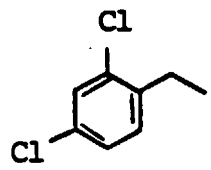 | $C_{32}H_{46}Cl_2O_4$(564); 571 (M+Li$^+$) |
| 16 | 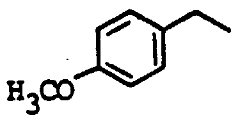 | $C_{33}H_{50}O_5$(526); 533 (M+Li$^+$) |
| 17 | 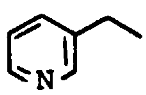 | $C_{31}H_{47}NO_4$(497); 504 (M+Li$^+$) |
| 18 | 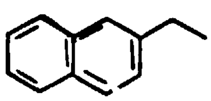 | $C_{36}H_{50}O_4$(546); 553 (M+Li$^+$) |

Tabelle 3

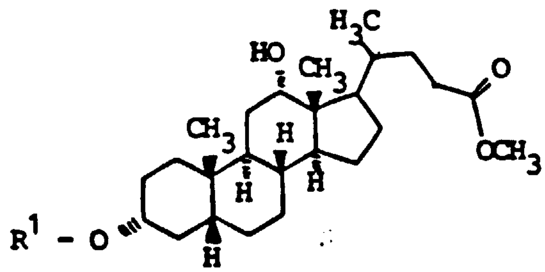

| Bsp. | $R^1$ | MS (FAB,3-NBA/LiCl oder LiJ) |
|---|---|---|
| 19 | 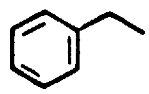 | $C_{32}H_{48}O_4$(496); 503 ($M+Li^+$) |
| 20 | 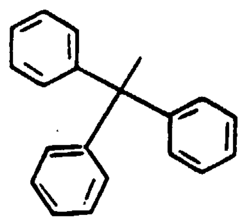 | $C_{44}H_{56}O_4$(648); 655 ($M+Li^+$) |
| 21 | 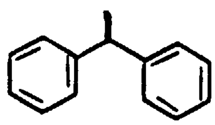 | $C_{38}H_{52}O_4$(572); 579 ($M+Li^+$) |
| 22 | 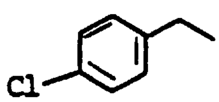 | $C_{32}H_{47}ClO_4$(530); 537 ($M+Li^+$) |
| 23 | 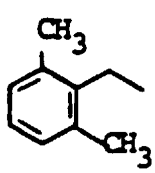 | $C_{34}H_{52}O_4$(524); 531 ($M+Li^+$) |

Fortsetzung Tabelle 3

| Bsp. | $R^1$ | MS (FAB,3-NBA/LiCl oder LiJ) |
|---|---|---|
| 24 | 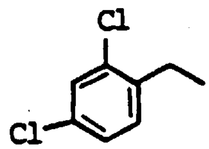 | $C_{32}H_{46}Cl_2O_4$(564); 571 (M+Li$^+$) |
| 25 | 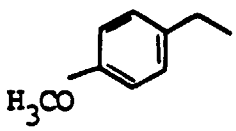 | $C_{33}H_{50}O_5$(526); 533 (M+Li$^+$) |
| 26 | 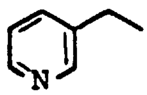 | $C_{31}H_{47}NO_4$(497); 504 (M+Li$^+$) |
| 27 | 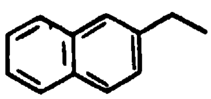 | $C_{36}H_{50}O_4$(546); 553 (M+Li$^+$) |

Tabelle 4

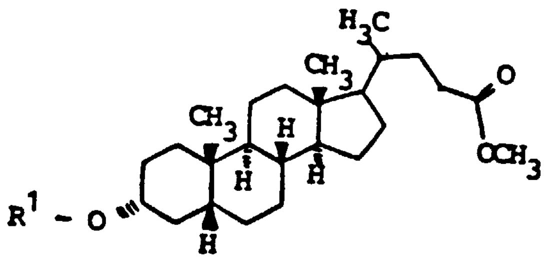

| Bsp. | $R^1$ | MS (FAB, 3-NBA/LiCl oder LiJ) |
|---|---|---|
| 28 | 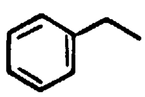 | $C_{32}H_{48}O_3$ (480); 487 $(M+Li^+)$ |
| 29 | 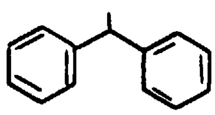 | $C_{38}H_{52}O_3$ (556); 563 $(M+Li^+)$ |
| 30 | 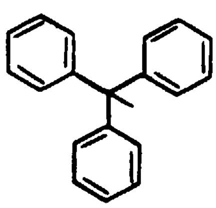 | $C_{44}H_{56}O_3$ (632); 639 $(M+Li^+)$ |
| 31 | 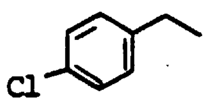 | $C_{32}H_{47}ClO_3$ (514); 521 $(M+Li^+)$ |
| 32 | 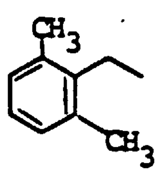 | $C_{34}H_{52}O_3$ (508); 515 $(M+Li^+)$ |

Fortsetzung Tabelle 3

| Bsp. | $R^1$ | MS (FAB,3-NBA/LiCl oder LiJ) |
|---|---|---|
| 33 | 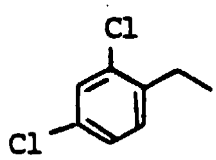 | $C_{32}H_{46}Cl_2O_3$(548); 555 ($M+Li^+$) |
| 34 | 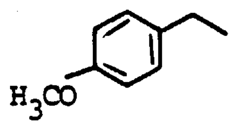 | $C_{33}H_{50}O_4$(510); 517 ($M+Li^+$) |
| 35 | 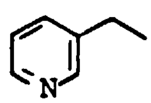 | $C_{31}H_{47}NO_3$(481); 488 ($M+Li^+$) |
| 36 | 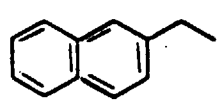 | $C_{36}H_{50}O_3$(530); 537 ($M+Li^+$) |

Beispiel 37

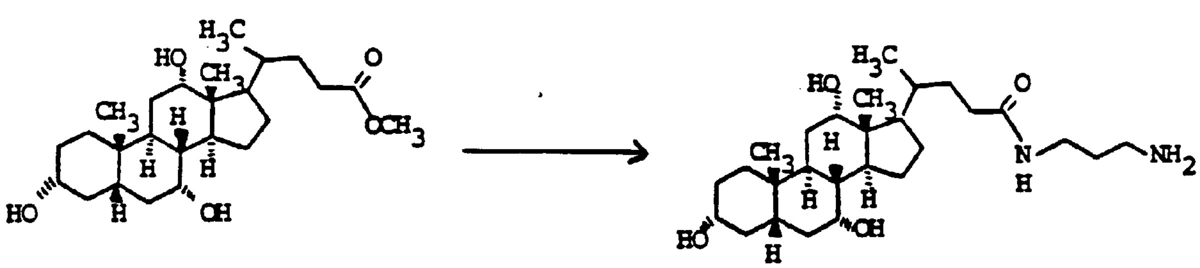

300 g (0,71 mol) Cholsäuremethylester wurden mit 2,5 l 1,3-Diaminopropan 5 Stunden unter Rückfluß gerührt.

Zur Aufarbeitung wurde der Ansatz eingedampft, mit 2 l Eiswasser versetzt und 1 Stunde intensiv gerührt. Den Rückstand saugte man ab und trocknete einen Tag im Vakuumtrockenschrank bei 75°C.

Ausbeute: 306 g (0,65 mol, 92 %)

$C_{27}H_{48}N_2O_4$ (464), MS (FAB, 3-NBA/LiCl) : 471 ($M^+Li^+$)

In Analogie zu Beispiel 37 wurden die Beispiele der Tabellen 5 bis 8 erhalten.

Tabelle 5

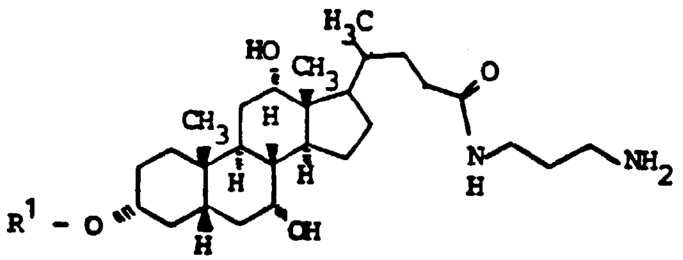

| Bsp. | $R^1$ | MS (FAB,3-NBA/LiCl oder LiJ) |
|---|---|---|
| 38 | 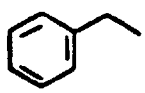 | $C_{34}H_{54}N_2O_4$(554); 561 (M+Li$^+$) |
| 39 | 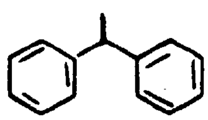 | $C_{40}H_{58}N_2O_4$(630; 637 (M+Li$^+$) |
| 40 | 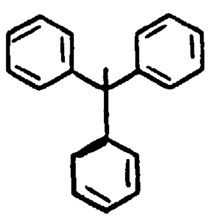 | $C_{46}H_{62}N_2O_4$(706); 713 (M+Li$^+$) |
| 41 | 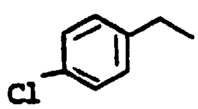 | $C_{34}H_{53}ClN_2O_4$(588); 595 (M+Li$^+$) |
| 42 | 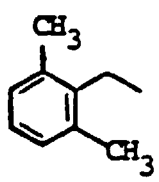 | $C_{36}H_{58}N_2O_4$(582); 589 (M+Li$^+$) |

Fortsetzung Tabelle 5

| Bsp. | $R^1$ | MS (FAB,3-NBA/LiCl oder LiJ) |
|---|---|---|
| 43 | 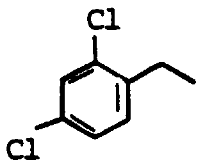 | $C_{32}H_{52}Cl_2N_2O_4$(622); 629 ($M+Li^+$) |
| 44 | 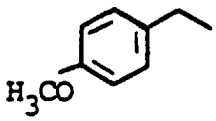 | $C_{32}H_{56}N_2O_4$(584); 591 ($M+Li^+$) |
| 45 | 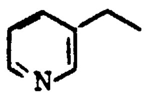 | $C_{33}H_{53}N_3O_4$(555); 562 ($M+Li^+$) |
| 46 | 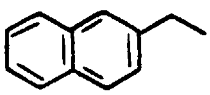 | $C_{38}H_{56}N_2O_4$(604); 611 ($M+Li^+$) |

**Tabelle 6**

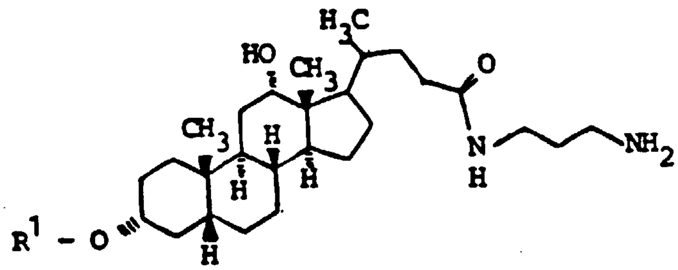

| Bsp. | $R^1$ | MS (FAB,3-NBA/LiCl oder LiJ) |
|---|---|---|
| 47 | H | $C_{27}H_{48}N_2O_3$(448); 455 ($M+Li^+$) |
| 48 | 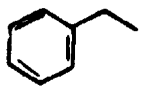 | $C_{34}H_{54}N_2O_3$(538); 545 ($M+Li^+$) |
| 49 | 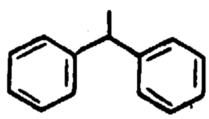 | $C_{40}H_{58}N_2O_3$(614); 621 ($M+Li^+$) |
| 50 | 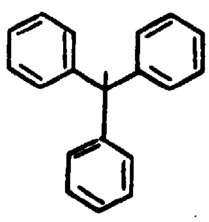 | $C_{46}H_{62}N_2O_3$(690); 697 ($M+Li^+$) |
| 51 | 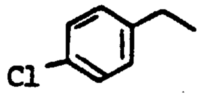 | $C_{34}H_{53}ClN_2O_3$(572); 579 ($M+Li^+$) |
| 52 | 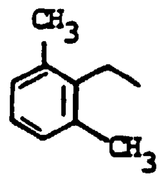 | $C_{36}H_{58}N_2O_3$(566); 573 ($M+Li^+$) |

Fortsetzung Tabelle 6

| Bsp. | $R^1$ | MS (FAB,3-NBA/LiCl oder LiJ) |
|---|---|---|
| 53 | 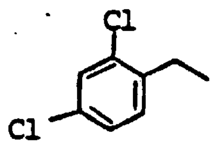 | $C_{34}H_{52}Cl_2N_2O_3$(606); 613 (M+Li$^+$) |
| 54 | 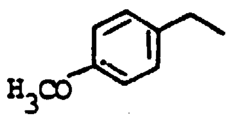 | $C_{35}H_{56}N_2O_4$(568); 575 (M+Li$^+$) |
| 55 | 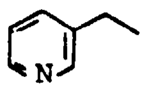 | $C_{33}H_{53}N_3O_3$(539); 546 (M+Li$^+$) |
| 56 | 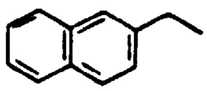 | $C_{38}H_{56}N_2O_3$(588); 595 (M+Li$^+$) |

Tabelle 7

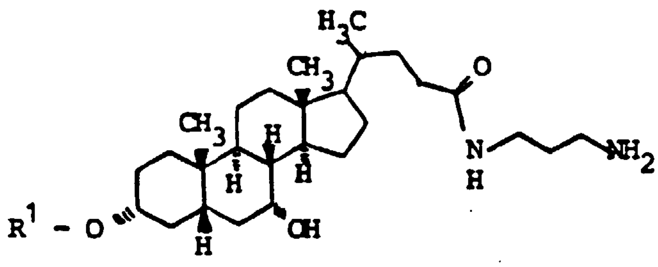

| Bsp. | $R^1$ | MS (FAB, 3-NBA/LiCl oder LiJ) |
|---|---|---|
| 57 | H | $C_{27}H_{48}N_2O_3$(448); 455 (M+Li$^+$) |
| 58 | 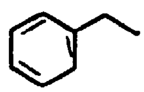 | $C_{34}H_{54}N_2O_3$(538); 545 (M+Li$^+$) |
| 59 | 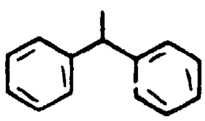 | $C_{40}H_{58}N_2O_3$(614); 621 (M+Li$^+$) |
| 60 | 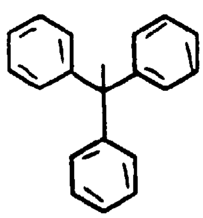 | $C_{46}H_{62}N_2O_3$(690); 697 (M+Li$^+$) |
| 61 | 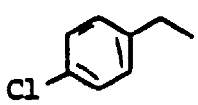 | $C_{34}H_{53}ClN_2O_3$(572); 579 (M+Li$^+$) |
| 62 | 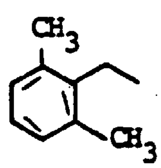 | $C_{36}H_{58}N_2O_3$(566); 573 (M+Li$^+$) |

Fortsetzung Tabelle 7

| Bsp. | $R^1$ | MS (FAB,3-NBA/LiCl oder LiJ) |
|---|---|---|
| 63 | 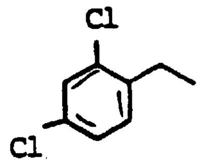 | $C_{34}H_{52}Cl_2N_2O_3$(606); 613 ($M+Li^+$) |
| 64 | 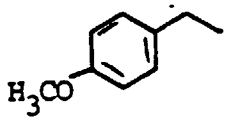 | $C_{35}H_{56}N_2O_4$(568); 575 ($M+Li^+$) |
| 65 | 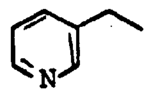 | $C_{33}H_{53}N_3O_3$(539); 546 ($M+Li^+$) |
| 66 | 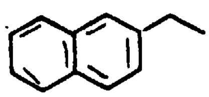 | $C_{38}H_{56}N_2O_3$(588); 595 ($M+Li^+$) |

Tabelle 8

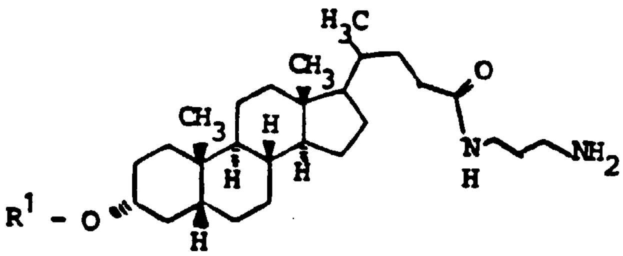

| Bsp. | $R^1$ | MS (FAB,3-NBA/LiCl oder LiJ) |
|---|---|---|
| 67 | H | $C_{27}H_{48}N_2O_2$(432); 439 (M+Li$^+$) |
| 68 | 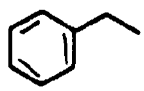 | $C_{34}H_{54}N_2O_2$(522); 529 (M+Li$^+$) |
| 69 | 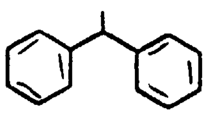 | $C_{40}H_{58}N_2O_2$(598); 605 (M+Li$^+$) |
| 70 | 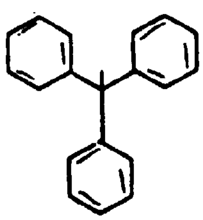 | $C_{46}H_{62}N_2O_2$(674); 681 (M+Li$^+$) |
| 71 | 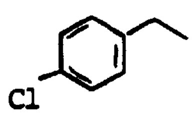 | $C_{34}H_{53}ClN_2O_2$(556); 563 (M+Li$^+$) |
| 72 | 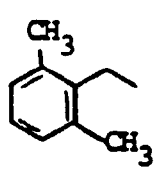 | $C_{36}H_{58}N_2O_2$(550); 557 (M+Li$^+$) |

**Fortsetzung Tabelle 8**

| Bsp. | $R^1$ | MS (FAB,3-NBA/LiCl oder LiJ) |
|---|---|---|
| 73 | 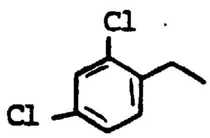 | $C_{34}H_{52}Cl_2N_2O_3$(590); 597 ($M+Li^+$) |
| 74 | 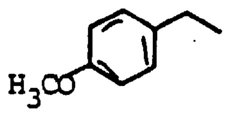 | $C_{35}H_{56}N_2O_3$(552); 559 ($M+Li^+$) |
| 75 | 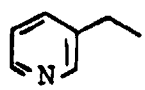 | $C_{33}H_{53}N_3O_2$(523); 530 ($M+Li^+$) |
| 76 | 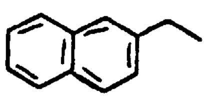 | $C_{38}H_{56}N_2O_2$(572); 579 ($M+Li^+$) |

In Analogie zu den Beispielen der Tabellen 5 bis 8 wurden die Beispiele der Tabellen 9 bis 12 erhalten.

Tablle 9

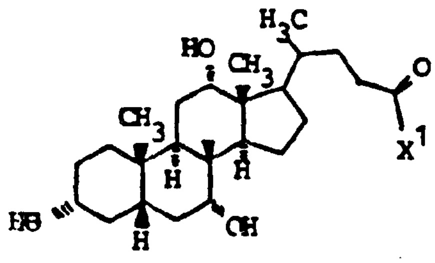

| Beispiel | $X^1$ | MS(FAB,3-NBA/LiCl oder LiJ) |
|---|---|---|
| 37 | $NH(CH_2)_3NH_2$ | $C_{27}H_{48}N_2O_4$(464); 471(M+$Li^+$) |
| 77 | $NH(CH_2)_4NH_2$ | $C_{28}H_{50}N_2O_4$(478); 485(M+$Li^+$) |
| 78 | $NH(CH_2)_5NH_2$ | $C_{29}H_{52}N_2O_4$(492); 499(M+$Li^+$) |
| 79 | $NH(CH_2)_6NH_2$ | $C_{30}H_{54}N_2O_4$(506); 513(M+$Li^+$) |
| 80 | $NH(CH_2)_7NH_2$ | $C_{31}H_{56}N_2O_4$(520); 527(M+$Li^+$) |
| 81 | $NH(CH_2)_8NH_2$ | $C_{32}H_{58}N_2O_4$(534); 541(M+$Li^+$) |
| 82 | $NH(CH_2)_9NH_2$ | $C_{33}H_{60}N_2O_4$(548); 555(M+$Li^+$) |
| 83 | $NH(CH_2)_{10}NH_2$ | $C_{34}H_{62}N_2O_4$(562); 569(M+$Li^+$) |
| 84 | $NH(CH_2)_{11}NH_2$ | $C_{35}H_{64}N_2O_4$(576); 583(M+$Li^+$) |
| 85 | $NH(CH_2)_{12}NH_2$ | $C_{36}H_{66}N_2O_4$(590); 597(M+$Li^+$) |
| 86 | $NH(CH_2)_2O(CH_2)_2O(CH_2)_2NH_2$ | $C_{30}H_{54}N_2O_6$(538); 545(M+$Li^+$) |
| 87 | $NH(C_6H_4)CH_2(C_6H_4)NH_2$ | $C_{37}H_{52}N_2O_4$(588); 595(M+$Li^+$) |
| 88 | $NH(C_6H_4)O(C_6H_4)NH_2$ | $C_{36}H_{50}N_2O_5$(590); 597(M+$Li^+$) |

Tabelle 10

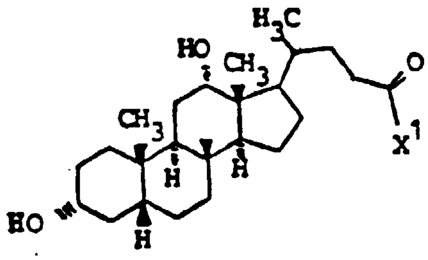

| Beispiel | $X^1$ | MS(FAB,3-NBA/LiCl oder LiJ) |
|---|---|---|
| 89 | $NH(CH_2)_3NH_2$ | $C_{27}H_{48}N_2O_3$(448); 455(M+Li$^+$) |
| 90 | $NH(CH_2)_4NH_2$ | $C_{28}H_{50}N_2O_3$(462); 469(M+Li$^+$) |
| 91 | $NH(CH_2)_5NH_2$ | $C_{29}H_{52}N_2O_3$(476); 483(M+Li$^+$) |
| 92 | $NH(CH_2)_6NH_2$ | $C_{30}H_{54}N_2O_3$(490); 497(M+Li$^+$) |
| 93 | $NH(CH_2)_7NH_2$ | $C_{31}H_{56}N_2O_3$(504); 511(M+Li$^+$) |
| 94 | $NH(CH_2)_8NH_2$ | $C_{32}H_{58}N_2O_3$(518); 525(M+Li$^+$) |
| 95 | $NH(CH_2)_9NH_2$ | $C_{33}H_{60}N_2O_3$(532); 539(M+Li$^+$) |
| 96 | $NH(CH_2)_{10}NH_2$ | $C_{34}H_{62}N_2O_3$(546); 553(M+Li$^+$) |
| 97 | $NH(CH_2)_{11}NH_2$ | $C_{35}H_{64}N_2O_3$(560); 567(M+Li$^+$) |
| 98 | $NH(CH_2)_{12}NH_2$ | $C_{36}H_{66}N_2O_3$(574); 581(M+Li$^+$) |
| 99 | $NH(CH_2)_2O(CH_2)_2O(CH_2)_2NH_2$ | $C_{30}H_{54}N_2O_5$(522); 529(M+Li$^+$) |
| 100 | $NH(C_6H_4)CH_2(C_6H_4)NH_2$ | $C_{37}H_{52}N_2O_3$(572); 579(M+Li$^+$) |
| 101 | $NH(C_6H_4)O(C_6H_4)NH_2$ | $C_{36}H_{50}N_2O_4$(574); 581(M+Li$^+$) |

Tabelle 11

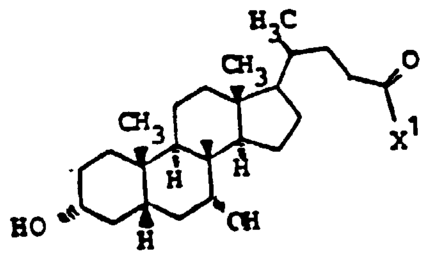

| Beispiel | $X^1$ | MS(FAB,3-NBA/LiCl oder LiJ) |
|---|---|---|
| 102 | $NH(CH_2)_3NH_2$ | $C_{27}H_{48}N_2O_3$(448); 455(M+Li$^+$) |
| 103 | $NH(CH_2)_4NH_2$ | $C_{28}H_{50}N_2O_3$(462); 469(M+Li$^+$) |
| 104 | $NH(CH_2)_5NH_2$ | $C_{29}H_{52}N_2O_3$(476); 483(M+Li$^+$) |
| 105 | $NH(CH_2)_6NH_2$ | $C_{30}H_{54}N_2O_3$(490); 497(M+Li$^+$) |
| 106 | $NH(CH_2)_7NH_2$ | $C_{31}H_{56}N_2O_3$(504); 511(M+Li$^+$) |
| 107 | $NH(CH_2)_8NH_2$ | $C_{32}H_{58}N_2O_3$(518); 525(M+Li$^+$) |
| 108 | $NH(CH_2)_9NH_2$ | $C_{33}H_{60}N_2O_3$(532); 539(M+Li$^+$) |
| 109 | $NH(CH_2)_{10}NH_2$ | $C_{34}H_{62}N_2O_3$(546); 553(M+Li$^+$) |
| 110 | $NH(CH_2)_{11}NH_2$ | $C_{35}H_{64}N_2O_3$(560); 567(M+Li$^+$) |
| 111 | $NH(CH_2)_{12}NH_2$ | $C_{36}H_{66}N_2O_3$(574); 581(M+Li$^+$) |
| 112 | $NH(CH_2)_2O(CH_2)_2O(CH_2)_2NH_2$ | $C_{30}H_{54}N_2O_5$(522); 529(M+Li$^+$) |
| 113 | $NH(C_6H_4)CH_2(C_6H_4)NH_2$ | $C_{37}H_{52}N_2O_3$(572); 579(M+Li$^+$) |
| 114 | $NH(C_6H_4)O(C_6H_4)NH_2$ | $C_{36}H_{50}N_2O_4$(574); 581(M+Li$^+$) |

Tabelle 12

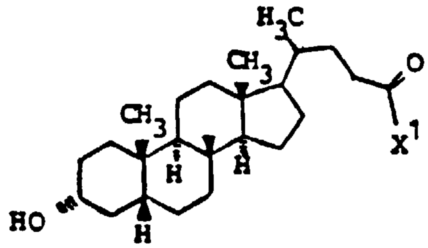

| Beispiel | $X^1$ | MS(FAB,3-NBA/LiCl oder LiJ) |
|---|---|---|
| 115 | $NH(CH_2)_3NH_2$ | $C_{27}H_{48}N_2O_2$(432); 439(M+Li$^+$) |
| 116 | $NH(CH_2)_4NH_2$ | $C_{28}H_{50}N_2O_2$(446); 453(M+Li$^+$) |
| 117 | $NH(CH_2)_5NH_2$ | $C_{29}H_{52}N_2O_2$(460); 467(M+Li$^+$) |
| 118 | $NH(CH_2)_6NH_2$ | $C_{30}H_{54}N_2O_2$(474); 481(M+Li$^+$) |
| 119 | $NH(CH_2)_7NH_2$ | $C_{31}H_{56}N_2O_2$(488); 495(M+Li$^+$) |
| 120 | $NH(CH_2)_8NH_2$ | $C_{32}H_{58}N_2O_2$(502); 509(M+Li$^+$) |
| 121 | $NH(CH_2)_9NH_2$ | $C_{33}H_{60}N_2O_2$(516); 523(M+Li$^+$) |
| 122 | $NH(CH_2)_{10}NH_2$ | $C_{34}H_{62}N_2O_2$(530); 537(M+Li$^+$) |
| 123 | $NH(CH_2)_{11}NH_2$ | $C_{35}H_{64}N_2O_2$(544); 551(M+Li$^+$) |
| 124 | $NH(CH_2)_{12}NH_2$ | $C_{36}H_{66}N_2O_2$(558); 565(M+Li$^+$) |
| 125 | $NH(CH_2)_2O(CH_2)_2O(CH_2)_2NH_2$ | $C_{30}H_{54}N_2O_4$(506); 513(M+Li$^+$) |
| 126 | $NH(C_6H_4)CH_2(C_6H_4)NH_2$ | $C_{37}H_{52}N_2O_2$(556); 563(M+Li$^+$) |
| 127 | $NH(C_6H_4)O(C_6H_4)NH_2$ | $C_{36}H_{50}N_2O_3$(558); 565(M+Li$^+$) |

Beispiel 128

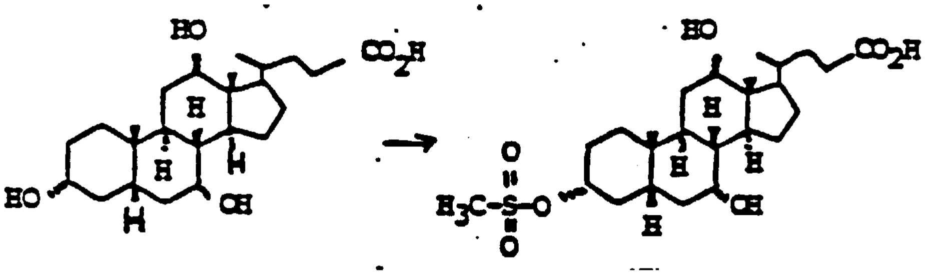

Zu 100 g (0.245 mol) Cholsäure in 500 ml Pyridin wurden bei 0°C 23.1 ml (0.294 mol) Methansulfonsäurechlorid getropft. Man ließ 30 min bei 0°C und 2 h bei Zimmertemperatur rühren. Die Mischung wurde auf 3000 ml Wasser/ 400 ml conc. $H_2SO_4$ gegossen und mit Essigester extrahiert (3 x). Die vereinigten organischen Phasen wurden mit $MgSO_4$ getrocknet und eingedampft. Chromatographie auf Kieselgel (Essigester/Cyclohexan HOAc= 5:5:1) ergab qantitativ Beispiel 128". Für präparative Zwecke war eine weitere Reinigung nicht erforderlich.

Beispiel 129

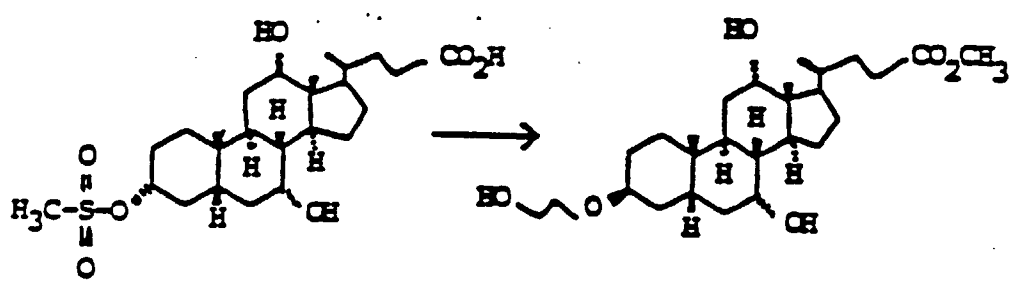

a) 119 g (0.245 mol) "Beispiel 128" wurden in 500 ml Ethylenglykol/100 ml Pyridin 2 h auf 100°C erhitzt. Die Mischung wurde auf 1500 ml Wasser/100 ml conc. $H_2SO_4$ gegossen und mit Essigester extrahiert (3x). Die vereinigten organischen Phasen wurden getrocknet ($MgSO_4$) und eingedampft.

b) Zur Veresterung wurde der Rückstand in 1100 ml methanolischer HCl (hergestellt durch Zutropfen von 100 ml Acetylchlorid zu 1000 ml Methanol) gelöst und über Nacht bei Zimmertemperatur gerührt. Die Lösung wurde auf 2000 ml Wasser gegossen und mit Ether extrahiert (3x). Die vereinigten organischen Phasen wurden mit gesättigter wäßriger $NaHCO_3$-Lösung gewaschen und getrocknet ($MgSO_4$). Abdampfen des Lösungsmittels und Flash-Chromatographie auf Kieselgel (Essigester dann Essigester/MeOH = 10/1) ergab 37.1 g (0.08 mol, 33 %) "Beispiel 129".
$C_{27}H_{46}O_6$ (466), MS (FAB,3-NBA/LiJ): 473 ($M^+Li^+$)
Das Produkt enthält bis zu 10 % des 3α-Isomeren, das gegebenenfalls nach entsprechender Derivatisierung abgetrennt werden kann.

Analog zu Beispiel 129 wurden die Verbindungen der Tabelle 13 hergestellt. (Neben kleineren Anteilen der α-Isomeren wurden überwiegend die β-Isomeren erhalten).

Tabelle 13

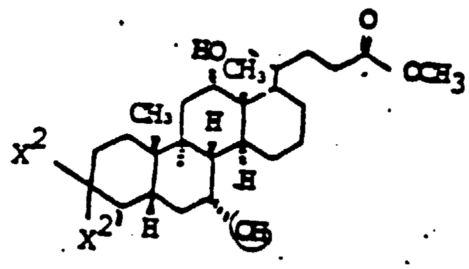

Tabelle 13

| Beisp. | ß-$X^2$ | α-$X^{2'}$ | MS (FAB, 3-NBA/LiJ oder LiCl) |
|---|---|---|---|
| 130 | $HO-(CH_2)_3-O-$ | H | $C_{28}H_{48}O_6$(480); 487 ($M+Li^+$) |
| 131 | $HO-(CH_2)_4-O-$ | H | $C_{29}H_{50}O_6$(494); 501 ($M+Li^+$) |
| 132 | $HO-(CH_2)_5-O-$ | H | $C_{30}H_{52}O_6$(508); 515 ($M+Li^+$) |
| 133 | $HO-(CH_2)_6-O-$ | H | $C_{31}H_{54}O_6$(522); 529 ($M+Li^+$) |
| 134 | $HO-(CH_2)_{10}-O-$ | H | $C_{35}H_{62}O_6$(578); 585 ($M+Li^+$)<br>601 ($M+Na^+$) |
| 135 | $HO-(CH_2)_2-O-(CH_2)_2-O-$ | H | $C_{29}H_{50}O_7$(510); 517 ($M+Li^+$) |
| 136 | $H_3C-CH(OH)-CH_2-O-$ | H | $C_{28}H_{48}O_6$(480); 487 ($M+Li^+$) |

Beispiel 137

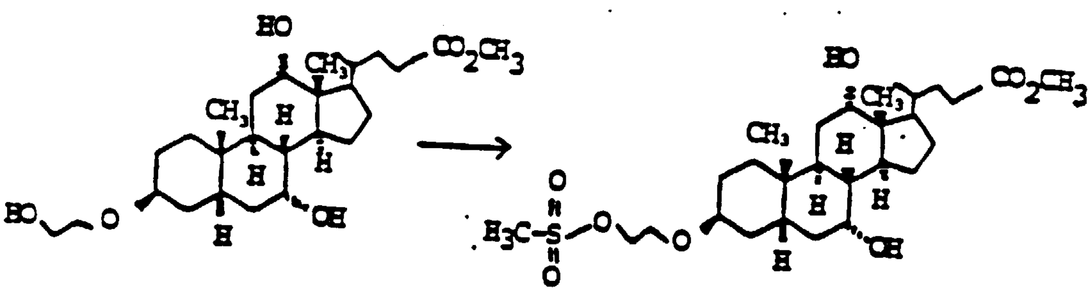

Zu 37.1 g (0.08 mol) "Beispiel 129" in 150 ml Pyridin wurden bei 0°C 6.6 mol (0.084 mol) Methansulfonsäurechlorid getropft. Man ließ 15 min bei 0°C und 1 h bei Zimmertemperatur rühren. Die Reaktionsmischung wurde auf 500 ml Wasser gegossen und mit Essigester extrahiert (3x). Trocknen der vereinigten organischen Phasen ($MgSO_4$), Abziehen des Lösungsmittels und Chromatographie auf Kieselgel (Essigester/Cyclohexan = 3/1) ergab 37.7 g (0.07 mol, 87 %) Mesylat "Beispiel 137".

$C_{28}H_{48}O_8S$ (544), MS (FAB, 3-NBA/LiJ): 551 ($M+Li^+$).

Beispiel 138

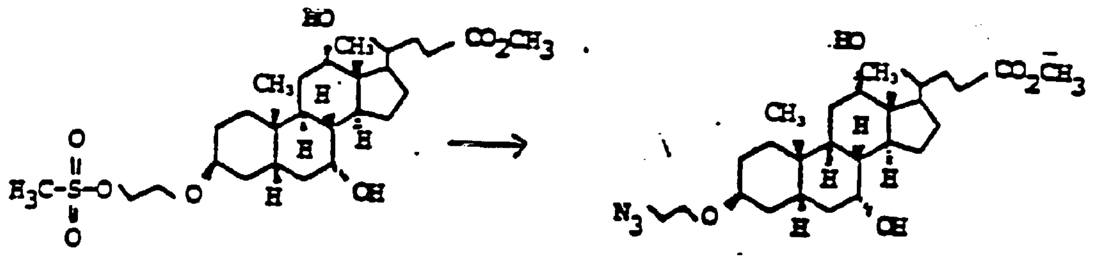

37.7 g (0.07 mol) Mesylat "Beispiel 137" wurden mit 4.95 g (0.076 mol) Natriumazid in 150 ml trockenem DMF 2 h bei 70°C gerührt. Das Reaktionsgemisch wurde auf Wasser gegossen und mit Essigester extrahiert (3x). Die vereinigten organischen Phasen wurden getrocknet ($MgSO_4$) und eingedampft. Der Rückstand wurde mit Toluol aufgenommen und das Toluol wieder im Rotationsverdampfer abgezogen (2x). Ausbeute 34.5 g "Beispiel 138" (quantitativ). Das Azid wurde ohne weitere Reinigung unmittelbar zur nächsten Stufe umgesetzt.

Beispiel 139

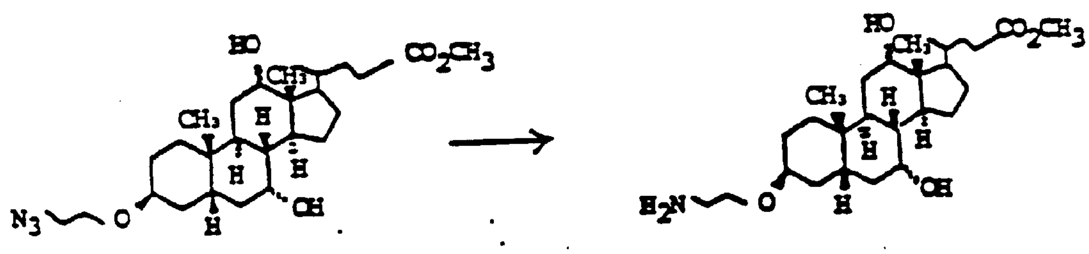

31.1 g (0.063 mol) "Beispiel 138" wurden in 500 ml Essigester mit 20 g Pd/C (10 ~) bei Zimmertemperatur und Normaldruck hydriert. Der Katalysator wurde abfiltriert und das Filtrat eingedampft. Chromatographie auf Kieselgel (Essigester/ Methanol/$NEt_3$ 5/1/1) ergab 21.0 g (0,45 mol, 71 %) Amin "Beispiel 139".
$C_{27}H_{47}NO_5$ (465), MS (FAB, 3-NBA/LiJ): 472 (M + $Li^+$).

Analog zu den Beispielen 137-139 wurden die Verbindungen der Tabelle 14 hergestellt.

Tabelle 14

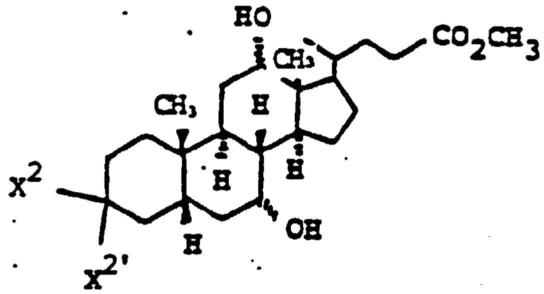

| Bsp. | ß-$X^2$ | α-$X^{2'}$ | MS (FAB, 3-NBA/LiJ) |
|---|---|---|---|
| 140 | $H_2N-(CH_2)_3-O-$ | H | $C_{28}H_{49}NO_5$(479); 486 (M+Li$^+$) |
| 141 | $H_2N-(CH_2)_4-O-$ | H | $C_{29}H_{51}NO_5$(493); 500 (M+Li$^+$) |
| 142 | $H_2N-(CH_2)_5-O-$ | H | $C_{30}H_{53}NO_5$(507); 514 (M+Li$^+$) |
| 143 | $H_2N-(CH_2)_6-O-$ | H | $C_{31}H_{55}NO_5$(521); 528 (M+Li$^+$) |
| 144 | $H_2N-(CH_2)_{10}-O-$ | H | $C_{35}H_{63}NO_5$(577); 584 (M+Li$^+$) |
| 145 | $H_2N-(CH_2)_2-O-(CH_2)_2-O-$ | H | $C_{29}H_{51}NO_6$(509); 516 (M+Li$^+$) |
| 146 | $H_3C-CH_2-CH_2-O-$<br>$NH_2$ | H | $C_{28}H_{49}NO_5$(479); 486 (M+Li$^+$) |

In Analogie zur Cholsäure wurden andere Gallensäuren entsprechend den Beispielen 128-146 umgesetzt und Verbindungen entsprechend Tabelle 15-17 erhalten.

a) ausgehend von Desoxycholoäure:

Tabelle 15

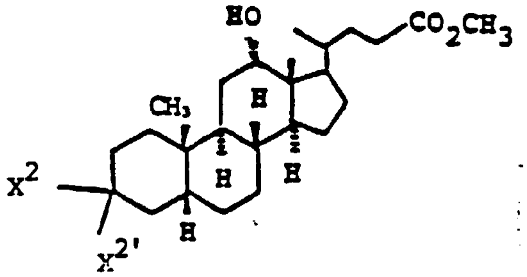

| Bsp. | ß-$X^2$ | α-$X^{2'}$ | MS (FAB, 3-NBA/LiJ) |
|---|---|---|---|
| 147 | $HO-(CH_2)_2-O-$ | H | $C_{27}H_{46}O_5$(450); 457 (M+$Li^+$) |
| 148 | $HO-(CH_2)_3-O-$ | H | $C_{28}H_{48}O_5$(464); 471 (M+$Li^+$) |
| 149 | $HO-(CH_2)_5-O-$ | H | $C_{30}H_{52}O_5$(492); 499 (M+$Li^+$) |
| 150 | $HO-(CH_2)_{10}-O-$ | H | $C_{35}H_{62}O_5$(562); 569 (M+$Li^+$) |
| 151 | $H_2N-(CH_2)_2-O-$ | H | $C_{27}H_{47}NO_4$(449); 456 (M+$Li^+$) |
| 152 | $H_2N-(CH_2)_5-O-$ | H | $C_{30}H_{53}NO_4$(491); 498 (M+$Li^+$) |

b) ausgehend von Chenodesoxycholsäure

Tabelle 16

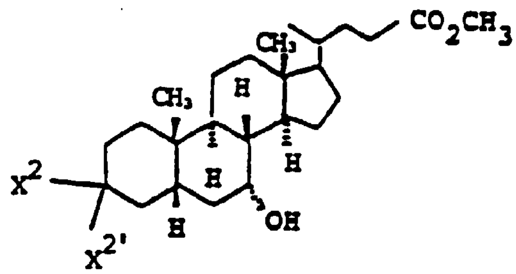

| Bsp. | ß-$X^2$ | α-$X^{2'}$ | MS(FAB, 3-NBA/LiJ) |
|---|---|---|---|
| 153 | $HO-(CH_2)_2-O-$ | H | $C_{27}H_{46}O_5$(450); 457 (M+$Li^+$) |
| 154 | $HO-(CH_2)_3-O-$ | H | $C_{28}H_{48}O_5$(464); 471 (M+$Li^+$) |
| 155 | $HO-(CH_2)_5-O-$ | H | $C_{30}H_{52}O_5$(492); 499 (M+$Li^+$) |
| 156 | $HO-(CH_2)_{10}-O-$ | H | $C_{35}H_{62}O_5$(562); 569 (M+$Li^+$) |
| 157 | $H_2N-(CH_2)_2-O-$ | H | $C_{27}H_{47}NO_4$(449); 456 (M+$Li^+$) |
| 158 | $H_2N-(CH_2)_5-O-$ | H | $C_{30}H_{53}NO_4$(491); 498 (M+$Li^+$) |

c) ausgehend von Lithocholsäure

Tabelle 17

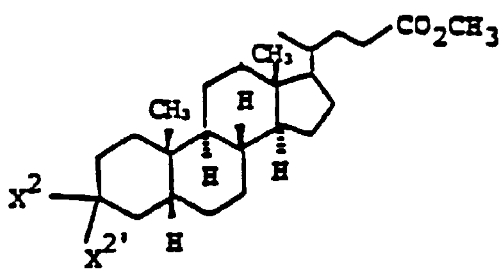

| Bsp. | β-$X^2$ | α-$X^{2'}$ | MA (FAB, 3-NBA/LiJ) |
|---|---|---|---|
| 159 | HO-$(CH_2)_2$-O- | H | $C_{27}H_{46}O_4$(434); 441 (M+Li$^+$) |
| 160 | HO-$(CH_2)_3$-O- | H | $C_{28}H_{48}O_4$(448); 455 (M+Li$^+$) |
| 161 | HO-$(CH_2)_5$-O- | H | $C_{30}H_{52}O_4$(476); 483 (M+Li$^+$) |
| 162 | HO-$(CH_2)_{10}$-O- | H | $C_{35}H_{62}O_4$(546); 653 (M+Li$^+$) |
| 163 | $H_2N$-$(CH_2)_2$-O- | H | $C_{27}H_{47}NO_3$(433); 440 (M+Li$^+$) |
| 164 | $H_2N$-$(CH_2)_5$-O- | H | $C_{30}H_{53}NO_3$(475); 482 (M+Li$^+$) |

Beispiel 165

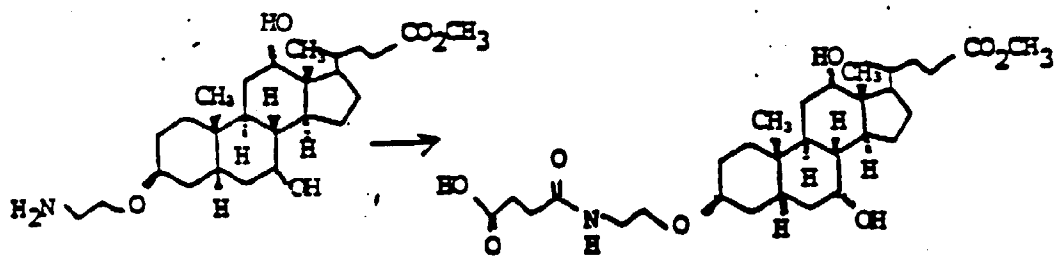

Beispiel 124 (X:g = 2 und n=2)

2.0 g (4.3 mmol) "Beispiel 139" wurden in 25 ml THF/5 ml Triethylamin mit 430 mg (4.3 mmol) Bernsteinsäureanhydrid 30 min bei Zimmertemperatur gerührt. Das Reaktionsgemisch wurde auf 2n HCl gegossen und mit Essigester extrahiert (3x). Trocknen der vereinigten organischen Phasen ($MgSO_4$) und Abziehen des Lösungsmittels ergab 2.4 g (4.2 mmol, 98 "Beispiel 165"

$C_{31}H_{51}NO_8$ (565) : MS (FAB, 3-NBA/LiJ): 578 (M+2Li$^+$-H)

Ebenso wurde die Verbindung, in denen im Rest X q = 0, 1, 3 und 5 und n = 3 bis 18 SiH, hergestellt.

Beispiel 166

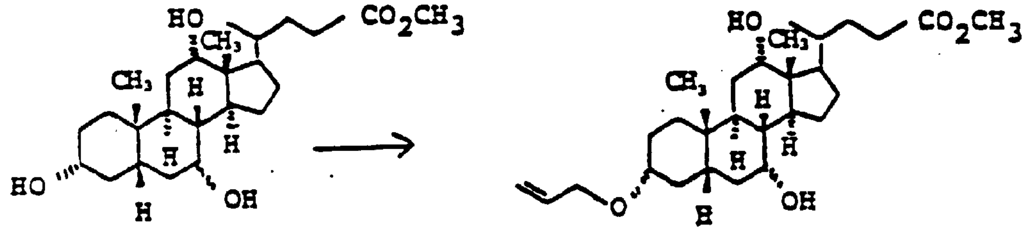

42,2 g (0.1 mol) Cholsäuremethylester, 300 ml (1.8 mol) N-Ethyldiisopropylamin und 10 ml (0.12 mol) Allylbromid wurden 8h unter Rückfluß erhitzt. Nach jeder Stunde Reaktionszeit wurden nochmals jeweils 5 ml Allylbromid zugegeben (DG-Kontrolle, Cyclohexan/Essigester = 1:1). Das Reaktionsgemisch wurde auf 400 ml konz. $H_2SO_4$/2000 ml Wasser gegossen und mit Essigester extrahiert (3x). Die vereinigten organischen Phasen wurden jeweils einmal mit 1 N HCl, Wasser und gesättigter $NaHCO_3$-Lösung gewaschen. Trocknen ($MgSO_4$), Abziehen des Lösungsmittels und Chromatographie des Rückstandes auf Kieselgel (n-Heptan/Essigester = 4:1 → 3:1 → 2:1) ergab 21,91 g (0,047 mol, 47 %) "Beispiel 166".
$C_{28}H_{46}O_5$ (462), MS (FAB, 3-NBA/LiCl): 469 ($M+Li^+$)
<<<MARK>>>

Beispiel 167

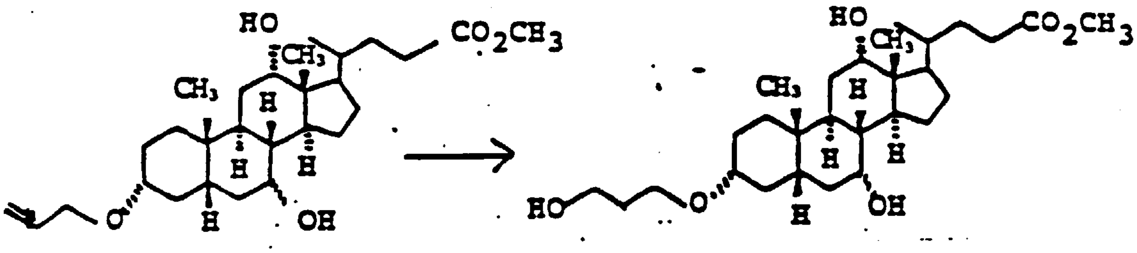

(1) Herstellung von Thexylboran: Unter Argonatmosphäre wurden bei 0°C zu 85 ml 1 molarer $BH_3$-THF-Lösung (THF) 85 ml 1 molare 2,3-Dimethylbutenlösung (THF) getropft. Man ließ bei 0°C rühren.

(2) Hydroborierung: Zu der nach (1) bereiteten Lösung wurden bei 0°C 8,6 g (18.59 mmol) Olefin Beispiel 166 in 25 ml THF getropft. Nach 3h bei 0°C ließ man auf Zimmertemperatur kommen (DG-Kontrolle). Nach 16 h bei Zimmertemperatur wurde frisch hergestellte Thexylboranlösung (THF) zugetropft. Man ließ erneut bei Zimmertemperatur rühren. Nachdem kein Ausgangsmaterial mehr nachweisbar war, wurde das Reaktionsgemisch unter Argonatmosphäre vorsichtig unter intensivem Rühren in wäßrige Natronlauge transferiert (pro Äquivalent Boran 1 Äquivalent NaOH). Anschließend wurde unter Eiskühlung 30 prozentige Wasserstoffperoxidlosung zugetropft. (2 Äquivalente pro 1 Äquivalent Boran). Nach 20 min bei 0°C wurde 30 min auf 50°C erwärmt. Zur besseren Phasentrennung wurde gesättigte Kochsalzlösung zugegeben. Die wäßrige Phase wurde mit Ethylacetat extrahiert (2x) und die vereinigten organischen Phasen mit gesättigter Natriumbisulfitlösung (2x) und anschließend mit Natriumchloridlösung (1x) gewaschen. Trocknen mit $MgSO_4$, Abziehen des Lösungsmittels und Chromatographie auf Kieselgel (Ethylacetat → Ethylacetat/MeOH = 20:1) ergab 5,0 g (10.4 mmol, 56 %) "Beispiel 167"
Rf (Ethylacetat): 0.18
$C_{28}H_{48}O_6$,(480); MS (FAB, 3-NBA/LiCl): 487 ($M+Li^+$)
Daneben wurden 1.0 g des sekundären Alkohols erhalten.
Rf (Ethylacetat): 0,27

Beispiel 168

In Analogie zu den Beispielen 137-146 wurde aus Beispiel 167 "Beispiel 168" erhalten.

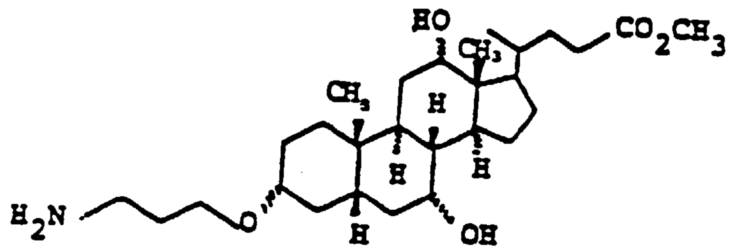

(X-G mit α-Konfiguration an 3-C)

$C_{28}H_{49}NO_5$ (479); MS (FAB, 3NBA/LiI): 486 ($M+Li^+$)

Die Verbindung gemäß Beispiel 168 wurde anschließend gemäß Beispiel 165 in ihr Dicarbonsäuremonoamid überführt.

Endverbindungen

Beispiel 169

Schritt a)

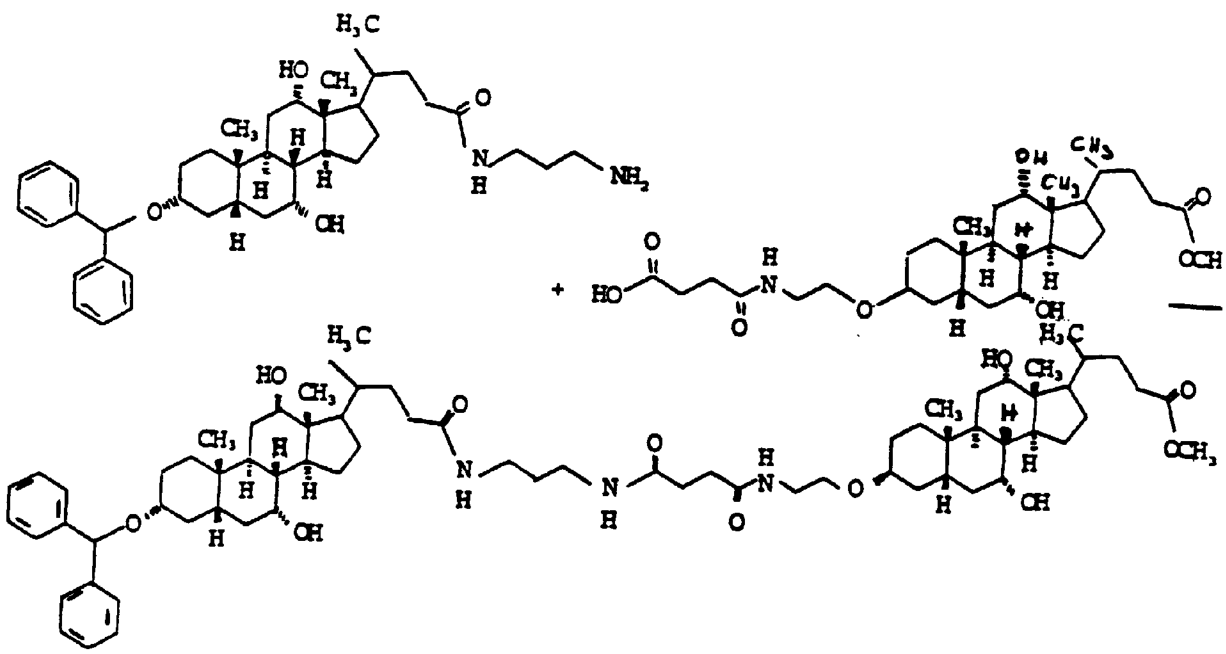

565 mg (1 mmol) "Beispiel 165" gelöst in 20 ml Tetrahydrofuran und 5 ml Triethylamin wurden vorgelegt und bei 0°C 96 µl (1 mmol) Ghlorameisensäureäthylester zugespritzt. Man ließ 15 Minuten bei 0°C rühren, anschließend gab man 630 mg (1 mmol) "Beispiel 39" fest zu. Man ließ 5 h bei Raumtemperatur rühren. Die Reaktionslosung wurde 10 Minuten mit 10 ml 1 molare Salzsäure heftig verquirlt, dann mit Essigester extrahiert (3x). Die vereinten organischen Phasen wurden mit gesättigter wäßriger $NaHCO_3$-Lösung gewaschen und getrocknet ($MgSO_4$).
Abdampfen des Lösungsmittels und Flash-Chromatographie auf Kieselgel (Essigester/Äthanol = 3/1) ergab 765 mg (0,65 mmol, 65 %) "Beispiel 169".

$C_{71}H_{107}N_3O_{11}$ (1177), MS (FAB, 3-NBA/LiCl): 1184 ($M+Li^+$)

Beispiel 170

Schritt b) Verseifung

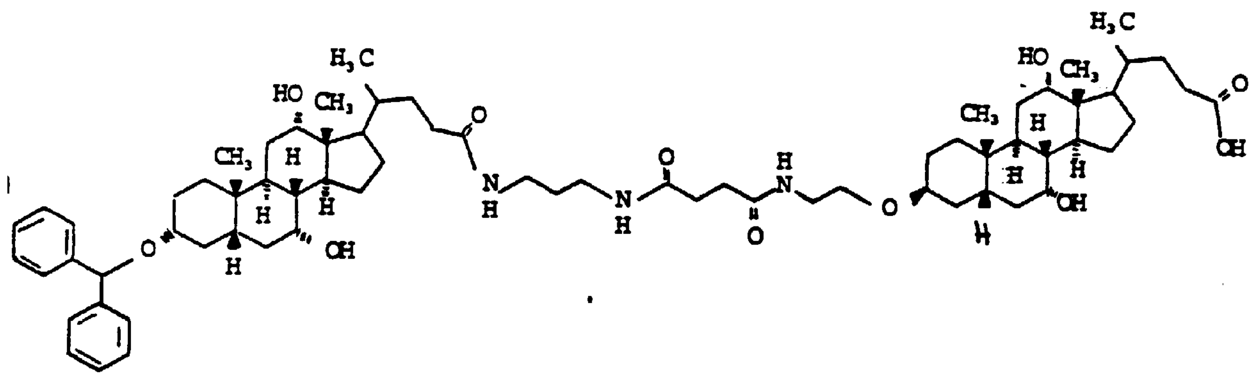

707 mg (0,6 mmol) "Beispiel 169" wurden mit 10 ml Äthanol und 5 ml 1 molare Natronlauge 1,5 h bei Raumtemperatur gerührt, anschließend mit 10 g Natriumdihydrogenphosphat 10 Minuten intensiv verquirlt, mit Essigester/Äthanol = 3/1 extrahiert (3x). Die vereinigten organischen Phasen wurden getrocknet ($MgSO_4$).

Abdampfen des Lösungsmittels, Verreiben mit n-Heptan und Absaugen ergab 665 mg (0,57 mmol 95 %) "Beispiel 170" $C_{70}H_{105}N_3O_{11}$ (1163), MS (FAB, 3-NBA/LiCl): 1170, ($M+Li^+$)

In Analogie zu den Beispielen 169 und 170 wurden die Verbindungen der Tabelle 18 erhalten.

Tabelle 18

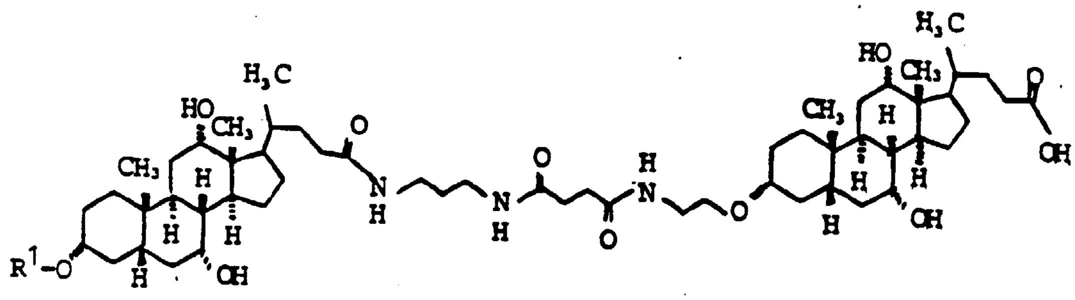

| Bsp. | $R^1$ | MS (FAB,3-NBA/LiCl oder LiJ) |
|---|---|---|
| 171 | H | $C_{57}H_{95}N_3O_{11}$ (997); 1004 ($M+Li^+$) |
| 172 | 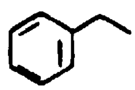 | $C_{64}H_{101}N_3O_{11}$ (1097); 1094 ($M+Li^+$) |
| 173 | 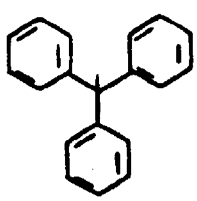 | $C_{76}H_{109}N_3O_{11}$ (1239); 1246 ($M+Li^+$) |
| 174 | 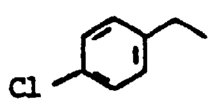 | $C_{64}H_{100}ClN_3O_{11}$ (1121); 1128 ($M+Li^+$) |
| 175 | 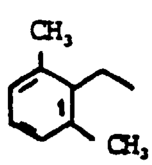 | $C_{66}H_{105}N_3O_{11}$ (1115); 1122 ($M+Li^+$) |

**Fortsetzung Tabelle 18**

| Bsp. | $R^1$ | MS (FAB,3-NBA/LiCl oder LiJ) |
|---|---|---|
| 176 | 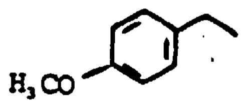 | $C_{65}H_{103}N_3O_{12}$(1117); 1124 (M+Li$^+$) |
| 177 | 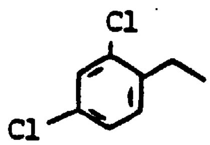 | $C_{64}H_{99}Cl_2N_3O_{11}$(1155); 1162 (M+Li$^+$) |
| 178 | 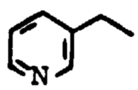 | $C_{63}H_{100}N_4O_{11}$(1088); 1095 (M+Li$^+$) |
| 179 | 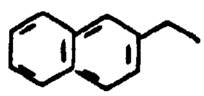 | $C_{68}H_{103}N_3O_{11}$(1137); 1144 (M+Li$^+$) |

In Analogie zu den Beispielen 169 und 170 ausgehend von Beispiel 151 wurden die Verbindungen der Tabelle 19 erhalten.

Tabelle 19

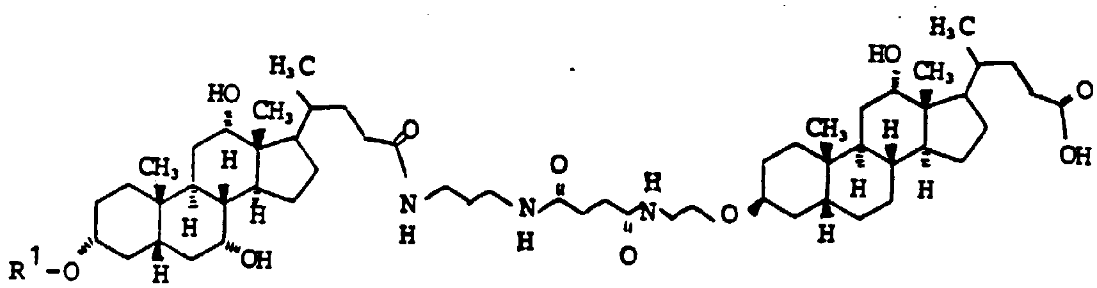

| Bsp. | $R^1$ | MS (FAB,3-NBA/LiCl oder LiJ) |
|---|---|---|
| 180 | H | $C_{57}H_{95}N_3O_{10}$(981); 988 ($M+Li^+$) |
| 181 | 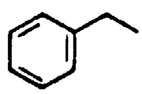 | $C_{64}H_{101}N_3O_{10}$(1071); 1078 ($M+Li^+$) |
| 182 | 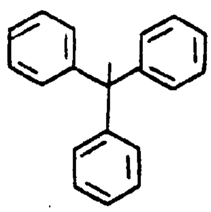 | $C_{76}H_{109}N_3O_{10}$(1223); 1230 ($M+Li^+$) |
| 183 | 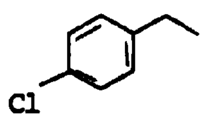 | $C_{64}H_{100}ClN_3O_{10}$(1105); 1112 ($M+Li^+$) |
| 184 | 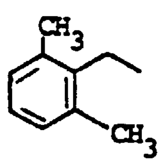 | $C_{66}H_{105}N_3O_{10}$(1099); 1106 ($M+Li^+$) |

**Fortsetzung Tabelle 19**

| Bsp. | $R^1$ | MS (FAB,3-NBA/LiCl oder LiJ) |
|---|---|---|
| 185 | 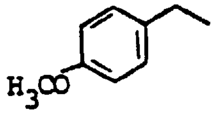 | $C_{65}H_{103}N_3O_{11}$(1101); 1108 ($M+Li^+$) |
| 186 | 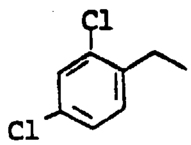 | $C_{64}H_{99}Cl_2N_3O_{10}$(1139); 1146 ($M+Li^+$) |
| 187 | 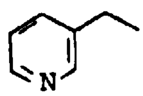 | $C_{63}H_{100}N_4O_{10}$(1072); 1079 ($M+Li^+$) |
| 188 | 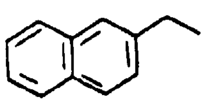 | $C_{68}H_{103}N_3O_{10}$(1121); 1128 ($M+Li^+$) |
| 189 | 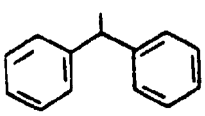 | $C_{70}H_{105}N_3O_{10}$(1147); 1154 ($M+Li^+$) |

In Analogie zu den Beispielen 169 und 170 ausgehend von Beispiel 116 wurden die Verbindungen der Tabelle 20 erhalten.

Tabelle 20

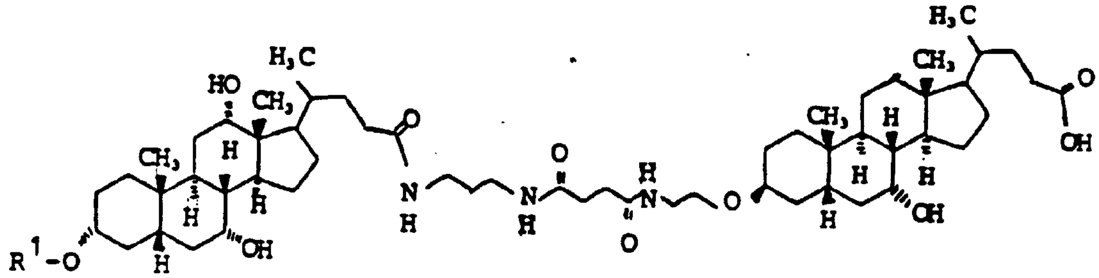

| Bsp. | $R^1$ | MS (FAB,3-NBA/LiCl oder LiJ) |
|---|---|---|
| 190 | H | $C_{57}H_{95}N_3O_{10}$(981); 988 ($M+Li^+$) |
| 191 | 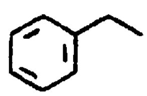 | $C_{64}H_{101}N_3O_{10}$(1071); 1078 ($M+Li^+$) |
| 192 | 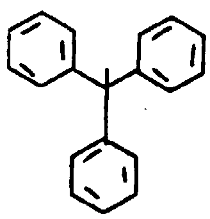 | $C_{76}H_{109}N_3O_{10}$(1223); 1230 ($M+Li^+$) |
| 193 | 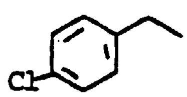 | $C_{64}H_{100}ClN_3O_{10}$(1105); 1112 ($M+Li^+$) |
| 194 | 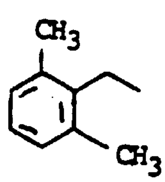 | $C_{66}H_{105}N_3O_{10}$(1099); 1106 ($M+Li^+$) |

Fortsetzung Tabelle 20

| Bsp. | $R^1$ | MS (FAB,3-NBA/LiCl oder LiJ) |
|---|---|---|
| 195 | 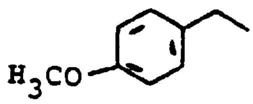 | $C_{65}H_{103}N_3O_{11}$(1101); 1108 ($M+Li^+$) |
| 196 | 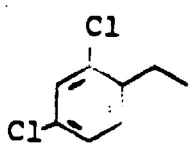 | $C_{64}H_{99}Cl_2N_3O_{10}$(1139); 1146 ($M+Li^+$) |
| 197 | 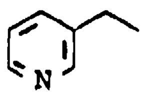 | $C_{63}H_{100}N_4O_{10}$(1072); 1079 ($M+Li^+$) |
| 198 | 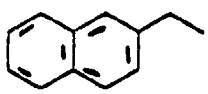 | $C_{68}H_{103}N_3O_{10}$(1121); 1128 ($M+Li^+$) |
| 199 | 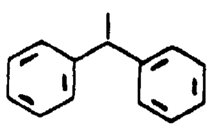 | $C_{70}H_{105}N_3O_{10}$(1147); 1154 ($M+Li^+$) |

In Analogie zu den Beispielen 169 und 170 ausgehend von Beispiel 163 wurden die Verbindungen der Tabelle 21 erhalten.

Tabelle 21

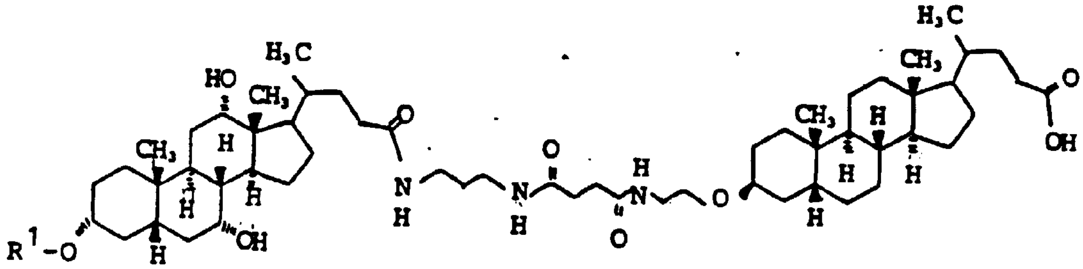

| Bsp. | $R^1$ | MS (FAB,3-NBA/LiCl oder LiJ) |
|---|---|---|
| 200 | H | $C_{57}H_{95}N_3O_9$(965); 972 ($M+Li^+$) |
| 201 | 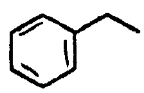 | $C_{64}H_{101}N_3O_9$(1055); 1062 ($M+Li^+$) |
| 202 | 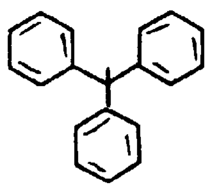 | $C_{76}H_{109}N_3O_9$(1207); 1214 ($M+Li^+$) |
| 203 | 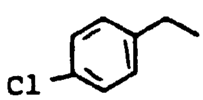 | $C_{64}H_{100}ClN_3O_{10}$(1089); 1096 ($M+Li^+$) |
| 204 | 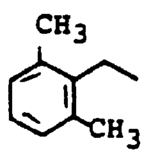 | $C_{66}H_{105}N_3O_9$(1083); 1090 ($M+Li^+$) |

**Fortsetzung Tabelle 21**

| Bsp. | $R^1$ | MS (FAB,3-NBA/LiCl oder LiJ) |
|---|---|---|
| 205 | 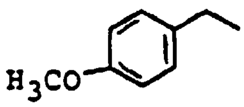 | $C_{65}H_{103}N_3O_{10}$(1085); 1092 (M+Li$^+$) |
| 206 | 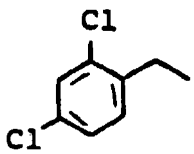 | $C_{64}H_{99}Cl_2N_3O_9$(1123); 1130 (M+Li$^+$) |
| 207 | 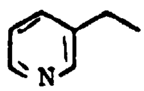 | $C_{63}H_{100}N_4O_9$(1056); 1063 (M+Li$^+$) |
| 208 | 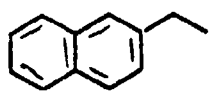 | $C_{68}H_{103}N_3O_9$(1105); 1112 (M+Li$^+$) |
| 209 | 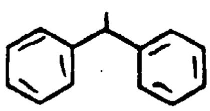 | $C_{70}H_{105}N_3O_9$(1131); 1138 (M+Li$^+$) |

In Analogie zu den Beispielen 169 und 170 wurden die Verbindungen der Tabelle 22 erhalten.

Tabelle 22

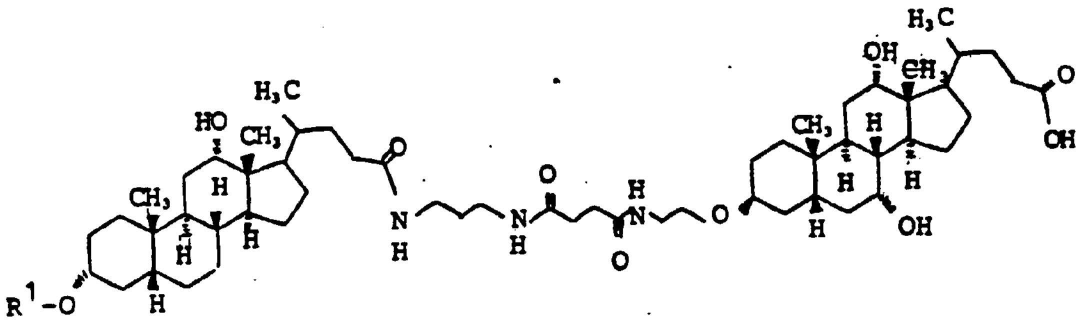

| Bsp. | $R^1$ | MS (FAB,3-NBA/LiCl oder LiJ) |
|---|---|---|
| 210 | H | $C_{57}H_{95}N_3O_{10}$(981); 988 (M+Li$^+$) |
| 211 | 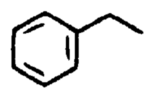 | $C_{64}H_{101}N_3O_{10}$(1071); 1078 (M+Li$^+$) |
| 212 | 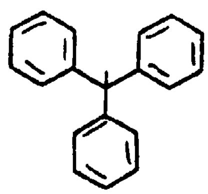 | $C_{76}H_{109}N_3O_{10}$(1223); 1230 (M+Li$^+$) |
| 213 | 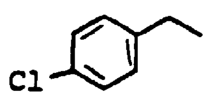 | $C_{64}H_{100}ClN_3O_{10}$(1105); 1112 (M+Li$^+$) |
| 214 | 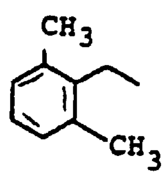 | $C_{66}H_{105}N_3O_{10}$(1099); 1106 (M+Li$^+$) |

Fortsetzung Tabelle 22

| Bsp. | $R^1$ | MS (FAB,3-NBA/LiCl oder LiJ) |
|---|---|---|
| 215 | 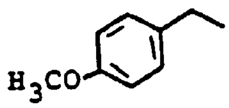 | $C_{65}H_{103}N_3O_{10}$(1101); 1108 ($M+Li^+$) |
| 216 | 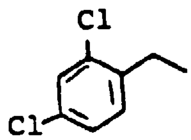 | $C_{64}H_{99}Cl_2N_3O_{10}$(1139); 1146 ($M+Li^+$) |
| 217 | 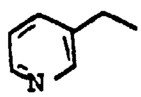 | $C_{63}H_{100}N_4O_{10}$(1072); 1079 ($M+Li^+$) |
| 218 | 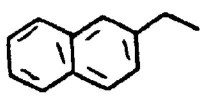 | $C_{68}H_{103}N_3O_{10}$(1121); 1128 ($M+Li^+$) |
| 219 | 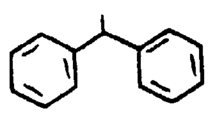 | $C_{70}H_{105}N_3O_{10}$(1147); 1154 ($M+Li^+$) |

In Analogie zu den Beispielen 169 und 170 wurden die Verbindungen der Tabelle 23 erhalten.

Tabelle 23

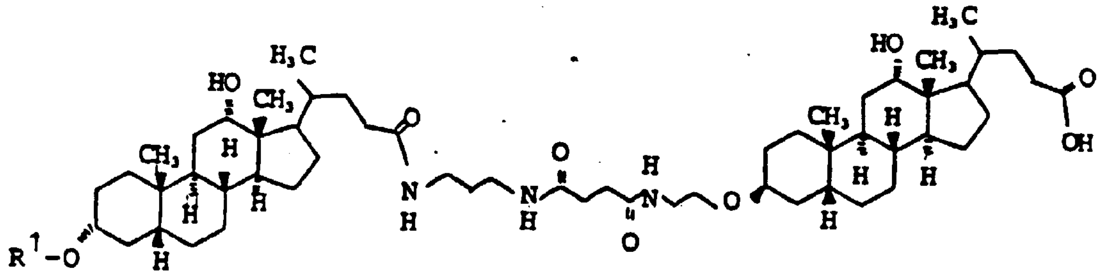

| Bsp. | $R^1$ | MS (FAB,3-NBA/LiCl oder LiJ) |
|---|---|---|
| 220 | H | $C_{57}H_{95}N_3O_9$(965); 972 (M+Li$^+$) |
| 221 | 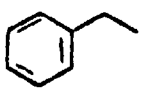 | $C_{64}H_{101}N_3O_9$(1055); 1062 (M+Li$^+$) |
| 222 | 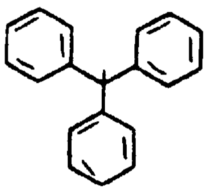 | $C_{76}H_{109}N_3O_9$(1207); 1214 (M+Li$^+$) |
| 223 | 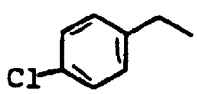 | $C_{64}H_{100}ClN_3O_{10}$(1089); 1096 (M+Li$^+$) |
| 224 | 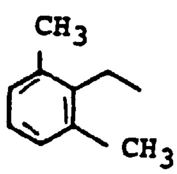 | $C_{66}H_{105}N_3O_9$(1083); 1090 (M+Li$^+$) |

Fortsetzung Tabelle 23

| Bsp. | $R^1$ | MS (FAB,3-NBA/LiCl oder LiJ) |
|---|---|---|
| 225 | 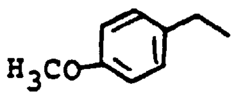 | $C_{65}H_{103}N_3O_{10}$(1085); 1092 ($M+Li^+$) |
| 226 | 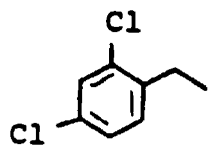 | $C_{64}H_{99}Cl_2N_3O_9$(1123); 1130 ($M+Li^+$) |
| 227 | 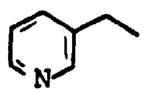 | $C_{63}H_{100}N_4O_9$(1056); 1063 ($M+Li^+$) |
| 228 | 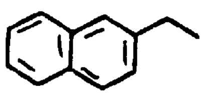 | $C_{68}H_{103}N_3O_9$(1105); 1112 ($M+Li^+$) |
| 229 | 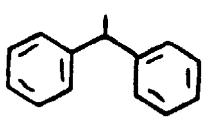 | $C_{70}H_{105}N_3O_9$(1131); 1138 ($M+Li^+$) |

In Analogie zu den Beispielen 169 und 170 wurden die Verbindungen der Tabelle 24 erhalten.

Tabelle 24

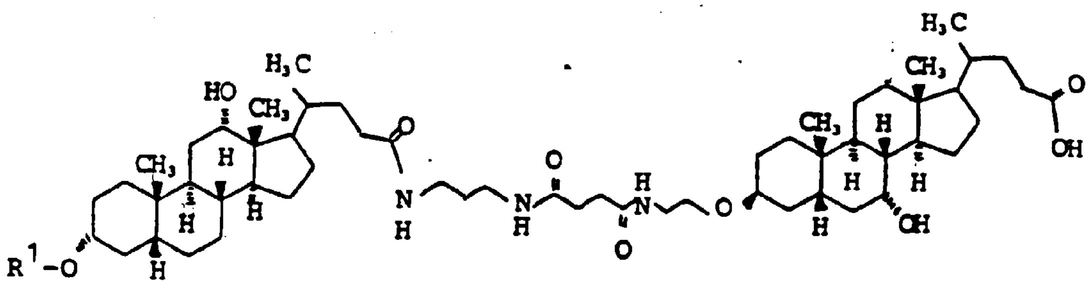

| Bsp. | $R^1$ | MS (FAB,3-NBA/LiCl oder LiJ) |
|---|---|---|
| 230 | H | $C_{57}H_{95}N_3O_9$(965); 972 (M+Li$^+$) |
| 231 | 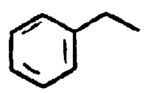 | $C_{64}H_{101}N_3O_9$(1055); 1062 (M+Li$^+$) |
| 232 | 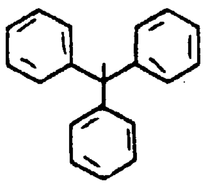 | $C_{76}H_{109}N_3O_9$(1207); 1214 (M+Li$^+$) |
| 233 | 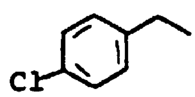 | $C_{64}H_{100}ClN_3O_9$(1089); 1096 (M+Li$^+$) |
| 234 | 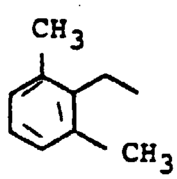 | $C_{66}H_{105}N_3O_9$(1083); 1090 (M+Li$^+$) |

**Fortsetzung Tabelle 24**

| Bsp. | $R^1$ | MS (FAB,3-NBA/LiCl oder LiJ) |
|---|---|---|
| 235 | 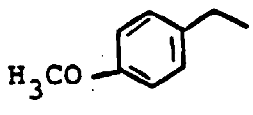 | $C_{65}H_{105}N_3O_{10}$(1085); 1092 $(M+Li^+)$ |
| 236 | 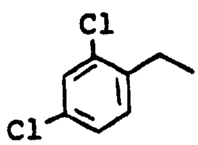 | $C_{64}H_{99}Cl_2N_3O_9$(1123); 1130 $(M+Li^+)$ |
| 237 | 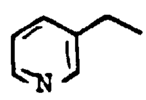 | $C_{63}H_{100}N_4O_9$(1056); 1063 $(M+Li^+)$ |
| 238 | 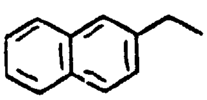 | $C_{68}H_{103}N_3O_9$(1105); 1112 $(M+Li^+)$ |
| 239 | 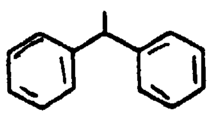 | $C_{70}H_{105}N_3O_9$(1131); 1138 $(M+Li^+)$ |

In Analogie zu den Beispielen 160 und 170 wurden die Verbindungen der Tabelle 25 erhalten.

Tabelle 25

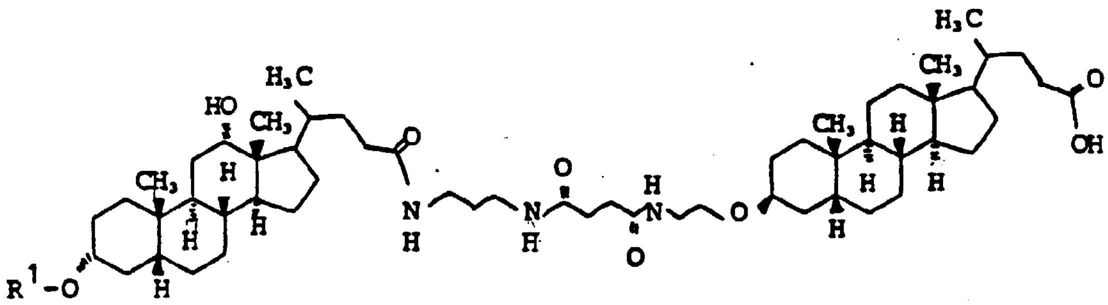

| Bsp. | $R^1$ | MS (FAB, 3-NBA/LiCl oder LiJ) |
|---|---|---|
| 240 | H | $C_{57}H_{95}N_3O_8$ (949); 956 ($M+Li^+$) |
| 241 | 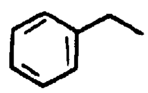 | $C_{64}H_{101}N_3O_8$ (1039); 1046 ($M+Li^+$) |
| 242 | 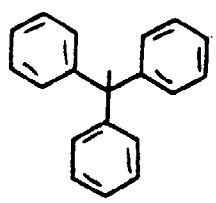 | $C_{76}H_{109}N_3O_8$ (1191); 1198 ($M+Li^+$) |
| 243 | 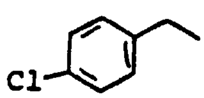 | $C_{64}H_{100}ClN_3O_8$ (1073); 1080 ($M+Li^+$) |
| 244 | 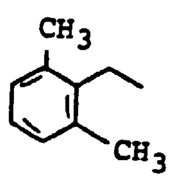 | $C_{66}H_{105}N_3O_8$ (1067); 1074 ($M+Li^+$) |

**Fortsetzung Tabelle 25**

| Bsp. | $R^1$ | MS (FAB,3-NBA/LiCl oder LiJ) |
|---|---|---|
| 245 | 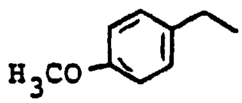 | $C_{65}H_{103}N_3O_9$(1069); 1076 ($M+Li^+$) |
| 246 | 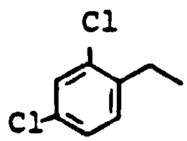 | $C_{64}H_{99}Cl_2N_3O_8$(1107); 1114 ($M+Li^+$) |
| 247 | 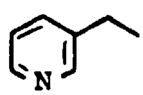 | $C_{63}H_{100}N_4O_8$(1040); 1047 ($M+Li^+$) |
| 248 | 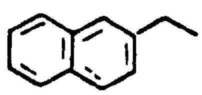 | $C_{68}H_{103}N_3O_8$(1089); 1096 ($M+Li^+$) |
| 249 | 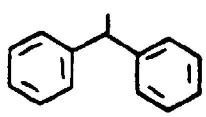 | $C_{70}H_{105}N_3O_8$(1115); 1122 ($M+Li^+$) |

In Analogie zu den Beispielen 169 und 170 wurden die Verbindungen der Tabelle 26 erhalten.

Tabelle 26

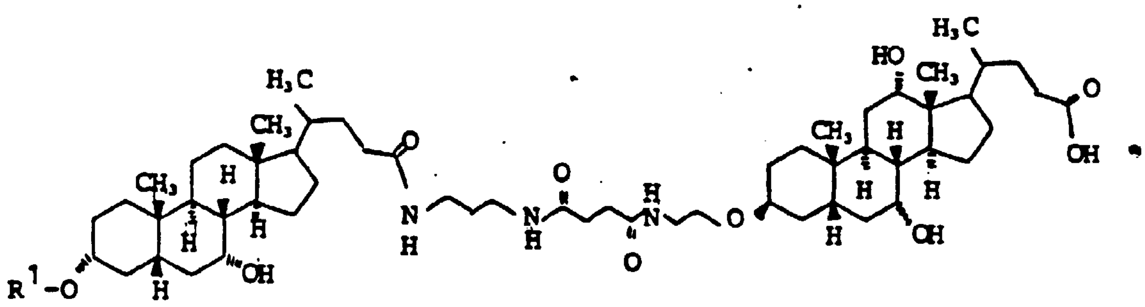

| Bsp. | $R^1$ | MS (FAB,3-NBA/LiCl oder LiJ) |
|---|---|---|
| 250 | H | $C_{57}H_{95}N_3O_{10}$(981); 988 $(M+Li^+)$ |
| 251 | 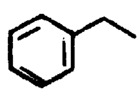 | $C_{64}H_{101}N_3O_{10}$(1071); 1078 $(M+Li^+)$ |
| 252 | 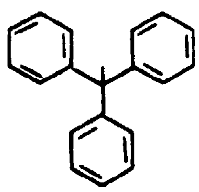 | $C_{76}H_{109}N_3O_{10}$(1223); 1230 $(M+Li^+)$ |
| 253 | 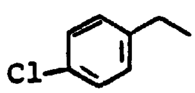 | $C_{64}H_{100}ClN_3O_{10}$(1105); 1112 $(M+Li^+)$ |
| 254 | 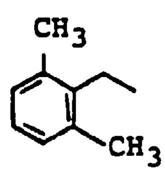 | $C_{66}H_{105}N_3O_{10}$(1099); 1106 $(M+Li^+)$ |

**Fortsetzung Tabelle 26**

| Bsp. | $R^1$ | MS (FAB,3-NBA/LiCl oder LiJ) |
|---|---|---|
| 255 | 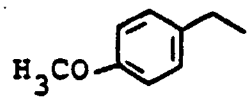 | $C_{65}H_{103}N_3O_{11}$(1101); 1108 ($M+Li^+$) |
| 256 | 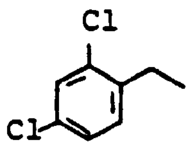 | $C_{64}H_{99}Cl_2N_3O_{10}$(1139); 1146 ($M+Li^+$) |
| 257 | 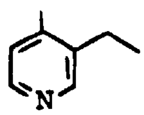 | $C_{63}H_{100}N_4O_{10}$(1072); 1079 ($M+Li^+$) |
| 258 | 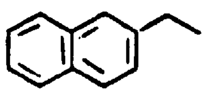 | $C_{68}H_{103}N_3O_{10}$(1121); 1128 ($M+Li^+$) |
| 259 | 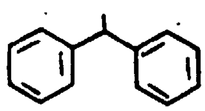 | $C_{70}H_{105}N_3O_{10}$(1147); 1154 ($M+Li^+$) |

In Analogie zu den Beispielen 169 und 170 wurden die Verbindungen der Tabelle 27 erhalten.

Tabelle 27

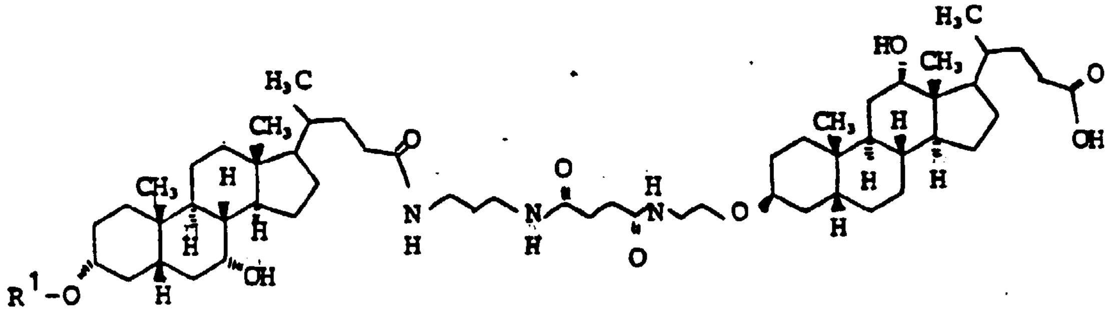

| Bsp. | $R^1$ | MS (FAB,3-NBA/LiCl oder LiJ) |
| --- | --- | --- |
| 260 | H | $C_{57}H_{95}N_3O_9$(965); 972 $(M+Li^+)$ |
| 261 | 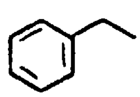 | $C_{64}H_{101}N_3O_9$(1055); 1062 $(M+Li^+)$ |
| 262 | 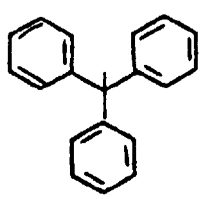 | $C_{76}H_{109}N_3O_9$(1207); 1214 $(M+Li^+)$ |
| 263 | 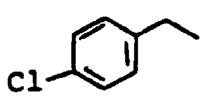 | $C_{64}H_{100}ClN_3O_9$(1089); 1096 $(M+Li^+)$ |
| 264 | 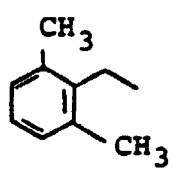 | $C_{66}H_{105}N_3O_9$(1083); 1090 $(M+Li^+)$ |

Fortsetzung Tabelle 27

| Bsp. | $R^1$ | MS (FAB,3-NBA/LiCl oder LiJ) |
|---|---|---|
| 265 | 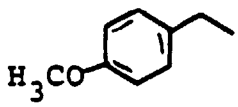 | $C_{65}H_{103}N_3O_{10}$(1085); 1092 ($M+Li^+$) |
| 266 | 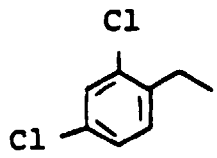 | $C_{64}H_{99}Cl_2N_3O_9$(1123); 1130 ($M+Li^+$) |
| 267 | 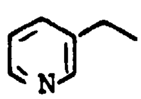 | $C_{63}H_{100}N_4O_9$(1056); 1063 ($M+Li^+$) |
| 268 | 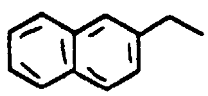 | $C_{68}H_{103}N_3O_9$(1105); 1112 ($M+Li^+$) |
| 269 | 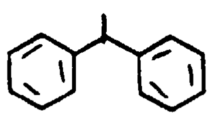 | $C_{70}H_{105}N_3O_9$(1105); 1112 ($M+Li^+$) |

In Analogie zu den Beispielen 169 und 170 werden die Verbindungen der Tabelle 28 erhalten.

Tabelle 28

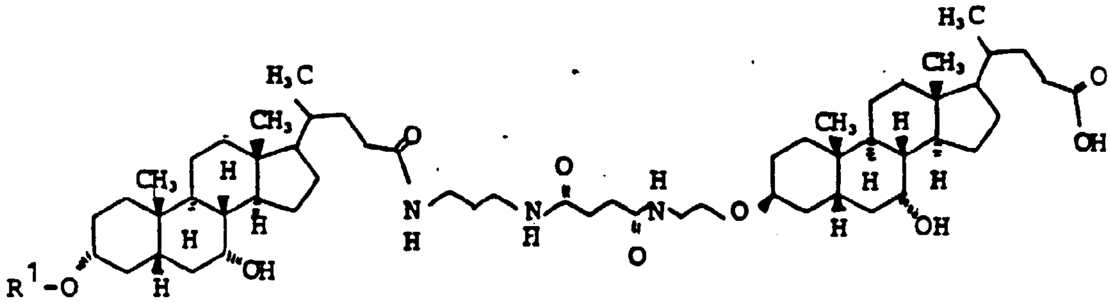

| Bsp. | $R^1$ | MS (FAB,3-NBA/LiCl oder LiJ) |
|---|---|---|
| 270 | H | $C_{57}H_{95}N_3O_9$(965); 972 (M+$Li^+$) |
| 271 | 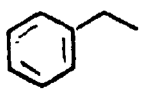 | $C_{64}H_{101}N_3O_9$(1055); 1062 (M+$Li^+$) |
| 272 | 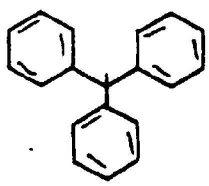 | $C_{76}H_{109}N_3O_9$(1207); 1214 (M+$Li^+$) |
| 273 | 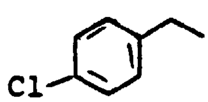 | $C_{64}H_{100}ClN_3O_9$(1089); 1096 (M+$Li^+$) |
| 274 | 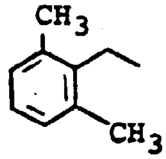 | $C_{66}H_{105}N_3O_9$(1083); 1090 (M+$Li^+$) |

Fortsetzung Tabelle 28

| Bsp. | $R^1$ | MS (FAB,3-NBA/LiCl oder LiJ) |
|---|---|---|
| 275 | 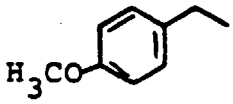 | $C_{65}H_{103}N_3O_{10}$(1085); 1092 ($M+Li^+$) |
| 276 | 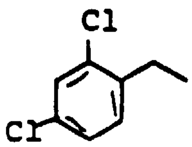 | $C_{64}H_{99}Cl_2N_3O_9$(1123); 1130 ($M+Li^+$) |
| 277 | 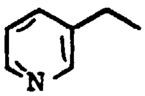 | $C_{63}H_{100}N_4O_9$(1056); 1063 ($M+Li^+$) |
| 278 | 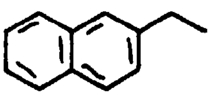 | $C_{68}H_{103}N_3O_9$(1105); 1112 ($M+Li^+$) |
| 279 | 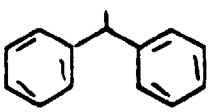 | $C_{70}H_{105}N_3O_9$(1131); 1138 ($M+Li^+$) |

In Analogie zu den Beispielen 160 und 170 wurden die Verbindungen der Tabelle 29 erhalten.

Tabelle 29

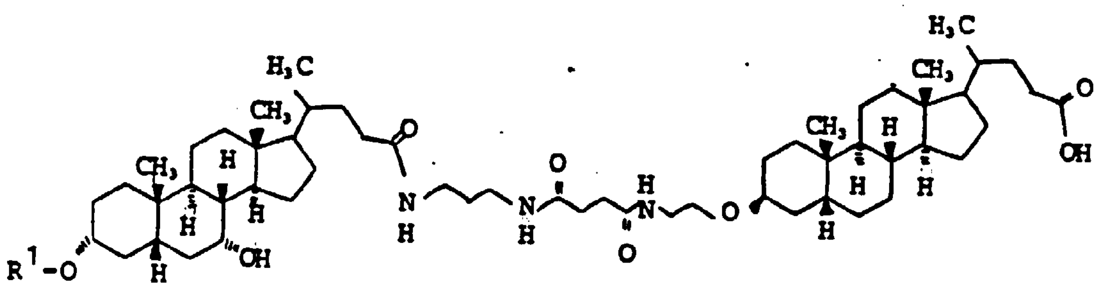

| Bsp. | $R^1$ | MS (FAB,3-NBA/LiCl oder LiJ) |
|---|---|---|
| 280 | H | $C_{57}H_{95}N_3O_8$(949); 956 (M+Li$^+$) |
| 281 | 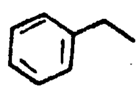 | $C_{64}H_{101}N_3O_8$(1039); 1046 (M+Li$^+$) |
| 282 | 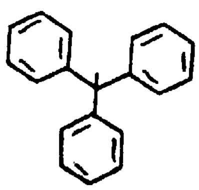 | $C_{76}H_{109}N_3O_8$(1191); 1198 (M+Li$^+$) |
| 283 | 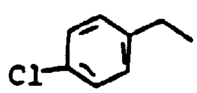 | $C_{64}H_{100}ClN_3O_8$(1073); 1080 (M+Li$^+$) |
| 284 | 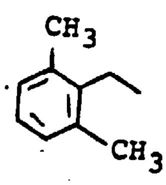 | $C_{66}H_{105}N_3O_8$(1067); 1074 (M+Li$^+$) |

**Fortsetzung Tabelle 29**

| Bsp. | $R^1$ | MS (FAB,3-NBA/LiCl oder LiJ) |
|---|---|---|
| 285 | 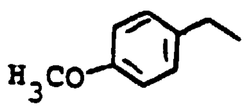 | $C_{65}H_{103}N_3O_9$(1069); 1076 (M+Li$^+$) |
| 286 | 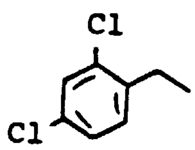 | $C_{64}H_{99}Cl_2N_3O_8$(1107); 1114 (M+Li$^+$) |
| 287 | 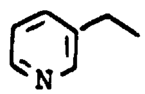 | $C_{63}H_{100}N_4O_8$(1040); 1047 (M+Li$^+$) |
| 288 | 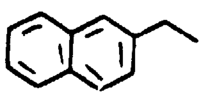 | $C_{68}H_{103}N_3O_8$(1089); 1096 (M+Li$^+$) |
| 289 | 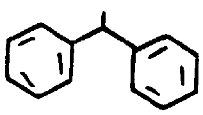 | $C_{70}H_{105}N_3O_8$(1115); 1122 (M+Li$^+$) |

In Analogie zu den Beispielen 169 und 170 wurden die Verbindungen der Tabelle 30 erhalten.

Tabelle 30

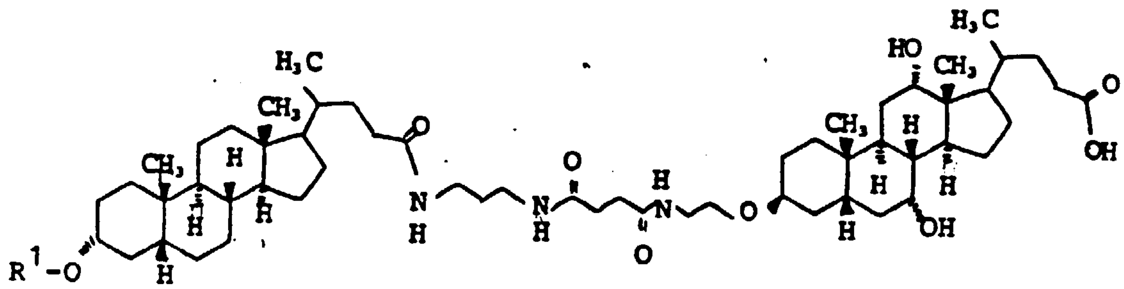

| Bsp. | $R^1$ | MS (FAB,3-NBA/LiCl oder LiJ) |
|---|---|---|
| 290 | H | $C_{57}H_{95}N_3O_9$(965); 972 (M+Li$^+$) |
| 291 | 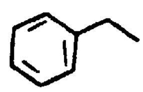 | $C_{64}H_{101}N_3O_9$(1055); 1062 (M+Li$^+$) |
| 292 | 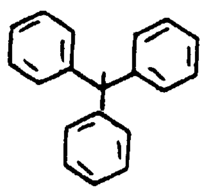 | $C_{76}H_{109}N_3O_9$(1207); 1214 (M+Li$^+$) |
| 293 | 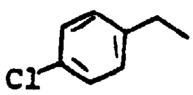 | $C_{64}H_{100}ClN_3O_9$(1089); 1096 (M+Li$^+$) |
| 294 | 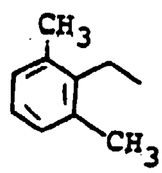 | $C_{66}H_{105}N_3O_9$(1083); 1090 (M+Li$^+$) |

**Fortsetzung Tabelle 30**

| Bsp. | $R^1$ | MS (FAB,3-NBA/LiCl oder LiJ) |
|---|---|---|
| 295 | 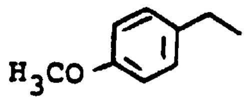 | $C_{65}H_{103}N_3O_{10}$(1085); 1092 ($M+Li^+$) |
| 296. | 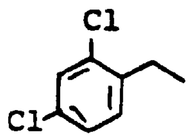 | $C_{64}H_{99}Cl_2N_3O_9$(1123); 1130 ($M+Li^+$) |
| 297 | 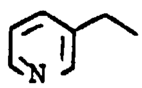 | $C_{63}H_{100}N_4O_9$(1056); 1063 ($M+Li^+$) |
| 298 | 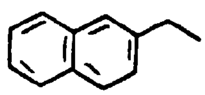 | $C_{68}H_{103}N_3O_9$(1105); 1112 ($M+Li^+$) |
| 299 | 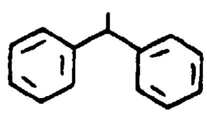 | $C_{68}H_{105}N_3O_9$(1131); 1138 ($M+Li^+$) |

In Analogie zu den Beispielen 160 und 170 wurden die Verbindungen der Tabelle 31 erhalten.

Tabelle 31

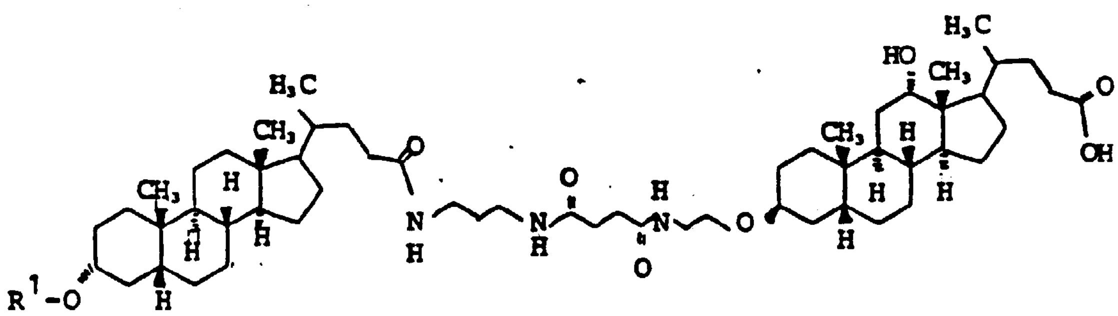

| Bsp. | $R^1$ | MS (FAB,3-NBA/LiCl oder LiJ) |
|---|---|---|
| 300 | H | $C_{57}H_{95}N_3O_8$(949); 956 ($M+Li^+$) |
| 301 | 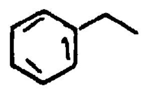 | $C_{64}H_{101}N_3O_8$(1039); 1046 ($M+Li^+$) |
| 302 | 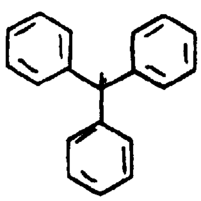 | $C_{76}H_{109}N_3O_8$(1191); 1198 ($M+Li^+$) |
| 303 | 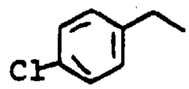 | $C_{64}H_{100}ClN_3O_8$(1073); 1080 ($M+Li^+$) |
| 304 | 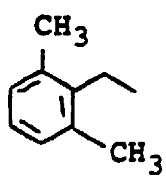 | $C_{66}H_{105}N_3O_8$(1067); 1074 ($M+Li^+$) |

Fortsetzung Tabelle 31

| Bsp. | $R^1$ | MS (FAB,3-NBA/LiCl oder LiJ) |
|---|---|---|
| 305 | 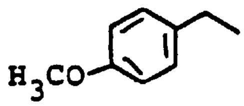 | $C_{65}H_{103}N_3O_9$(1069); 1076 ($M+Li^+$) |
| 306 | 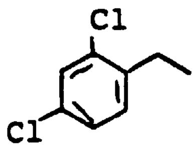 | $C_{64}H_{99}Cl_2N_3O_8$(1107); 1114 ($M+Li^+$) |
| 307 | 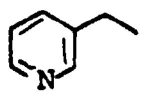 | $C_{63}H_{100}N_4O_8$(1040); 1047 ($M+Li^+$) |
| 308 | 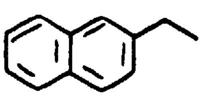 | $C_{68}H_{103}N_3O_8$(1089); 1096 ($M+Li^+$) |
| 309 | 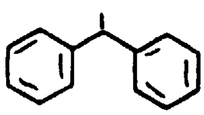 | $C_{70}H_{105}N_3O_8$(1115); 1022 ($M+Li^+$) |

In Analogie zu den Beispielen 169 und 170 wurden die Verbindungen oder Tabelle 32 erhalten.

Tabelle 32

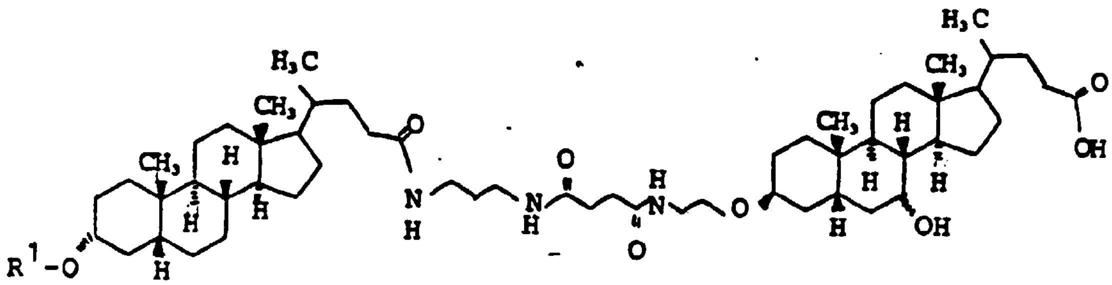

| Bsp. | $R^1$ | MS (FAB, 3-NBA/LiCl oder LiJ) |
|---|---|---|
| 310 | H | $C_{57}H_{95}N_3O_8$(949); 956 (M+$Li^+$) |
| 311 | 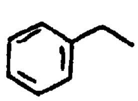 | $C_{64}H_{101}N_3O_8$(1039); 1046 (M+$Li^+$) |
| 312 | 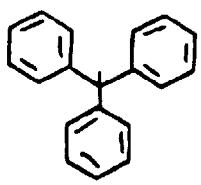 | $C_{76}H_{109}N_3O_8$(1191); 1198 (M+$Li^+$) |
| 313 | 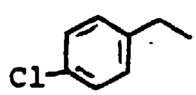 | $C_{64}H_{100}ClN_3O_8$(1073); 1080 (M+$Li^+$) |
| 314 | 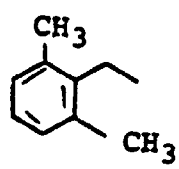 | $C_{66}H_{105}N_3O_8$(1067); 1074 (M+$Li^+$) |

**Fortsetzung Tabelle 32**

| Bsp. | $R^1$ | MS (FAB,3-NBA/LiCl oder LiJ) |
|---|---|---|
| 315 | 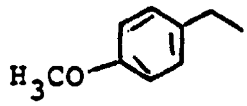 | $C_{65}H_{103}N_3O_9$(1069); 1076 (M+Li$^+$) |
| 316 | 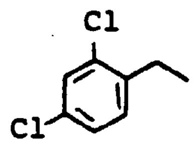 | $C_{64}H_{99}Cl_2N_3O_8$(1107); 1114 (M+Li$^+$) |
| 317 | 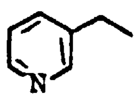 | $C_{63}H_{100}N_4O_8$(1040); 1047 (M+Li$^+$) |
| 318 | 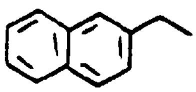 | $C_{68}H_{103}N_3O_8$(1089); 1096 (M+Li$^+$) |
| 319 | 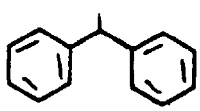 | $C_{70}H_{105}N_3O_8$(1115); 1122 (M+Li$^+$) |

In Analogie zu den Beispielen 169 und 170 wurden die Verbindungen der Tabelle 33 erhalten.

Tabelle 33

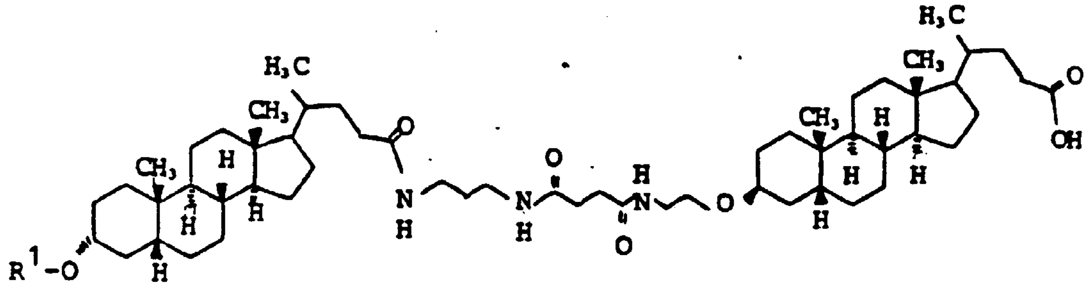

| Bsp. | $R^1$ | MS (FAB,3-NBA/LiCl oder LiJ) |
|---|---|---|
| 320 | H | $C_{57}H_{95}N_3O_7$(933); 940 (M+$Li^+$) |
| 322 | 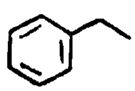 | $C_{64}H_{101}N_3O_7$(1023); 1030 (M+$Li^+$) |
| 323 | 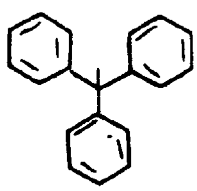 | $C_{76}H_{109}N_3O_7$(1171); 1082 (M+$Li^+$) |
| 324 | 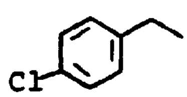 | $C_{64}H_{100}ClN_3O_7$(1057); 1064 (M+$Li^+$) |
| 325 | 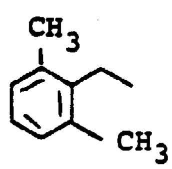 | $C_{66}H_{105}N_3O_7$(1051); 1058 (M+$Li^+$) |

**Fortsetzung Tabelle 33**

| Bsp. | $R^1$ | MS (FAB,3-NBA/LiCl oder LiJ) |
|---|---|---|
| 325 | 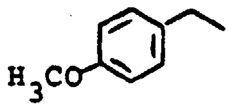 | $C_{65}H_{103}N_3O_8$(1053); 1060 ($M+Li^+$) |
| 326 | 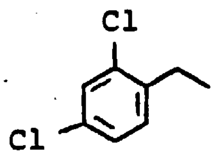 | $C_{64}H_{99}Cl_2N_3O_7$(1091); 1098 ($M+Li^+$) |
| 327 | 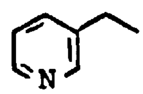 | $C_{63}H_{100}N_4O_7$(1024); 1031 ($M+Li^+$) |
| 328 | 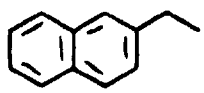 | $C_{68}H_{103}N_3O_7$(1073); 1080 ($M+Li^+$) |
| 329 | 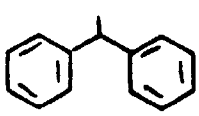 | $C_{70}H_{105}N_3O_7$(1099); 1106 ($M+Li^+$) |

Beispiel 330

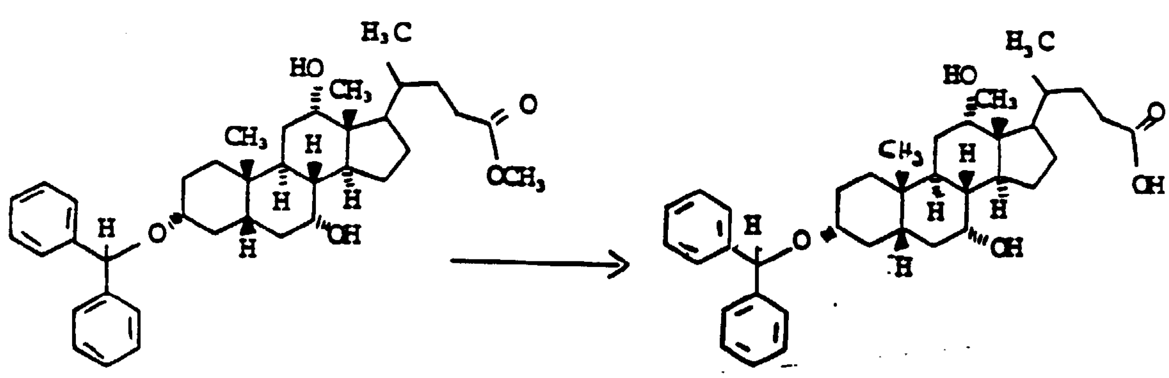

10 g (17 mmol) "Beispiel 1" wurden in 10 ml Äthanol gelöst, 50 ml 1 molare Natronlauge zugegeben und 2h bei Raumtemperatur gerührt, anschließend mit 100 g Natriumdihydrogenphosphat 15 Minuten intensiv verrührt, mit Essigester/Äthanol = 5/1 extrahiert (3x). Die vereinten organischen Phasen wurden getrocknet ($MgSO_4$).

Abdampfen des Lösungsmittels, Verreiben mit Diisopropyläther und Absaugen ergab 9,08 g (15,8 mmol, 93 %) "Beispiel 330" $C_{37}H_{50}O_5$(574),
MS(FAB,2-NBA/LiGI): 581 ($M+Li^+$)

In Analogie zu Beispiel 330 wurden die Beispiele der Tabellen 34- 37 erhalten.

Tabelle 34

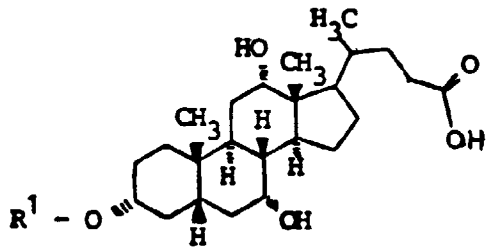

| Beispiel | $R^1$ | MS(FAB,3-NBA/LiCl oder LiJ) |
|---|---|---|
| 331 | 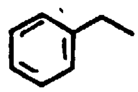 | $C_{31}H_{46}O_5$(498); 505(M+Li$^+$) |
| 332 | 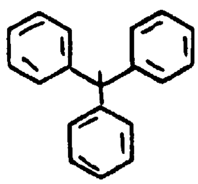 | $C_{43}H_{54}O_5$(650); 657(M+Li$^+$) |
| 333 | 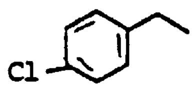 | $C_{31}H_{45}ClO_5$(532); 539(M+Li$^+$) |
| 334 | 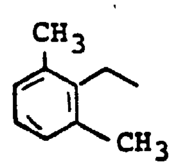 | $C_{33}H_{50}O_5$(526); 533(M+Li$^+$) |
| 335 | 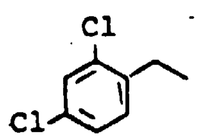 | $C_{31}H_{44}Cl_2O_5$(566); 573(M+Li$^+$) |
| 336 | 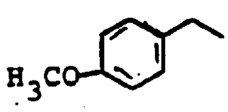 | $C_{32}H_{48}O_6$(528); 535(M+Li$^+$) |
| 337 | 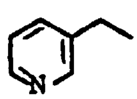 | $C_{30}H_{45}NO_5$(499); 506(M+Li$^+$) |
| 338 | 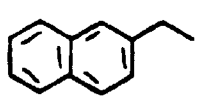 | $C_{35}H_{48}O_5$(538); 545(M+Li$^+$) |

Tabelle 35

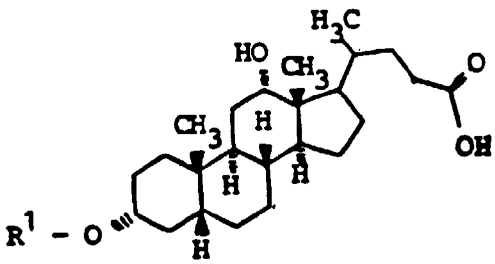

| Beispiel | $R^1$ | MS(FAB,3-NBA/LiCl oder LiJ) |
|---|---|---|
| 339 | 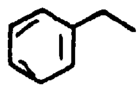 | $C_{31}H_{46}O_4$(482); 489(M+Li$^+$) |
| 340 | 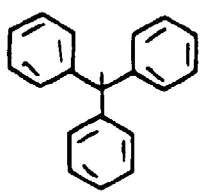 | $C_{43}H_{54}O_4$(634); 641(M+Li$^+$) |
| 341 | 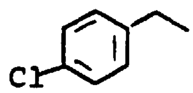 | $C_{31}H_{45}ClO_4$(516); 523(M+Li$^+$) |
| 342 | 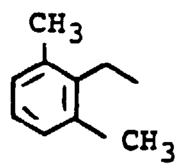 | $C_{33}H_{50}O_4$(510); 517(M+Li$^+$) |
| 343 | 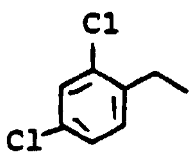 | $C_{31}H_{44}Cl_2O_4$(550); 557(M+Li$^+$) |
| 344 | 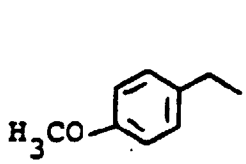 | $C_{32}H_{48}O_5$(512); 519(M+Li$^+$) |

Fortsetzung Tabelle 35

| Beispiel | $R^1$ | MS(FAB,3-NBA/LiCl oder LiJ) |
|---|---|---|
| 345 | 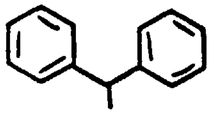 | $C_{30}H_{45}NO_4$(483); 490(M+Li$^+$) |
| 346 | 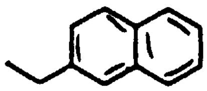 | $C_{35}H_{48}O_4$(522); 529(M+Li$^+$) |
| 347 | 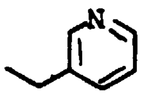 | $C_{37}H_{50}O_4$(558); 565(M+Li$^+$) |

Tabelle 36

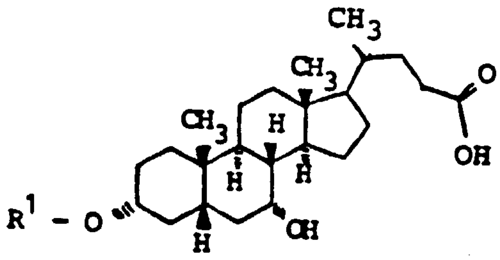

| Beispiel | $R^1$ | MS(FAB,3-NBA/LiCl oder LiJ) |
|---|---|---|
| 348 | 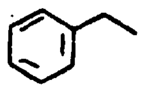 | $C_{31}H_{46}O_4$(482); 489(M+Li$^+$) |
| 349 | 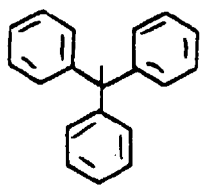 | $C_{43}H_{54}O_4$(634); 641(M+Li$^+$) |
| 350 | 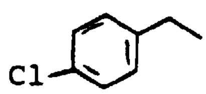 | $C_{31}H_{45}ClO_4$(516); 523(M+Li$^+$) |
| 351 | 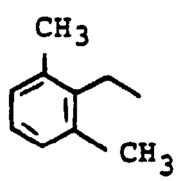 | $C_{33}H_{50}O_4$(510); 517(M+Li$^+$) |
| 352 | 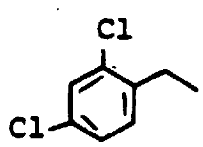 | $C_{31}H_{44}Cl_2O_4$(550); 557(M+Li$^+$) |
| 353 | 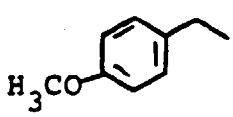 | $C_{32}H_{48}O_5$(512); 519(M+Li$^+$) |

Fortsetzung Tabelle 36

| Beispiel | $R^1$ | MS(FAB,3-NBA/LiCl oder LiJ) |
|---|---|---|
| 354 | 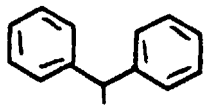 | $C_{30}H_{45}NO_4$(483); 490($M+Li^+$) |
| 355 | 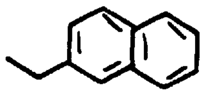 | $C_{35}H_{48}O_4$(522); 529($M+Li^+$) |
| 356 | 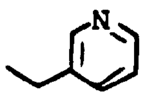 | $C_{37}H_{50}O_4$(558); 565($M+Li^+$) |

Tabelle 37

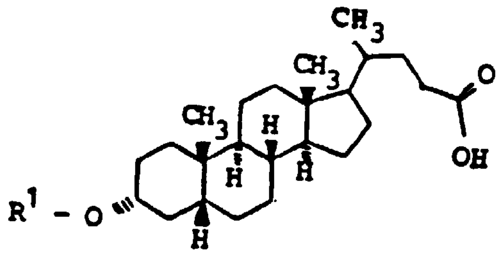

| Beispiel | $R^1$ | MS(FAB,3-NBA/LiCl oder LiJ) |
|---|---|---|
| 357 | 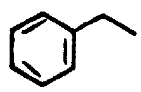 | $C_{31}H_{46}O_3$(466); 473(M+Li$^+$) |
| 358 | 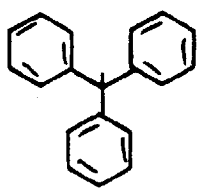 | $C_{43}H_{54}O_3$(618); 625(M+Li$^+$) |
| 359 | 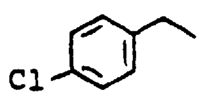 | $C_{31}H_{45}ClO_3$(500); 507(M+Li$^+$) |
| 360 | 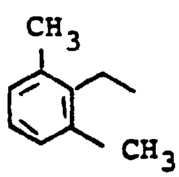 | $C_{33}H_{50}O_3$(494); 501(M+Li$^+$) |
| 361 | 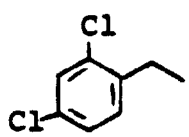 | $C_{31}H_{44}Cl_2O_3$(534); 541(M+Li$^+$) |
| 362 | 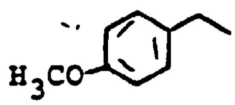 | $C_{32}H_{48}O_4$(496); 503(M+Li$^+$) |

**Fortsetzung Tabelle 37**

| Beispiel | $R^1$ | MS(FAB,3-NBA/LiCl oder LiJ) |
|---|---|---|
| 363 | 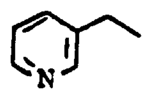 | $C_{30}H_{45}NO_3$(467); 474(M+Li$^+$) |
| 364 | 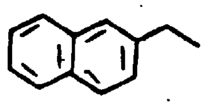 | $C_{35}H_{48}O_3$(506); 513(M+Li$^+$) |
| 365 | 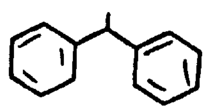 | $C_{37}H_{50}O_3$(542); 549(M+Li$^+$) |

Beispiel 366

Schritt a)

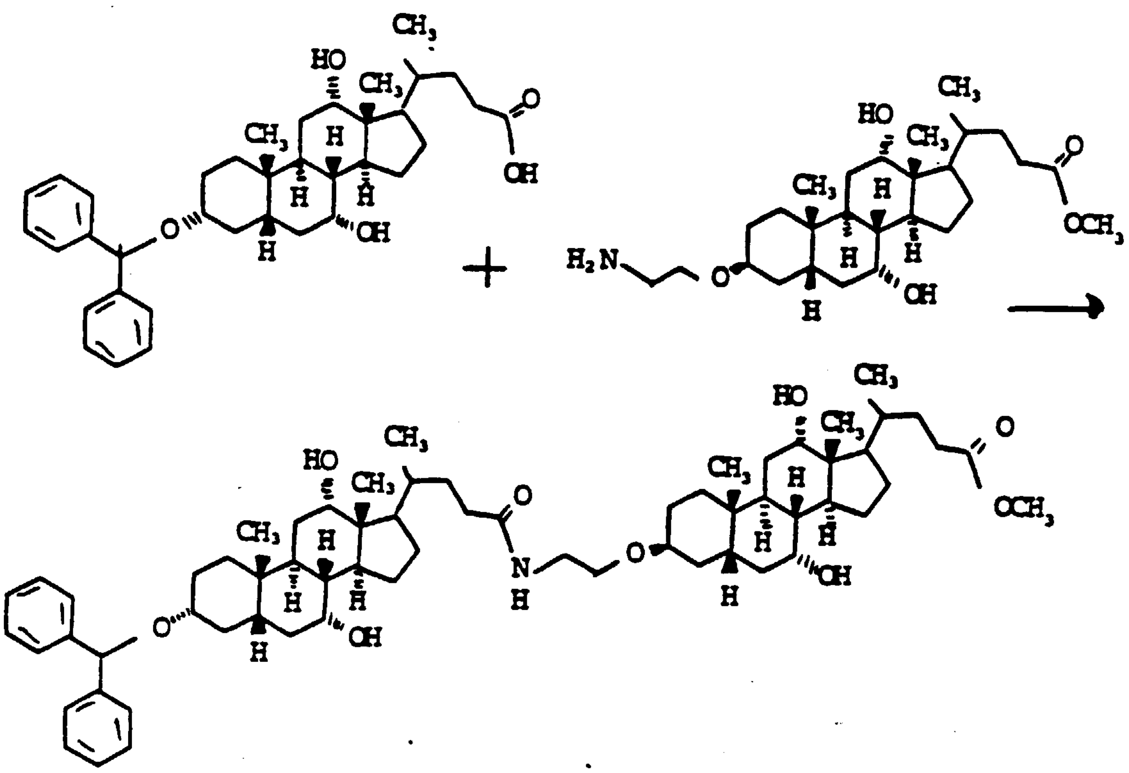

574 mg (1 mmol) "Beispiel 330" gelöst in 20 ml Tetrahydrofuran und 5 ml Triethylamin wurden vorgelegt und bei 0°C 108 µl (1,1 mmol) Chlorameisensäureäthylester zugespritzt. Man ließ 15 Minuten bei 0°C rühren, anschließend gab man 465 mg (1 mmol) "Beispiel 139" fest zu.

Man ließ 4 h bei Raumtemperatur rühren.

Die Reaktionslösung wurde 10 Minuten mit 10 ml 1 molare Salzsäure heftig verquirlt, dann mit Essigester extrahiert (3x). Die vereinten organischen Phasen wurden mit gesättigter wäßriger $NaHCO_3$-Lösung gewaschen und getrocknet ($MgSO_4$).

Abdampfen des Lösungsmittels und Flash-Chromatographie auf Kieselgel (Essigester/Äthanol = 9/1) ergab 755 mg (0,74 mmol, 74 %) "Beispiel 366".

$C_{64}H_{95}NO_9$(1021), MS(FAB,3-NBA/LiCl): 1028 (M+Li+)

Schritt b) Verseifung

Beispiel 367

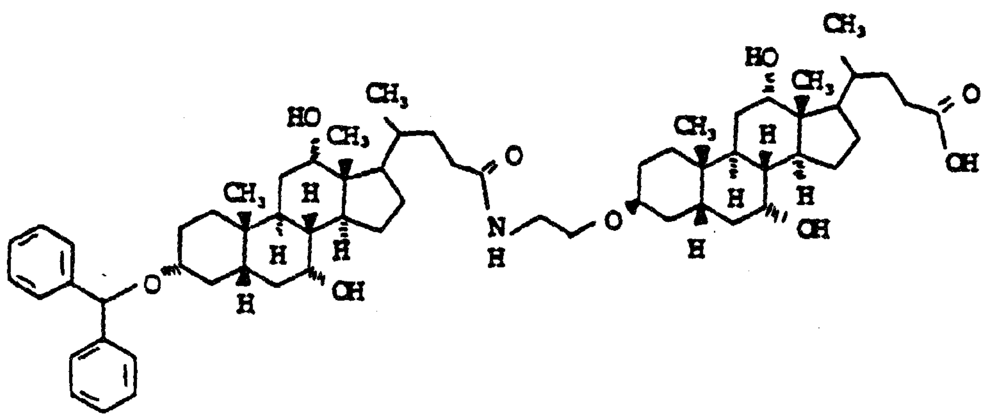

715 mg (0,7 mmol) "Beispiel 366" wurden mit 10 ml Äthanol und 5 ml 1 molare Natronlauge 1,5 h bei Raumtemperatur gerührt, anschließend mit 10 g Natriumdihydrogenphosphat 10 Minuten intensiv verquirlt und mit Essigester/Äthanol= 3/1 extrahiert (3x). Die vereinigten organischen Phasen wurden getrocknet ($MgSO_4$).

Abdampfen des Lösungsmittels, Verreiben mit Diisopropyläther und Absaugen ergab 670 mg (0,67 mmol, 95 %) "Beispiel 367".

$C_{63}H_{93}NO_9$(1007), MS(FAB,3-NBA/LiCl): 1014 (M+Li+)

In Analogie zu den Beispielen 325 und 326 wurden die Verbindungen der Tabelle 35 erhalten.

Beispiel 368

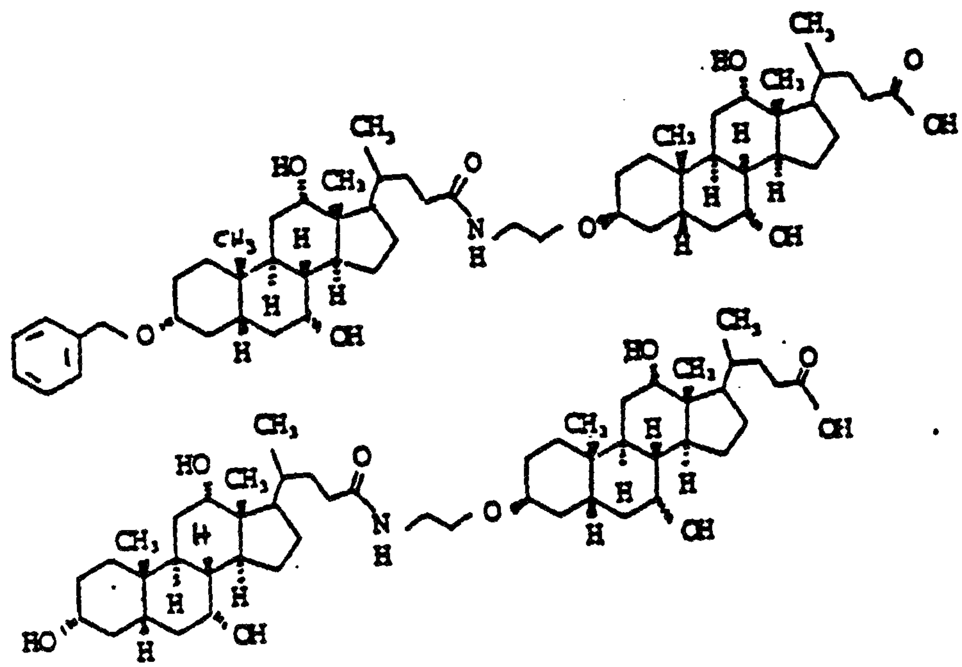

1024 mg (1,1 mmol) "Beispiel 328" wurden in 5 ml Äthanol gelöst, 200 mg Palladium auf Kohle (10 %) zugegeben und 1 h bei Raumtemperatur unter Wasserstoffatmosphäre geschüttelt.

Zur Aufarbeitung wurde der Katalysator abfiltriert und das Filtrat eingedampft. Chromarographie auf Kieselgel (Ethylacetat/Äthanol = 1/1) ergab 780 mg (0,92 mmol, 84 % "Beispiel 490"
$C_{50}H_{83}NO_9$(841), MS(FAB,3-NBA/LiCl); 848 (M+Li$^+$)

In Analogie zu den Beispielen 366 und 367 wurden die Verbindungen der Tabelle 38 erhalten.

Tabelle 38

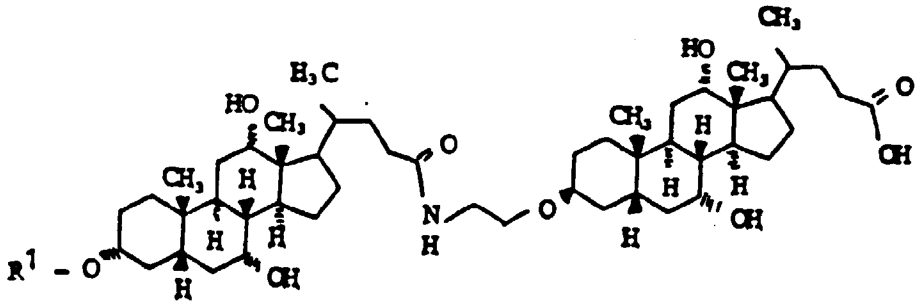

| Beispiel | $R^1$ | MS(FAB,3-NBA/LiCl oder LiJ) |
|---|---|---|
| 369 | 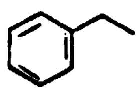 | $C_{57}H_{89}NO_9$(931); 938(M+Li$^+$) |
| 370 | 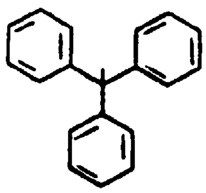 | $C_{69}H_{97}NO_9$(1083); 1090(M+Li$^+$) |
| 371 | 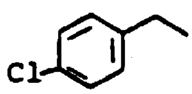 | $C_{57}H_{88}ClNO_9$(965); 972(M+Li$^+$) |
| 372 | 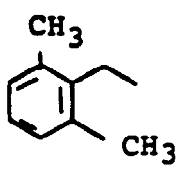 | $C_{59}H_{93}NO_9$(959); 966(M+Li$^+$) |
| 373 | 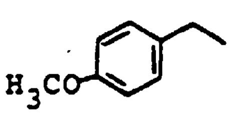 | $C_{58}H_{91}NO_{10}$(961); 968(M+Li$^+$) |

Fortsetzung Tabelle 38

| Beispiel | $R^1$ | MS(FAB,3-NBA/LiCl oder LiJ) |
|---|---|---|
| 374 | 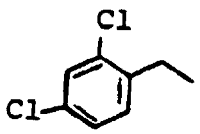 | $C_{57}H_{87}Cl_2NO_9$(999); 1006(M+Li$^+$) |
| 375 | 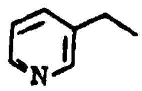 | $C_{56}H_{88}N_2O_9$(932); 939(M+Li$^+$) |
| 376 | 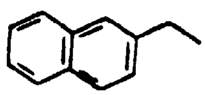 | $C_{61}H_{91}NO_9$(981); 988(M+Li$^+$) |

In Analogie zu den Beispielen 366, 367 und 368 wurden die Verbindungen der Tabelle 39 erhalten.

Tabelle 39

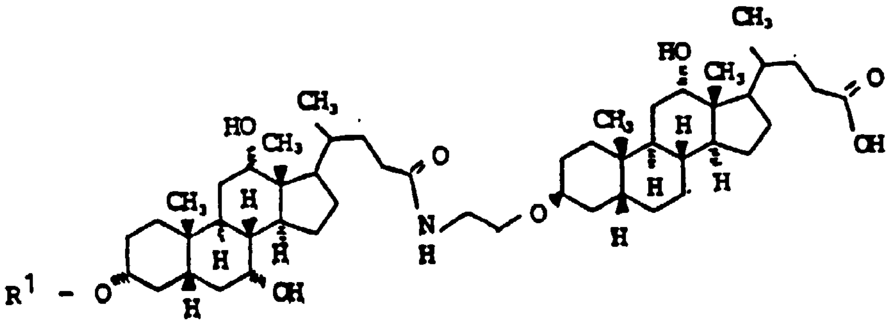

| Beispiel | $R^1$ | MS(FAB,3-NBA/LiCl oder LiJ) |
|---|---|---|
| 377 | H | $C_{50}H_{83}NO_8$(825); 832(M+Li$^+$) |
| 378 | 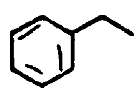 | $C_{57}H_{89}NO_8$(915); 922(M+Li$^+$) |
| 379 | 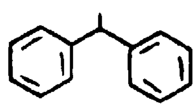 | $C_{63}H_{93}NO_8$(991); 998(M+Li$^+$) |
| 380 | 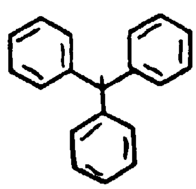 | $C_{69}H_{97}NO_8$(1067); 1074(M+Li$^+$) |
| 381 | 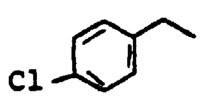 | $C_{57}H_{88}ClNO_8$(949); 956(M+Li$^+$) |

**Fortsetzung Tabelle 39**

| Beispiel | $R^1$ | MS(FAB,3-NBA/LiCl oder LiJ) |
|---|---|---|
| 382 | 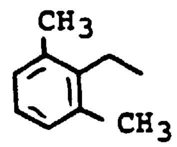 | $C_{58}H_{94}NO_8$(943); 950(M+Li$^+$) |
| 383 | 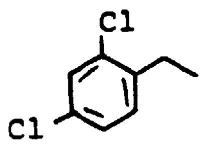 | $C_{57}H_{87}Cl_2NO_8$(983); 990(M+Li$^+$) |
| 384 | 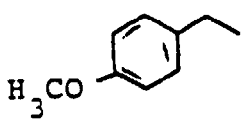 | $C_{58}H_{91}NO_9$(945); 952(M+Li$^+$) |
| 385 | 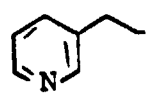 | $C_{56}H_{88}N_2O_8$(916); 923(M+Li$^+$) |
| 386 | 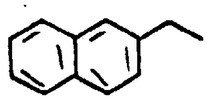 | $C_{61}H_{91}NO_8$(965); 972(M+Li$^+$) |

In Analogie zu den Beispielen 366, 367 und 368 wurden die Verbindungen der Tabelle 40 erhalten.

Tabelle 40

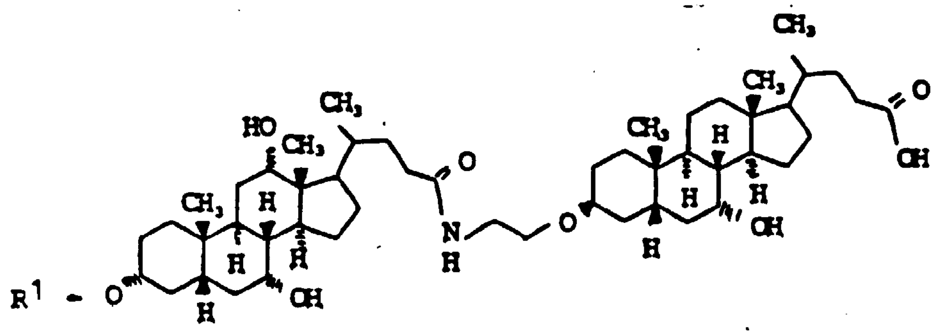

| Beispiel | $R^1$ | MS(FAB,3-NBA/LiCl oder LiJ) |
|---|---|---|
| 387 | H | $C_{50}H_{83}NO_8$(825); 832(M+Li$^+$) |
| 388 | 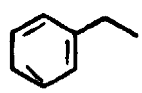 | $C_{57}H_{89}NO_8$(915); 922(M+Li$^+$) |
| 389 | 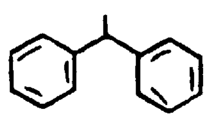 | $C_{63}H_{93}NO_8$(991); 998(M+Li$^+$) |
| 390 | 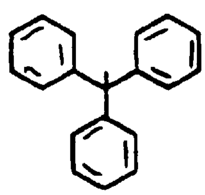 | $C_{69}H_{97}NO_8$(1067); 1074(M+Li$^+$) |
| 391 | 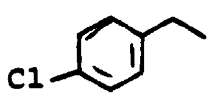 | $C_{57}H_{88}ClNO_8$(949); 956(M+Li$^+$) |

**Fortsetzung Tabelle 40**

| Beispiel | $R^1$ | MS(FAB,3-NBA/LiCl oder LiJ) |
|---|---|---|
| 392 | 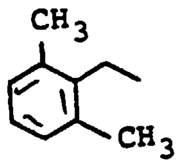 | $C_{59}H_{94}NO_8$(943); 950(M+Li$^+$) |
| 393 | 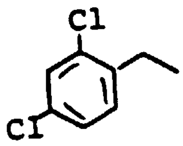 | $C_{57}H_{87}Cl_2NO_8$(983); 990(M+Li$^+$) |
| 394 | 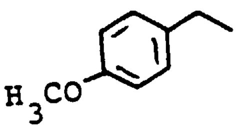 | $C_{58}H_{91}NO_9$(945); 952(M+Li$^+$) |
| 395 | 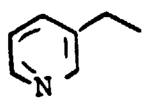 | $C_{56}H_{88}N_2O_8$(916); 923(M+Li$^+$) |
| 396 | 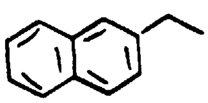 | $C_{61}H_{91}NO_9$(965); 972(M+Li$^+$) |

In Analogie zu den Beispielen 366, 367 und 368 wurden die Verbindungen der Tabelle 41 erhalten.

Tabelle 41

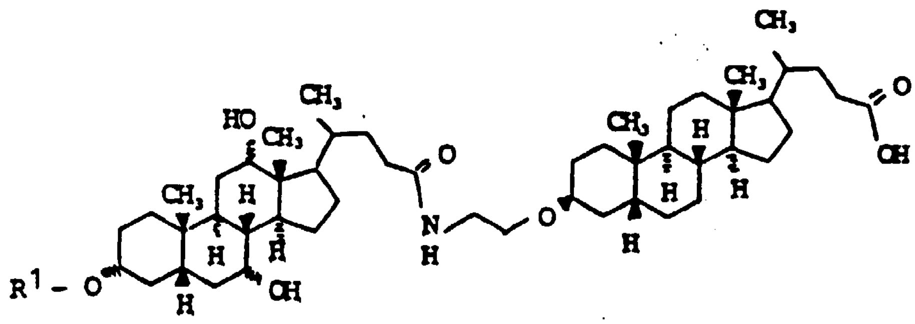

| Beispiel | $R^1$ | MS(FAB,3-NBA/LiCl oder LiJ) |
|---|---|---|
| 397 | H | $C_{50}H_{83}NO_7$(809); 816(M+Li$^+$) |
| 398 | 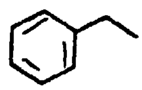 | $C_{57}H_{89}NO_7$(899); 906(M+Li$^+$) |
| 399 | 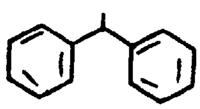 | $C_{63}H_{93}NO_7$(975); 982(M+Li$^+$) |
| 400 | 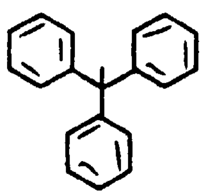 | $C_{69}H_{97}NO_7$(1051); 1058(M+Li$^+$) |
| 401 | 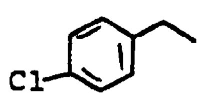 | $C_{57}H_{88}ClNO_7$(933); 940(M+Li$^+$) |

**Fortsetzung Tabelle 41**

| Beispiel | $R^1$ | MS(FAB,3-NBA/LiCl oder LiJ) |
| --- | --- | --- |
| 402 | 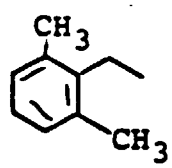 | $C_{59}H_{94}NO_7$(927); 934(M+Li$^+$) |
| 403 | 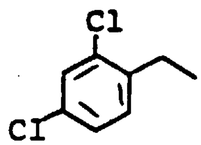 | $C_{57}H_{87}Cl_2NO_7$(967); 974(M+Li$^+$) |
| 404 | 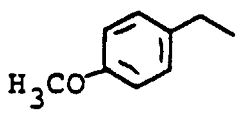 | $C_{58}H_{91}NO_8$(929); 936(M+Li$^+$) |
| 405 | 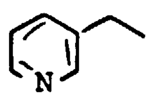 | $C_{56}H_{88}N_2O_7$(900); 907(M+Li$^+$) |
| 406 | 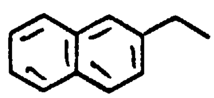 | $C_{61}H_{91}NO_7$(949); 956(M+Li$^+$) |

In Analogie zu den Beispielen 366, 367 und 368 wurden die Verbindungen der Tabelle 42 erhalten.

Tabelle 42

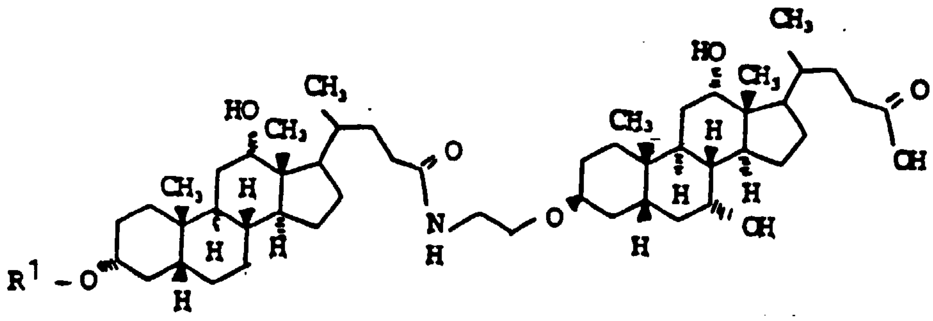

| Beispiel | $R^1$ | MS(FAB,3-NBA/LiCl oder LiJ) |
|---|---|---|
| 407 | H | $C_{50}H_{83}NO_8$(825); 832(M+Li$^+$) |
| 408 | 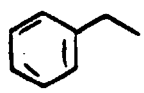 | $C_{57}H_{89}NO_8$(915); 922(M+Li$^+$) |
| 409 | 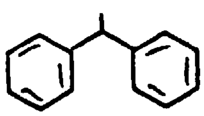 | $C_{63}H_{93}NO_8$(991); 998(M+Li$^+$) |
| 410 | 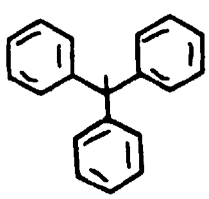 | $C_{69}H_{97}NO_8$(1067); 1074(M+Li$^+$) |
| 411 | 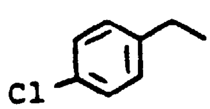 | $C_{57}H_{88}ClNO_8$(949); 956(M+Li$^+$) |

Fortsetzung Tabelle 42

| Beispiel | $R^1$ | MS(FAB,3-NBA/LiCl oder LiJ) |
|---|---|---|
| 412 | 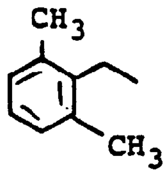 | $C_{59}H_{94}NO_8$(943); 950(M+Li$^+$) |
| 413 | 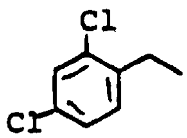 | $C_{57}H_{87}Cl_2NO_8$(983); 990(M+Li$^+$) |
| 414 | 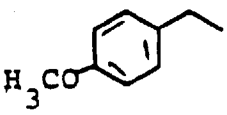 | $C_{58}H_{91}NO_9$(945); 952(M+Li$^+$) |
| 415 | 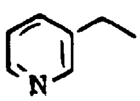 | $C_{56}H_{88}N_2O_8$(916); 923(M+Li$^+$) |
| 416 | 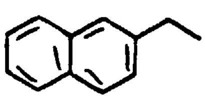 | $C_{61}H_{91}NO_8$(965); 972(M+Li$^+$) |

In Analogie zu den Beispielen 366, 367 und 368 wurden die Verbindungen der Tabelle 43 erhalten.

Tabelle 43

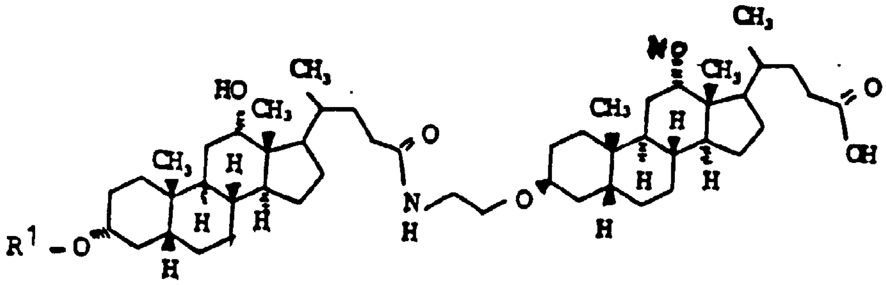

| Beispiel | $R^1$ | MS(FAB,3-NBA/LiCl oder LiJ) |
|---|---|---|
| 417 | H | $C_{50}H_{83}NO_7$(809); 816(M+Li$^+$) |
| 418 | 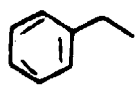 | $C_{57}H_{89}NO_7$(899); 906(M+Li$^+$) |
| 419 | 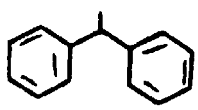 | $C_{63}H_{93}NO_7$(975); 982(M+Li$^+$) |
| 420 | 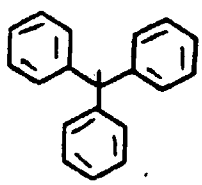 | $C_{69}H_{97}NO_7$(1051); 1058(M+Li$^+$) |
| 421 | 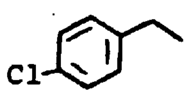 | $C_{57}H_{88}ClNO_7$(933); 940(M+Li$^+$) |

Fortsetzung Tabelle 43

| Beispiel | $R^1$ | MS(FAB,3-NBA/LiCl oder LiJ) |
|---|---|---|
| 422 | 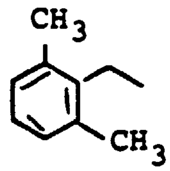 | $C_{59}H_{94}NO_7$(927); 934(M+Li$^+$) |
| 423 | 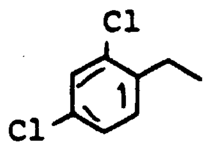 | $C_{57}H_{87}Cl_2NO_7$(967); 974(M+Li$^+$) |
| 424 | 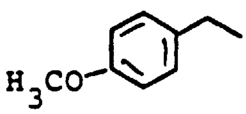 | $C_{58}H_{91}NO_8$(929); 936(M+Li$^+$) |
| 425 | 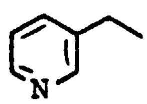 | $C_{56}H_{88}N_2O_7$(900); 907(M+Li$^+$) |
| 426 | 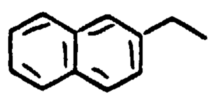 | $C_{61}H_{91}NO_7$(949); 956(M+Li$^+$) |

In Analogie zu den Beispielen 366, 367 und 368 wurden die Verbindungen der Tabelle 44 erhalten.

Tabelle 44

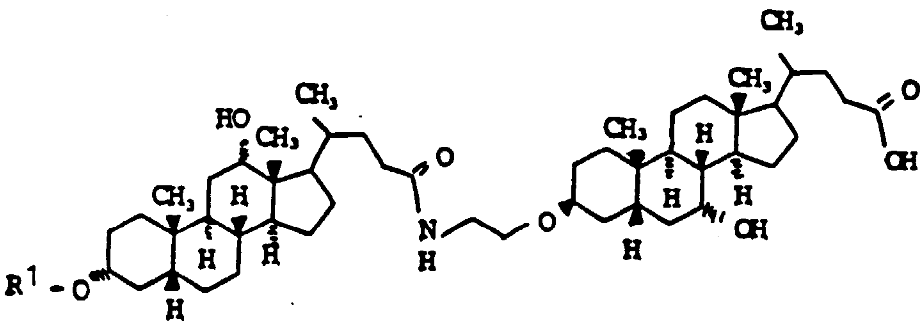

| Beispiel | $R^1$ | MS(FAB,3-NBA/LiCl oder LiJ) |
|---|---|---|
| 427 | H | $C_{50}H_{83}NO_7$(809); 816(M+Li$^+$) |
| 428 | 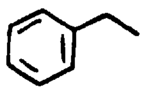 | $C_{57}H_{89}NO_7$(899); 906(M+Li$^+$) |
| 429 | 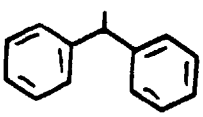 | $C_{63}H_{93}NO_7$(975); 982(M+Li$^+$) |
| 430 | 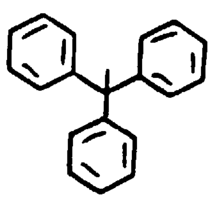 | $C_{69}H_{97}NO_7$(1051); 1058(M+Li$^+$) |
| 431 | 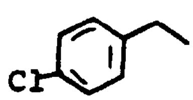 | $C_{57}H_{88}ClNO_7$(933); 940(M+Li$^+$) |

**Fortsetzung Tabelle 44**

| Beispiel | $R^1$ | MS(FAB,3-NBA/LiCl oder LiJ) |
|---|---|---|
| 432 | 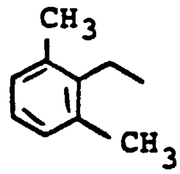 | $C_{59}H_{94}NO_7$(927); 934(M+Li$^+$) |
| 433 | 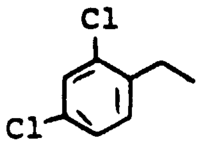 | $C_{57}H_{87}Cl_2NO_7$(967); 974(M+Li$^+$) |
| 434 | 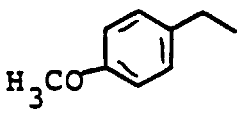 | $C_{58}H_{91}NO_8$(929); 936(M+Li$^+$) |
| 435 | 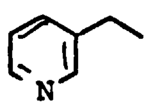 | $C_{56}H_{88}N_2O_7$(900); 907(M+Li$^+$) |
| 436 | 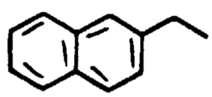 | $C_{61}H_{91}NO_7$(949); 956(M+Li$^+$) |

In Analogie zu den Beispielen 366,367 und 368 wurden die Verbindungen der Tabelle 45 erhalten.

Tabelle 45

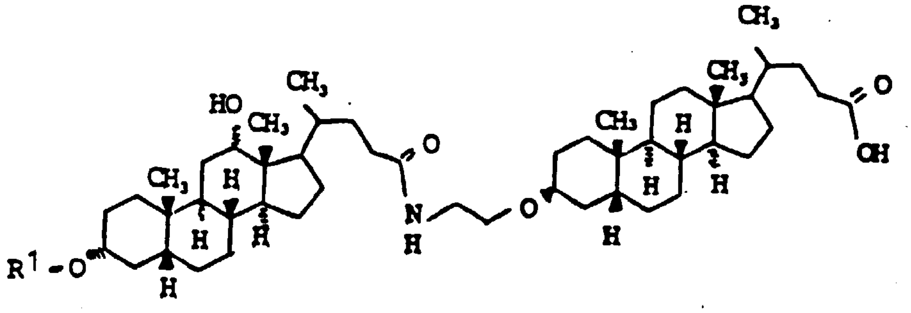

| Beispiel | $R^1$ | MS(FAB,3-NBA/LiCl oder LiJ) |
|---|---|---|
| 437 | H | $C_{50}H_{83}NO_6$(793); 800(M+Li$^+$) |
| 438 | 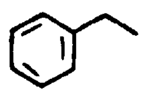 | $C_{57}H_{89}NO_6$(883); 890(M+Li$^+$) |
| 439 | 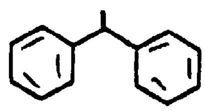 | $C_{63}H_{93}NO_6$(959); 966(M+Li$^+$) |
| 440 | 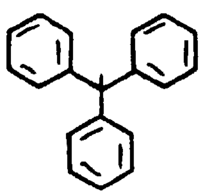 | $C_{69}H_{97}NO_6$(1035); 1042(M+Li$^+$) |
| 441 | 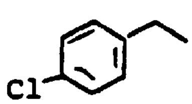 | $C_{57}H_{88}ClNO_6$(917); 924(M+Li$^+$) |

Fortsetzung Tabelle 45

| Beispiel | $R^1$ | MS(FAB,3-NBA/LiCl oder LiJ) |
|---|---|---|
| 442 | 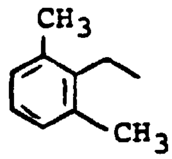 | $C_{59}H_{94}NO_6$(911); 918(M+Li$^+$) |
| 443 | 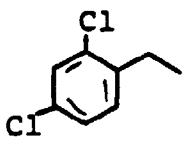 | $C_{57}H_{87}Cl_2NO_6$(951); 958(M+Li$^+$) |
| 444 | 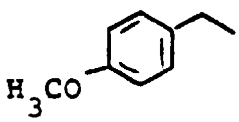 | $C_{58}H_{91}NO_7$(913); 920(M+Li$^+$) |
| 445 | 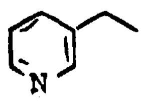 | $C_{56}H_{88}N_2O_6$(884); 891(M+Li$^+$) |
| 446 | 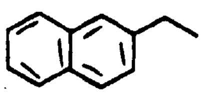 | $C_{61}H_{91}NO_6$(933); 940(M+Li$^+$) |

In Analogie zu den Beispielen 366, 367 und 368 wurden die Verbindungen der Tabelle 46 erhalten.

Tabelle 46

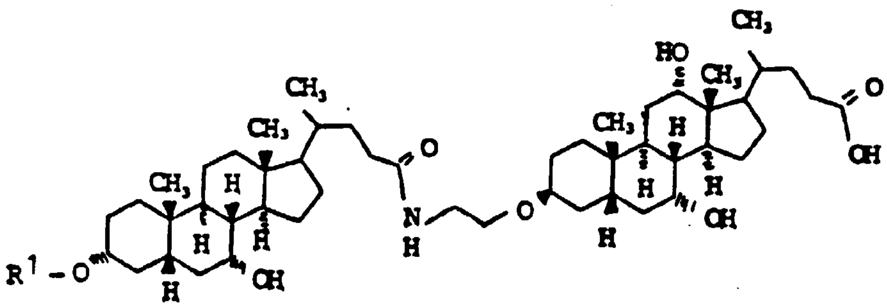

| Beispiel | $R^1$ | MS(FAB,3-NBA/LiCl oder LiJ) |
|---|---|---|
| 447 | H | $C_{50}H_{83}NO_9$(825); 832$(M+Li^+)$ |
| 448 | 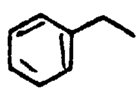 | $C_{57}H_{89}NO_8$(915); 922$(M+Li^+)$ |
| 449 | 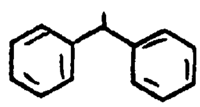 | $C_{63}H_{93}NO_8$(991); 998$(M+Li^+)$ |
| 450 | 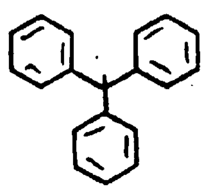 | $C_{69}H_{97}NO_8$(1067); 1074$(M+Li^+)$ |
| 451 | 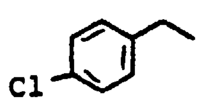 | $C_{57}H_{88}ClNO_8$(949); 956$(M+Li^+)$ |

**Fortsetzung Tabelle 46**

| Beispiel | $R^1$ | MS(FAB,3-NBA/LiCl oder LiJ) |
|---|---|---|
| 452 | 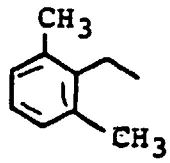 | $C_{59}H_{94}NO_8$(943); 950(M+Li$^+$) |
| 453 | 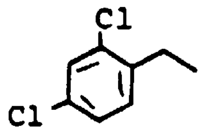 | $C_{57}H_{87}Cl_2NO_8$(983); 990(M+Li$^+$) |
| 454 | 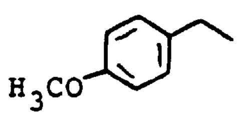 | $C_{58}H_{91}NO_9$(945); 952(M+Li$^+$) |
| 455 | 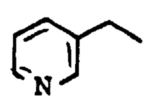 | $C_{56}H_{88}N_2O_8$(916); 923(M+Li$^+$) |
| 456 | 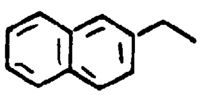 | $C_{61}H_{91}NO_8$(965); 972(M+Li$^+$) |

In Analogie zu den Beispielen 366, 367 und 368 wurden die Verbindungen der Tabelle 47 erhalten.

Tabelle 47

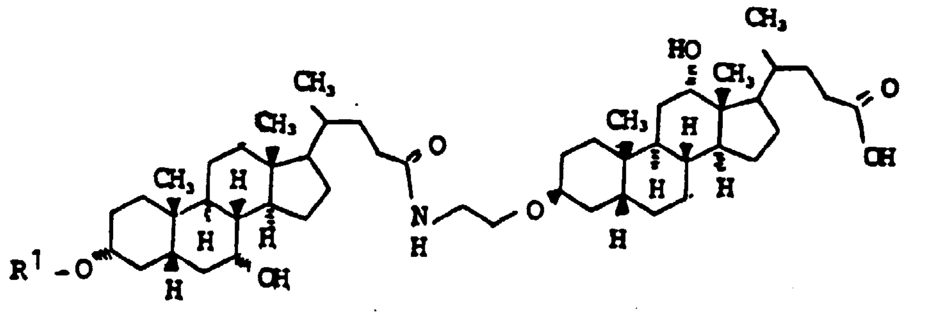

| Beispiel | $R^1$ | MS(FAB,3-NBA/LiCl oder LiJ) |
|---|---|---|
| 457 | H | $C_{50}H_{83}NO_7$(809); 816(M+Li$^+$) |
| 458 | 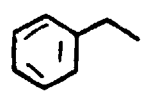 | $C_{57}H_{89}NO_7$(899); 906(M+Li$^+$) |
| 459 | 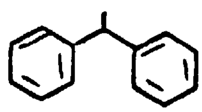 | $C_{63}H_{93}NO_7$(975); 982(M+Li$^+$) |
| 460 | 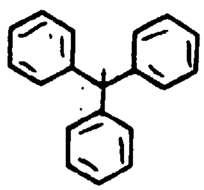 | $C_{69}H_{97}NO_7$(1051); 1058(M+Li$^+$) |
| 461 | 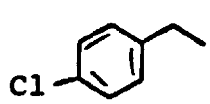 | $C_{57}H_{88}ClNO_7$(933); 940(M+Li$^+$) |

Fortsetzung Tabelle 47

| Beispiel | $R^1$ | MS(FAB,3-NBA/LiCl oder LiJ) |
|---|---|---|
| 462 | 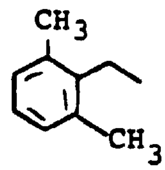 | $C_{59}H_{94}NO_7$(927); 934(M+Li$^+$) |
| 463 | 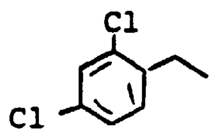 | $C_{57}H_{87}Cl_2NO_7$(967); 974(M+Li$^+$) |
| 464 | 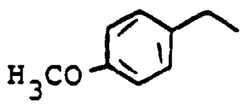 | $C_{58}H_{91}NO_8$(929); 936(M+Li$^+$) |
| 465 | 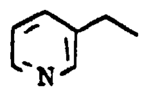 | $C_{56}H_{88}N_2O_7$(900); 907(M+Li$^+$) |
| 466 | 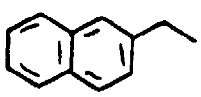 | $C_{61}H_{91}NO_7$(949); 956(M+Li$^+$) |

In Analogie zu den Beispielen 366, 367 und 368 wurden die Verbindungen der Tabelle 48 erhalten.

Tabelle 48

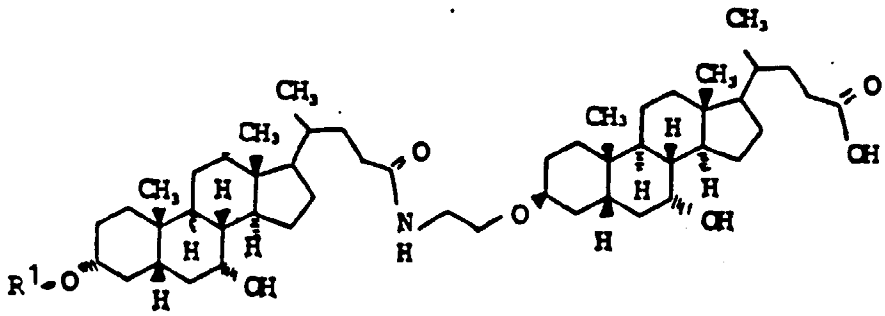

| Beispiel | $R^1$ | MS(FAB,3-NBA/LiCl oder LiJ) |
|---|---|---|
| 467 | H | $C_{50}H_{83}NO_7$(809); 816(M+Li$^+$) |
| 468 | 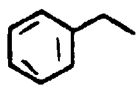 | $C_{57}H_{89}NO_7$(899); 906(M+Li$^+$) |
| 469 | 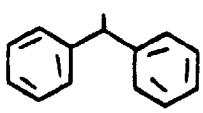 | $C_{63}H_{93}NO_7$(975); 982(M+Li$^+$) |
| 470 | 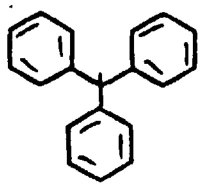 | $C_{69}H_{97}NO_7$(1051); 1058(M+Li$^+$) |
| 471 | 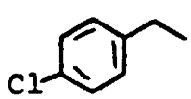 | $C_{57}H_{88}ClNO_7$(933); 940(M+Li$^+$) |

**Fortsetzung Tabelle 48**

| Beispiel | $R^1$ | MS(FAB,3-NBA/LiCl oder LiJ) |
|---|---|---|
| 472 | 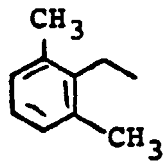 | $C_{59}H_{94}NO_7$(927); 934(M+Li$^+$) |
| 473 | 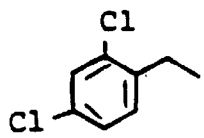 | $C_{57}H_{87}Cl_2NO_7$(967); 974(M+Li$^+$) |
| 474 | 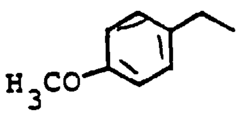 | $C_{58}H_{91}NO_8$(929); 936(M+Li$^+$) |
| 475 | 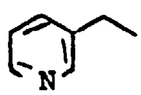 | $C_{56}H_{88}N_2O_7$(900); 907(M+Li$^+$) |
| 476 | 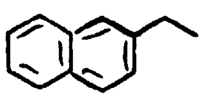 | $C_{61}H_{91}NO_7$(949); 956(M+Li$^+$) |

In Analogie zu den Beispielen 366, 367 und 368 wurden die Verbindungen der Tabelle 49 erhalten.

Tabelle 49

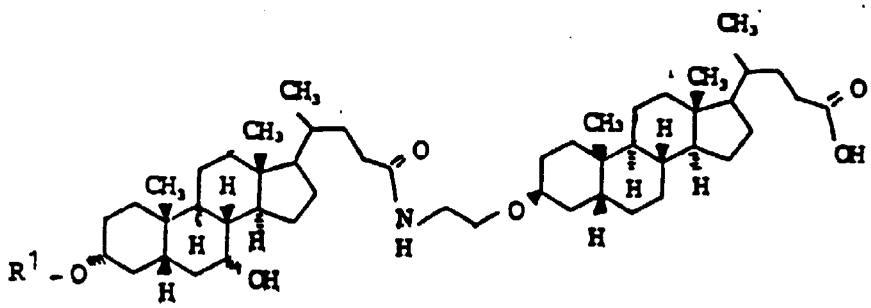

| Beispiel | $R^1$ | MS(FAB,3-NBA/LiCl oder LiJ) |
|---|---|---|
| 477 | H | $C_{50}H_{83}NO_6$(793); 800(M+Li$^+$) |
| 478 | 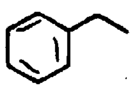 | $C_{57}H_{89}NO_6$(883); 890(M+Li$^+$) |
| 479 | 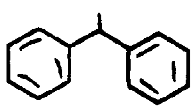 | $C_{63}H_{93}NO_6$(959); 966(M+Li$^+$) |
| 480 | 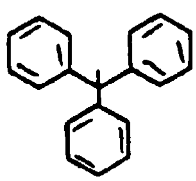 | $C_{69}H_{97}NO_6$(1035); 1042(M+Li$^+$) |
| 481 | 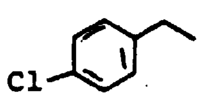 | $C_{57}H_{88}ClNO_6$(917); 924(M+Li$^+$) |

**Fortsetzung Tabelle 49**

| Beispiel | $R^1$ | MS(FAB,3-NBA/LiCl oder LiJ) |
|---|---|---|
| 482 | 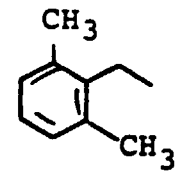 | $C_{59}H_{94}NO_6$(911); 918(M+Li$^+$) |
| 483 | 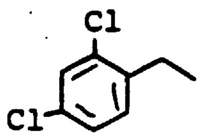 | $C_{57}H_{87}Cl_2NO_6$(951); 958(M+Li$^+$) |
| 484 | 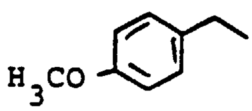 | $C_{58}H_{91}NO_7$(913); 920(M+Li$^+$) |
| 485 | 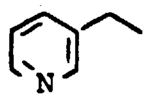 | $C_{56}H_{88}N_2O_6$(884); 891(M+Li$^+$) |
| 486 | 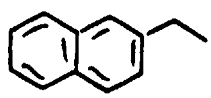 | $C_{61}H_{91}NO_6$(933); 940(M+Li$^+$) |

In Analogie zu den Beispielen 366,367 und 368 wurden die Verbindungen der Tabelle 50 erhalten.

Tabelle 50

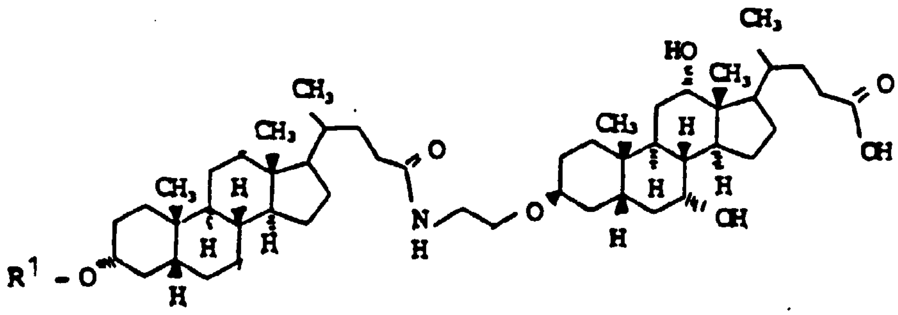

| Beispiel | $R^1$ | MS(FAB,3-NBA/LiCl oder LiJ) |
|---|---|---|
| 487 | H | $C_{50}H_{83}NO_7$(809); 816(M+Li$^+$) |
| 488 | 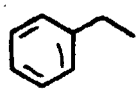 | $C_{57}H_{89}NO_7$(899); 906(M+Li$^+$) |
| 489 | 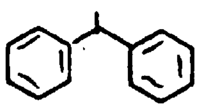 | $C_{63}H_{93}NO_7$(975); 982(M+Li$^+$) |
| 490 | 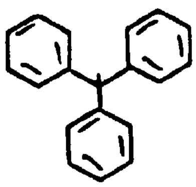 | $C_{69}H_{97}NO_7$(1051); 1058(M+Li$^+$) |
| 491 | 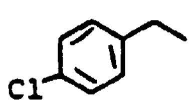 | $C_{57}H_{88}ClNO_7$(933); 940(M+Li$^+$) |

Fortsetzung Tabelle 50

| Beispiel | $R^1$ | MS(FAB,3-NBA/LiCl oder LiJ) |
|---|---|---|
| 492 | 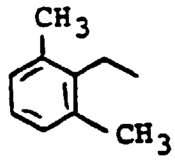 | $C_{59}H_{94}NO_7$(927); 934(M+Li$^+$) |
| 493 | 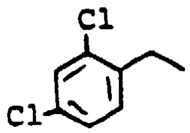 | $C_{57}H_{87}Cl_2NO_7$(967); 974(M+Li$^+$) |
| 494 | 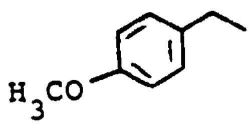 | $C_{58}H_{91}NO_8$(929); 936(M+Li$^+$) |
| 495 | 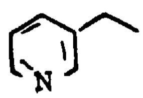 | $C_{56}H_{88}N_2O_7$(900); 907(M+Li$^+$) |
| 496 | 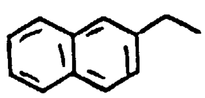 | $C_{61}H_{91}NO_7$(949); 956(M+Li$^+$) |

In Analogie zu den Beispielen 366, 367 und 358 wurden die Verbindungen der Tabelle 51 erhalten.

Tabelle 51

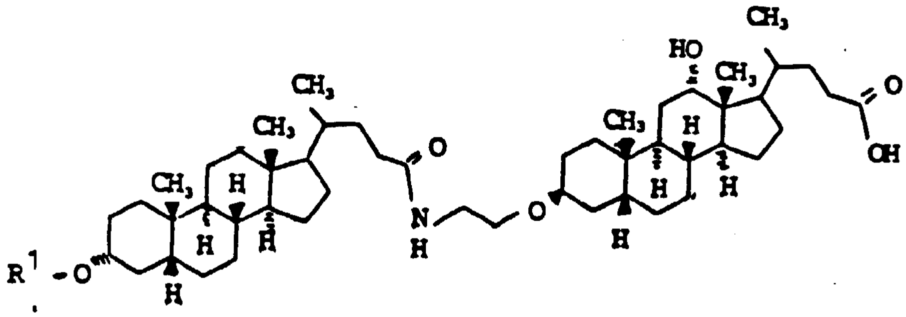

| Beispiel | $R^1$ | MS(FAB,3-NBA/LiCl oder LiJ) |
|---|---|---|
| 497 | H | $C_{50}H_{83}NO_6$(793); 800(M+Li$^+$) |
| 498 | 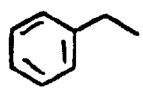 | $C_{57}H_{89}NO_6$(883); 890(M+Li$^+$) |
| 499 | 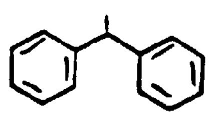 | $C_{63}H_{93}NO_6$(959); 966(M+Li$^+$) |
| 500 | 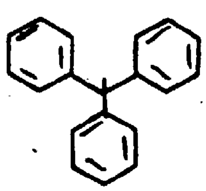 | $C_{69}H_{97}NO_6$(1035); 1042(M+Li$^+$) |
| 501 | 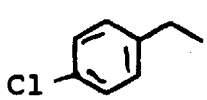 | $C_{57}H_{88}ClNO_6$(917); 924(M+Li$^+$) |

**Fortsetzung Tabelle 51**

| Beispiel | $R^1$ | MS(FAB,3-NBA/LiCl oder LiJ) |
|---|---|---|
| 502 | 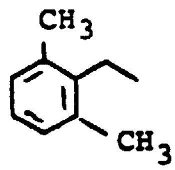 | $C_{59}H_{94}NO_6$(911); 918(M+Li$^+$) |
| 503 | 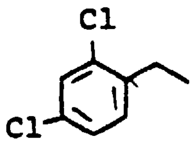 | $C_{57}H_{87}Cl_2NO_6$(951); 958(M+Li$^+$) |
| 504 | 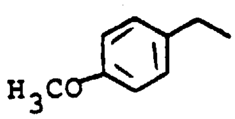 | $C_{58}H_{91}NO_7$(913); 920(M+Li$^+$) |
| 505 | 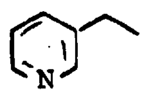 | $C_{56}H_{88}N_2O_6$(884); 891(M+Li$^+$) |
| 506 | 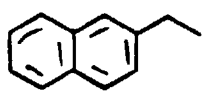 | $C_{61}H_{91}NO_6$(933); 940(M+Li$^+$) |

In Analogie zu den Beispielen 366, 367 und 368 wurden die Verbindungen der Tabelle 52 erhalten.

Tabelle 52

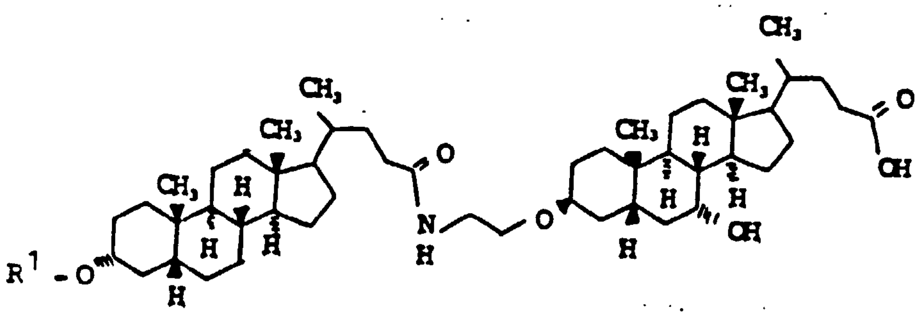

| Beispiel | $R^1$ | MS(FAB,3-NBA/LiCl oder LiJ) |
|---|---|---|
| 507 | H | $C_{50}H_{83}NO_6$(793); 800(M+Li$^+$) |
| 508 | 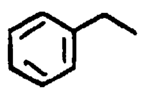 | $C_{57}H_{89}NO_6$(883); 890(M+Li$^+$) |
| 509 | 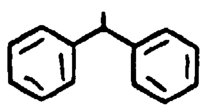 | $C_{63}H_{93}NO_6$(959); 966(M+Li$^+$) |
| 510 | 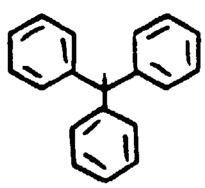 | $C_{69}H_{97}NO_6$(1035); 1042(M+Li$^+$) |
| 511 | 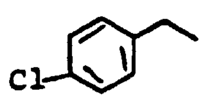 | $C_{57}H_{88}ClNO_6$(917); 924(M+Li$^+$) |

Fortsetzung Tabelle 52

| Beispiel | $R^1$ | MS(FAB,3-NBA/LiCl oder LiJ) |
|---|---|---|
| 512 | 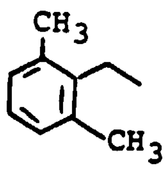 | $C_{59}H_{94}NO_6$(911); 918(M+Li$^+$) |
| 513 | 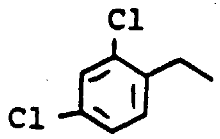 | $C_{57}H_{87}Cl_2NO_6$(951); 958(M+Li$^+$) |
| 514 | 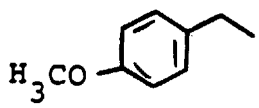 | $C_{58}H_{91}NO_7$(913); 920(M+Li$^+$) |
| 515 | 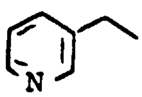 | $C_{56}H_{88}N_2O_6$(884); 891(M+Li$^+$) |
| 516 | 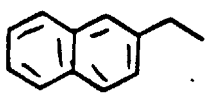 | $C_{61}H_{91}NO_6$(933); 940(M+Li$^+$) |

In Analogie zu den Beispielen 366, 367 und 368 wurden die Verbindungen der Tabelle 53 erhalten.

Tabelle 53

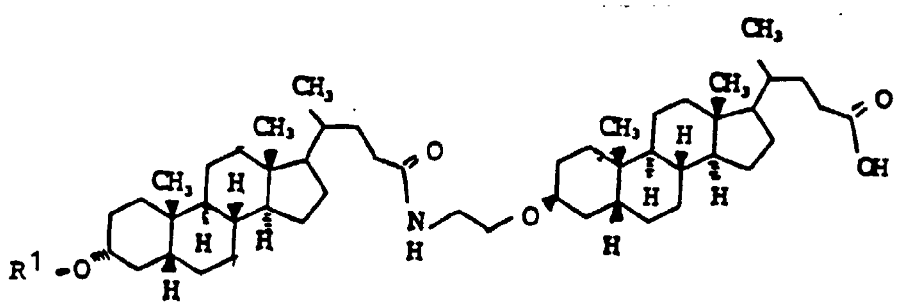

| Beispiel | $R^1$ | MS(FAB,3-NBA/LiCl oder LiJ) |
|---|---|---|
| 517 | H | $C_{50}H_{83}NO_5$(777); 784(M+Li$^+$) |
| 518 | 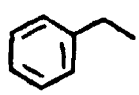 | $C_{57}H_{89}NO_5$(867); 874(M+Li$^+$) |
| 519 | 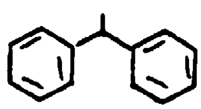 | $C_{63}H_{93}NO_5$(943); 950(M+Li$^+$) |
| 520 | 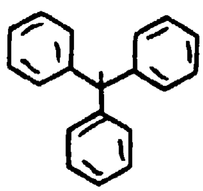 | $C_{69}H_{97}NO_5$(1019); 1026(M+Li$^+$) |
| 521 | 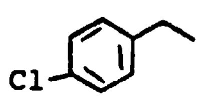 | $C_{57}H_{88}ClNO_5$(901); 908(M+Li$^+$) |

Fortsetzung Tabelle 53

| Beispiel | $R^1$ | MS(FAB,3-NBA/LiCl oder LiJ) |
|---|---|---|
| 522 | 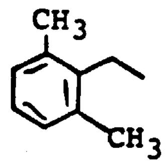 | $C_{59}H_{94}NO_5$(895); 902($M+Li^+$) |
| 523 | 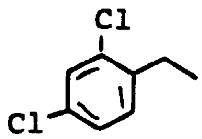 | $C_{57}H_{87}Cl_2NO_5$(935); 942($M+Li^+$) |
| 524 | 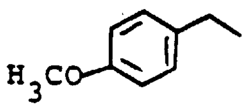 | $C_{58}H_{91}NO_6$(897); 904($M+Li^+$) |
| 525 | 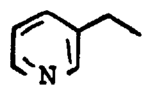 | $C_{56}H_{88}N_2O_5$(868); 875($M+Li^+$) |
| 526 | 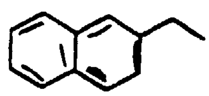 | $C_{61}H_{91}NO_5$(917); 924($M+Li^+$) |

Beispiel 527

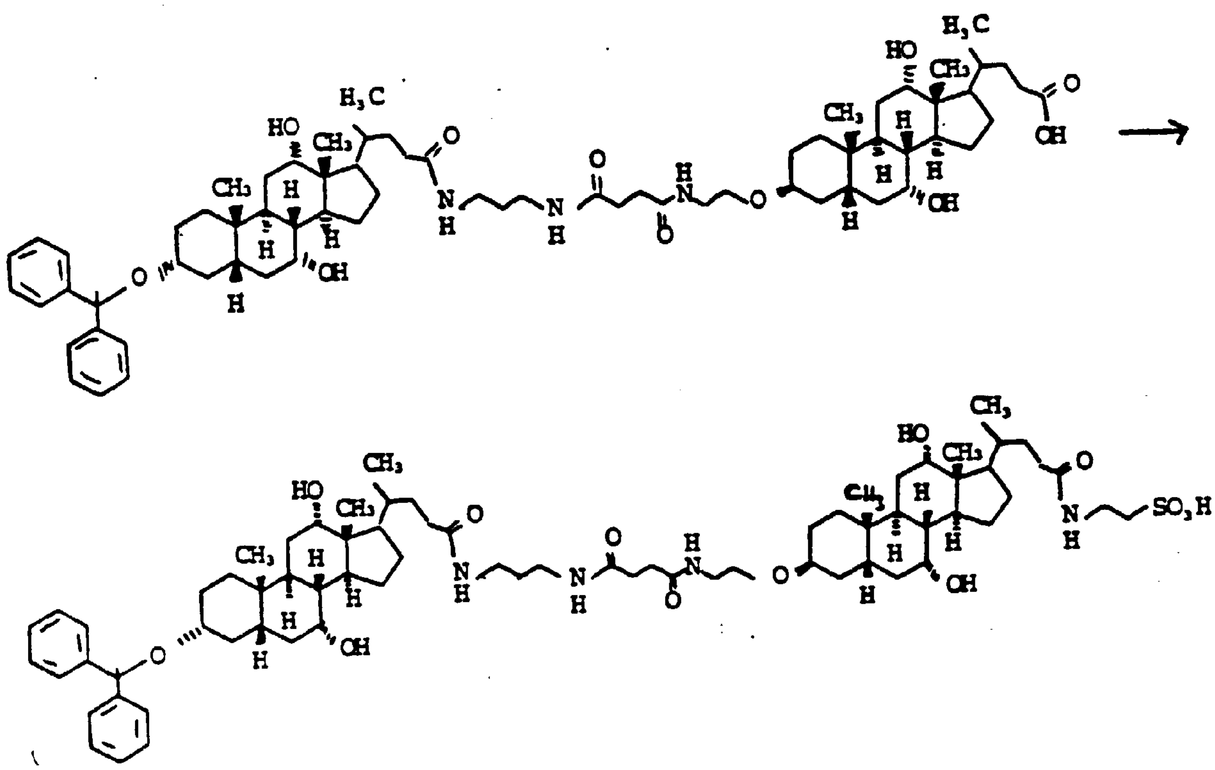

116 mg (0,1 mmol) "Beispiel 170" wurden in 5 ml Tetrahydrofuran vorgelegt, 28,2 µl (0,2 mmol) Triethylamin zugespritzt, auf 0°C abgekühlt, 14,5 µl (0,15 mmol) Chlorameisensäureäthylester zugespritzt, 15 Minuten bei Raumtemperatur gerührt, anschließend 44 mg (0,35 mmol) Taurin gelöst in 3 ml 0,1molare Natronlauge zugetropft, 1 h bei Raumtemperatur gerührt, mit 10 g Natriumdihydrogenphosphat 10 Minuten verquirlt und mit Ethylacetat/Äthanol 4/1 extrahiert (3x). Die vereinten organischen Phasen wurden getrocknet (MgSO4) und eingedampft.

Chromatographie auf Kieselgel (Ethylacetat/Äthanol = 3/2) ergab 76 mg (0,06 mmol, 60 %) "Beispiel 527"

$C_{72}H_{110}N_4SO_{13}$(1270), MS(FAB,3-NBA/LiI); 1277(M+Li+)

In Analogie zu Beispiel 527 wurden die Beispiele der Tabellen 18 - 33 in Taurinkonjugate überführt.

Beispiel 528

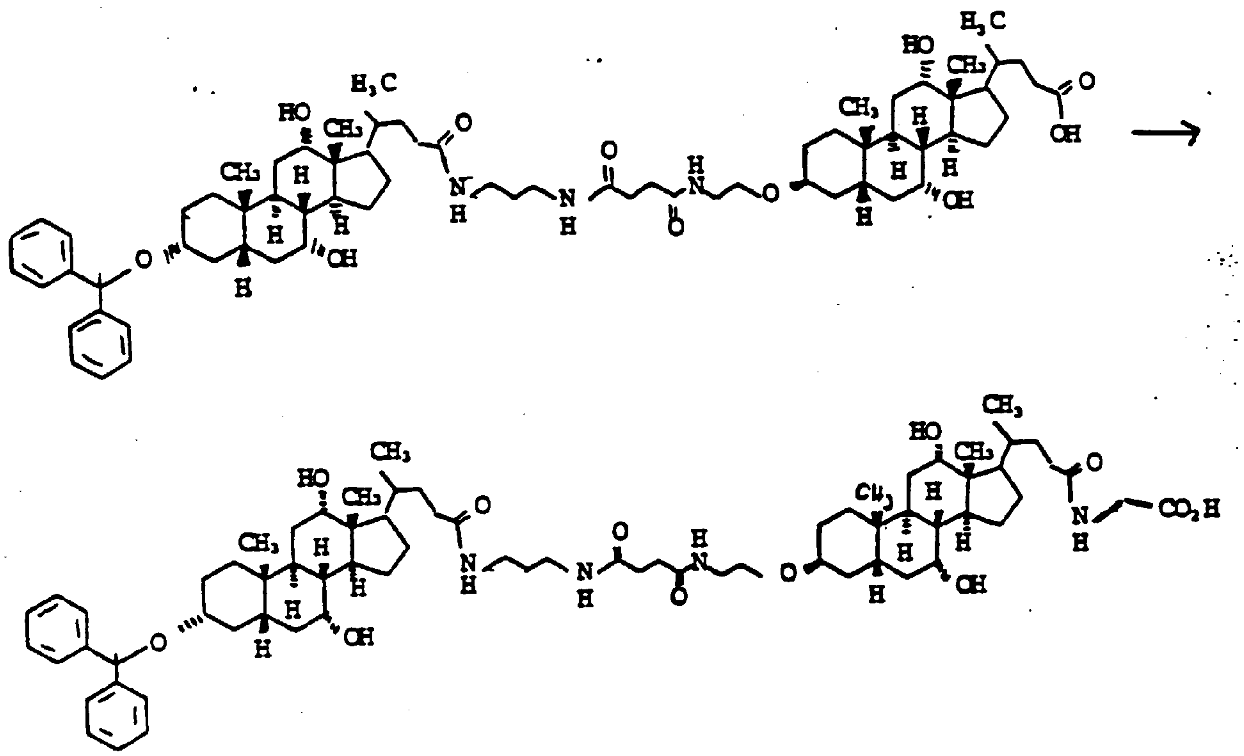

116 mg (0,1 mmol) "Beispiel 170" wurden in 5 ml Tetrahydrofuran vorgelegt, 28,2 µl (0,2 mmol) Triethylamin zugespritzt, auf 0°C abgekühlt, 14,5 µl (0,15 mmol) Chlorameisensäureäthylester zugespritzt, 15 Minuten bei Raumtemperatur gerührt und anschließend 26,5 mg (0,35 mmol) Glycin, gelöst in 3 ml 0,1molare Natronlauge, zugetropft. Gerührt wurde dann 1 h bei Raumtemperatur, mit 10 g Natriumdihydrogenphosphat 10 Minuten verquirlt und mit Ethylacetat/Äthanol 4/1 extrahiert (3x). Die vereinten organischen Phasen wurden getrocknet ($MgSO_4$) und eingedampft.Chromatographie auf Kieselgel (Ethylacetat/Äthanol = 3/2) ergab 74 mg (0,0606 mmol, 60,6 %) "Beispiel 528"
$C_{72}H_{108}N_4O_{12}$(1220), MS(FAB,3-NBA/LiI); 1227($M+Li^+$)

In Analogie zu Beispiel 528 wurden die Beispiele der Tabellen 18 - 33 in Glycinkonjugate überführt.

Beispiel 529

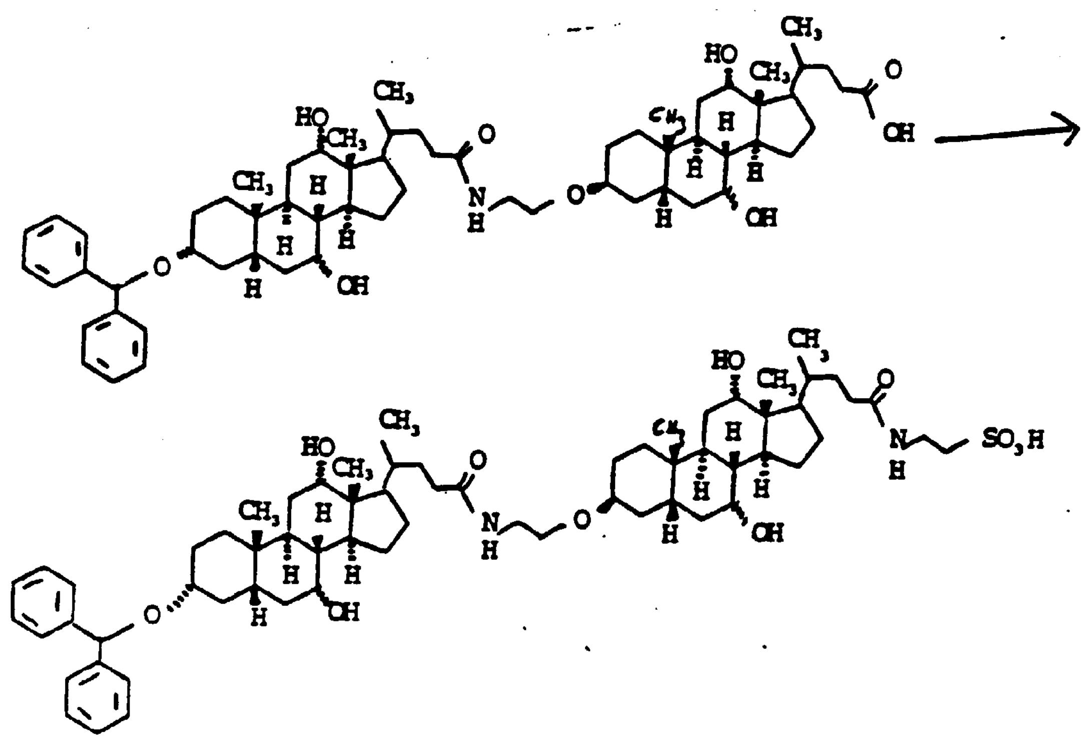

202 mg (0,2 mmol) "Beispiel 367" wurden in 10 ml Tetrahydrofuran vorgelegt, 56,5 µl (0,4 mmol) Triethylamin zugespritzt, auf 0°C abgekühlt, 29 µl (0,3 mmol) Chlorameisensäureäthylester zugespritzt, 15 Minuten bei Raumtemperatur gerührt und anschließend 88 mg (0,7 mmol) Taurin gelöst in 6 ml 0,1 molare Natronlauge, zugetropft. Gerührt wurde 1 h bei Raumtemperatur, mit 20 g Natriumdihydrogenphosphat 10 Minuten verquirlt und mit Ethylacetat/Äthanol 4/1 extrahiert (3x). Die vereinten organischen Phasen wurden getrocknet ($MgSO_4$) und eingedampft. Chromatographie auf Kieselgel (Ethylacetat/Äthanol = 3/2) ergab 194 mg (0,17 mmol, 87 %) "Beispiel 529"
$C_{65}H_{98}N_2O_{11}S$(1114), MS(FAB,3-NBA/LiI); 1121 ($M+Li^+$)

In Analogie zu Beispiel 529 wurden die Beispiele der Tabellen 18 - 33 in Taurinkonjugate überführt.

Beispiel 530

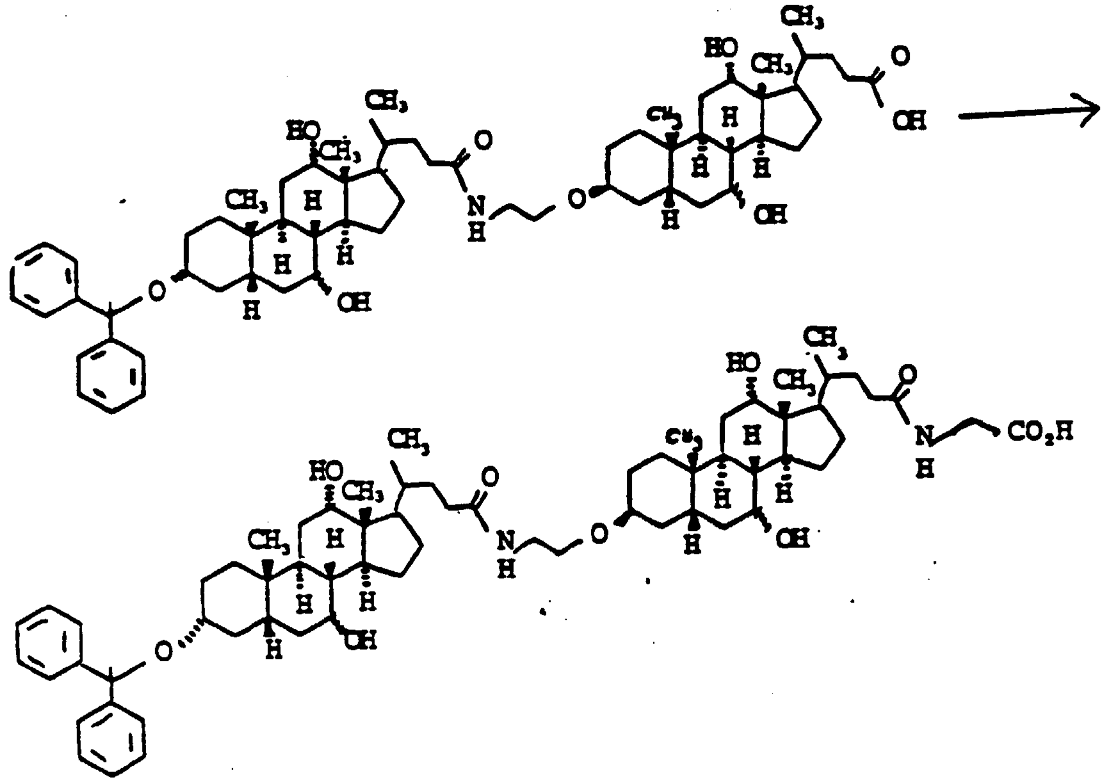

202 mg (0,2 mmol) "Beispiel 367" wurden in 10 ml Tetrahydrofuran vorgelegt, 56,5 µl (0,4 mmol) Triethylamin zugespritzt, auf 0°C abgekühlt, 29 µl (0,3 mmol) Chlorameisensäureäthylester zugespritzt, 15 Minuten bei Raumtemperatur gerührt und anschließend 53 mg (0,7 mmol) Glycin, gelöst in 6 ml 0,1 molare Natronlauge, zugetropft. Gerührt wurde 1 h bei Raumtemperatur, mit 20 g Natriumdihydrogenphosphat 10 Minuten verquirlt und mit Ethylacetat/Äthanol 4/1 extrahiert (3x). Die vereinten organischen Phasen wurden getrocknet ($MgSO_4$) und eingedampft. Chromatographie auf Kieselgel (Ethylacetat/Äthanol = 3/2) ergab 181 mg (0,17 mmol, 85 % "Beispiel 530"
$C_{65}H_{96}N_2O_{10}$(1064), MS(FAB,3-NBA/LiCl): 1071 ($M+Li^+$)

In Analogie zu Beispiel 530 wurden die Beispiele der Tabellen 38 - 53 in Glycinkonjugate überführt.

Beispiel 531

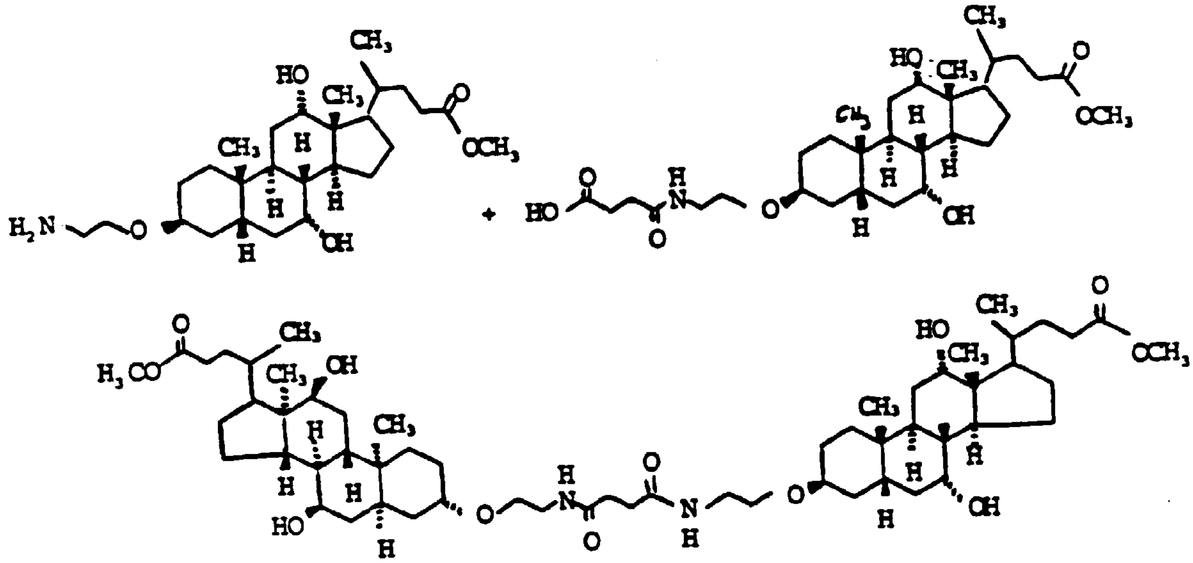

565 mg (1 mmol) "Beispiel 165", gelöst in 20 ml Tetrahydrofuran und 5 ml Triethylamin, wurden vorgelegt und bei 0°C 96 µl (1 mmol) Chlorameisensäureäthylester zugespritzt. Man ließ 15 Minuten bei 0°C rühren. Anschließend gab man 465 mg (1 mmol) "Beispiel 139" gelöst in 10 ml Tetrahydrofuran tropfenweise zu. Man ließ 1,5 h bei Raumtemperatur rühren. Die Reaktionslösung wurde auf 1 molare Salzsäure gegossen, dann mit Essigester extrahiert (3x). Die vereinten organischen Phasen wurden mit gesättigter $NaHCO_3$-Lösung gewaschen und getrocknet ($MgSO_4$). Abdampfen des Lösungsmittels und Flash-Chromatographie auf Kieselgel (Essigester/Methanol = 5/1) ergab 608 mg (0,601 mmol, 60.1 %) "Beispiel 531"

$C_{58}H_{96}N_2O_{12}$(1012), MS(FAB,3-NBA/LiCl): 1019 (M+Li+)

Beispiel 532

Schritt b)

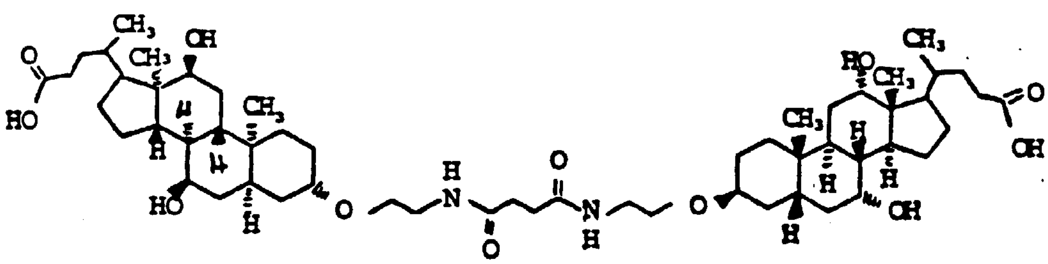

300 mg (0,296 mmol) "Beispiel 531" wurden in 10 ml Äthanol gelöst und 3 ml lmolare Natronlauge zugegeben 24 h bei Raumtemperatur gerührt, anschließend mit 6 g Natriumdihydrogenphosphat 15 Minuten intensiv verquirlt und mit Essigester/Äthanol = 4/1 extrahiert (,3x). Die vereinten organischen Phasen wurden getrocknet ($MgSO_4$). Abdampfen des Lösungsmittels, Verreiben mit Diisopropyläther und Absaugen abgesaugt ergab 268 mg (0,272 mmol, 92 %) "Beispiel 532"

$C_{56}H_{92}N_2O_{12}$(984), MS(FAB,3NB/LiCl); 991 (M+Li+)

In Analogie zu den Beispielen 531 und 532 wurden die Beispiele der Tabelle 54 erhalten.

Tabelle 54

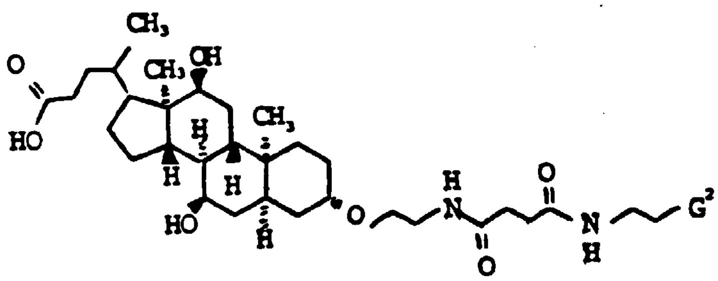

| Bsp. | $G^2$ | MS(FAB,3-NBA/LiCl oder Li) |
|---|---|---|
| 533 | 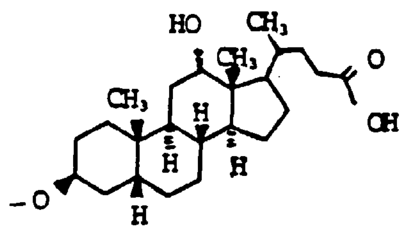 | $C_{56}H_{92}N_2O_{11}$(968); 975(M+Li$^+$) |
| 534 | 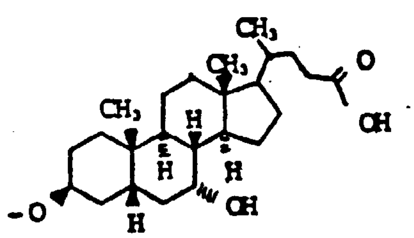 | $C_{56}H_{92}N_2O_{11}$(968); 975(M+Li$^+$) |
| 535 | 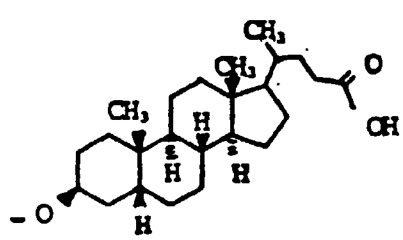 | $C_{56}H_{92}N_2O_{10}$(952); 959(M+Li$^+$) |

In Analogie zu den Beispielen 531 und 532 wurden die Beispiele der Tabelle 55 erhalten.

**Tabelle 55**

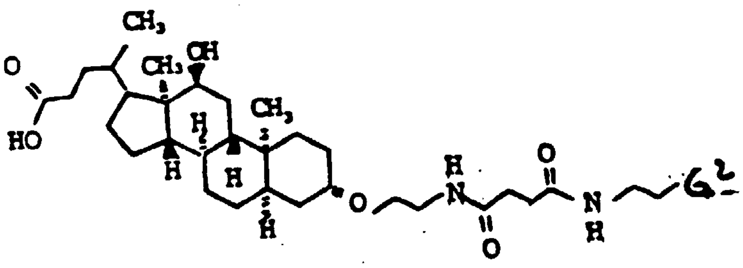

| Bsp. | $G^2$ | MS(FAB,3-NBA/LiCl oder Li) |
|---|---|---|
| 536 | 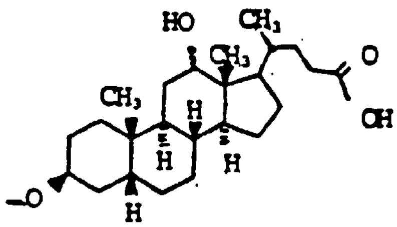 | $C_{56}H_{92}N_2O_{10}$(952); 959(M+Li$^+$) |
| 537 | 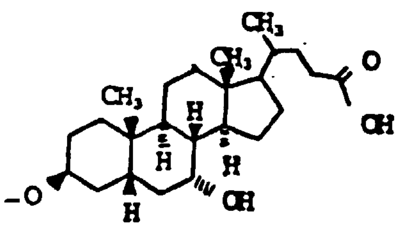 | $C_{56}H_{92}N_2O_{10}$(952); 959(M+Li$^+$) |
| 538 | 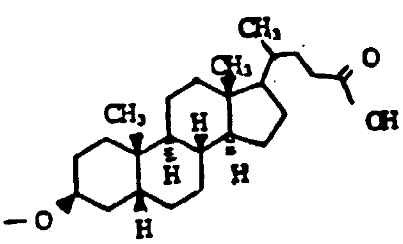 | $C_{56}H_{92}N_2O_9$(936); 943(M+Li$^+$) |

In Analogie zu den Beispielen 531 und 532 wurden die Beispiele der Tabelle 56 erhalten.

Tabelle 56

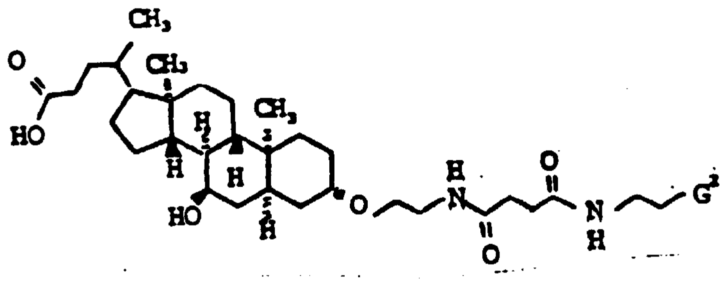

| Bsp. | $G^2$ | MS(FAB,3-NBA/LiCl oder Li) |
|---|---|---|
| 539 | 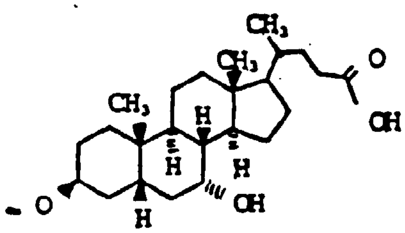 | $C_{56}H_{92}N_2O_{10}$(952); 959(M+Li$^+$) |
| 540 | 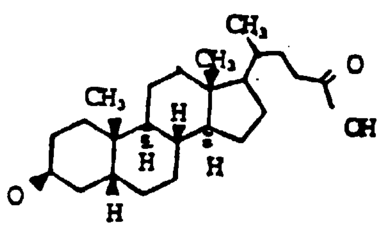 | $C_{56}H_{92}N_2O_9$(936); 943(M+Li$^+$) |

In Analogie zu den Beispielen 531 und 532 wurde das Beispiel der Tabelle 57 erhalten.

Tabelle 57

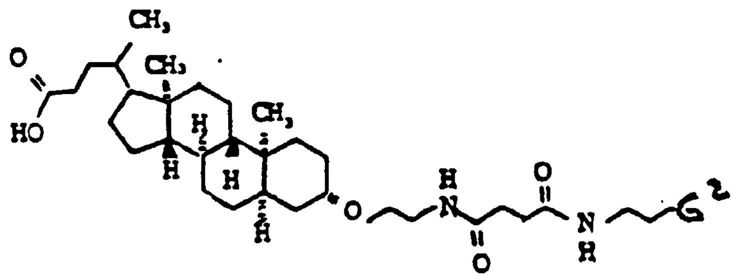

| Bsp. | $G^2$ | MS(FAB,3-NBA/LiCl oder Li) |
|---|---|---|
| 541 | 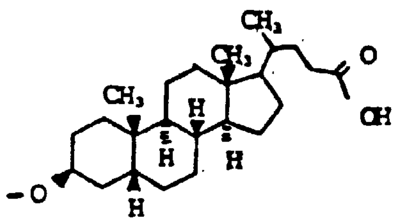 | $C_{56}H_{92}N_2O_8$(920); 927(M+Li$^+$) |

Zur weiteren Variation der Gruppe X wurden folgende Verbindungen hergestellt (L = H)

Tabelle 58

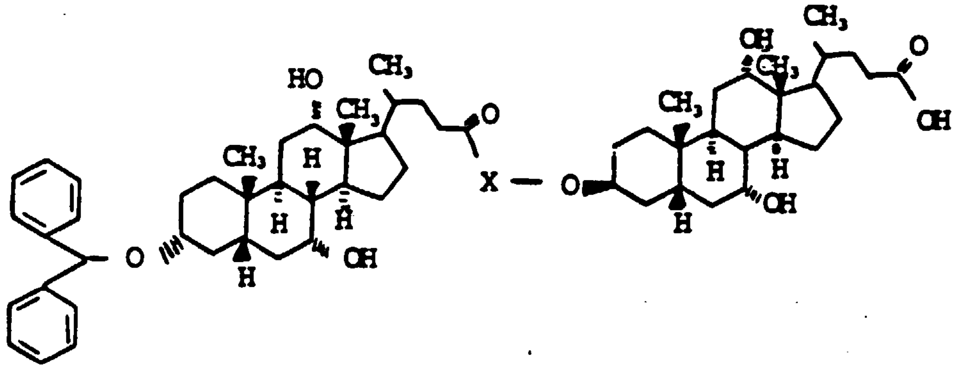

| Beispiel | X |
|---|---|
| 542 | 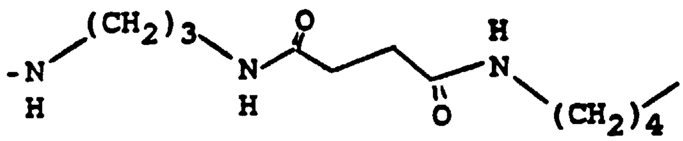 |
| 543 | 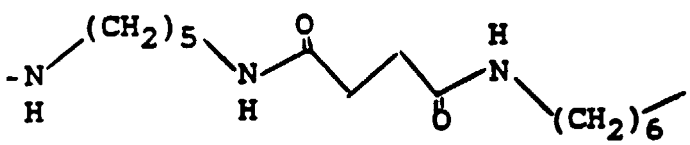 |
| 544 | 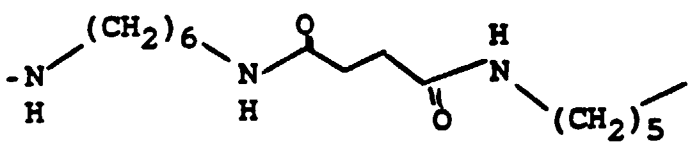 |
| 545 | 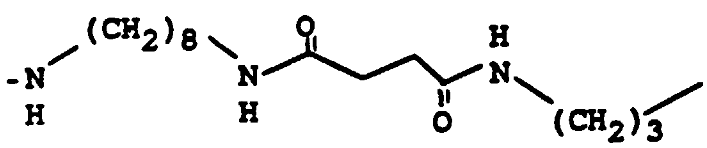 |
| 546 | 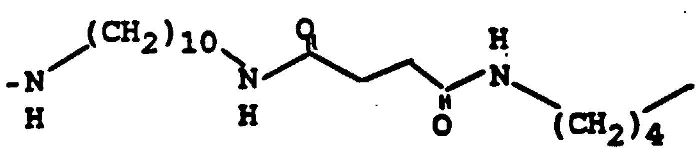 |

Fortsetzung Tabelle 58

| Beispiel | X |
|---|---|
| 547 | 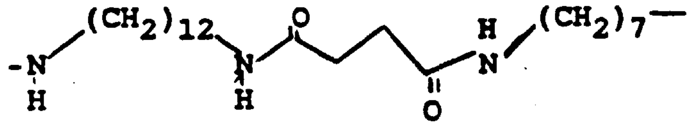 |
| 548 | 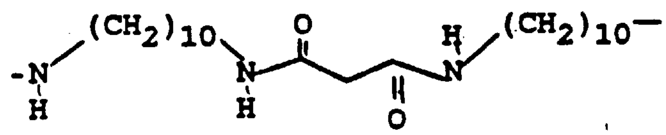 |
| 549 | 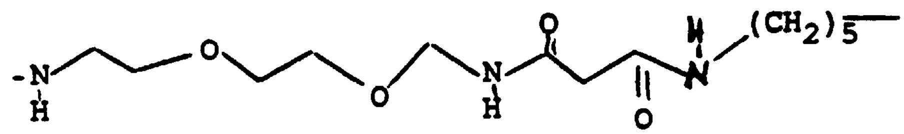 |
| 550 | 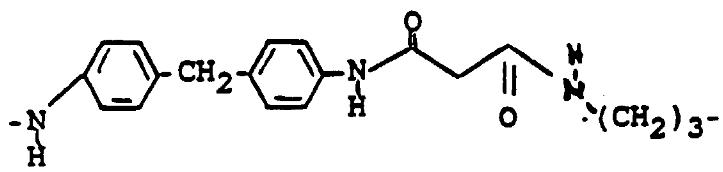 |
| 551 | 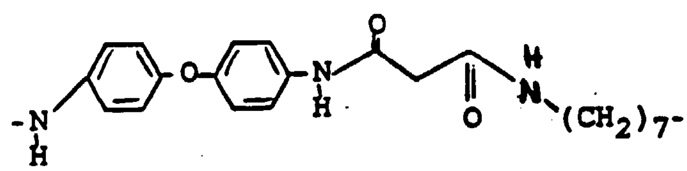 |
| 552 | 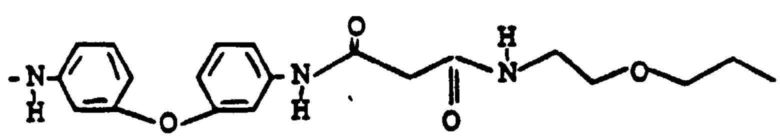 |
| 553 | 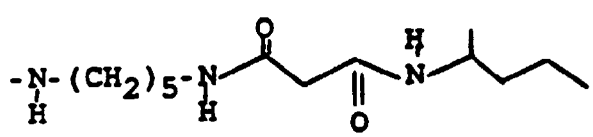 |
| 554 | direkte Bindung |

Fortsetzung Tabelle 58

| Beispiel | X |
| --- | --- |
| 555 | 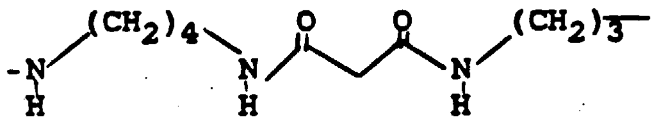 |
| 556 | 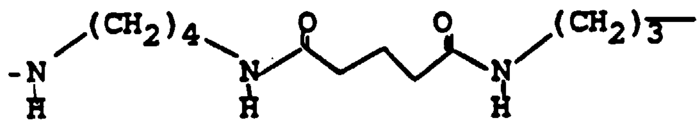 |
| 557 | 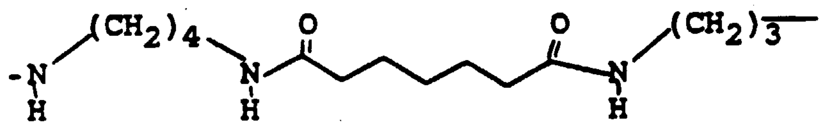 |

Tabelle 59 zeigt Verbindungen der Tabelle 58, in denen zusätzlich $L_1$, $L_2$, $L_3$ variiert worden sind

| Bsp. | X | $L_1$ | $L_2$ | $L_3$ |
|---|---|---|---|---|
| 558 | 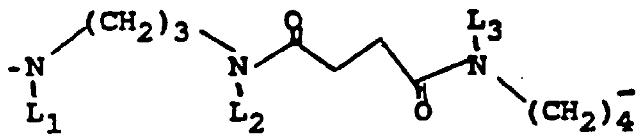 | H- | $CH_3$- | $CH_3$- |
| 559 | " | $CH_3$- | H- | $C_3H_7$- |
| 560 | " | H- | H- | $C_5H_{11}$- |
| 561 | " | $C_3H_7$- | H- | 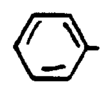 |
| 562 | " | H- | 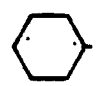 | 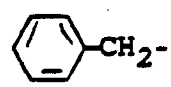 |
| 563 | " | $CH_3$- | $C_2H_5$- | $C_2H_5$- |
| 564 | " | $C_2H_5$- | 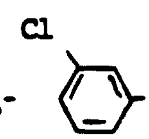 | $CH_3$- |

Tabelle 60 zeigt $IC_{50}$ und $IC_{50}$ Na.-Werte einiger dimeren Gallensäuren:

Tabelle 60

| Verbindungen aus Beispiel | $IC_{50}$ | $IC_{50}$ Na |
|---|---|---|
| 527 | 20 | 10 |
| 191 (Taurinkon.) | 18 | 14 |
| 170 | 25 | 14 |
| 528 | 26 | 16 |
| 191 (Glycinkon.) | 27 | 18 |
| 172 (Taurinkon.) | 32 | 20 |
| 368 (Glycinkon.) | 48 | 16 |
| 368 | 45 | 18 |
| 369 (Taurinkon.) | 50 | 18 |
| 369 | 55 | 20 |
| 191 | 58 | 29 |
| 531 | > 50 | 18 |
| 532 | 30 | 10 |

Beispiel 565

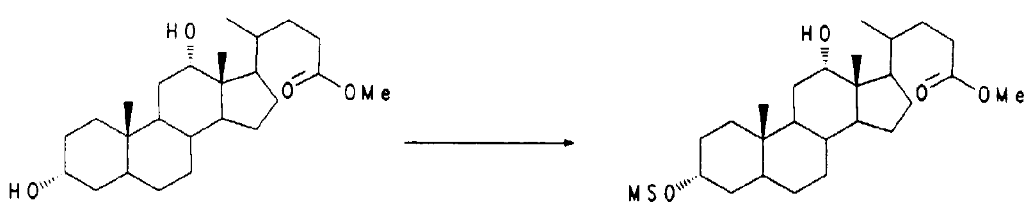

50 g (123 mmol) Desoxycholsäuremethylester wurden in 300 ml Pyridin gelöst und auf 5°C gekühlt. Unter Rühren wurden 15 ml (193 mmol) Methansulfonylchlorid zugetropft und anschließend 12 h bei Zimmertemperatur gerührt. Es wurde auf Wasser gegossen und mit Essigester extrahiert. Nach Trocknen und Einengen der organischen Phase wurde über Kieselgel (Essigester) chromatographiert. Ausbeute 29,5 g (49 %) "Beispiel 565"
$C_{26}H_{44}O_6S$ (484), MS (FAB, 3-NBA/LiCl): 491 ($M+Li^+$).

Beispiel 566

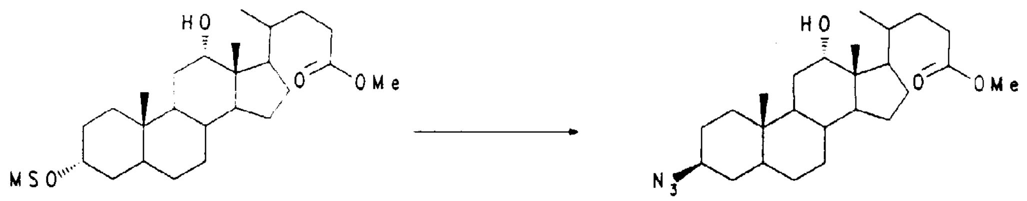

28,0 g (57,8 mmol) Beispiel 565 wurden in 350 ml DMF in Gegenwart von 7,5 g (115 mmol) Natriumazid 1,5 h bei 130°C gerührt. Es wurde auf Wasser gegossen und mit Essigester extrahiert. Nach Trocknen und Einengen der organischen Phase wurde über Kieselgel filtriert (Cyclohexan/Essigester 1:1). Ausbeute 18,5 g (74 %) "Beispiel 566".
$C_{25}H_{41}N_3O_3$ (431), MS (FAB, 3-NBA/LiCl): 438 ($M+Li^+$).

Beispiel 567

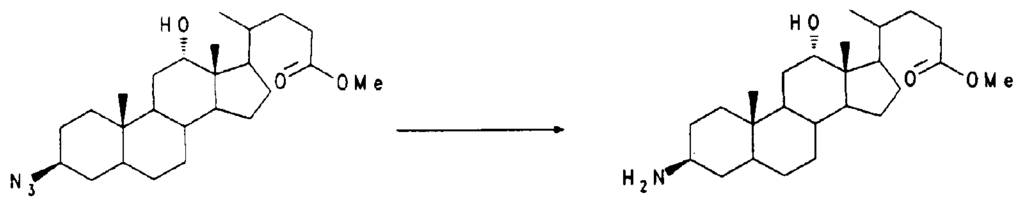

5,0 g (11,6 mmol) Beispiel 566 wurden in 150 ml Essigester in Gegenwart von 0,5 g Pd/C (10 %) bei Zimmertemperatur und Normaldruck hydriert. Es wurde vom Katalysator abfiltriert und eingeengt. Chromatographie über Kieselgel (Methanol, dann Methanol/Triethylamin 98:2) ergaben 3,1 g (66 %) "Beispiel 567".
$C_{25}H_{43}NO_3$ (405), MS (FAB, 3-NBA/LiCl) : 412 ($M+Li^+$).

Analog zu den Beispielen 565 - 567 wurden die Beispiele 568 und 569 hergestellt.

| Bsp. | | MS (FAB, 3-NBA/LiCl oder LiJ |
|---|---|---|
| 568 | 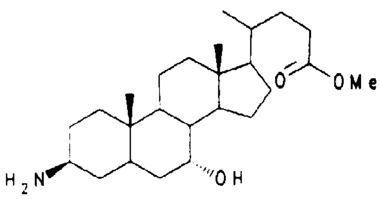 | $C_{25}H_{43}NO_3$; 412 $(M+Li^+)$ 405 |
| 569 | 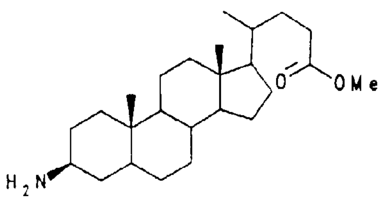 | $C_{25}H_{43}NO_2$; 396 $(M+Li^+)$ 389 |

Beispiel 570

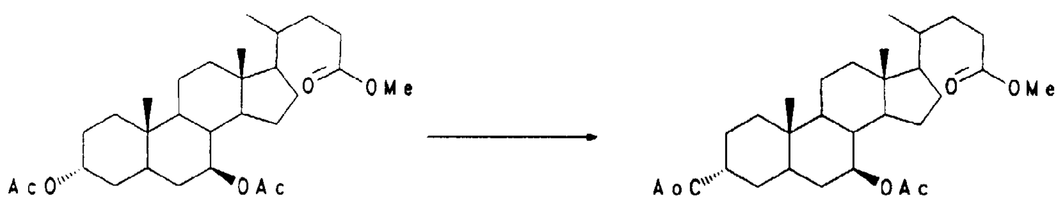

Zu einer aus 5,75 g (0,25 mol) Natrium und 400 ml Methanol bereiteten Lösung wurden 14,4 g (28,4 mmol) 3α, 7β-Diacetoxy-cholansäuremethylester in 100 ml Methanol zugegeben und bei Zimmertemperatur gerührt. Nach 15 min wurde gesättigte Natriumdihydrogenphosphatlösung zugegeben und mehrmals mit Essigester extrahiert. Trocknen und Einengen der organischen Phase ergab 1,18 g (90 %) "Beispiel 570" das ohne weitere Reinigung weiter umgesetzt wurde.

$C_{27}H_{44}NO_5$, MS (FAB, 3-NBA/LiCl) : 455 $(M+Li^+)$.

Beispiel 571

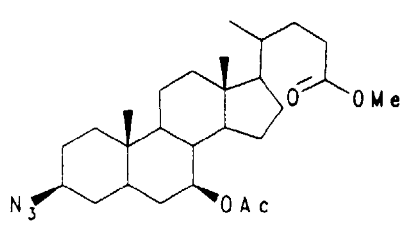

In Analogie zu den Beispielen 565 und 566 wurde aus Beispiel 570 "Beispiel 571" erhalten.

$C_{27}H_{43}N_3O_4$ (473), MS (FAB, 3-NBA/LiCl) : 480 (M+Li+).

Beispiel 572

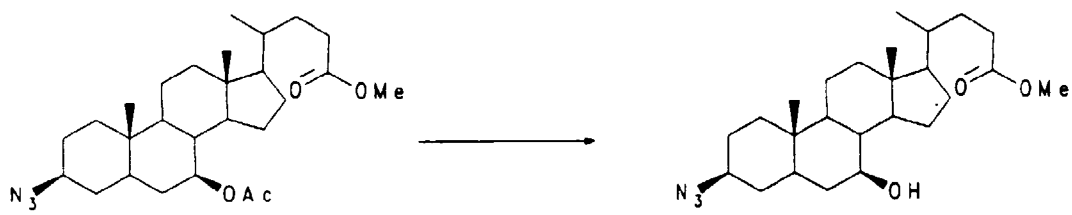

5,7 g (12,0 mmol) Beispiel 571 wurden in 300 ml 2 M Natriummethylatlösung in Methanol 1 h unter Rückfluß erhitzt. Die Aufarbeitung erfolgte nach dem für Beispiel 570 beschriebenen Verfahren. Chromatographie über Kieselgel (Cyclohexan/Essigester 1:1) ergab 4,3 g (83 %) "Beispiel 572".
$C_{25}H_{41}N_3O_3$ (431), MS (FAB, 3-NBA/LiCl) : 438 (M+Li+).

Beispiel 573

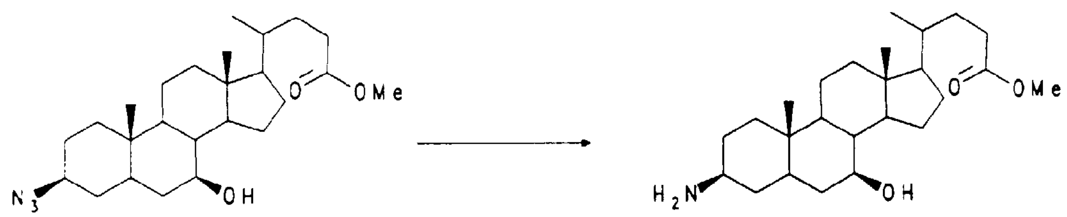

Analog zu Beispiel 567 wurde aus Beispiel 572 "Beispiel 573" hergestellt.
$C_{25}H_{43}NO_3$ (405), MS (FAB, 3-NBA/LiCl), 412 (M+Li+).

Beispiel 574

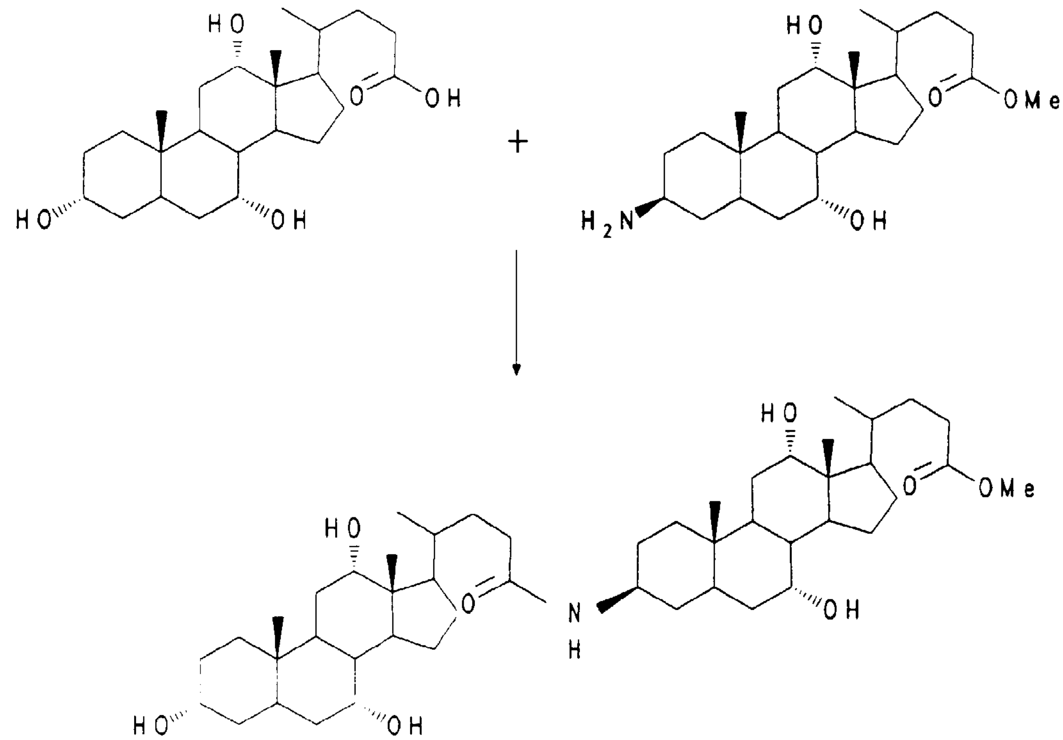

1,0 g (2,45 mmol) Cholsäure, 1,03 g (2,45 mmol) 3β-Amino-7α,12α-dihydroxycholansäuremethylester und 550 mg (4,07 mmol) Hydroxybenzotriazol wurden in 40 ml THF 20 min bei Zimmertemperatur gerührt. Nach Abkühlen auf 0°C wurden 610 mg (2,96 mmol) Dicyclohexylcarbodiimid in 10 ml THF zugetropft und 12 h bei Zimmertemperatur weitergerührt. Vom Feststoff wurde abfiltriert, das Lösungsmittel eingeengt und der Rückstand über Kieselgel ($CHCl_3$/ Methanol 9:1) chromatographiert. Ausbeute 1,2 g (60 %) "Beispiel 574".
$C_{49}H_{81}NO_8$ (811), MS (FAB, 3-NBA/LiCl) : 818 ($M+Li^+$).

Beispiel 575

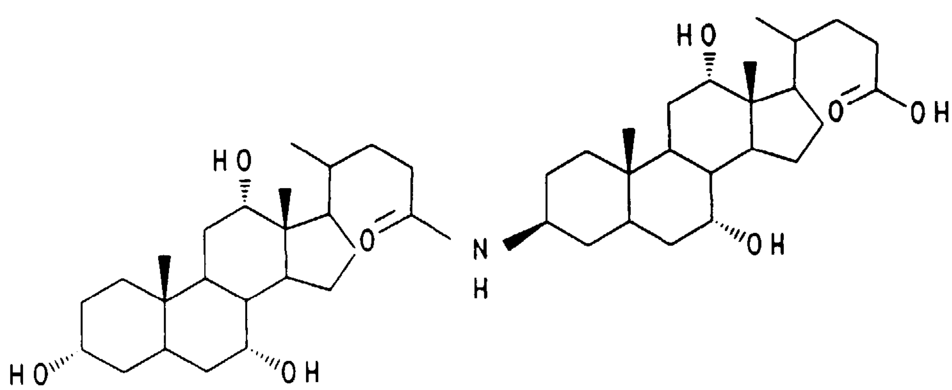

530 mg (0,65 mmol) Beispiel 574 wurden in 10 ml Ethanol und 2 ml 1 M Natriumhydroxidlösung 18 h bei Zimmertemperatur gerührt. Nach Zusatz von Wasser wurde der Alkohol abdestilliert. Die wäßrige Lösung wurde mit 1,3 ml 2 M HCl angesäuert. Der Niederschlag wurde abgesaugt und getrocknet, man erhielt 510 mg (98 %) "Beispiel 575".
$C_{48}H_{79}NO_8$ (797), MS (FAB, 3-NBA/LiCl) : 804 ($M+Li^+$).

In Analogie zu den Beispielen 574 und 575 wurden die Beispiele der Tabelle 61 hergestellt.

Tabelle 61

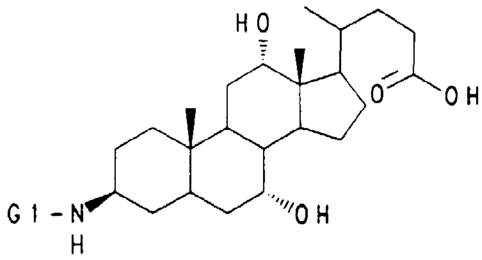

| Bsp. | G1 | MS (FAB, 3-NBA/LiCl oder LiJ) |
|---|---|---|
| 576 | 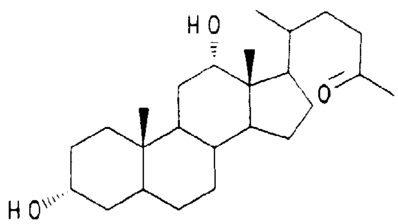 | $C_{48}H_{79}NO_7$ (781) 788 (M+Li$^+$) |
| 577 | 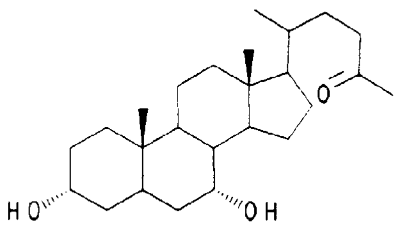 | $C_{48}H_{79}NO_7$ (781) 788 (M+Li$^+$) |
| 578 | 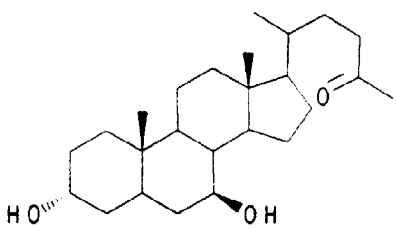 | $C_{48}H_{79}NO_7$ (781) 788 (M+Li$^+$) |
| 579 | 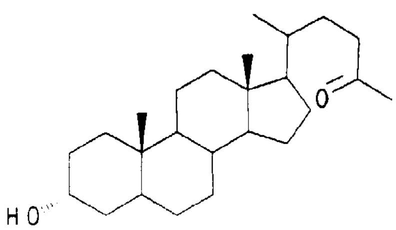 | $C_{48}H_{79}NO_6$ (765) 772 (M+Li$^+$) |

In Analogie zu den Beispielen 574 und 575 wurden die Beispiele der Tabelle 62 hergestellt.

Tabelle 62

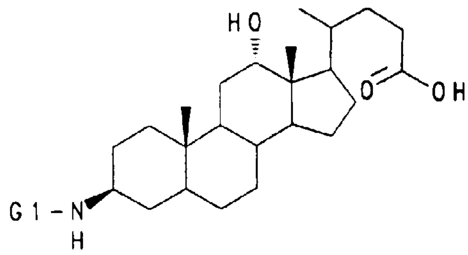

| Bsp. | G1 | MS (FAB, 3-NBA/LiCl oder LiJ) |
|---|---|---|
| 580 | 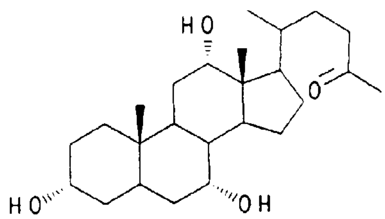 | $C_{48}H_{79}NO_7$ (781) 788 ($M+Li^+$) |
| 581 | 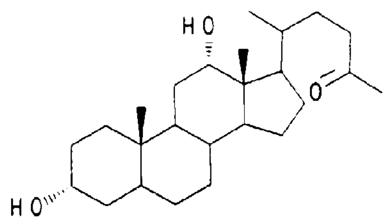 | $C_{48}H_{79}NO_6$ (765) 772 ($M+Li^+$) |
| 582 | 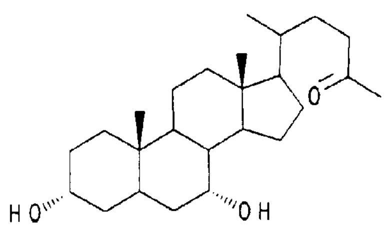 | $C_{48}H_{79}NO_6$ (765) 772 ($M+Li^+$) |

| Bsp. | G1 | MS (FAB, 3-NBA/LiCl oder LiJ) |
|---|---|---|
| 583 | 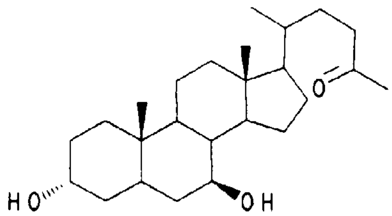 | $C_{48}H_{79}NO_6$ (765) 772 (M+Li$^+$) |
| 584 | 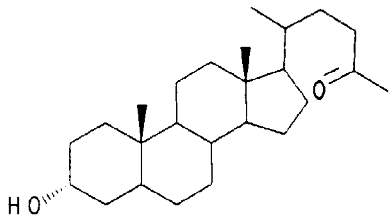 | $C_{48}H_{79}NO_5$ (749) 756 (M+Li$^+$) |

In Analogie zu den Beispielen 574 und 575 wurden die Beispiele der Tabelle 63 hergestellt.

Tabelle 63

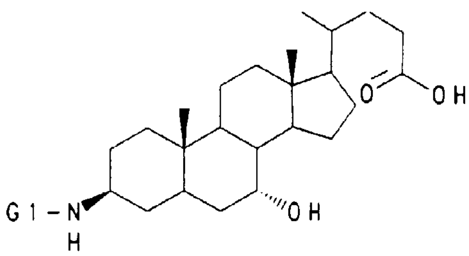

| Bsp. | G1 | MS (FAB, 3-NBA/LiCl oder LiJ) |
|---|---|---|
| 585 | 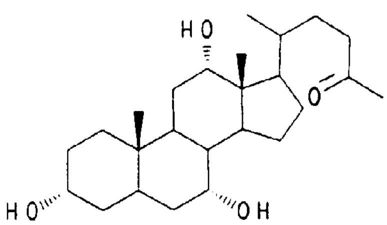 | $C_{48}H_{79}NO_7$ (781) 788 (M+Li$^+$) |

| Bsp. | G1 | MS (FAB, 3-NBA/LiCl oder LiJ) |
|---|---|---|
| 586 | 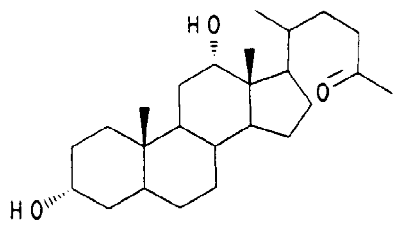 | $C_{48}H_{79}NO_6$ (765) 772 (M+Li$^+$) |
| 587 | 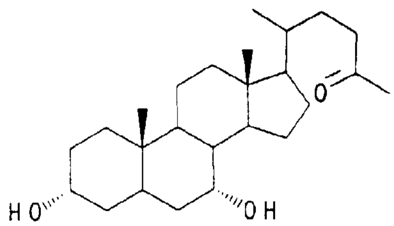 | $C_{48}H_{79}NO_6$ (765) 772 (M+Li$^+$) |
| 588 | 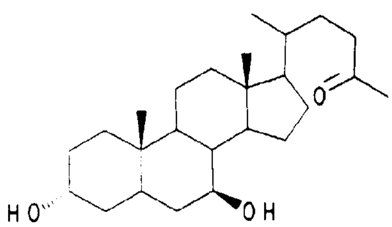 | $C_{48}H_{79}NO_6$ (765) 772 (M+Li$^+$) |
| 589 | 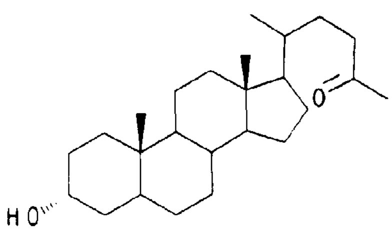 | $C_{48}H_{79}NO_5$ (749) 756 (M+Li$^+$) |

In Analogie zu den Beispielen 574 und 575 wurden die Beispiele der Tabelle 64 hergestellt.

Tabelle 64

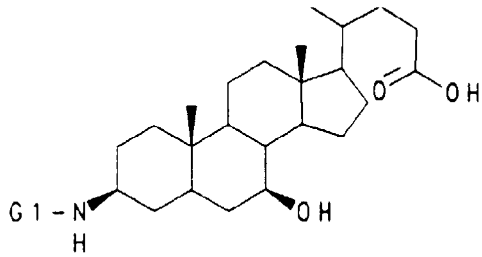

| Bsp. | G1 | MS (FAB, 3-NBA/LiCl oder LiJ) |
| --- | --- | --- |
| 590 | 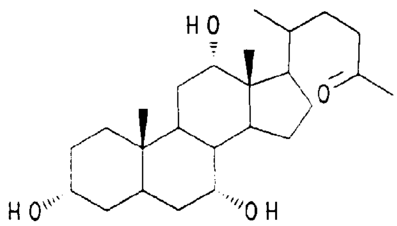 | $C_{48}H_{79}NO_7$ (781) 788 ($M+Li^+$) |
| 591 | 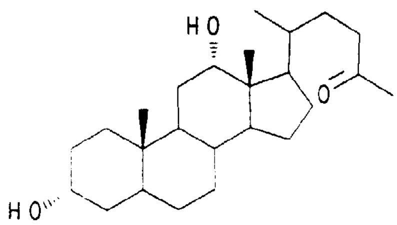 | $C_{48}H_{79}NO_6$ (765) 772 ($M+Li^+$) |
| 592 | 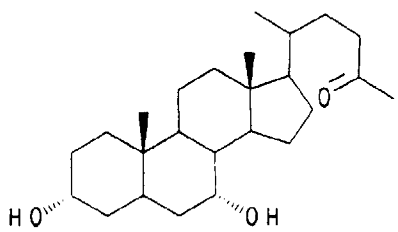 | $C_{48}H_{79}NO_6$ (765) 772 ($M+Li^+$) |
| 593 | 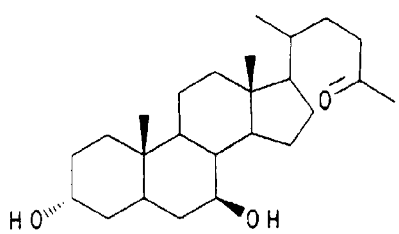 | $C_{48}H_{79}NO_6$ (765) 772 ($M+Li^+$) |

| Bsp. | G1 | MS (FAB, 3-NBA/LiCl oder LiJ) |
|---|---|---|
| 594 | 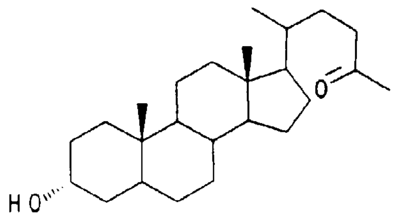 | $C_{48}H_{79}NO_5$ (749) 756 (M+Li$^+$) |

In Analogie zu den Beispielen 574 und 575 wurden die Beispiele der Tabelle 65 hergestellt.

Tabelle 65

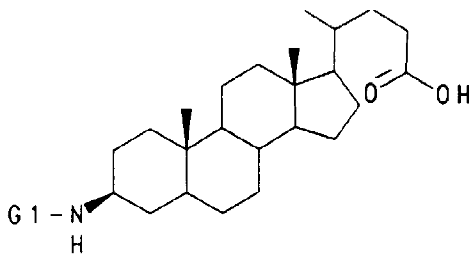

| Bsp. | G1 | MS (FAB, 3-NBA/LiCl oder LiJ) |
|---|---|---|
| 595 | 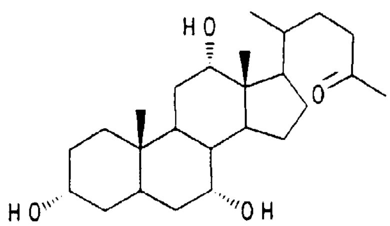 | $C_{48}H_{79}NO_6$ (765) 772 (M+Li$^+$) |
| 596 | 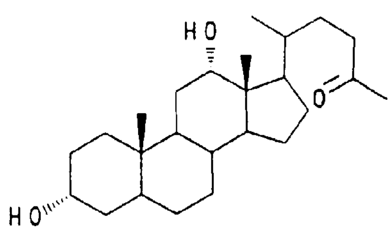 | $C_{48}H_{79}NO_5$ (749) 756 (M+Li$^+$) |

| Bsp. | G1 | MS (FAB, 3-NBA/LiCl oder LiJ) |
|---|---|---|
| 597 | 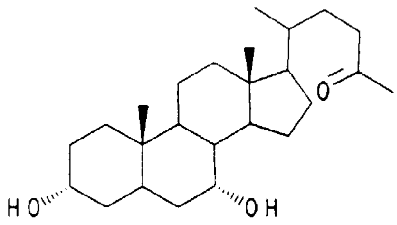 | $C_{48}H_{79}NO_5$ (749) 756 ($M+Li^+$) |
| 598 | 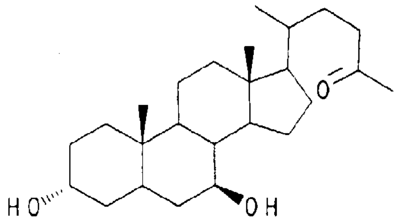 | $C_{48}H_{79}NO_5$ (749) 756 ($M+Li^+$) |
| 599 | 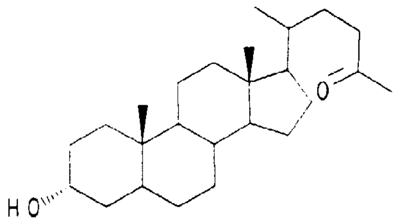 | $C_{48}H_{79}NO_4$ (733) 740 ($M+Li^+$) |

Beispiel 600

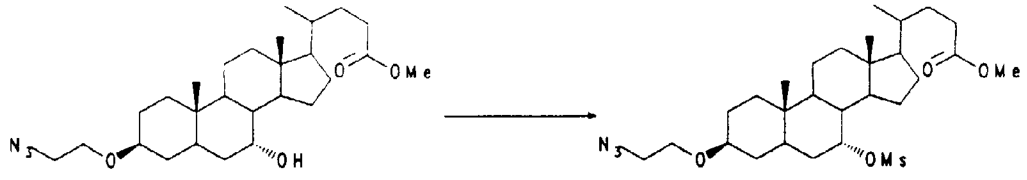

Zu einer Lösung von 15,0 g (31,5 mmol) 3β-(2-Azidoethoxy)-7α-hydroxycholansäuremethylester in 150 ml Pyridin wurden bei 0°C 25 ml (0,32 mol) Methansulfonsäurechlorid zugetropft. Nach 3 h bei Zimmertemperatur wurde auf Eiswasser gegossen und mit Essigester extrahiert. Nach Trocknen und Einengen der organischen Phasen wurde über Kieselgel chromatographiert (Cyclohexan/Essigester 2:1). Man erhielt 13,8 g (79 %) "Beispiel 600".
$C_{28}H_{47}N_3O_6S$ (553), MS (FAB, 3-NBA/LiCl) : 560 ($M+Li^+$).

Beispiel 601

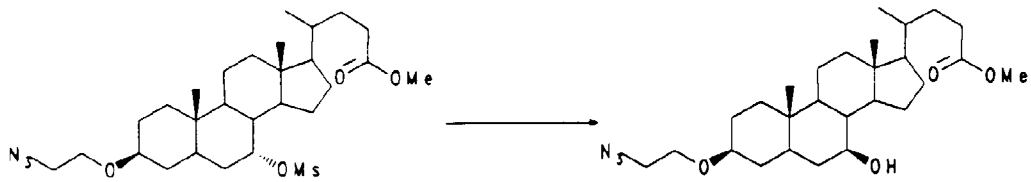

16,8 g (237 mmol) $KO_2$ und 7,2 g (27,2 mmol) 18-Crown-6 wurden in 300 ml DMSO 15 min bei Zimmertemperatur gerührt. Bei 0°C wurden 13,0 g (23,5 mmol) Beispiel 600 in 50 ml DMSO zugetropft. Nach 1,5 h bei Zimmertemperatur wurde wieder auf 0°C gekühlt, langsam gesättigte NaCl-Lösung zugegeben und mit 2 N HCl angesäuert. Nach mehrmaliger Extraktion mit Essigester wurden die organischen Phasen getrocknet und eingeengt. Das Rohprodukt wurde in einer aus 130 ml Methanol und 13 ml Acetylchlorid hergestellten Lösung verestert. Nach der Aufarbeitung wurde das Rohprodukt über Kieselgel chromatographiert (Cyclohexan/Essigester 6:4) Ausbeute: 5,65 g (51 %) "Beispiel 601".
$C_{27}H_{45}N_3O_4$ (475), MS (FAB, 3-NBA/LiCl) : 482 ($M+Li^+$)

Beispiel 602

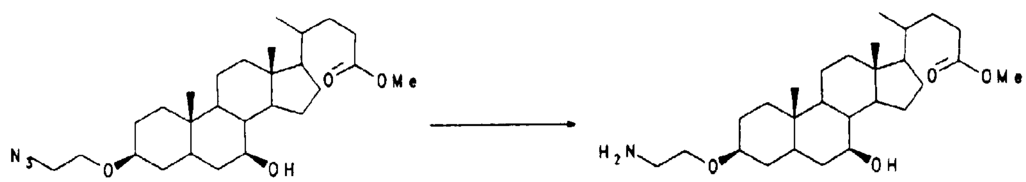

Analog zu Beispiel 567 wurde aus Beispiel 601 "Beispiel 602" hergestellt.
$C_{27}H_{47}NO_4$ (449), MS (FAB, 3-NBA/LiCl) : 456 ($M+Li^+$)

Analog zu den Beispielen 600 - 602 wurden die Beispiele der Tabelle 66 hergestellt.

Tabelle 66

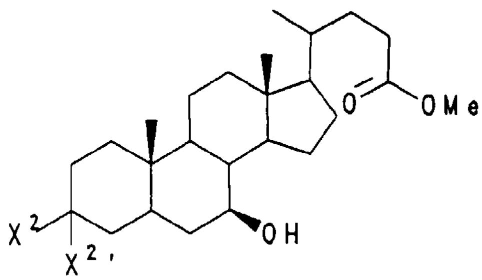

| Bsp. | ß-$X^2$ | α-$X^{2'}$ | MS (FAB, 3-NBA/LiCl oder LiJ) |
|---|---|---|---|
| 603 | $H_2N-(CH_2)_3-O-$ | H | $C_{28}H_{49}NO_4$ (463), 470 ($M+Li^+$) |
| 604 | $H_2N-(CH_2)_4-O-$ | H | $C_{29}H_{51}NO_4$ (477), 484 ($M+Li^+$) |
| 605 | $H_2N-(CH_2)_5-O-$ | H | $C_{30}H_{53}NO_4$ (491), 498 ($M+Li^+$) |
| 606 | $H_2N-(CH_2)_6-O-$ | H | $C_{31}H_{55}NO_4$ (505), 512 ($M+Li^+$) |
| 607 | $H_2N-(CH_2)_{10}-O-$ | H | $C_{25}H_{63}NO_4$ (561), 568 ($M+Li^+$) |
| 608 | $H_2N-(CH_2)_2-O-(CH_2)_2-O-$ | H | $C_{29}H_{51}NO_5$ (493), 500 ($M+Li^+$) |
| 609 | $H_3C-CH(NH_2)-CH_2-O-$ | H | $C_{28}H_{49}NO_4$ (463), 470 ($M+Li^+$) |

Beispiel 610

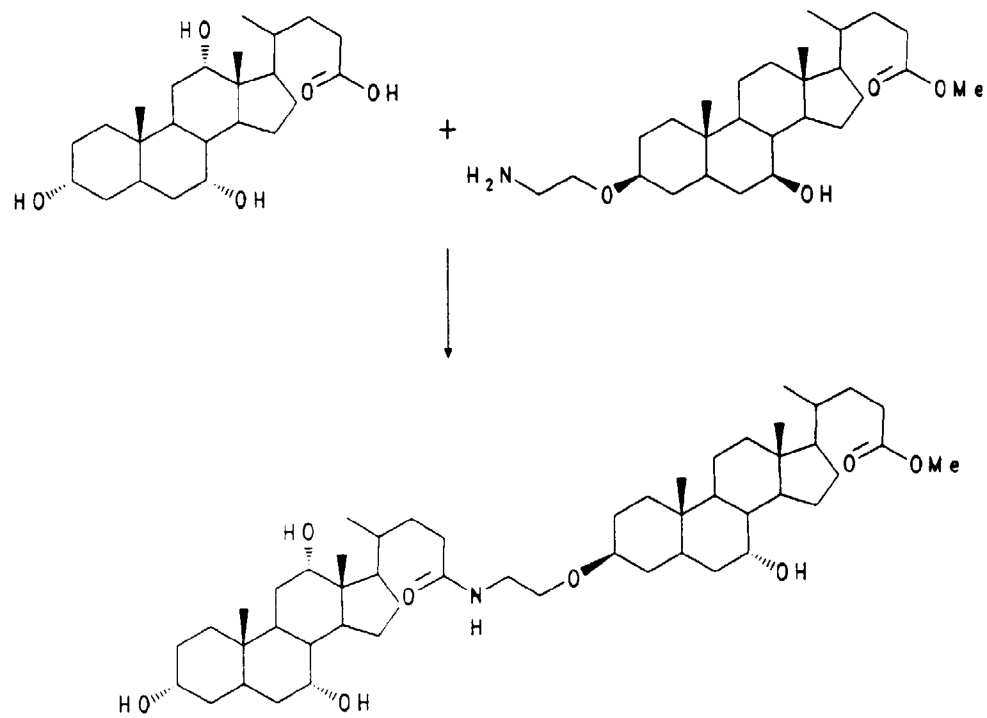

Aus 1,21 g (2,69 mmol) Beispiel 602 und 1,0 g (2,45 mmol) Cholsäure wurde mit 0,55 g (4,07 mmol) Hydroxybenzotriazol und 0,61 g (2,96 mmol) dicyclohexylcarbodiimid nach dem für Beispiel 574 beschriebenen Verfahren 1,7 g (82 %) "Beispiel 610" hergestellt.

$C_{51}H_{85}NO_8$ (839), MS (FAB, 3-NBA/LiCl) : 836 (M+Li$^+$)

Beispiel 611

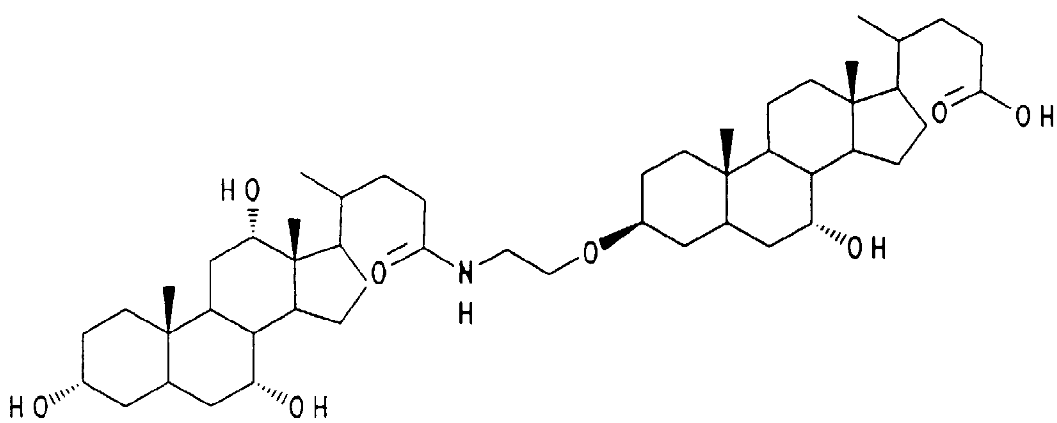

1,5 g (1,79 mmol) Beispiel 610 wurden nach dem für Beispiel 575 beschriebenen Verfahren zu 1,14 g (77 %) "Beispiel 611" verseift.

$C_{50}H_{83}NO_3$ (825), MS (FAB, 3-NBA/LiCl) : 832 (M+Li$^+$)

In Analogie zu den Beispielen 610 und 611 wurden die Beispiele der Tabelle 67 erhalten.

Tabelle 67

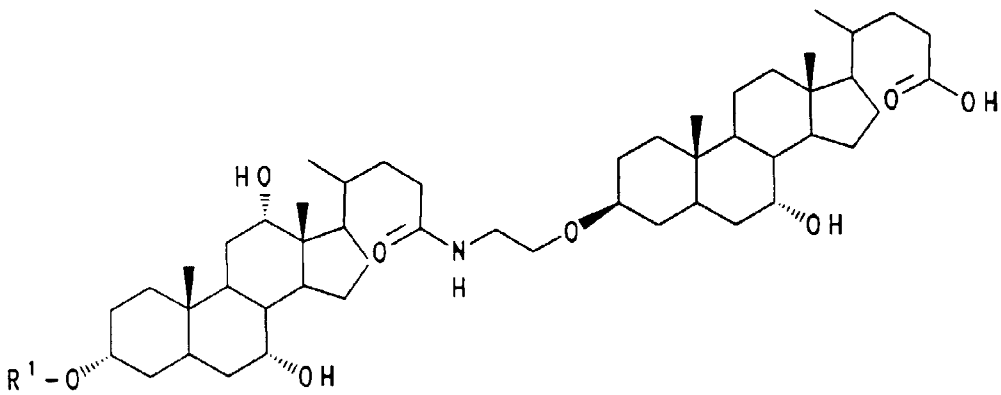

| Bsp. | $R^1$ | MS (FAB, 3-NBA/LiCl oder LiJ) |
|---|---|---|
| 612 | 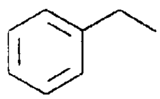 | $C_{57}H_{89}NO_8$ (915), 922 (M+Li$^+$) |
| 613 | 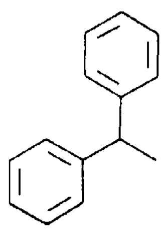 | $C_{63}H_{93}NO_8$ (991), 998 (M+Li$^+$) |
| 614 | 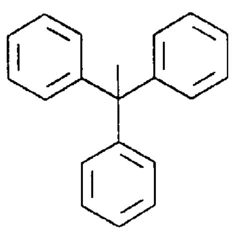 | $C_{69}H_{97}NO_8$ (1067), 1074 (M+Li$^+$) |

| Bsp. | $R^1$ | MS (FAB, 3-NBA/LiCl oder LiJ) |
|---|---|---|
| 615 | 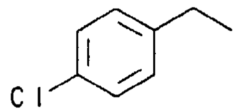 | $C_{57}H_{88}ClNO_8$ (949, 956 ($M+Li^+$) |
| 616 | 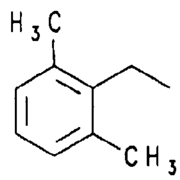 | $C_{59}H_{94}NO_8$ (944), 951 ($M+Li^+$) |
| 617 | 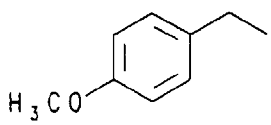 | $C_{58}H_{91}NO_9$ (945), 952 ($M+Li^+$) |
| 618 | 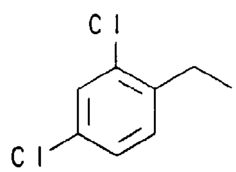 | $C_{57}H_{87}Cl_2NO_8$ (983), 990 ($M+Li^+$) |
| 619 | 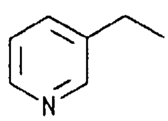 | $C_{56}H_{88}N_2O_8$ (916), 923 ($M+Li^+$) |

| Bsp. | $R^1$ | MS (FAB, 3-NBA/LiCl oder LiJ) |
|---|---|---|
| 620 | 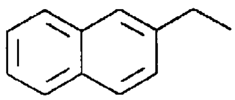 | $C_{61}H_{91}NO_8$ (965), 972 (M+Li$^+$) |

In Analogie zu den Beispielen 610 und 611 wurden die Beispiele der Tabelle 68 erhalten.

Tabelle 68

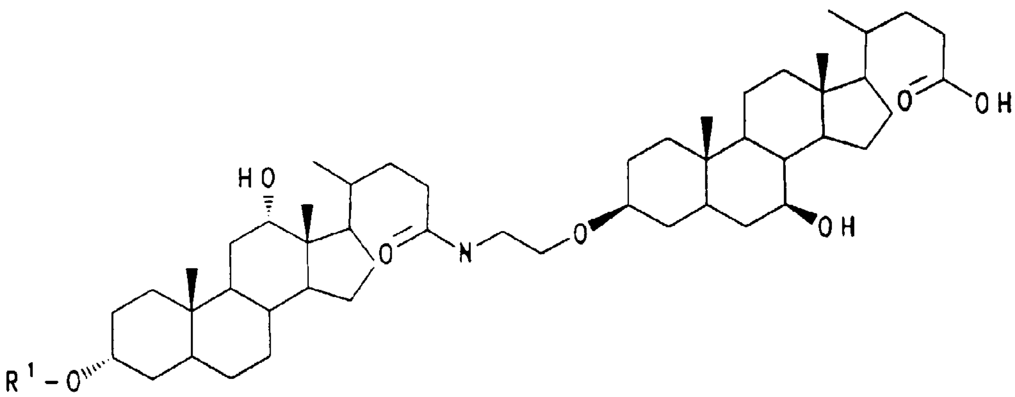

| Bsp. | $R^1$ | MS (FAB, 3-NBA/LiCl oder LiJ) |
|---|---|---|
| 621 | H | $C_{50}H_{83}NO_7$ (809), 816 (M+Li$^+$) |
| 622 | 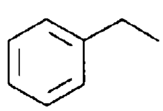 | $C_{57}H_{89}NO_7$ (899), 906 (M+Li$^+$) |

| Bsp. | R[1] | MS (FAB, 3-NBA/LiCl oder LiJ) |
|---|---|---|
| 623 | 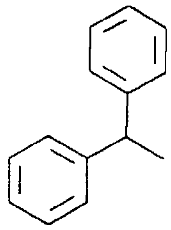 | $C_{63}H_{93}NO_7$ (975), 982 ($M+Li^+$) |
| 624 | 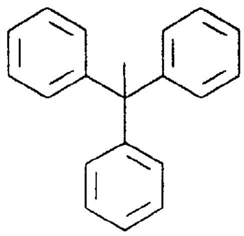 | $C_{69}H_{97}NO_7$ (1051), 1058 ($M+Li^+$) |
| 625 | 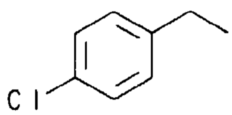 | $C_{57}H_{88}ClNO_7$ (933), 940 ($M+Li^+$) |
| 626 | 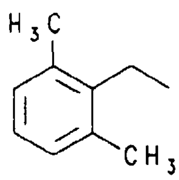 | $C_{59}H_{94}NO_7$ (928), 935 ($M+Li^+$) |
| 627 | 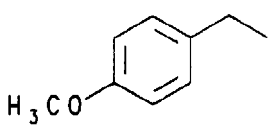 | $C_{58}H_{91}NO_8$ (929), 936 ($M+Li^+$) |

| Bsp. | $R^1$ | MS (FAB, 3-NBA/LiCl oder LiJ) |
|---|---|---|
| 628 | 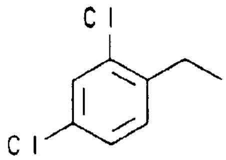 | $C_{57}H_{87}Cl_2NO_7$ (967), 974 ($M+Li^+$) |
| 629 | 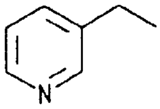 | $C_{56}H_{88}N_2O_7$ (900), 907 ($M+Li^+$) |
| 630 | 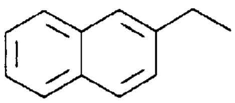 | |

In Analogie zu den Beispielen 610 und 611 wurden die Beispiele der Tabelle 69 erhalten.

Tabelle 69

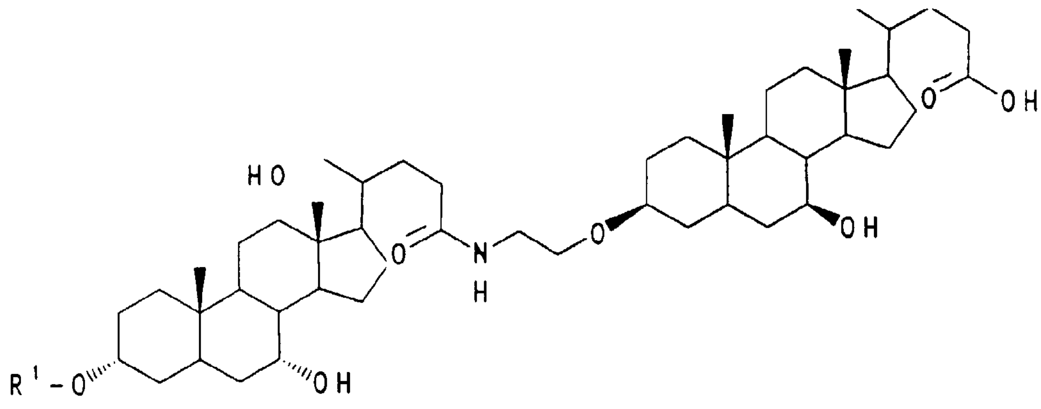

| Bsp. | $R^1$ | MS (FAB, 3-NBA/Licl oder LiJ) |
|---|---|---|
| 631 | H | $C_{50}H_{83}NO_7$ (809), 816 (M+Li$^+$) |
| 632 | 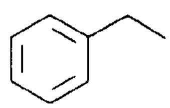 | $C_{57}H_{89}NO_7$ (899), 906 (M+Li$^+$) |
| 633 | 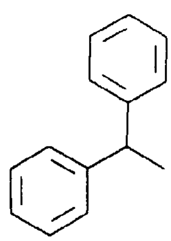 | $C_{63}H_{93}NO_7$ (975), 982 (M+Li$^+$) |
| 634 | 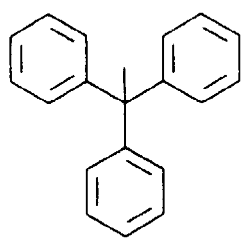 | $C_{69}H_{97}NO_7$ (1051), 1058 (M+Li$^+$) |

| Bsp. | $R^1$ | MS (FAB, 3-NBA/Licl oder LiJ) |
|---|---|---|
| 635 | 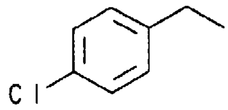 | $C_{57}H_{88}ClNO_7$ (933), 940 (M+Li$^+$) |
| 636 | 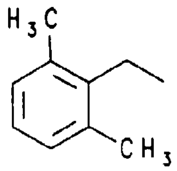 | $C_{59}H_{84}NO_7$ (928), 935 (M+Li$^+$) |
| 637 | 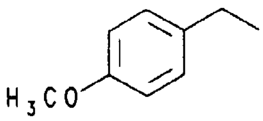 | $C_{58}H_{91}NO_8$ (929), 936 (M+Li$^+$) |
| 638 | 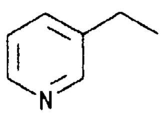 | $C_{56}H_{88}N_2O_7$ (900), 907 (M+Li$^+$) |
| 639 | 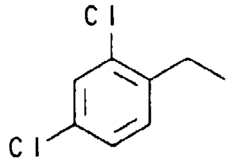 | $C_{57}H_{87}Cl_2NO_7$ (967), 974 (M+Li$^+$) |

| Bsp. | $R^1$ | MS (FAB, 3-NBA/Licl oder LiJ) |
|---|---|---|
| 640 | 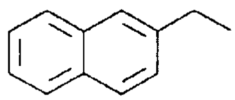 | $C_{61}H_{91}NO_7$ (949), 956 ($M+Li^+$) |

In Analogie zu den Beispielen 610 und 611 wurden die Beispiele der Tabelle 70 erhalten.

Tabelle 70

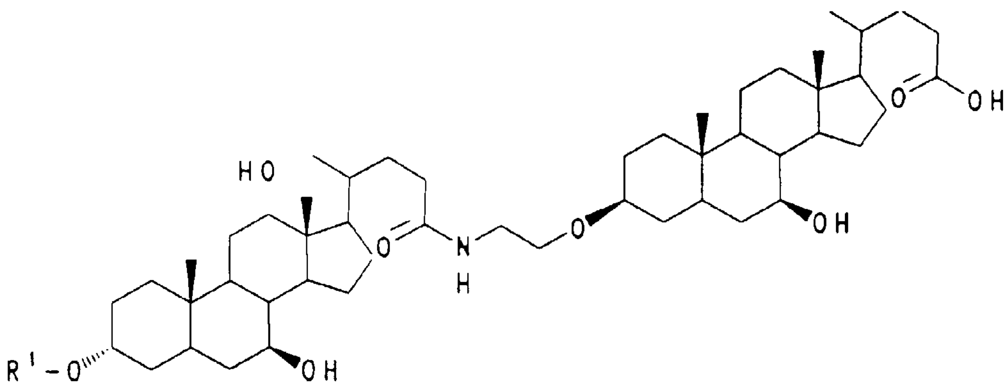

| Bsp. | $R^1$ | MS (FAB, 3-NBA/LiCl oder LiJ) |
|---|---|---|
| 641 | H | $C_{50}H_{83}NO_7$ (809), 816 ($M+Li^+$) |
| 642 | 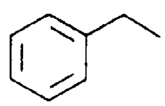 | $C_{57}H_{89}NO_7$ (899), 906 ($M+Li^+$) |

| Bsp. | $R^1$ | MS (FAB, 3-NBA/LiCl oder LiJ) |
|---|---|---|
| 643 | 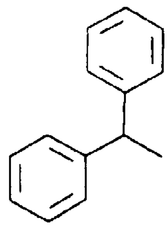 | $C_{63}H_{93}NO_7$ (975), 982 ($M+Li^+$) |
| 644 | 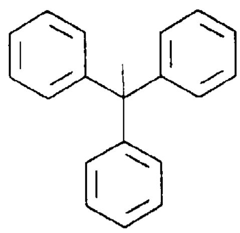 | $C_{69}H_{97}NO_7$ (1051), 1058 ($M+Li^+$) |
| 645 | 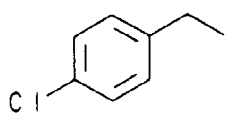 | $C_{57}H_{88}ClNO_7$ (933), 940 ($M+Li^+$) |
| 646 | 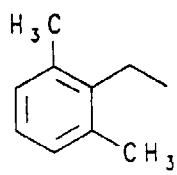 | $C_{59}H_{94}NO_7$ (928), 935 ($M+Li^+$) |
| 647 | 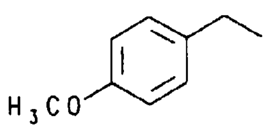 | $C_{58}H_{91}NO_8$ (929), 936 ($M+Li^+$) |

| Bsp. | $R^1$ | MS (FAB, 3-NBA/LiCl oder LiJ) |
|---|---|---|
| 648 | 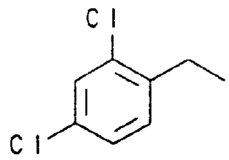 | $C_{57}H_{87}Cl_2NO_7$ (967), 974 ($M+Li^+$) |
| 649 | 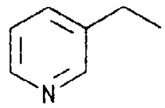 | $C_{56}H_{88}N_2O_7$ (900), 907 ($M+Li^+$) |
| 650 | 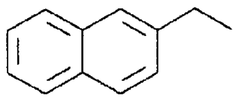 | $C_{61}H_{91}NO_7$ (949), 956 ($M+Li^+$) |

In Analogie zu den Beispielen 610 und 611 wurden die Beispiele der Tabelle 71 erhalten.

Tabelle 71

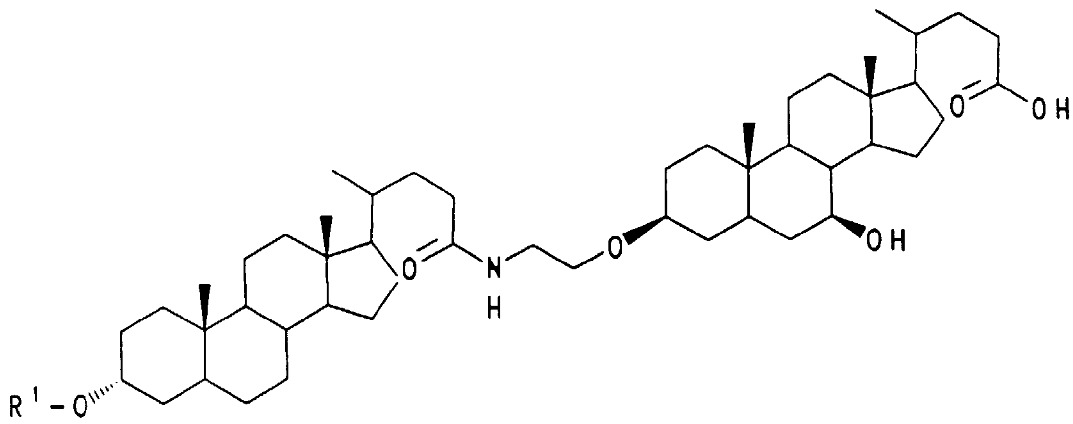

| Bsp. | $R^1$ | MS (FAB, 3-NBA/LiCl oder LiJ) |
|---|---|---|
| 651 | H | $C_{50}H_{83}NO_6$ (793), 800 $(M+Li^+)$ |
| 652 | 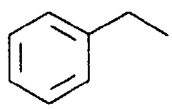 | $C_{57}H_{89}NO_6$ (883), 890 $(M+Li^+)$ |
| 653 | 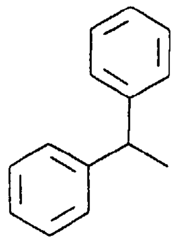 | $C_{63}H_{93}NO_6$ (959), 966 $(M+Li^+)$ |
| 654 | 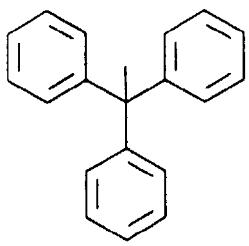 | $C_{69}H_{97}NO_6$ (1035), 1042 $(M+Li^+)$ |

| Bsp. | $R^1$ | MS (FAB, 3-NBA/LiCl oder LiJ) |
|---|---|---|
| 655 | 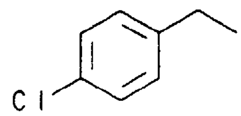 | $C_{57}H_{88}ClNO_6$ (917), 924 (M+Li$^+$) |
| 656 | 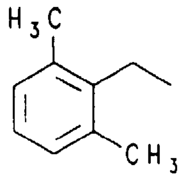 | $C_{59}H_{94}NO_6$ (912), 919 (M+Li$^+$) |
| 657 | 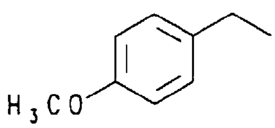 | $C_{58}H_{91}NO_7$ (913), 920 (M+Li$^+$) |
| 658 | 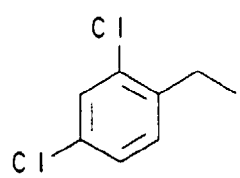 | $C_{57}H_{87}Cl_2NO_6$ (951), 958 (M+Li$^+$) |
| 659 | 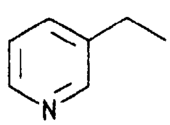 | $C_{56}H_{88}N_2O_6$ (884), 891 (M+Li$^+$) |

| Bsp. | $R^1$ | MS (FAB, 3-NBA/LiCl oder LiJ) |
|---|---|---|
| 660 | 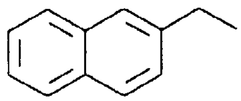 | $C_{61}H_{91}NO_6$ (933), 940 ($M+Li^+$) |

Beispiel 661

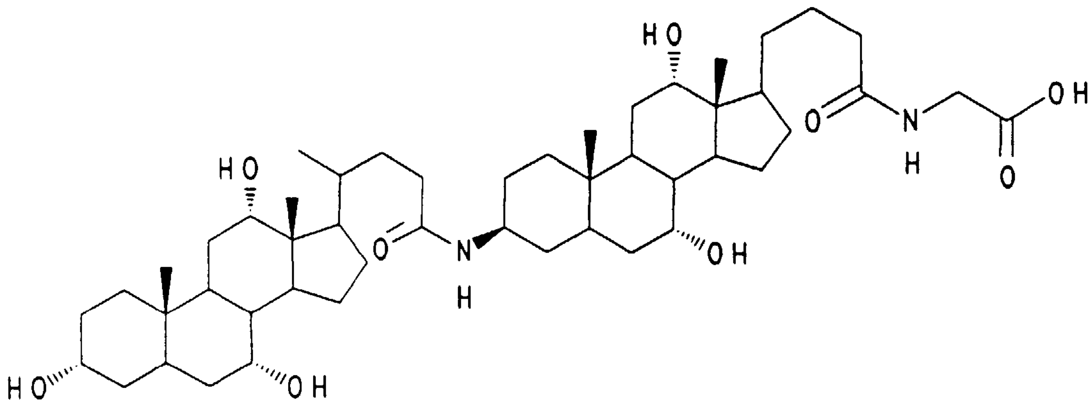

Zu 200 mg (0,25 mmol) Beispiel 575 und 0,052 ml (0,38 mmol) Triethylamin in 30 ml THF wurden bei 0°C 0,036 ml (0,38 mmol) Chlorameisensäureethylester zugetropft. Nach 15 min bei 0°C wurde eine Lösung von 66 mg (0,88 mmol) Glycin in 7,55 ml 0,1 M NaOH zugetropft und noch 5 h bei Zimmertemperatur gerührt. Es wurde gesättigte Natriumdihydrogenphosphatlösung zugegeben und 3x mit THF extrahiert. Nach Trocknen und Einengen der organischen Phasen wurde der Rückstand über Kieselgel chromatographiert ($CHCl_3$/Methanol/Essigsäure 16:4:1). Ausbeute 180 mg (84 %) "Beispiel 661".

$C_{50}H_{82}N_2O_9$ (854), MS (FAB, 3-NBA/LiCl) : 861 ($M+Li^+$).

In Analogie zu Beispiel 661 wurden die Beispiele der Tabellen 61 - 71 in die entsprechenden Glycinderivate überführt.

Beispiel 662

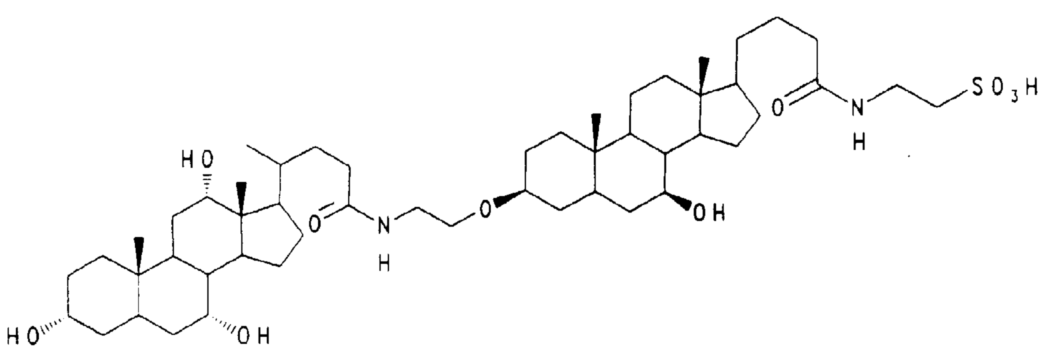

Zu 300 mg (0,38 mmol) Beispiel 611 und 0,19 ml (1,38 mmol) Triethylamin in 50 ml THF wurden bei 0°C 0,13 ml

(1,36 mmol) Chlorameisensäureethylester zugetropft. Nach 15 min bei 0°C wurde eine Lösung von 300 mg (2,4 mmol) Taurin in 12 ml 1 M NaOH zugegeben. Nach 24 h bei Zimmertemperatur wurde aufgearbeitet wie für Beispiel 661 beschrieben. Man erhielt 200 mg (59 %) "Beispiel 662".

$C_{52}H_{88}N_2O_{10}S$ (932), MS (FAB, 3-NBA/LiCl) : 939 (M+Li$^+$).

In Analogie zu Beispiel 662 wurden die Beispiele der Tabellen 61 - 71 in die entsprechenden Taurinderivate überführt.

Beispiel 663

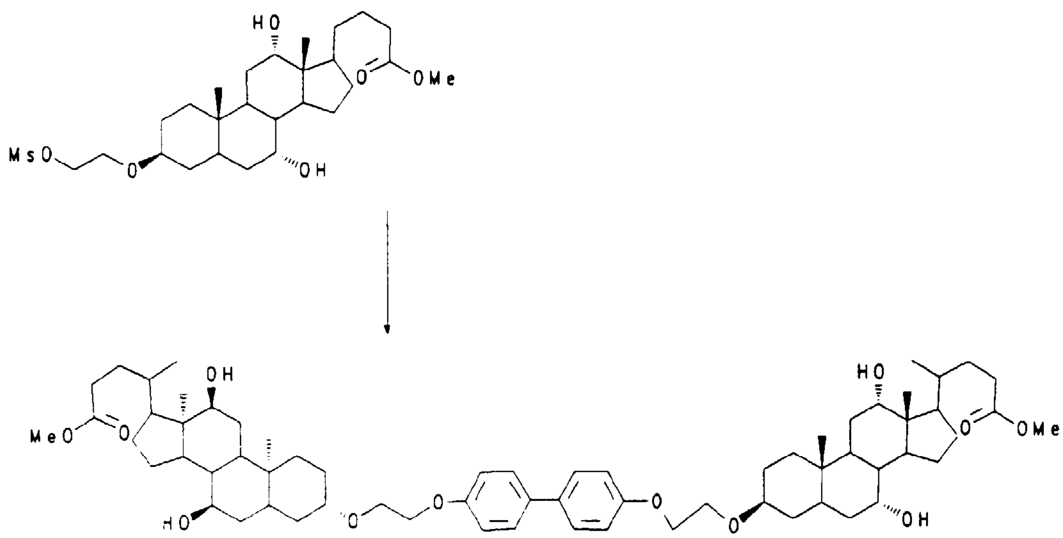

1,0 g (1,84 mmol) Beispiel 137, 170 mg (0,91 mmol) 4,4'-Dihydroxybiphenyl und 400 mg (2,9 mmol) Kaliumcarbonat wurden in 20 ml DMSO 5 h bei 60°C gerührt. Es wurde Wasser zugegeben und mit Essigester extrahiert. Nach Trocknen und Einengen der organischen Phase wurde der Rückstand über Kieselgel chromatographiert (Cyclohexan/Essigester 1:4). Ausbeute 340 mg (34 %) "Beispiel 663".

$C_{66}H_{98}O_{12}$ (1082), MS (FAB, 3-NBA/LiCl) 1089 (M+Li$^+$).

Beispiel 664

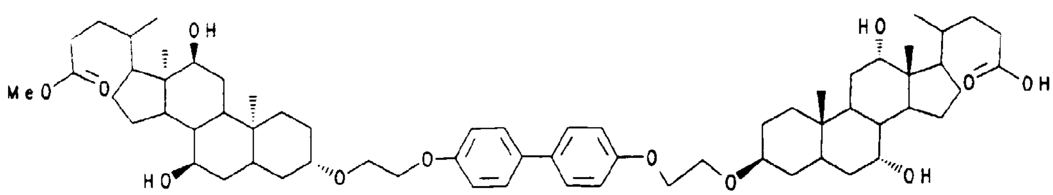

Beispiel 663 wurde nach dem für Beispiel 663 beschriebenen Verfahren zu "Beispiel 664" verseift.

$C_{64}H_{94}O_{12}$ (1054), MS (FAB, 3-NBA/LiCl) : 1061 (M+Li$^+$)

Beispiel 665

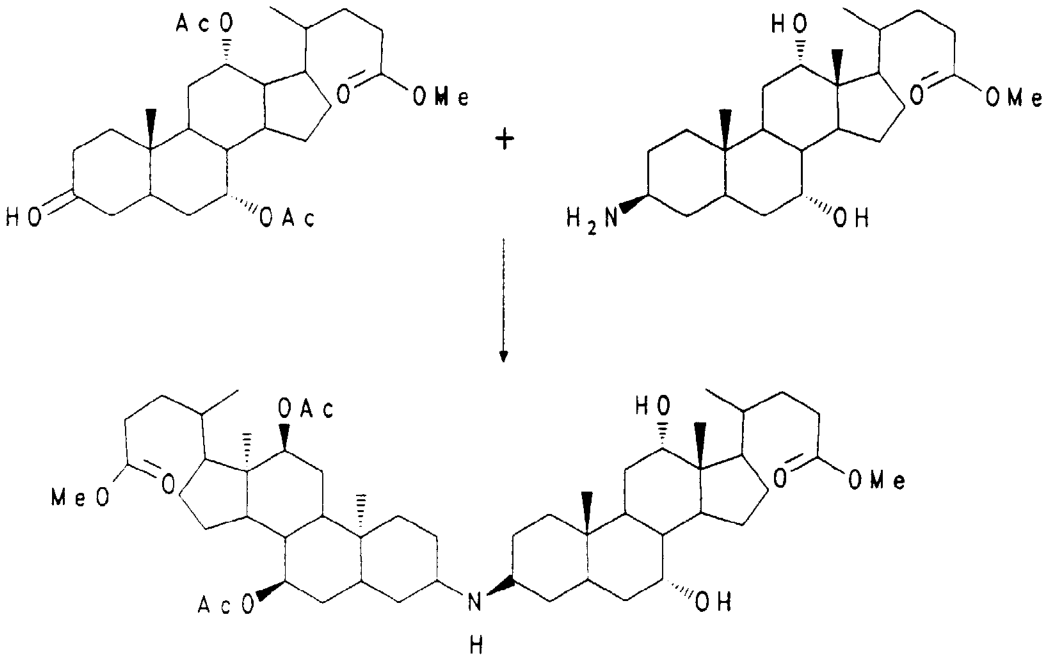

530 mg (1,16 mmol) 3-Amino-7α,12α-dihydroxycholansäuremethylesterhydrochlorid, 600 mg (1,19 mmol) 7α,12α-Diacetoxy-3-ketocholansäuremethylester und 180 mg (2,86 mmol) Natriumcyanoborhydrid wurden in 30 ml absolutem Methanol 24 h bei Zimmertemperatur gerührt. Es wurde auf Wasser gegossen, mit 0,1 M NaOH auf pH 9 gebracht und mit Essigester extrahiert. Die organischen Phasen wurden getrocknet und eingeengt. Chromatographie über Kieselgel (Cyclohexan/Essigester/Triethylamin 50:50:2) ergab 360 mg (34 %) "Beispiel 665".
$C_{54}H_{87}NO_{10}$ (909), MS (FAB, 4-NBA/LiCl) : 916 ($M+Li^+$).

Beispiel 666

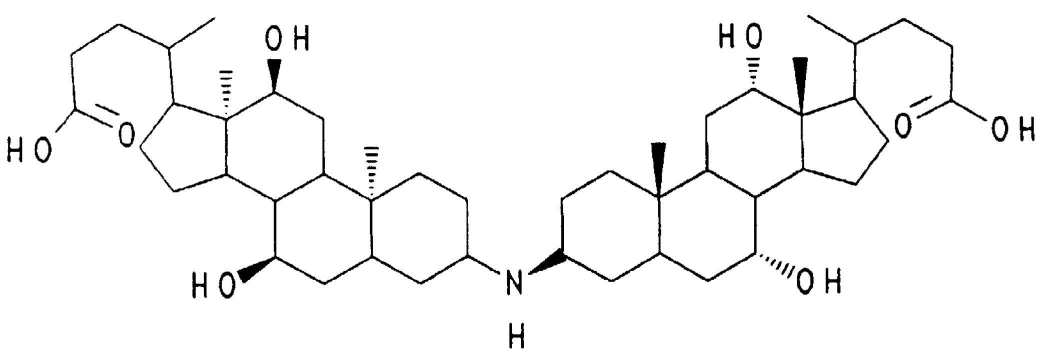

200 mg (0,22 mmol) Beispiel 665 wurden in 5 ml Ethanol/10 ml 5 M NaOH 3 h unter Rückfluß erhitzt. Anschließend wurde der Alkohol eingedampft, mit 1 M HCl angesäuert und der entstandene Niederschlag abgesaugt und getrocknet. Man erhielt 170 mg (97 %) "Beispiel 666".
$C_{49}H_{79}NO_8$ (797), MS (FAB, 3-NBA/LiCl) : 804 ($M+Li^+$).

Beispiel 667

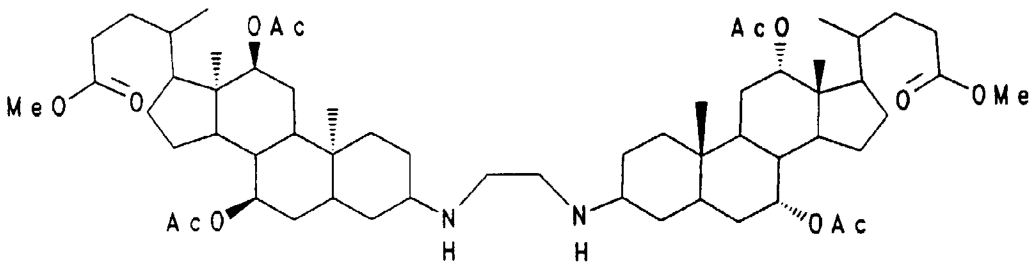

1,0 g (1,98 mmol) 7α,12α-Diacetoxy-3-ketocholansäuremethylester, 130 mg (0,98 mmol) 1,2-Diaminoethan-dihydrochlorid und 300 mg (4,8 mmol) Natriumcyanoborhydrid wurden nach dem für Beispiel 665 beschriebenen Verfahren umgesetzt. Man erhielt 740 mg (71 %) "Beispiel 667".
$C_{58}H_{91}NO_{12}$ (993), MS (FAB, 3-NBA/LiCl) : 1000 (M+Li$^+$)

Beispiel 668

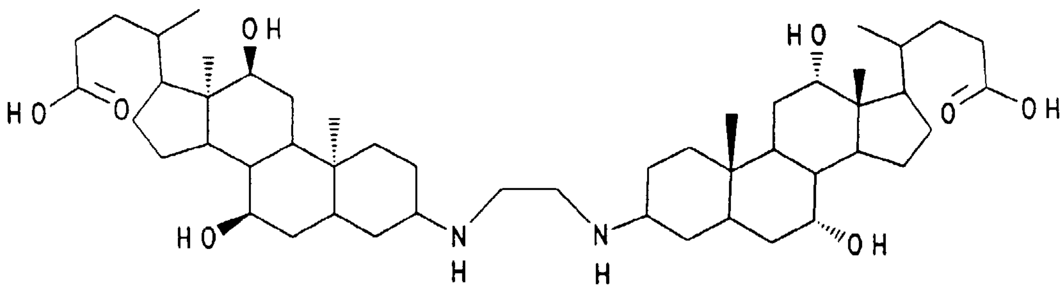

200 mg (1,93 mmol) Beispiel 667 wurden wie für Beispiel 666 beschrieben verseift. Man erhielt 140 mg (86 %) "Beispiel 668".
$C_{50}H_{84}N_2O_8$ (840), MS (FAB, 3-NBA/LiCl) : 847 (M+Li$^+$)

Analog zu den Beispielen 667 und 668 wurden die Beispiele der Tabelle 72 erhalten.

Tabelle 72

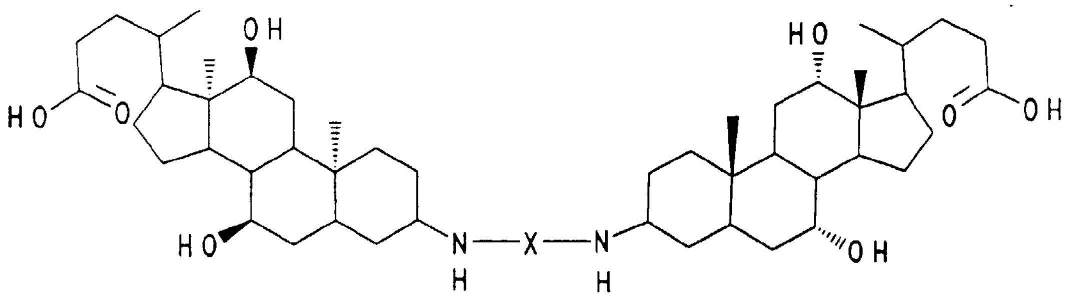

| Bsp. | X | MS (FAB, 3-NBA/LiCl oder LiJ) |
|---|---|---|
| 669 | $-(CH_2)_3-$ | $C_{51}H_{86}N_2O_8$ (854), 861 (M+Li$^+$) |
| 670 | $-(CH_2)_6-$ | $C_{54}H_{92}N_2O_8$ (896), 903 (M+Li$^+$) |
| 671 | $-(CH_2)_{12}-$ | $C_{60}H_{104}N_2O_8$ (980), 987 (M+Li$^+$) |
| 672 | $-(CH_2)_2-O-(CH_2)_2-$ | $C_{52}H_{88}N_2O_9$ (884), 891 (M+Li$^+$) |

Beispiel 673

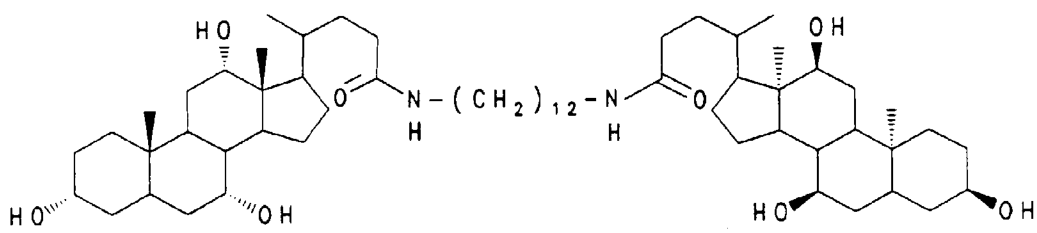

4,0 g (9,8 mmol) Cholsäure, 1,0 g (5 mmol) 1,12-Diaminododecan und 2,46 g (10 mmol) 2-Ethoxy-1-ethoxy-carbonyl-1,2-dihydrochinolin wurden 3 h in 80 ml Toluol unter Rückfluß erhitzt. Das Lösungsmittel wurde eingedampft und der Rückstand über Kieselgel chromatographiert (Chloroform/Methanol 6:1). Ausbeute 3,0 g (63 %) "Beispiel 673". $C_{60}H_{104}N_2O_2$ (980), MS (FAB, 3-NBA/LiCl) : 987 (M+Li$^+$)

Analog zu Beispiel 673 wurden die Beispiele der Tabelle 73 erhalten.

Tabelle 73

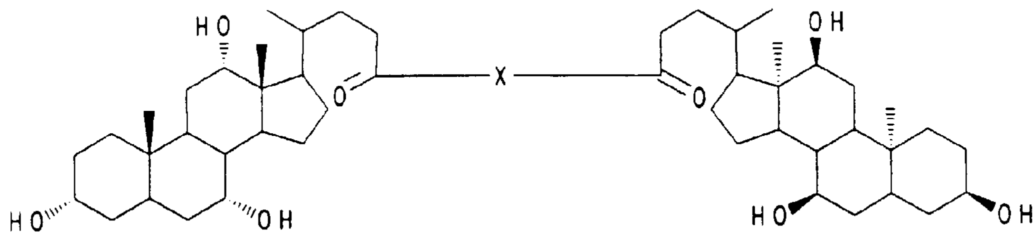

| Bsp. | | MS (FAB, 3-NBA/LiCl oder LiJ) |
|---|---|---|
| 674 | $-N-(CH_2)_3-N-$<br>\|   \|<br>H   H | $C_{51}H_{88}N_2O_8$ (856), 863 $(M+Li^+)$ |
| 675 | $-N-(CH_2)_6-N-$<br>\|   \|<br>H   H | $C_{54}H_{94}N_2O_8$ (898), 905 $(M+Li^+)$ |

| Bsp. | | MS (FAB, 3-NBA/LiCl oder LiJ) |
|---|---|---|
| 676 | 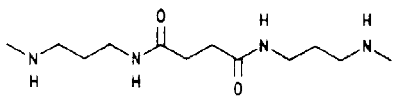 | $C_{58}H_{100}N_4O_{10}$ (1012, 1019 $(M+Li^+)$ |
| 677 | 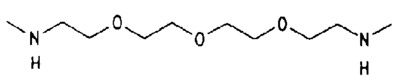 | $C_{56}H_{98}N_2O_{11}$ (974), 981 $(M+Li^+)$ |

Beispiel 678

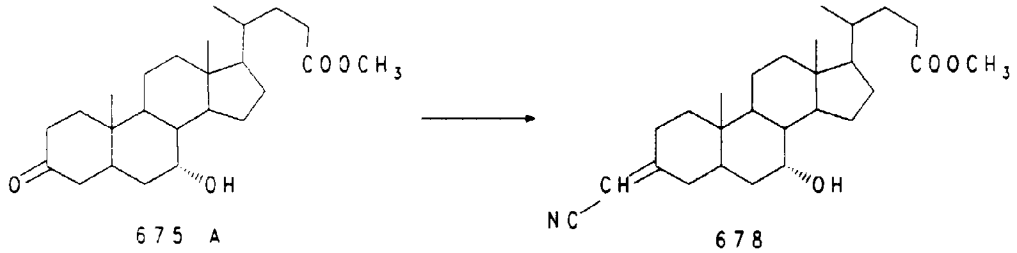

675 A 678

2,2 g (0,055 Mol) 60 % Natriumhydridsuspension wurden unter Argon zu 150 trockenem Methanol gegeben. Dazu gibt man tropfenweise unter Kühlung 9 ml (0,055 Mol) Cyanmethanphophonsäurediethylester in 50 ml Methanol. Nach 1 h bei Raumtemperatur gibt man 20,7 g (0,05 Mol) 678 A in 300 ml Methanol dazu und rührt unter DC-Kontrolle 1 - 2 h bei Raumtemperatur. Die Mischung wird in der Kälte eingeengt und dann zwischen Wasser und Dichlormethan verteilt. Nach Abtrennung wird mit Dichlormethan extrahiert, die organische Phase gewaschen, getrocknet und eingeengt. Der Rückstand wird durch Chromatographie an $SiO_2$ gereinigt.
Ausbeute 17,5 g (82 %) 678 MS (FAB, 3-NBA, LiCl) : 434 ($M+Li^+$)

Beispiel 679

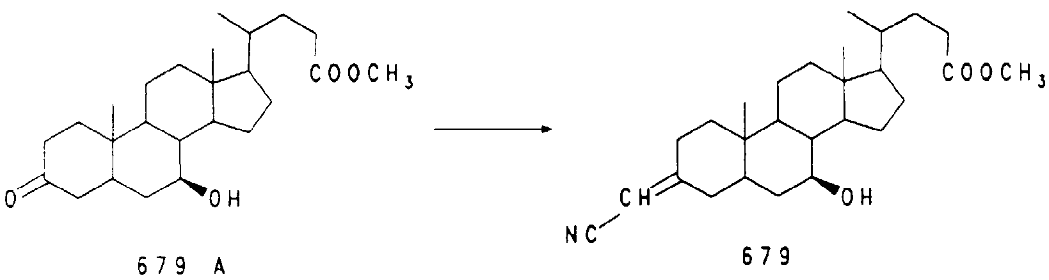

679 A 679

Analog 678 aus 679 A. Ausbeute 49 % MS (FAB, 3-NBA, LiCl) : 434 ($M+Li^+$)

Man erhält nach Chromatographie in 43 % Ausbeute ein doppelbindungsisomeres Produkt 679 B mit gleichem Molekulargewicht, das bei der nachfolgend beschriebenen Hydrierung zum gleichen Punkt wie 679 führt.

Beispiel 680

678

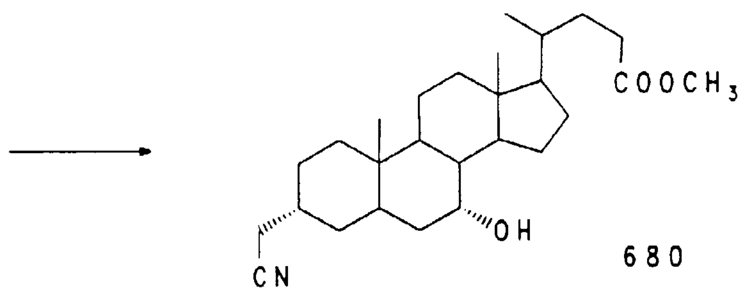

680

15 g (0,035 mol) 678 wurden in 500 ml Methanol gelöst und unter Zusatz von 5 g 10 % Palladium-Kohle bei Raumtemperatur in der Schüttelente hydriert. Man trennt den Katalysator ab, engt ein und chromatographiert den Rückstand an $SiO_2$.
Ausbeute 13,7 g (91 %) 680 (3α-Isomer nach Analyse von 684)
MS (Fab, 3-NBA, LiCl) : 436 ($M+Li^+$).

Beispiel 681

679

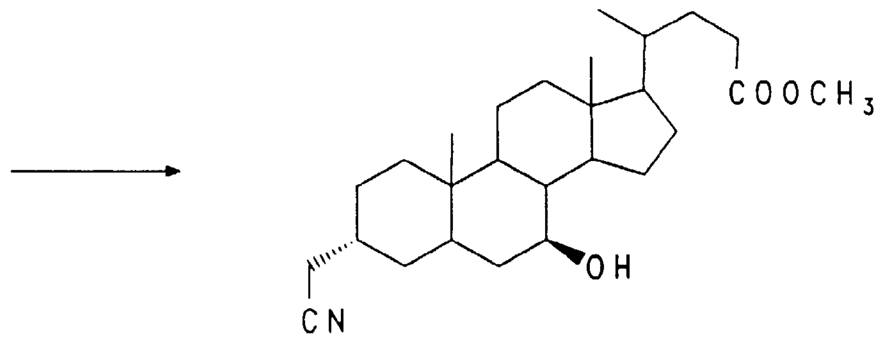

Analog 680 aus 679. Ausbeute 85 % MS (FAB, 3-NBA, LiCl) : 436 (M+Li+) (3α-Isomer nach Angabe von 685)

Beispiel 682

680

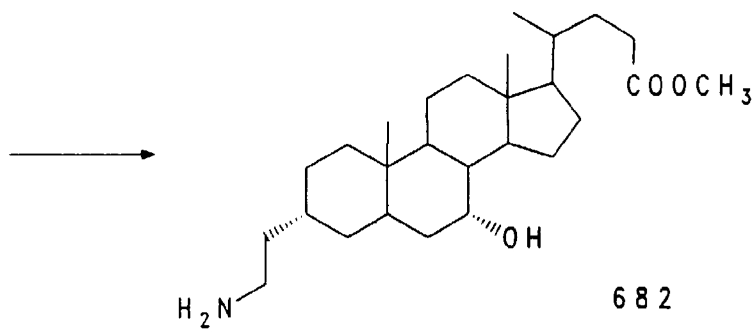

682

12 g (0,028 mol) 680 werden in 300 ml Methanol gelöst und unter Zusatz von 30 ml konzentrierter Ammoniaklösung und 3,5 g 5 % Rhodium auf $Al_2O_3$ bei 20 bar und Raumtemperatur 24 h hydriert. Nach Abtrennung des Katalysators, Einengen und Chromatographie des Rückstands ($SiO_2$) erhält man 682. Ausbeute 9,8 g (81 %)
MS (FAB, 3-NBA, LiCl) : 440 (M+Li+), 434 (M+H+) (3α-Isomer nach Analyse von 684).

Beispiel 683

681

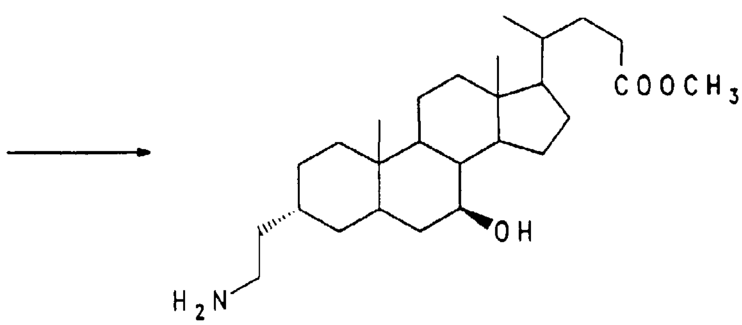

Analog 682 aus 681. Ausbeute 6,1 g (67 %) MS (FAB, 3-NBA, LiCl) 440 (M+Li+), 434 (M+H+) (3α-Isomer nach Analyse von 685).

Beispiel 684

678

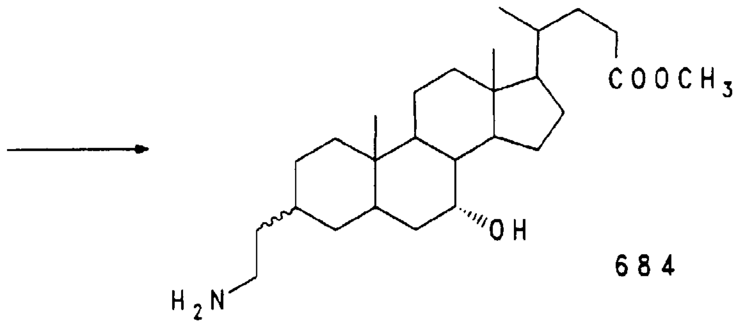

684

15 g (0,035 mol) 678 werden in 50 ml Methanol gelöst und unter Zugabe von 50 ml konz. Ammoniaklösung und 4 g 5 % Rhodium auf $Al_2O_3$ bei 25 bar und Raumtemperatur 24 h hydriert. Nach Aufarbeitung wie bei 682 resultiert ein Rohprodukt, das durch Chromatograpie an $SiO_2$ mit Dichlormethan/Methanol/konz. Ammoniaklösung = 100:15:5 gereinigt wird. Man erhält 6,4 g (42 %) weniger polares 3β-684 und 4,2 g (27,6 %) polareres 3α-684
MS (FAB, 3-NBA, LiCl) : 440 ($M + Li^+$), 424 ($M+ H^+$).
Dünnschichtchromatographischer Vergleich mit 682 ergibt Identität mit 3α-684 und Unterschied zu 3β-684.

Beispiel 685

679

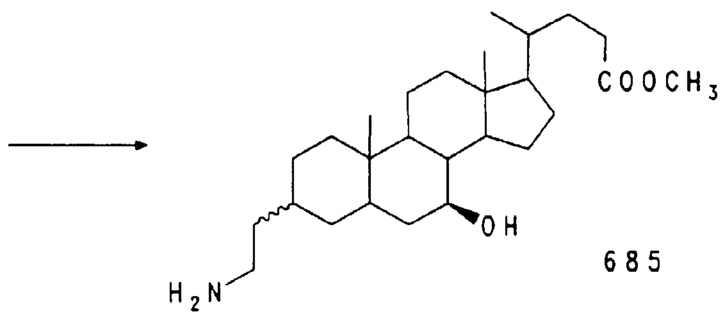

685

Analog zu 684 aus 679. Ausbeute 35 % (weniger polares) 3β-685 und 29 % (polareres) 3α-685. DC-Vergleich mit 683 ergibt Identität mit 3α-685.
MS (FAB, 3-NBA, LiCl) : 440 ($M + Li^+$), 434 ($M+H^+$)

Beispiel 686

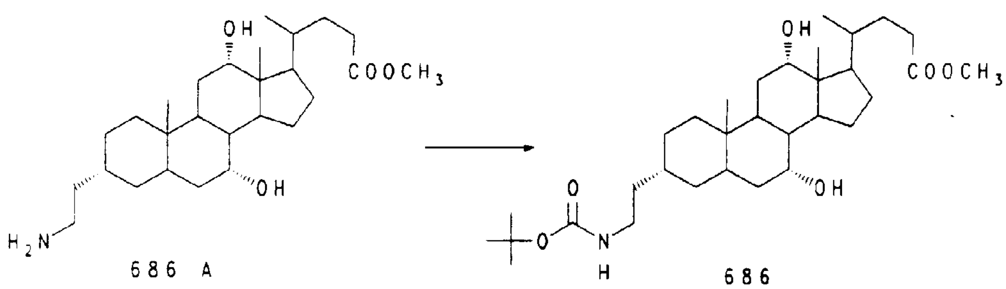

686 A

686

899,4 mg (2 mmol) Amin 678 werden in 15 ml Dioxan/Wasser = 2/1 gelöst und unter Eiskühlung mit 5 ml 1 N NaOH versetzt. Dazu gibt man bei 0°C 480 mg (2,2 mmol) Pyrokohlensäure-di-tert.-Butylester und rührt 30 min bei Raumtemperatur nach. Nach beendeter Umsetzung wird Dioxan im Vakuum entfernt, die wäßrige Phase mit Essigester überschichtet und unter Eiskühlung mit verdünnter $KHSO_4$-Lösung bis pH 2 angesäuert. Man extrahiert neutral, trocknet, engt ein und reinigt den Rückstand durch Chromatographie an $SiO_2$. Ausbeute 792 mg (72 %) Beispiel 686, MS (FAB, 3-NBA, LiCl) : 556 (M+Li+).

Beispiel 687

686

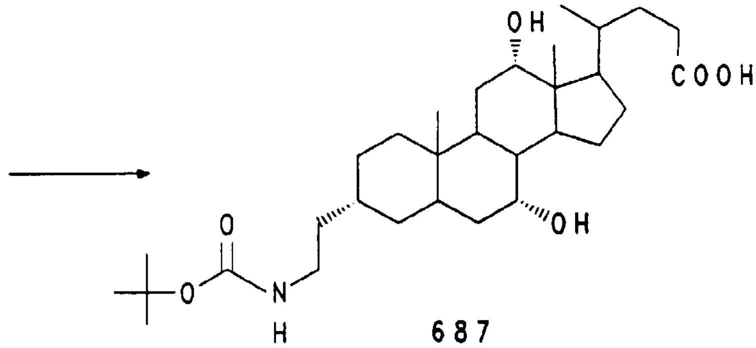

687

2,75 g (5 mmol) 686 werden in 20 ml Methanol gelöst und mit 2 ml 2 N NaOH über Nacht bei Raumtemperatur gerührt. Man verdünnt mit Wasser, entfernt Methanol im Vakuum und säuert durch tropfenweise Zugabe von $KHSO_4$-Lösung bis zur Niederschlagsbildung an. Man saugt ab und filtriert den Rückstand über $SiO_2$.
Ausbeute 1,79 g (67 %) 687. MS (FAB, 3-NBA, LiCl) : 543 (M+Li+).

Beispiel 688

687

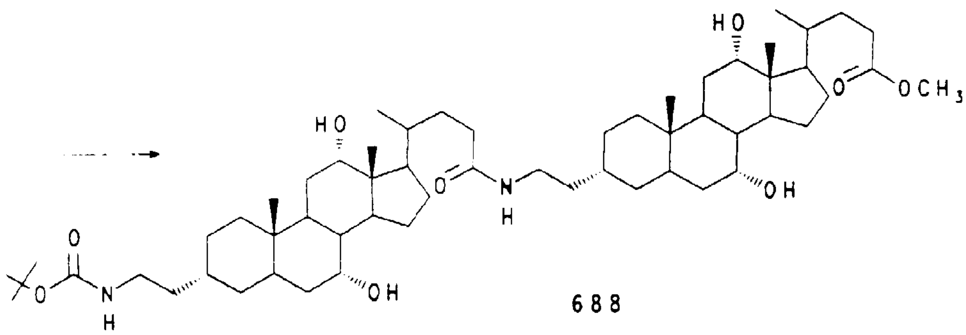

688

1,35 g (3 mmol) 686 A werden in 50 ml Essigester gelöst und mit 0,5 ml Triethylamin versetzt. Dazu gibt man 0,85 g 1,2-Dihydro-2-ethoxychinolin-1-carbonsäureethylester (EEDQ) und 1,6 g (3 mmol) 687 und rührt 4 - 5 h unter Rückfluß. Nach beendeter Reaktion wird mit Essigester verdünnt, mit gesättigter Kaliumhydrogensulfatlösung und Wasser gewaschen, getrocknet, eingeengt und der Rückstand an $SiO_2$ chromatographiert. Ausbeute 2,46 g (85 %) 688.
MS (FAB, 3-NBA, LiCl) : 974 (M+Li+).

Beispiel 689

688

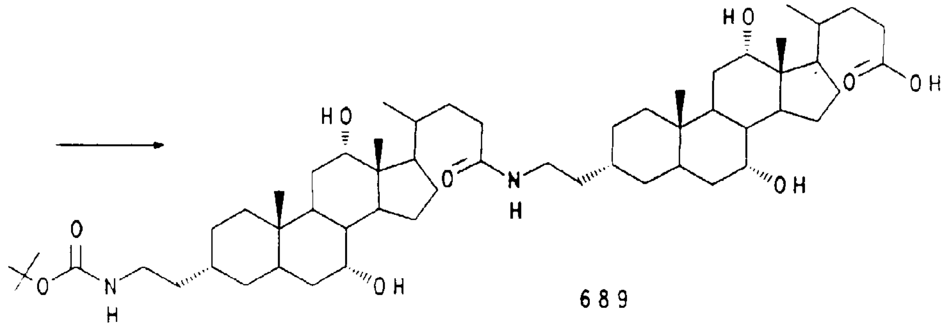

689

2 g (2,07 mmol) 688 werden in 20 ml Methanol mit 2 ml 2 N NaOH wie unter 687 beschrieben verseift. Ausbeute 1,66 g (84 %) 689. MS (FAB, 3-NBA, LiCl) : 960 (M+Li+)

Analog zur Reaktionsfolge 686 A → 686 → 687 → 688 → 689 werden folgende Beispiele hergestellt:

Beispiel 690 aus 682

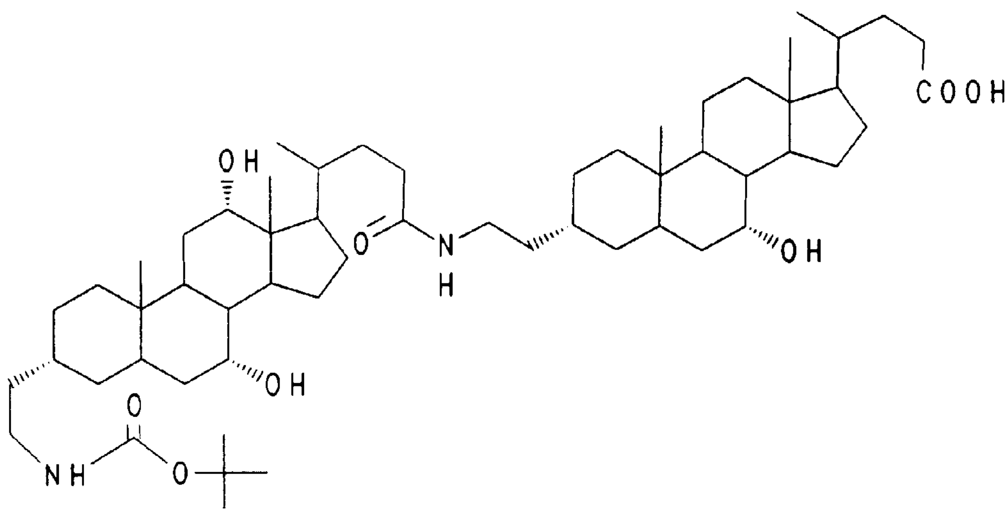

MS (FAB, 3-NBA, LiCl) : 944 (M+Li+)

Beispiel 691 aus 683

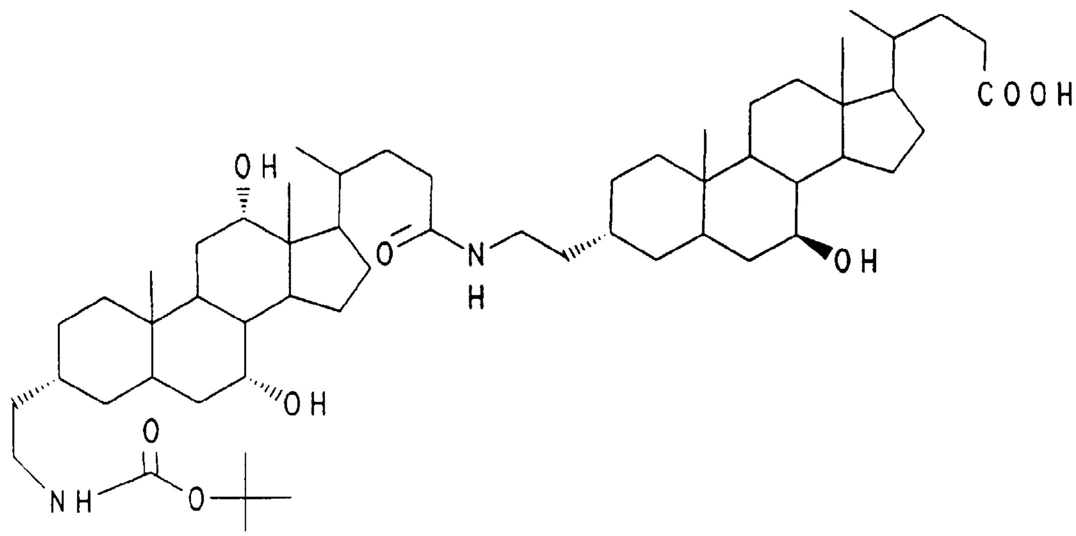

MS (FAB, 3-NBA, LiCl) : 944 (M+Li+)

Beispiel 692

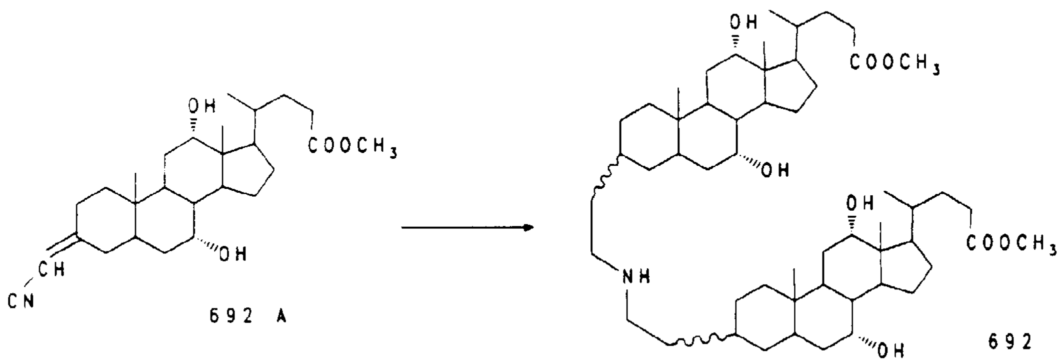

692 A

692

30 g (0,067 mol) 692 A werden in 1 l Methanol gelöst und analog zu Beispiel 684 hydriert. Die chromatographische Trennung des Rohprodukts ergibt neben den Hauptprodukten 1,28 g (4,3 %) 692
MS (FAB, 3-NBA, LiCl) : 889 (M+Li+)

Beispiel 693

692

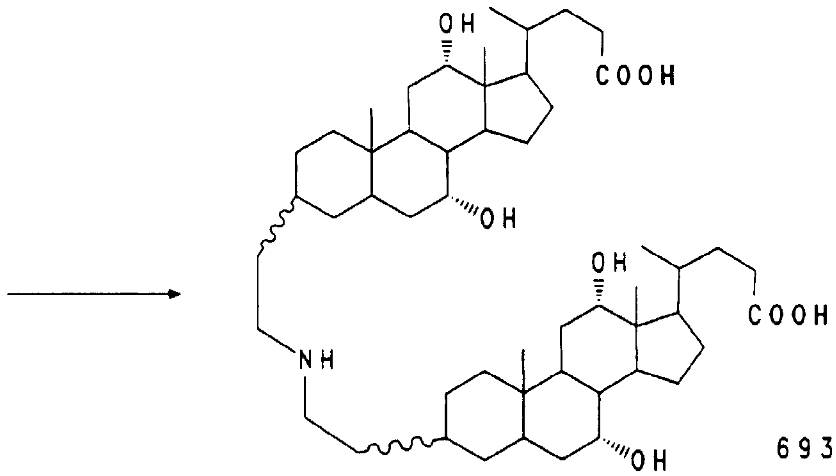

693

1,2 g( 1,36 mmol) 692 werden in 10 ml Methanol gelöst und mit 1 ml 2 N NaOH durch Rühren über Nacht verseift. Man verdünnt mit Wasser, entfernt Methanol im Vakuum und fällt das Produkt durch Zugabe von 2 N HCl aus. Das Rohprodukt wird durch Säulenfiltration gereinigt. Ausbeute 0,96 g (83 %) 693.
MS (FAB, 3-NBA, LiCl) : 861 (M+Li+)

Beispiel 694

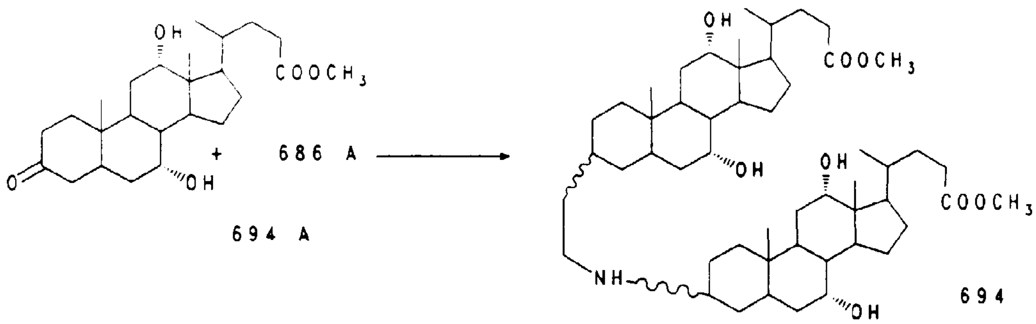

686 A

694 A

694

449 (1 mmol) 686A werden in 15 ml trockenem Methanol gelöst. Dazu gibt man 420 mg (1 mmol) 694A und 80 mg (1,3 mmol) Natriumcyanoborhydrid und rührt über Nacht bei Raumtemperatur. Danach wird eingeengt, der Rückstand zwischen Wasser und Dichlormethan verteilt und der Rückstand der organischen Phase durch Chromatographie ($SiO_2$) gereinigt.
Ausbeute: 450 mg (53 %) 694.
MS (FAB, 3-NBA, LiCl): 861 (M+Li+)

Beispiel 695

694

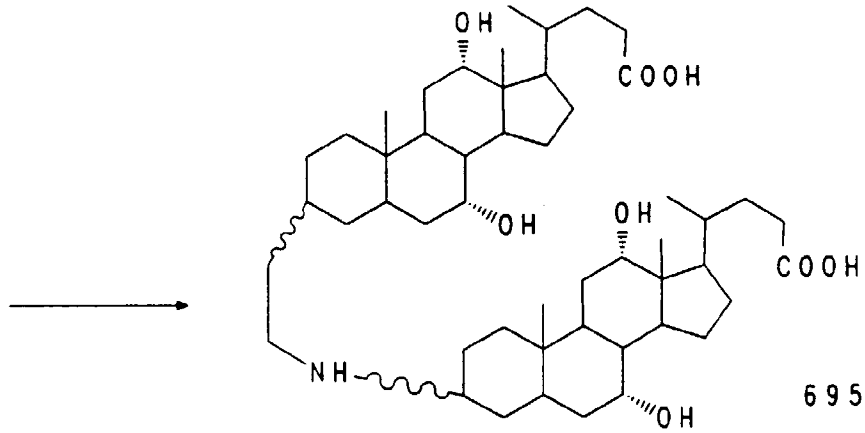

695

200 mg (0,23 mmol) 694 werden in 5 ml Methanol gelöst und mit 0,5 ml 2 N NaOH wie unter 693 beschrieben verseift. Ausbeute 180 mg (95 %) 695.
MS (FAB, 3-NBA, LiCl) : 823 (M + $Li^+$), 827 ($M+H^+$)

Beispiel 696

686 A

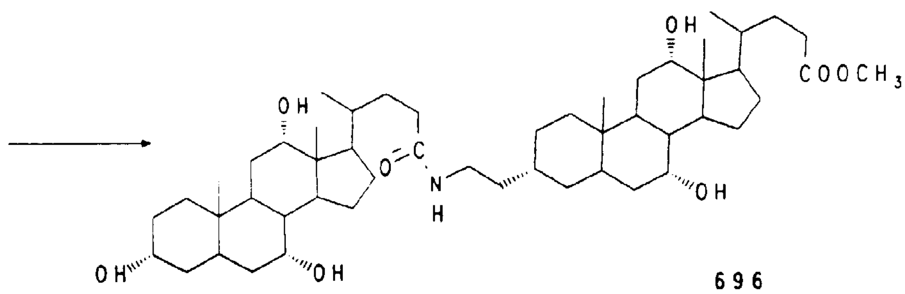

696

4,49 mg (1 mmol) 686 A werden in 30 ml trockenen Essigester mit 0,15 ml Triethylamin, 273 mg (1,1 mol) EEDQ und 408 mg (1 mmol) Cholsäure versetzt und 4 h zum Rückfluß erwärmt. Nach beendeter Umsetzung wird mit ca. 100 ml Essigester verdünnt, mit $KHSO_4$-Lösung gewaschen und der Rückstand der organischen Phase durch Chromatographie gereinigt. Ausbeute 597 mg (71 %) 696
MS (FAB, 3-NBA, LiCl) : 847 ($M+Li^+$)

Beispiel 697

696

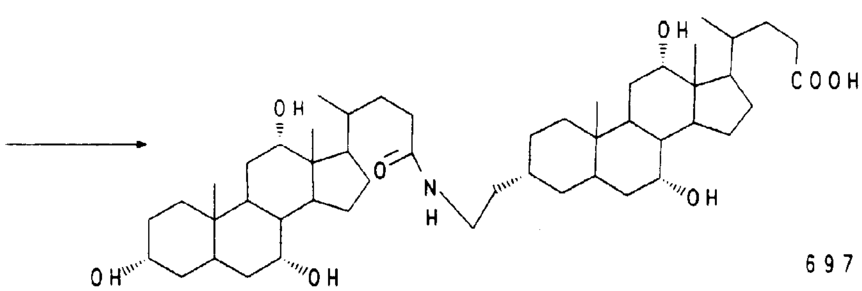

697

500 mg (0,6 mmol) 696 werden in 15 ml Ethanol mit 1,5 ml 2 N NaOH wie unter 693 beschrieben verseift. Ausbeute 452 mg (91 %) 697

MS (FAB, 3-NBA, LiCl) : 833 ($M+Li^+$)

Beispiel 698

697 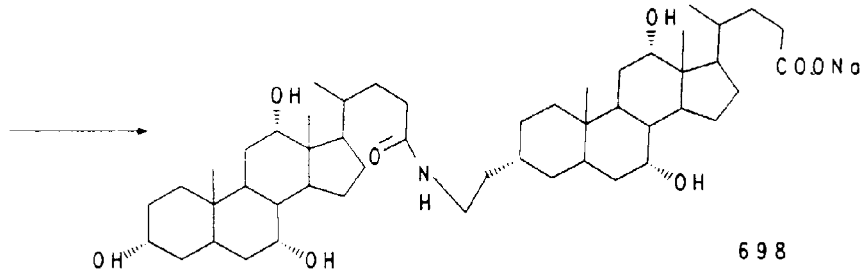 698

413 mg (0,5 mmol) 697 werden in 3 - 5 ml trockenen Methanol gelöst und mit einem Äquivalent einer 1 M Lösung von NaOH in Methanol versetzt. Durch Zugabe von trockenem Ether wird das Natriumsalz 698 ausgefällt, anschließend abgesaugt und getrocknet. Ausbeute 390 mg (92 %) 698.

MS (FAB, 3-NBA) : 849 ($M+H^+$)

Analog zur Reaktionsfolge 686 A $\rightarrow$ 696 $\rightarrow$ 697 werden folgende Beispielsubstanzen hergestellt (699 bis 713)

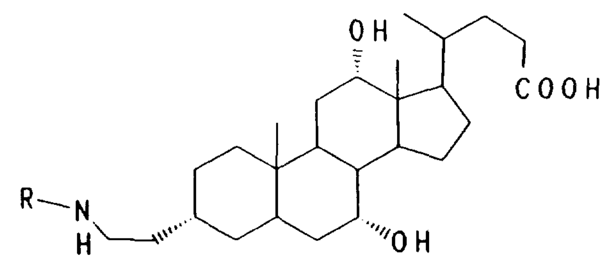

| Bsp. | R | Edukte: 686 A + | MS (FAB, 3-NBA, LiCl) |
|---|---|---|---|
| 699 | 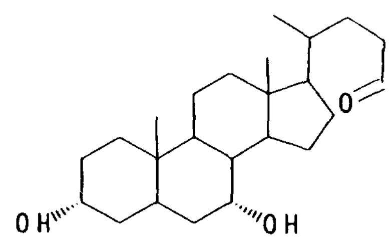 | Chenodesoxycholsäure | 817 $(M+Li^+)$ |
| 700 | 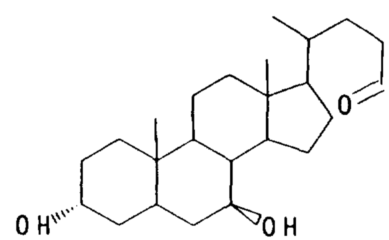 | Ursodesoxycholsäure | 817 $(M+Li^+)$ |

| Bsp. | R | Edukte: 686 A + | MS (FAB, 3-NBA, LiCl) |
|---|---|---|---|
| 701 | 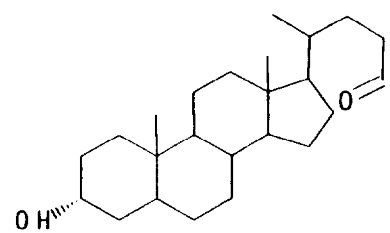 | Lithocholsäure | 801 ($M+Li^+$) |

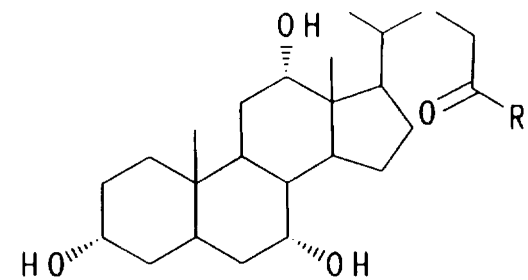

| Bsp. | R | Edukte: Cholsäure + | MS (FAB, 3-NBA, LiCl) |
|---|---|---|---|
| 702 | 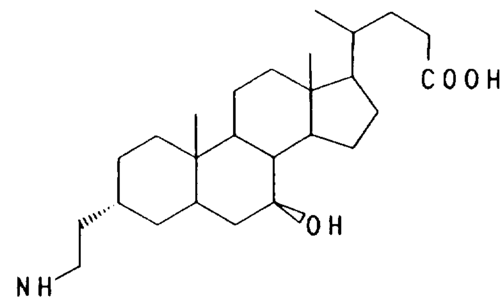 | 682 | 817 (M+Li$^+$) |

| Bsp. | R | Edukte: Cholsäure + | MS (FAB, 3-NBA, LiCl) |
|---|---|---|---|
| 703 | 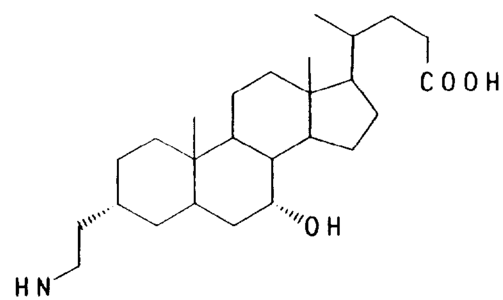 | 683 | 817 $(M+Li^+)$ |

Beispiel 704

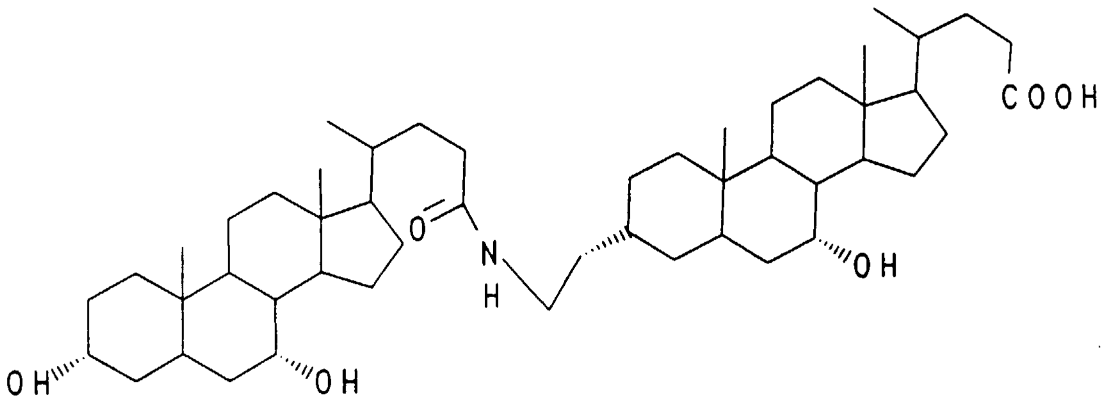

aus Chenodesoxycholsäure + 682
MS (FAB, 3-NBA, LiCl) : 801 (M+Li+)

Beispiel 705

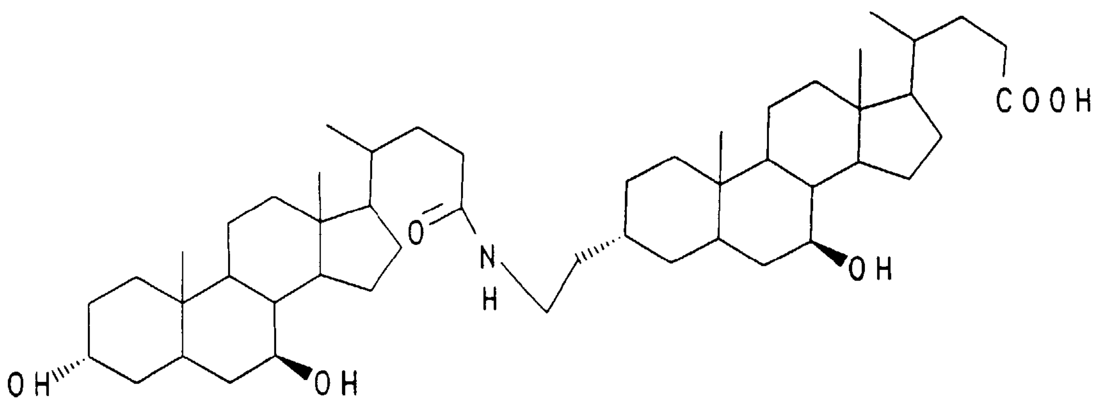

aus Ursodesoxycholsäure + 683
MS (FAB, 3-NBA, LiCl) 801 (M+Li+)

Beispiel 706

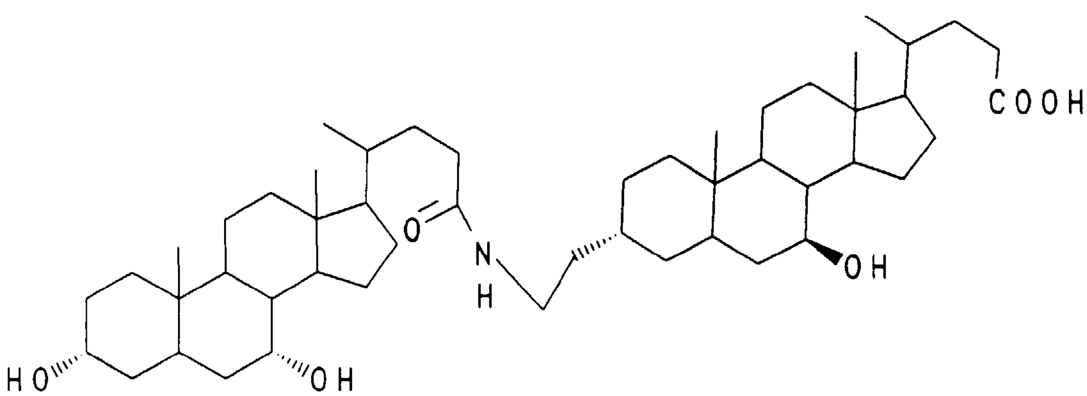

aus Ursodesoxycholsäure + 682
MS (FAB, 3-NBA, LiCl) : 801 (M+Li+)

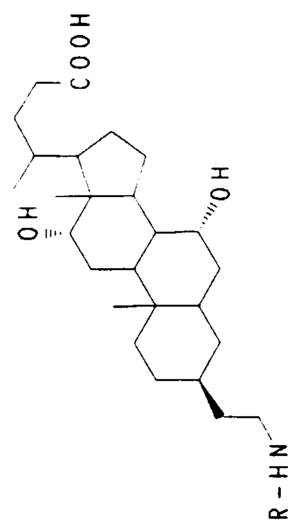

| Bsp. | R | aus | MS (FAB, 3-NBA, LiCl) |
|---|---|---|---|
| 707 | 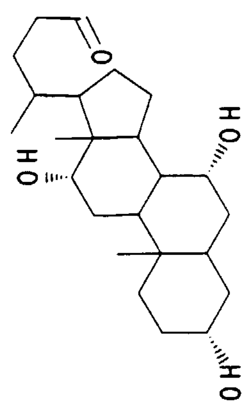 | 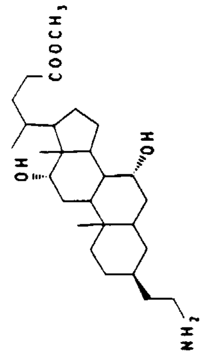 + Cholsäure | 833 (M+Li+) |

| Bsp. | R | aus | MS (FAB, 3-NBA, LiCl) |
|---|---|---|---|
| | | 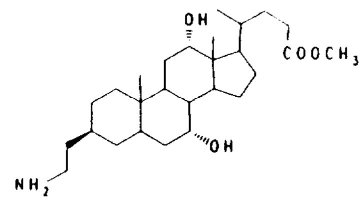 | |
| 708 | 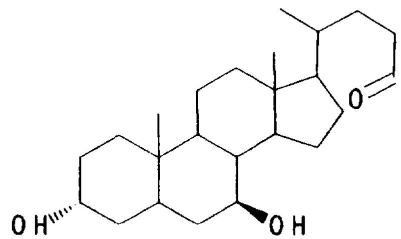 | + Ursodesoxy-cholsäure | 817 $(M+Li^+)$ |
| 709 | 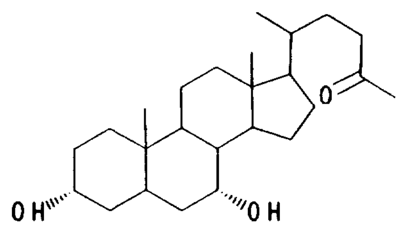 | + Chenodesoxy-cholsäure | 817 $(M+Li^+)$ |

| Bsp. | R | aus | MS (FAB, 3-NBA, LiCl) |
|---|---|---|---|
| | | 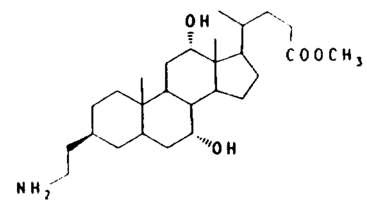 | |
| 710 | 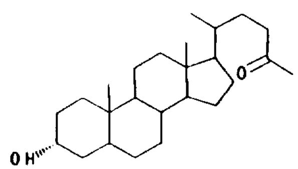 | + Lithocholsäure | 801 $(M+Li^+)$ |

Beispiel 711

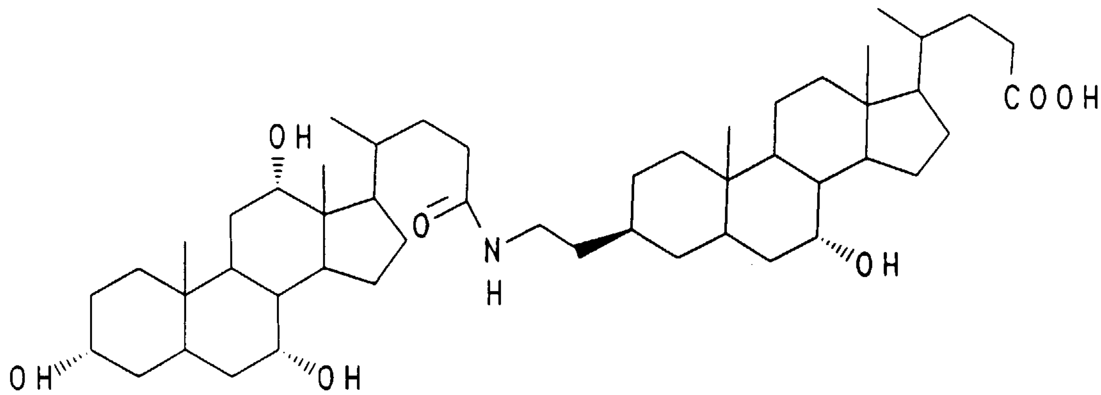

aus Cholsäure + 3β-684 MS (FAB, 3-NBA, LiCl) : 817 ($M+Li^+$)

Beispiel 712

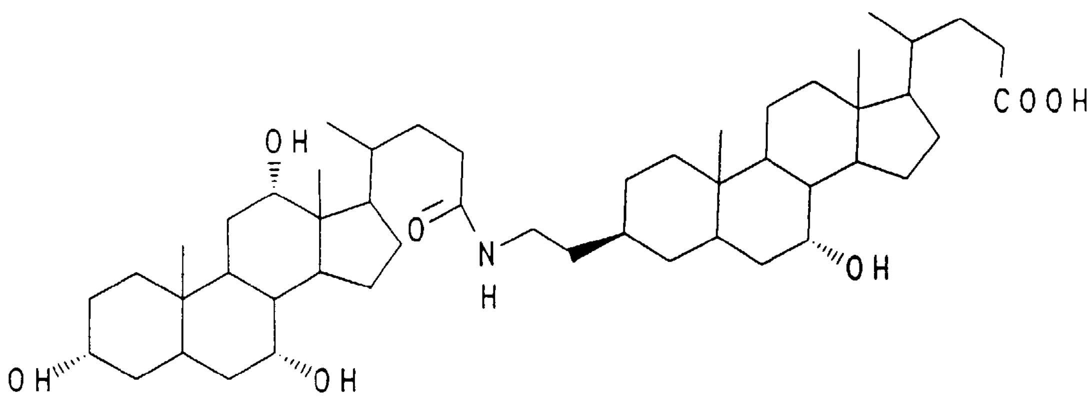

aus Cholsäure + 3β-685 MS (FAB, 3-NBA, LiCl) : 817 ($M+Li^+$)

Beispiel 713

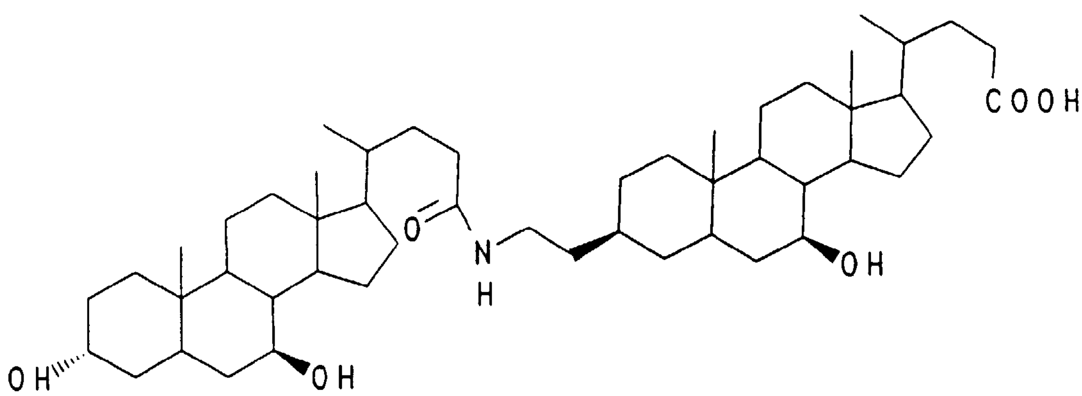

aus Ursodesoxycholsäure + 3β-685 MS (FAB, 3-NBA, LiCl) : 801 ($M+Li^+$)

Beispiel 714

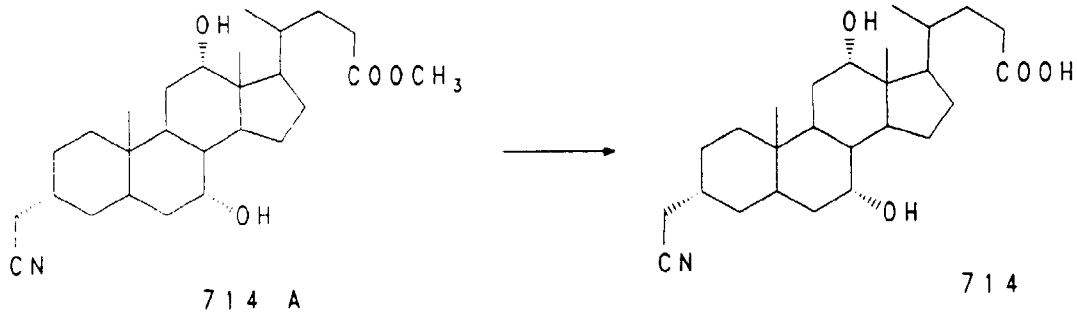

714 A

714

446 mg (1 mmol) 714 A wurden in 40 ml Ethanol mit 2 ml halbkonzentrierter Natronlauge durch Rühren über Nacht verseift. Man verdünnt mit Wasser, entfernt Ethanol im Vakuum und fällt 714 durch Ansäuern mit verdünnter Salzsäure aus. Das Produkt wird abgesaugt, und mit Wasser nachgewaschen und getrocknet. Ausbeute: 420 mg (97 %).
MS (FAB, 3-NBA, LiCl) : 438 (M +$Li^+$)

Beispiel 715

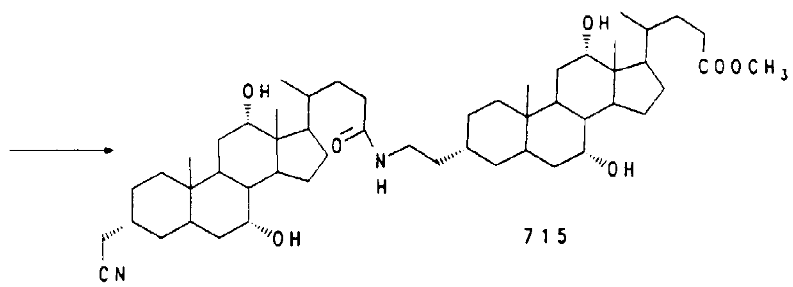

714 + 686 A

715

449 mg (1 mmol) 686 A wurden in 30 ml trockenen THF gelöst und mit 0,14 ml Triethylamin und 1,1 mol EEDQ versetzt. Dazu gibt man 432 mg (1 mmol) 714 und erwärmt 6 h zum Rückfluß. Nach beendeter Reaktion wird eingeengt, mit Essigester aufgenommen und mit $KHSO_4$-Lösung und Wasser gewaschen. Der Rückstand der organischen Phase wird chromatographisch gereinigt. Man erhält 587 mg (68 %) 715.
MS (FAB, 3-NBA, LiCl) : 870 (M+$Li^+$)

Beispiel 716a

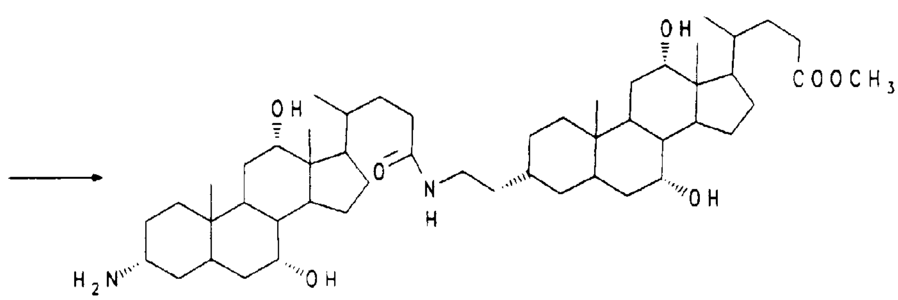

715

716

2 g (2,3 mol) 715 werden in 50 ml Methanol und 5 ml konzentrierter $NH_3$-Lösung mit 0,3 g 5 % Rhodium auf $Al_2O_3$ bei 20 bar und Raumtemperatur 24 h hydriert. Man saugt vom Katalysator ab, engt ein und reinigt den Rückstand chromatographisch. Ausbeute 1,5 g (75 %) 716.
MS (FAB, 3-NBA, LiCl) : 874 (M+$Li^+$)

Beispiel 716b

688 → 716

484 mg (0,5 mmol) 688 wurden zu einer unter Kühlung hergestellten Mischung aus 25 ml Methanol in 1,5 ml Acetylchlorid gegeben und bei Raumtemperatur 2 h gerührt (Reaktionskontrolle durch Dünnschichtchromatographie (DC)). Nach beendeter Reaktion wird mit konzentrierter $NH_3$-Lösung neutralisiert, im Vakuum eingeengt und der Rückstand chromatographisch ($SiO_2$) gereinigt. Man erhält 247 mg (58 %) 716, nach DC und MS identisch mit der nach a) hergestellten Substanz.

Beispiel 717

716

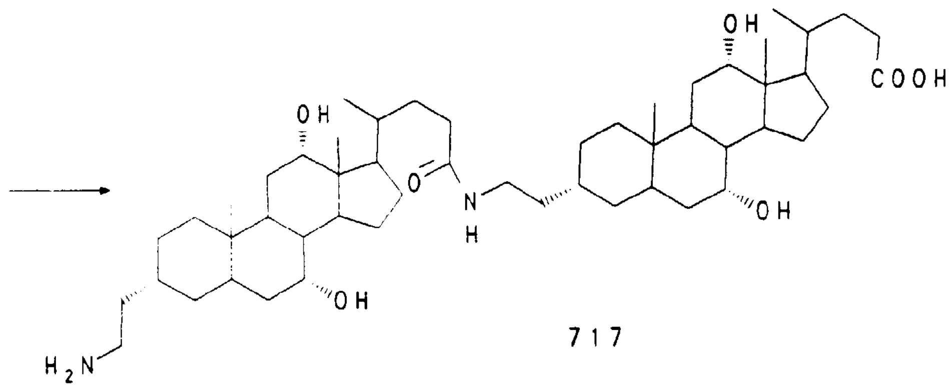

717

1,2 g (1,38 mmol) 716 werden in 20 ml Methanol gelöst und mit 2 ml halbkonzentrierter Natronlauge durch Rühren über Nacht verseift. Man verdünnt mit Wasser, entfernt Methanol im Vakuum und fällt die Aminosäure 717 durch vorsichtiges Ansäuern aus. Der Niederschlag wird abgesaugt, mit Wasser nachgewaschen und getrocknet. Ausbeute 1,1 g (93 %).

MS (FAB, 3-NBA, LiCl) : 860 ($M+Li^+$).

Analog zur Reaktionsfolge (714 A) → 714 → 715 → 716 → 717 werden folgende Beispielsubstanzen hergestellt (718 - 725).

G1 - NH - G2

| Bsp. | G1 G2 | Edukte | MS (FAB, 3-NBA, LiCl oder LiJ) |
|---|---|---|---|
| 718 | 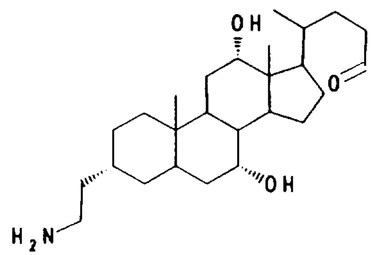 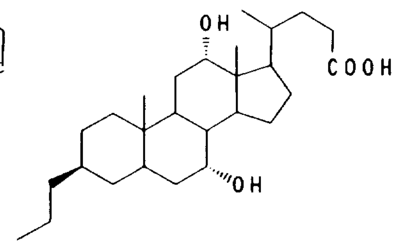 | 714 + 686 B | 860 (M+Li$^+$) |
| 719 | 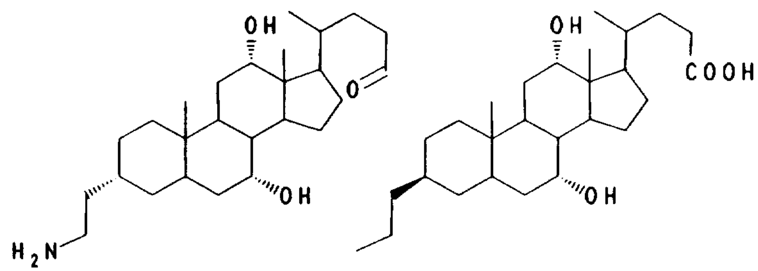 | 681 + 686 A | 844 (M+Li$^+$) |

| Bsp. | G1 G2 | Edukte | MS (FAB, 3-NBA, LiCl oder LiJ) |
|---|---|---|---|
| 720 | 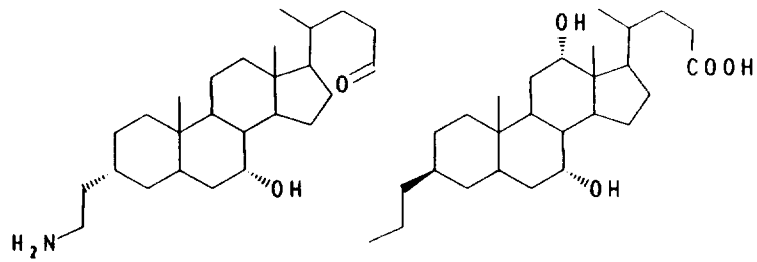 | 680 + 686 B | 844 $(M+Li^+)$ |
| 721 | 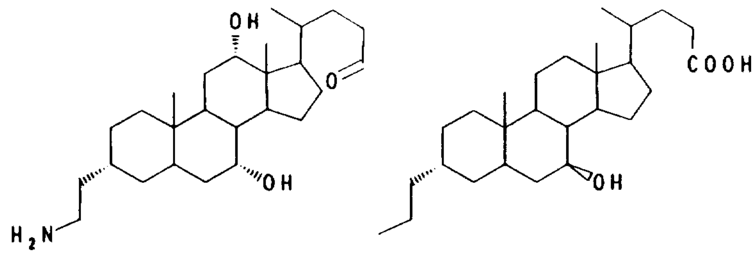 | 714 + 683 | 844 $(M+Li^+)$ |
| 722 | 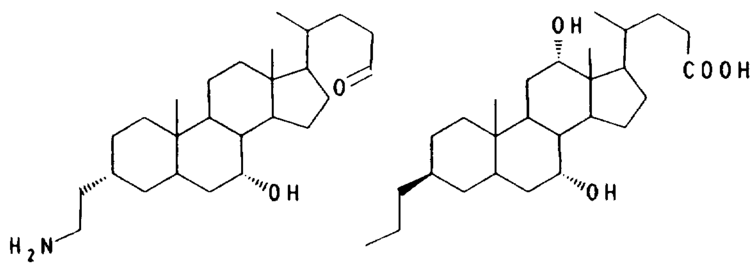 | 714 + 682 | 844 $(M+Li^+)$ |

| Bsp. | G1 G2 | Edukte | MS (FAB, 3-NBA, LiCl oder LiJ) |
|---|---|---|---|
| 723 | 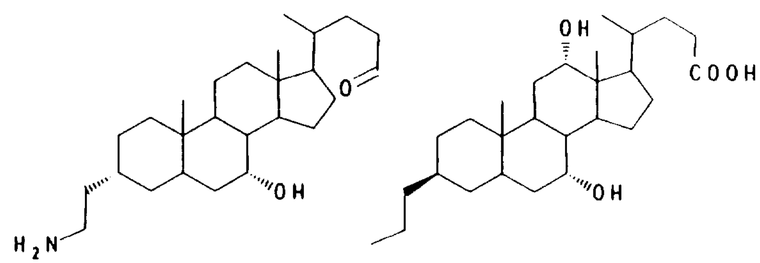 | 681 + 683 | 828 (M+Li$^+$) |

Beispiel 724

715

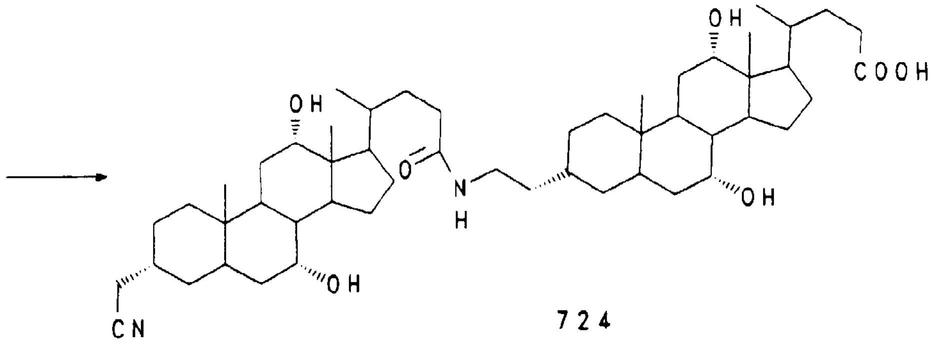

724

432 mg (0,5 mmol) 715 werden in 15 ml Methanol mit 1,5 ml 12 N NaOH wie unter 717 beschrieben verseift. Ausbeute 318 mg (75 %) 724.

MS (FAB, 3-NBA, LiCl) : 856 ($M+Li^+$).

Analog zur Reaktionsfolge 714 → 715 → 724 werden folgende Beispiel Substanzen hergestellt (725 - 729):

G1 - NH - G2

| Bsp. | G1 | G2 | Redukte | MS (FAB, 3-NBA LiCl) |
|---|---|---|---|---|
| 725 | 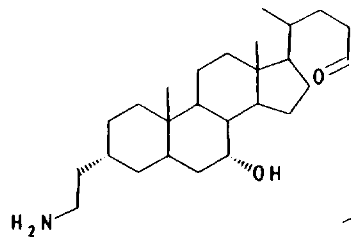 | 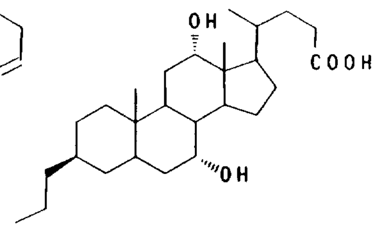 | 714 + 686 B | 856 $(M+Li^{+})$ |
| 726 | 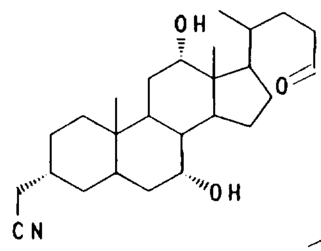 | 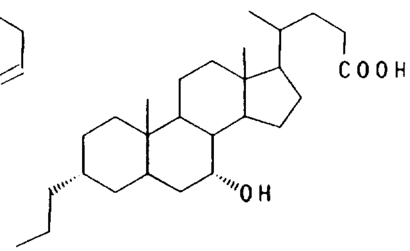 | 714 + 682 | 840 $(M+Li^{+})$ |

| Bsp. | G1 G2 | Redukte | MS (FAB, 3-NBA LiCl) |
|---|---|---|---|
| 727 | 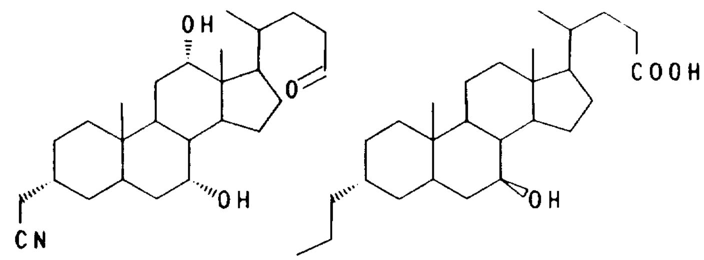 | 714 + 683 | 840 (M+Li$^+$) |
| 728 | 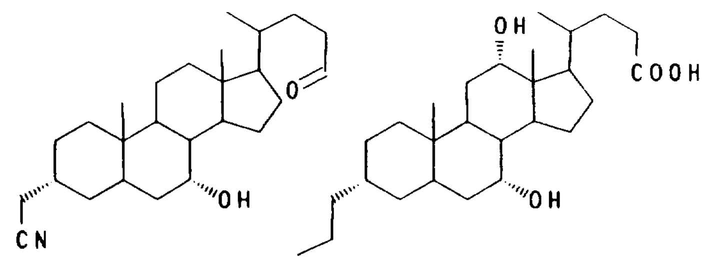 | 680 + 686 A | 840 (M+Li$^+$) |
| 729 | 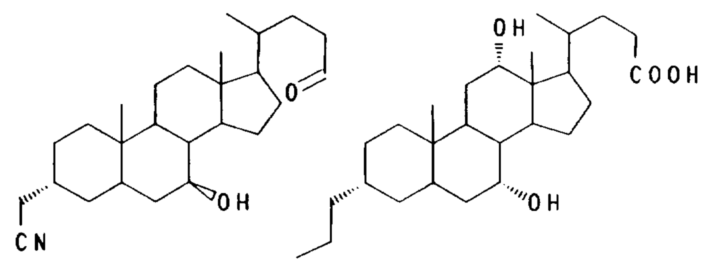 | 681 + 686 A | 840 (M+Li$^+$) |

Beispiel 730

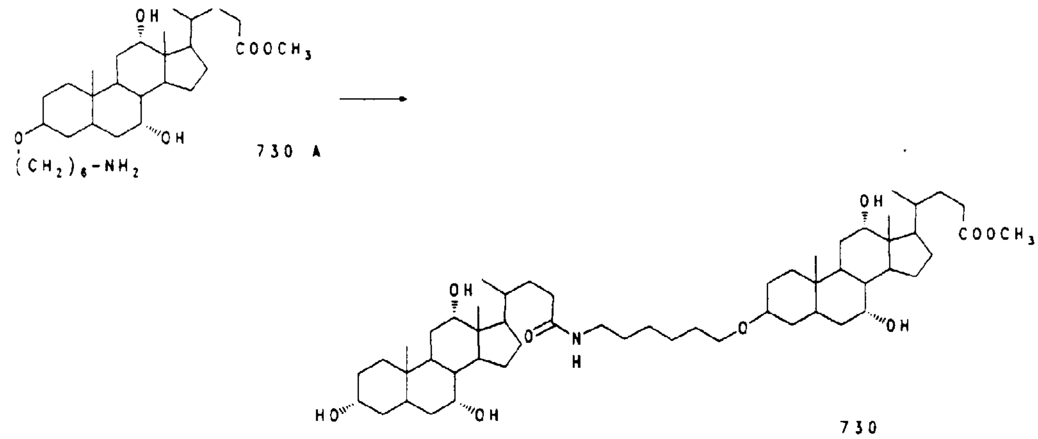

730 A

730

2,1 g (4 mmol) 730 A werden in 50 ml trockenem THF mit 0,6 ml Triethylamin, 1,1 g (4,4 mmol) EEDQ und 1,64 g (4 mmol) Cholsäure 8 h zum Rückfluß erwärmt. Nach beendeter Reaktion wird eingeengt, mit Essigester aufgenommen, die Lösung mit $KHSO_4$-Lösung und Wasser gewaschen und der Rückstand der organischen Phase chromatographisch gereinigt. Ausbeute 2,48 g (68 %) 730.
MS (FAB, 3-NBA, LiCl) : 919 ($M+H^+$)

Beispiel 731

730

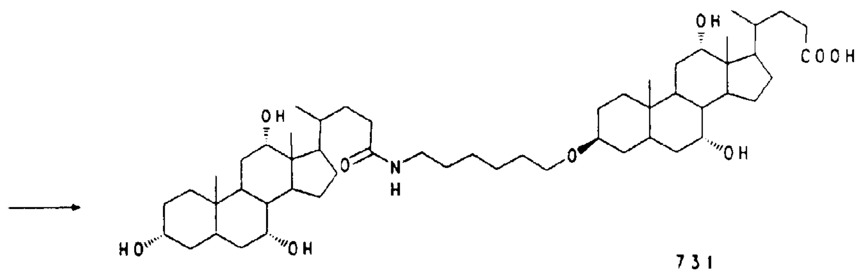

731

2 g (2,2 mmol) 730 werden in 30 ml Methanol mit 3 ml 2 N NaOH wie unter 724 beschrieben verseift. Ausbeute: 168 g (85 %). 731
MS (FAB, 3-NBA, LiCl) : 905 ($M+Li^+$)

Analog zur Reaktionsfolge 730 A → 730 → 731 werden folgende Beispielsubstanzen hergestellt (732 - 734).

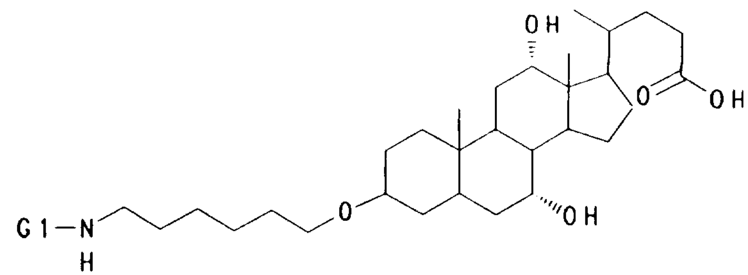

| Bsp. | G1 | Edukte | MS (FAB, 3-NBA, LiCl) |
|---|---|---|---|
| 732 | 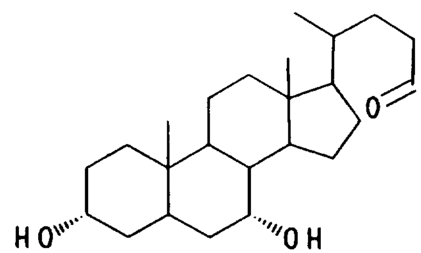 | 730 A + Cheno-desoxycholsäure | 889 ($M+Li^+$) |
| 733 | 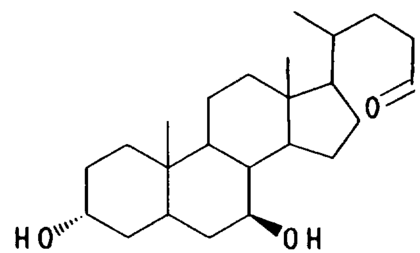 | 730 A + Urso-desoxycholsäure | 889 ($M+Li^+$) |

| Bsp. | G1 | Edukte | MS (FAB, 3-NBA, LiCl) |
|---|---|---|---|
| 734 | 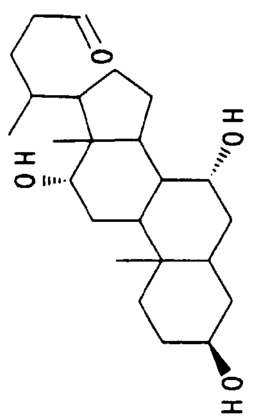 | 730 A + 3ß-OH-Cholsäure | 905 (M+Li$^+$) |

Beispiel 735

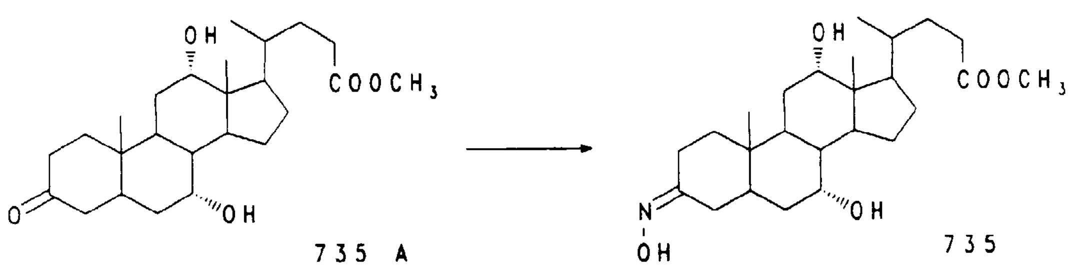

735 A

735

3,1 g (7,4 mmol) 735 A werden in 30 ml Isopropanol unter Rückfluß mit einer Lösung von 2,33 g Hydroxylaminhydrochlorid und 4,57 g Natriumacetat in 10 ml Wasser versetzt und 4 h zum Rückfluß erwärmt. Nach beendeter Reaktion wird mit Wasser versetzt, Isopropanol im Vakuum teilweise entfernt und mit viel Dichlormethan ausgeschüttelt. Der Rückstand der organischen Phase wird chromatographisch gereinigt. Ausbeute: 2,7 g (84 %) 735.
MS (FAB, 3-NBA, LiCl) : 442 (M + Li+), 436 (M +H+).

Beispiel 736

735

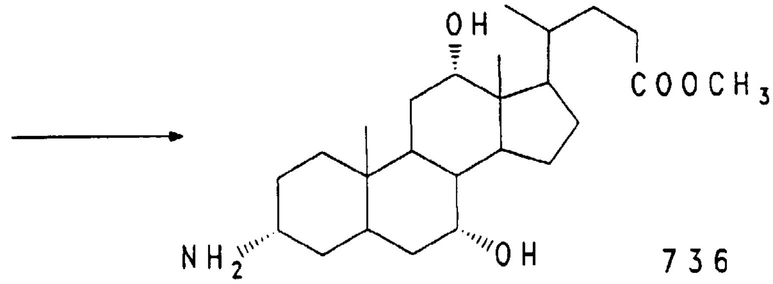

736

2 g (4,6 mmol) 735 werden in 50 ml Methanol mit 200 mg 10 % Palladium/Kohle bei 20 bar $H_2$ und Raumtemperatur 24 h hydriert. Man trennt den Katalysator ab, engt ein und reinigt den Rückstand chromatographisch. Ausbeute 1,56 g (80 %) 736.
MS 8FAB, 3-NBA, LiCl) : 428 (M + Li+), 422 (M + H+).
736 ist nach DC ($SiO_2$; Dichlormethan/Methanol/konz. $NH_3$-Lösung = 100:15:5) nicht identisch mit dem 3β-Isomeren Amin.

Beispiel 737

736

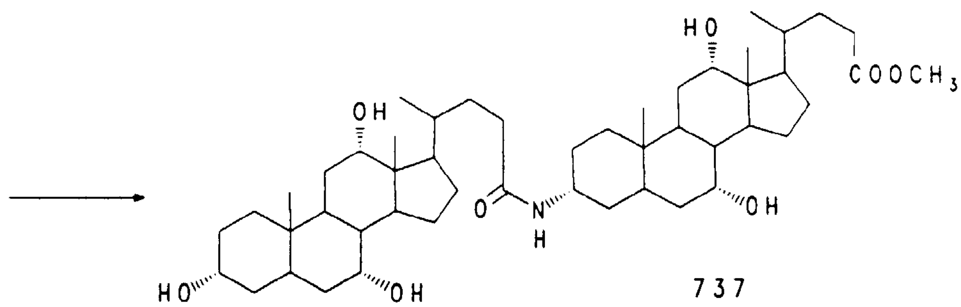

737

842 mg (2 mmol) werden in 50 ml trockenen THF mit 0,15 ml Triethylamin, 2,2 mmol EEDQ und 820 mg (2 mmol) Cholsäure 6 h zum Rückfluß erhitzt. Man engt ein, nimmt mit Essigester auf und schüttelt mit $KHSO_4$-Lösung und Wasser aus und reinigt den Rückstand der organischen Phase chromatographisch. Ausbeute 926 mg (57 %).
MS (FAB, 3-NBA, LiCl) : 819 (M+Li+)

Beispiel 738

737 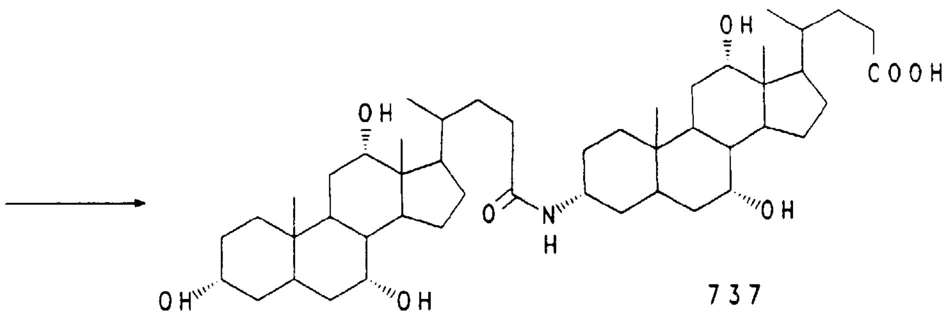

737

200 mg (0,25 mmol) 737 werden in 15 ml Methanol mit 1,5 ml halbkonzentrierter NaOH wie unter 724 beschrieben verseift. Ausbeute: 162 mg (82 %) 738.

MS (FAB, 3-NBA) : 798,6 ($M+H^+$)

Analog zur Reaktionsfolge 735 A → 735 → 736 → 737 → 738 werden folgende Beispielsubstanzen hergestellt.

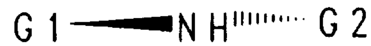

| Bsp. | G1 | G2 | Edukte | MS (FAB, 3-NBA) |
|---|---|---|---|---|
| 739 | 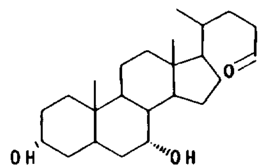 | 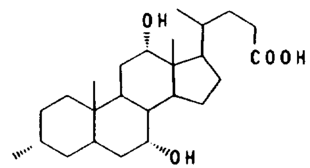 | 736 + Cheno-desoxychol-säure | 783 (M+H$^+$) |
| 740 | 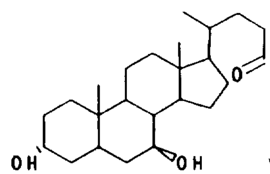 | 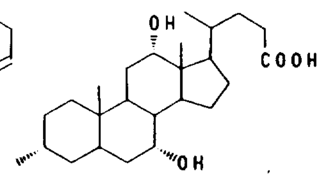 | 736 + Urso-desoxychol-säure | 783 (M+H$^+$) |
| 741 | 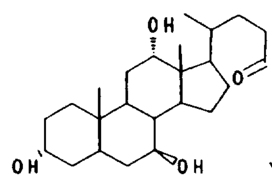 | 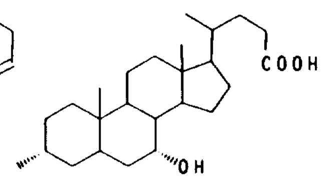 | 678 A + Cholsäure | 783 (M+H$^+$) |

| Bsp. | G1 | G2 | Edukte | MS (FAB, 3-NBA) |
|---|---|---|---|---|
| 742 | 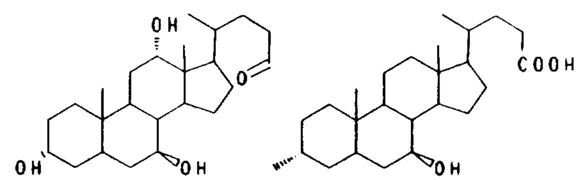 | | 679 A + Cholsäure | 783 (M+H⁺) |

Beispiel 743

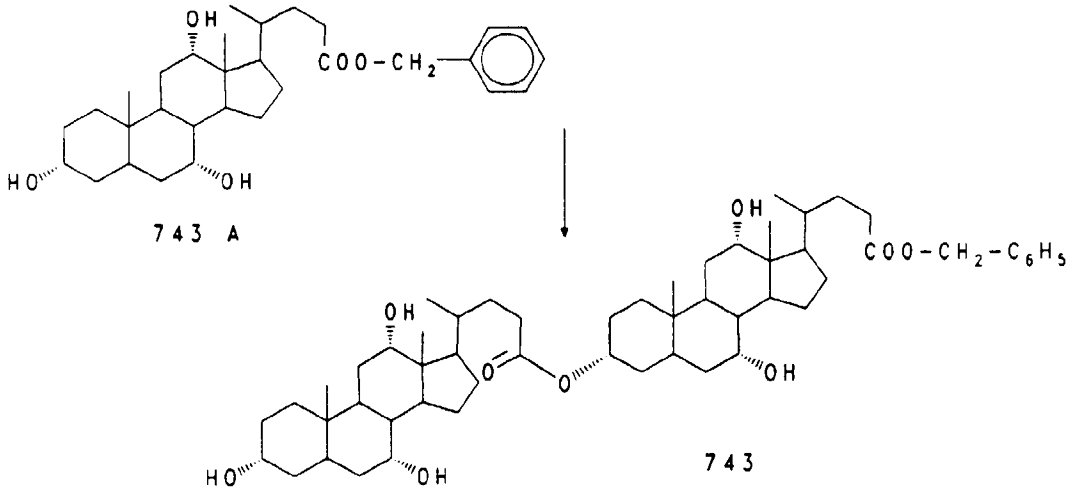

743 A

743

1 g Cholsäure (2,45 mmol) werden in 15 ml trockenem THF bei 0°C mit 4 Äquivalenten Trifluoressigsäureanhydrid versetzt und 2 h bei Raumtemperatur gerührt. Unter Eiskühlung gibt man 1,04 g (2,01 mmol) Cholsäurebenzylester in THF hinzu und rührt bei Raumtemperatur über Nacht. Unter Eiskühlung gibt man ca. 2,5 ml konzentrierter $NH_3$-Lösung zu und rührt mehrere h unter DC-Kontrolle. Nach beendeter Reaktion wird weitgehend in der Kälte eingeengt und der Rückstand zwischen viel Ether und $NaHCO_3$-Lösung verteilt. Die organische Phase wird mit $NaHCO_3$-Lösung und Wasser gewaschen, eingeengt und der Rückstand chromatographisch gereinigt. Ausbeute 1,14 g (64 %) 743
MS (FAB, 3-NBA, LiCl) : 896 ($M+H^+$)

Beispiel 744

743

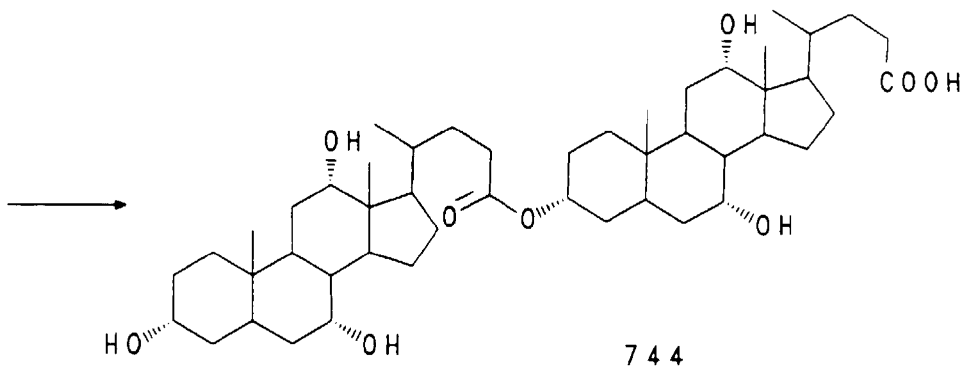

744

1 g (1,12 mmol) 743 werden in 10 ml THF mit 200 mg 10 % Palladium auf Kohle in der Schüttelente bei Raumtemperatur hydriert. Nach beendeter Reaktion trennt man den Katalysator ab, engt ein und reinigt den Rückstand chromatographisch.
Ausbeute: 828 mg (92 %) 744.
MS (FAB, 3-NBA, LiCl) : 806 ($M+Li^+$)

Analog zur Reaktionsfolge 743 A → 743 → 744 wurden folgende Beispielsubstanzen hergestellt (745 - 747):

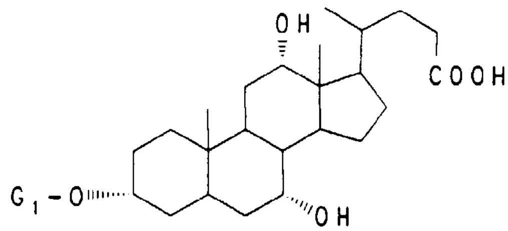

| Bsp. | G1 | Edukt | MS (FAB, 3-NBA, LiCl) |
|---|---|---|---|
| 745 | 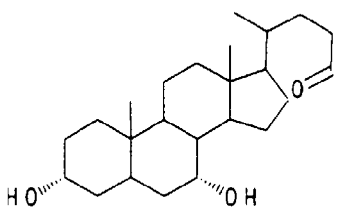 | 743 A + Chenodesoxy-cholsäure | |

| Bsp. | G1 | Edukt | MS (FAB, 3-NBA, LiCl) |
|---|---|---|---|
| 746 | 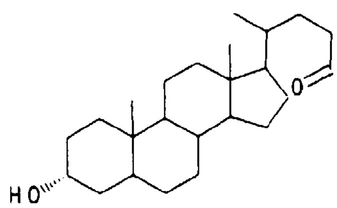 | 743 A + Lithochol-säure | |

Beispiel 747

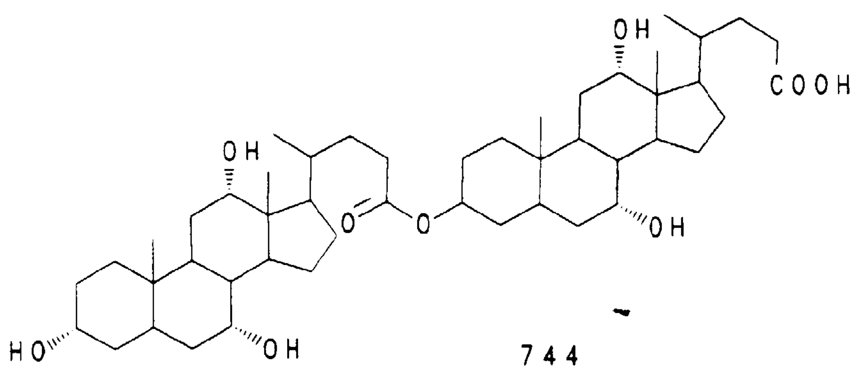

744

MS (FAB, 3-NBA, LiCl) : 806 (M+Li$^+$)
aus 3-β-OH-Cholsäurebenzylester + Cholsäure

Beispiel 748

707

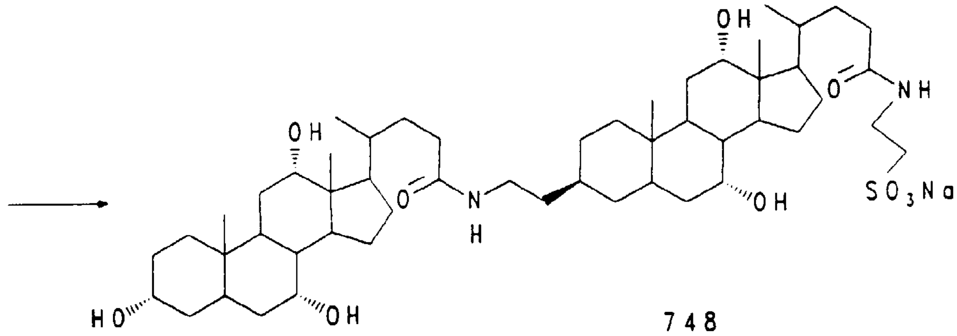

748

826 mg (1 mmol) 707 und 1,4 mmol EEDQ wurden in 8 ml trockenem, gereinigtem Dimethylformamid gelöst und mit 0,18 ml Triethylamin und 140 mg (1 mmol) Taurin versetzt. Die Lösung wird 15 min auf 90°C erwärmt. Nach Abkühlung gibt man 40 ml trockenen Ether zu. Die ProdukfläIlung wird durch Stehen über Nacht im Kühlschrank komplettiert. Die Lösung wird dekantiert, der Niederschlag mit Ether gewaschen, abgesaugt und luftgetrocknet. Das Produkt wird in 5 ml 0,2 N methanolische NaOH gelöst, die Lösung mit 40 ml trockenem Ether versetzt und 1 h unter Eiskühlung gerührt. Der gebildete Niederschlag wird abgesaugt und im Exsikkator getrocknet. Weitere Reinigung erfolgt durch Reversed Phase-Chromatographie. Ausbeute 806 mg (84 %) 748

MS (FAB, 3-NBA) : 956 ($M+H^+$)

Analog zur Reaktionsfolge 707 → 748 folgende Beispielsubstanzen hergestellt.

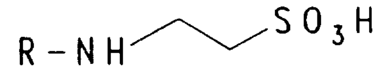

| Bsp. | R | Edukt | MS (FAB, 3-NBA) |
|---|---|---|---|
| 749 | 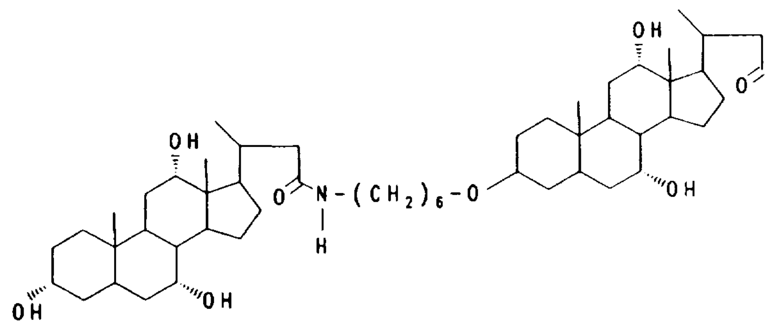 | 731 | 1028 $(M+H^+)$ |
| 750 | 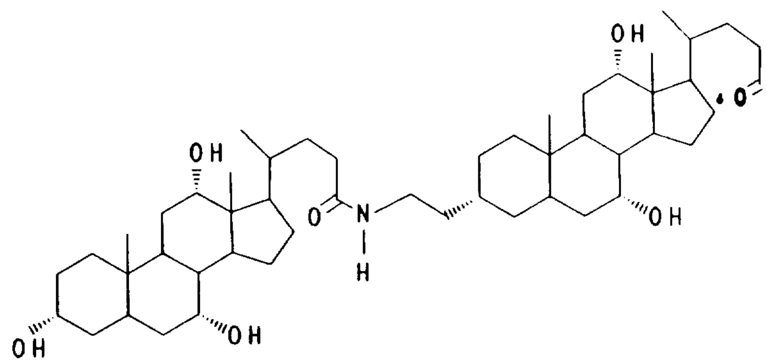 | 697 | 956 $(M+H^+)$ |

| Bsp. | R | Edukt | MS (FAB, 3-NBA) |
|---|---|---|---|
| 751 | 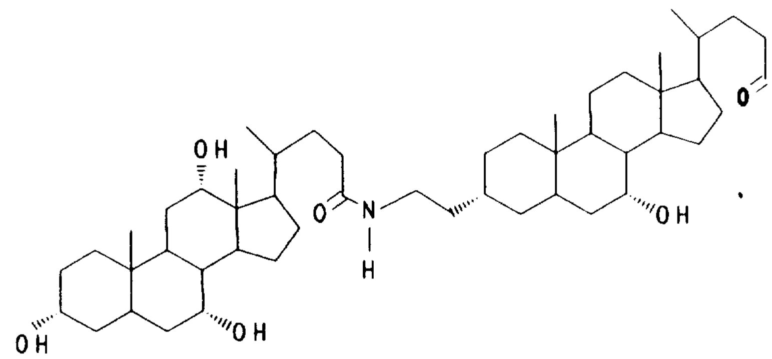 | 702 | 940 $(M+H^+)$ |
| 752 | 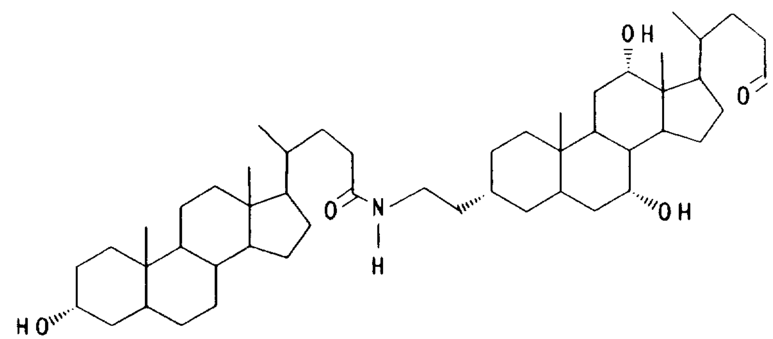 | 701 | 924 $(M+H^+)$ |

| Bsp. | R | Edukt | MS (FAB, 3-NBA) |
|---|---|---|---|
| 753 | 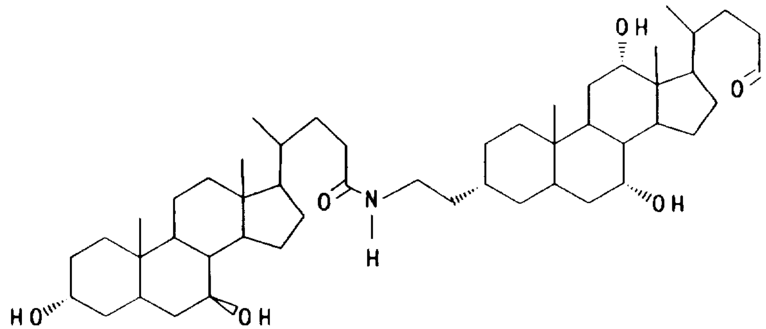 | 700 | 940 $(M+H^+)$ |
| 754 | 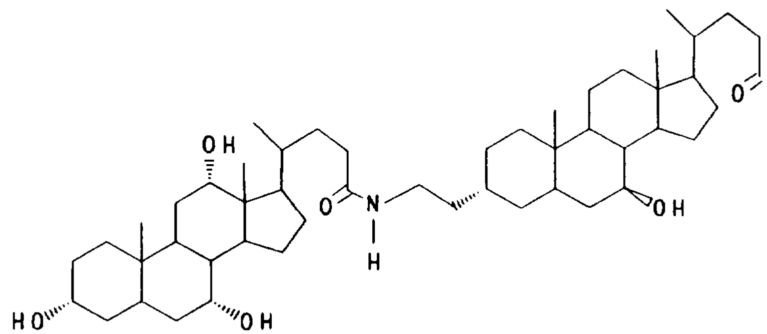 | 703 | 940 $(M+H^+)$ |

| Bsp. | R | Edukt | MS (FAB, 3-NBA) |
|---|---|---|---|
| 755 | 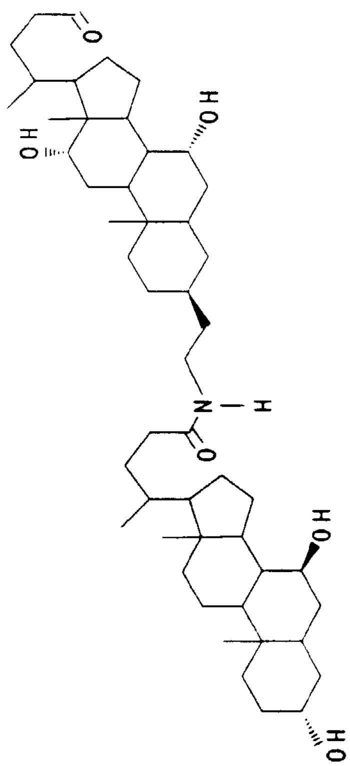 | 708 | 940 $(M+H^+)$ |

**Patentansprüche**

**Patentansprüche für folgende Vertragsstaaten : AT, BE, CH, LI, DE, DK, FR, GB, IT, LU, NL, SE**

**1.** Dimere Gallensäurederivate der Formel I

G1-X-G2 (I)

in der G1 eine Verbindung der allgemeinen Formel II

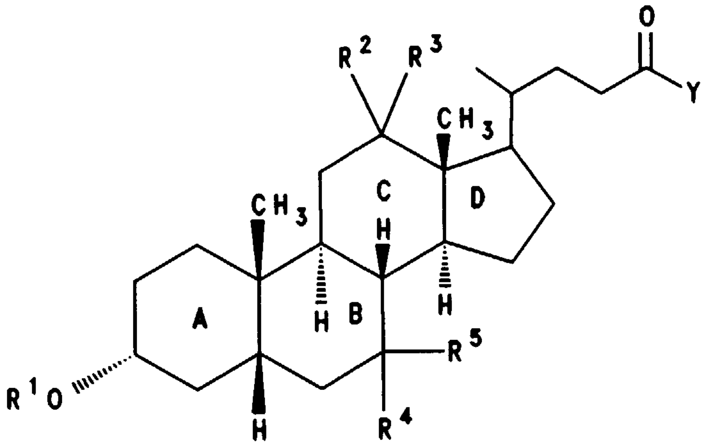

bedeutet, worin

Y eine freie Valenz zur Bindung der Gruppe X oder die folgenden Bedeutungen hat -OL, -NHL, $-NL_2$, eine über die Aminogruppe gebundene Aminosäure oder Aminosulfonsäure, wie z.B.

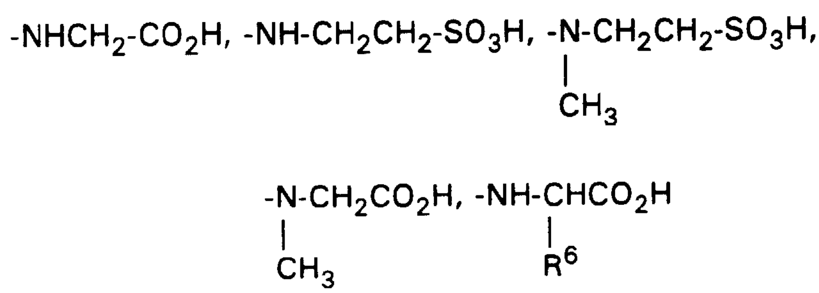

und deren $(C_1-C_4)$-Alkylester und Alkali- und Erdalkalisalze, -OKa, wobei Ka ein Kation bedeutet wie z.B. ein Alkali oder Erdalkaliion oder auch ein quartäres Ammonium und
wobei L

H, einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, $(C_1-C_4)$-Alkyl oder $(C_1-C_4)$-Alkoxy,
einen Benzylrest, der unsubstituiert oder 1-3fach substituiert ist mit F, Cl, Br, $(C_1-C_4)$-Alkyl oder $(C_1-C_4)$-Alkoxy,
und $R^6$
Methyl, Isopropyl, Isobutyl, 2-Butyl, Benzyl, 4-Hydroxybenzyl,
Hydroxymethyl, 1-Hydroxyethyl, $H_3CSCH_2CH_2$-, $HO_2CCH_2$-, $HO_2CCH_2CH_2$-

bedeutet,

$R^1$ eine freie Valenz zur Bindung der Gruppe X oder H,

einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen Benzylrest, der im Kern unsubstituiert oder 1-3fach substituiert ist mit F, Cl, Br, ($C_1$-$C_4$)-Alkyl, ($C_1$-$C_4$)-Alkoxy, -$N^{\oplus}H_3$, -$OPO_3^{\ominus}$ oder Phenyl, das seinerseits 1-3fach substituiert sein kann mit F, Cl, Br, ($C_1$-$C_4$)-Alkyl oder ($C_1$-$C_4$)-Alkoxy, -$N^{\oplus}H_3$, -$OPO_3^{\ominus}$,
einen Biphenylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen Triphenylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen 1- oder 2-Naphthylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen 9-Fluorenylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen 2-, 3- oder 4-Pyridylrest,
einen

Rest 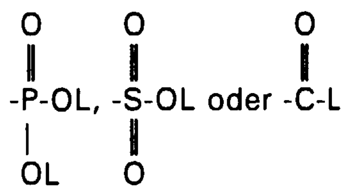

wobei L die oben angegebene Bedeutung hat

$R^2$ bis $R^5$, wobei $R^2$ und $R^3$ bzw. $R^4$ und $R^5$ jeweils gemeinsam der Sauerstoff einer Carbonylgruppe, oder einzeln und jeweils unabhängig voneinander

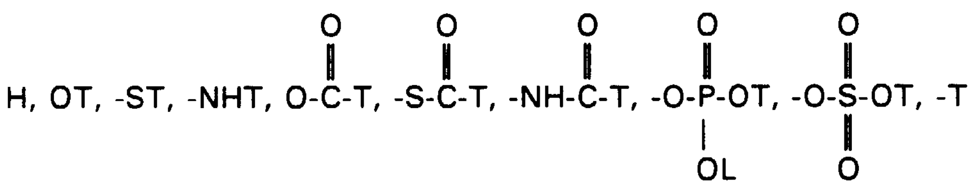

wobei L die obengenannte Bedeutung hat und T die Bedeutung von L aufweist oder eine freie Valenz zur Bindung der Gruppe X,

mit der Beschränkung, daß insgesamt nur eine freie Valenz zur Bindung der Gruppe X ausgeht,

X eine Einfachbindung oder eine Gruppe der allgemeinen Formel III

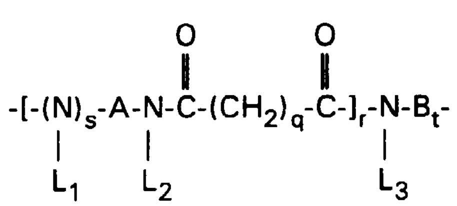 (III)

wobei

A eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

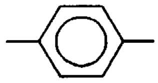 , 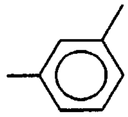 oder 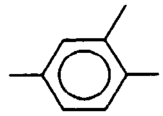

erfolgt und die Kette insgesamt 2 bis 12, vorzugsweise 2 bis 6 Kettenglieder p umfaßt.

B eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

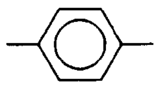 , 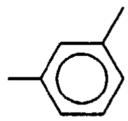 oder 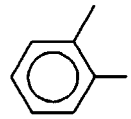

erfolgt und

die Kette insgesamt 2 bis 18, vorzugsweise 2 bis 12 Kettenglieder n enthält,

| | |
|---|---|
| $L_1$, $L_2$, $L_3$ | gleich oder verschieden sind und die Bedeutung von L haben, sowie |
| q | 0 bis 5, |
| r | 0 oder 1 und |
| s | 0 oder 1 |
| t | 0 oder 1 |

bedeuten,

G2 eine Verbindung der allgemeinen Formel IV bedeutet

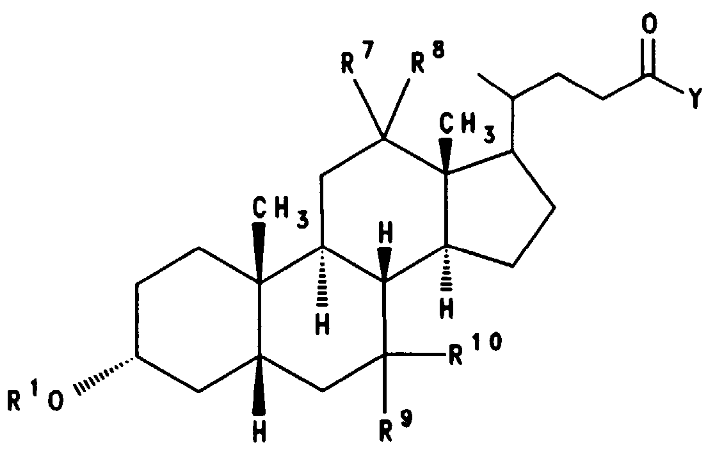

wobei

| | |
|---|---|
| Y und $R^1$ | die unter G1 angegebene Bedeutung haben sowie |
| $R^7$ bis $R^{10}$, | wobei $R^7$ und $R^8$ bzw. $R^9$ und $R^{10}$ jeweils gemeinsam der Sauerstoff einer Carbonylgruppe oder einzeln und jeweils unabhängig voneinander H, |

OT, -ST, -NHT,

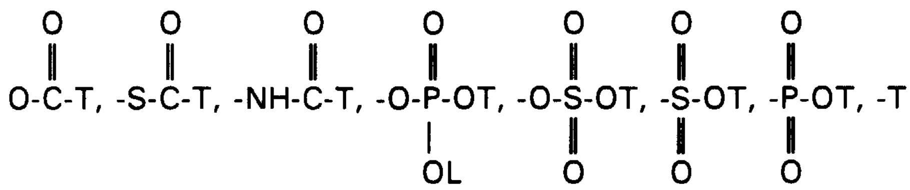

bedeutet, wobei L und T die unter G1 angegebene Bedeutung haben, ebenfalls mit der Beschränkung, daß insgesamt von G2 nur eine freie Valenz zur Bindung der Gruppe X ausgeht, wobei Gallensäurederivate der folgenden Formeln ausgeschlossen sind:

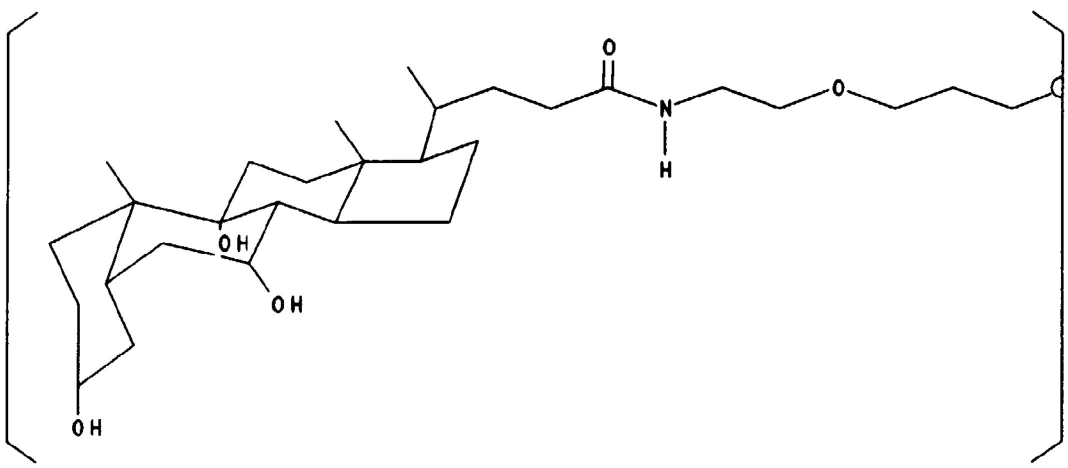

oder

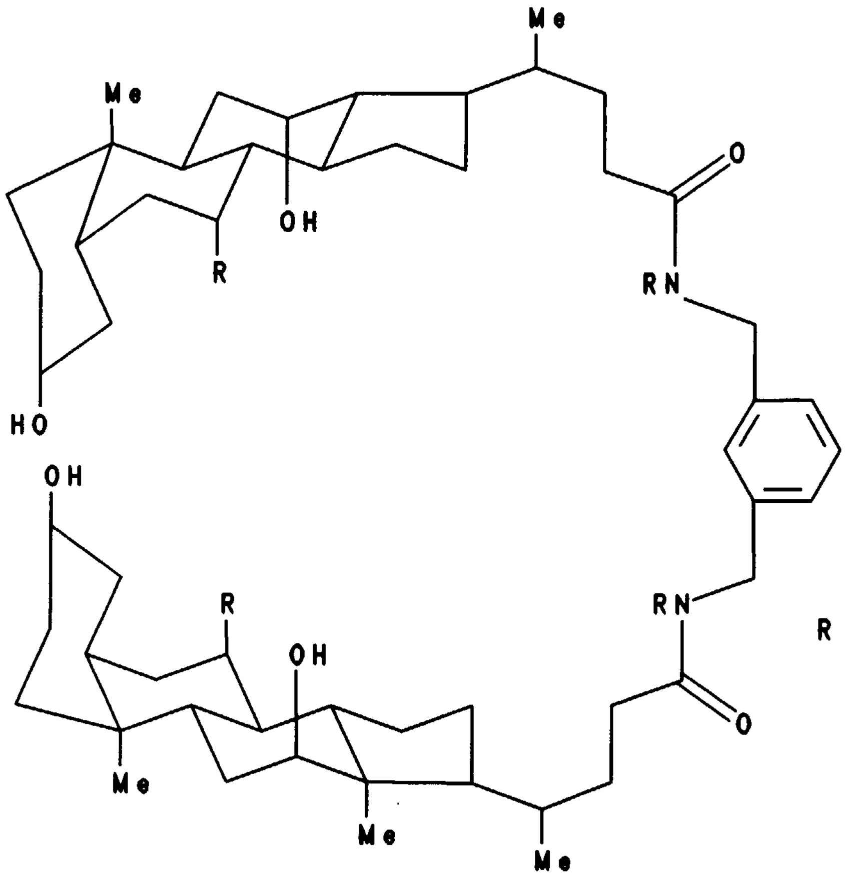

R = H oder OH

2. Verbindung nach Anspruch 1, dadurch gekennzeichnet, daß die Verknüpfung der Reste G1 und G2 nicht symmetrisch, d.h. nicht über die gleichen Ringe erfolgt.

3. Verbindung nach den Ansprüchen 1 bis 2, dadurch gekennzeichnet, daß die Verknüpfung des Restes G1 durch C-24 (Ring D) über X an eine der Positionen C-3 (Ring A), C-7 (Ring B) oder C-12 (Ring G) des Restes G2 erfolgt.

4. Verfahren zur Herstellung einer Verbindung gemäß einem oder mehreren der Ansprüche 1 bis 3, dadurch gekennzeichnet, daß man

a) im Falle von X = Einfachbindung geeignete reaktionsfähige Formen von G1 und G2 nach im Prinzip bekannten Verfahren miteinander zur Reaktion bringt oder

b) im Falle von X = Brückengruppe

α) reaktionsfähige Formen von G1-X mt G2 bzw.
β) reaktionsfähige Formen von G2-X mit G1

nach im Prinzip bekannten Verfahren miteinander zur Reaktion bringt oder

c) aus G1-X1 nach bekannten Verfahren Verbindungen der allgemeinen Formel I (G1-X-G2) herstellt, wobei X aus X1 und X2 entsteht durch Ausbildung einer kovalenten Bindung, insbesondere innerhalb einer Kondensations- oder Substitutionsreaktion.

5. Verwendung der Verbindung gemäß den Ansprüchen 1 bis 3 zur Herstellung eines Heilmittels.

6. Verbindung gemäß den Ansprüchen 1 bis 3 als Heilmittel.

**7.** Pharmazeutische Zubereitung, enthaltend Verbindungen gemäß den Ansprüchen 1 bis 3.

**Patentansprüche für folgende Vertragsstaaten : ES, GR**

**1.** Verfahren zur Herstellung von dimeren Gallensäurederivaten der Formel I

$$G1\text{-}X\text{-}G2 \qquad (I)$$

in der G1 eine Verbindung der allgemeinen Formel II

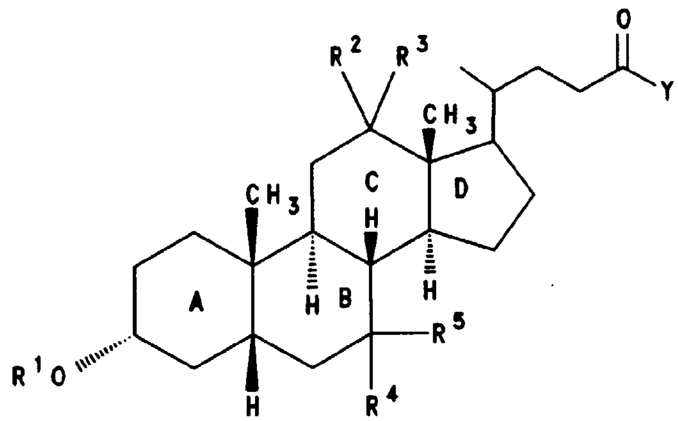

bedeutet, worin

Y eine freie Valenz zur Bindung der Gruppe X oder die folgenden Bedeutungen hat -OL, -NHL, $-NL_2$, eine über die Aminogruppe gebundene Aminosäure oder Aminosulfonsäure, wie z.B.

$-NHCH_2-CO_2H$, $-NH-CH_2CH_2-SO_3H$, $-N-CH_2CH_2-SO_3H$,
|
$CH_3$

$-N-CH_2CO_2H$, $-NH-CHCO_2H$
| |
$CH_3$ $R^6$

und deren $(C_1\text{-}C_4)$-Alkylester und Alkali- und Erdalkalisalze, -OKa, wobei Ka ein Kation bedeutet wie z.B. ein Alkali oder Erdalkaliion oder auch ein quartäres Ammonium und wobei L

H, einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, $(C_1\text{-}C_4)$-Alkyl oder $(C_1\text{-}C_4)$-Alkoxy,
einen Benzylrest, der unsubstituiert oder 1-3fach substituiert ist mit F, Cl, Br, $(C_1\text{-}C_4)$-Alkyl oder $(C_1\text{-}C_4)$-Alkoxy,
und $R^6$
Methyl, Isopropyl, Isobutyl, 2-Butyl, Benzyl, 4-Hydroxybenzyl, Hydroxymethyl, 1-Hydroxyethyl,

$H_3CSCH_2CH_2$-, $HO_2CCH_2$-, $HO_2CCH_2CH_2$-

bedeutet,

$R^1$ eine freie Valenz zur Bindung der Gruppe X oder H,

einen gesättigten oder ungesättigten Alkylrest mit 1 bis 10 C-Atomen, der verzweigt oder unverzweigt ist,
einen Cycloalkylrest mit 3 bis 8 C-Atomen,
einen Phenylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen Benzylrest, der im Kern unsubstituiert oder 1-3fach substituiert ist mit F, Cl, Br, ($C_1$-$C_4$)-Alkyl, ($C_1$-$C_4$)-Alkoxy, -$N^{\oplus}H_3$, -$OPO_3^{\ominus}$ oder Phenyl, das seinerseits 1-3fach substituiert sein kann mit F, Cl, Br, ($C_1$-$C_4$)-Alkyl oder ($C_1$-$C_4$)-Alkoxy, -$N^{\oplus}H_3$, -$OPO_3^{\ominus}$,
einen Biphenylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$, einen Triphenylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$, einen 1- oder 2-Naphthylmethylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen 9-Fluorenylrest, der unsubstituiert oder 1-3fach substituiert ist mit F, Cl, Br, ($C_1$-$C_4$)-Alkyl oder ($C_1$-$C_4$)-Alkoxy, -$N^{\oplus}H_3$, -$OPO_3^{\ominus}$,
einen 2-, 3- oder 4-Pyridylrest,
einen

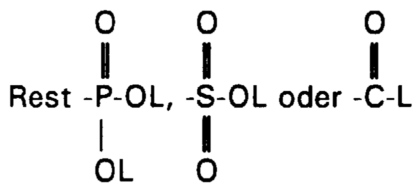

wobei L die oben angegebene Bedeutung hat

$R^2$ bis $R^5$, wobei $R^2$ und $R^3$ bzw. $R^4$ und $R^5$ jeweils gemeinsam der Sauerstoff einer Carbonylgruppe, oder einzeln und jeweils unabhängig voneinander

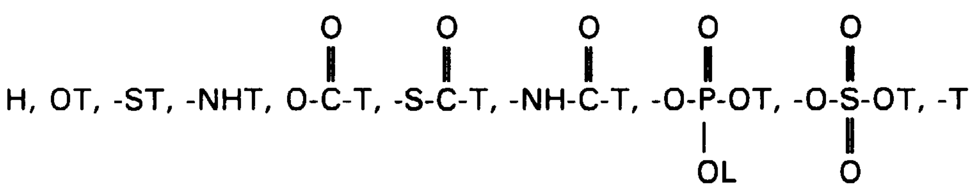

wobei L die obengenannte Bedeutung hat und T die Bedeutung von L aufweist oder eine freie Valenz zur Bindung der Gruppe X,

mit der Beschränkung, daß insgesamt nur eine freie Valenz zur Bindung der Gruppe X ausgeht,

X eine Einfachbindung oder eine Gruppe der allgemeinen Formel III

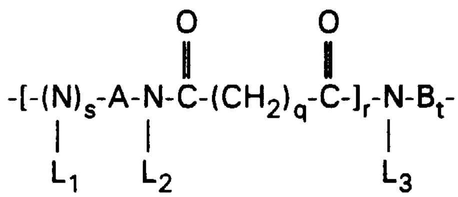 (III)

wobei

A eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

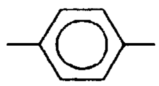 , 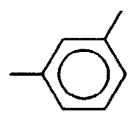 oder 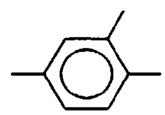

erfolgt und die Kette insgesamt 2 bis 12, vorzugsweise 2 bis 6 Kettenglieder p umfaßt.

B eine Alkylenkette, die verzweigt oder unverzweigt, gesättigt oder ungesättigt ist und in der Kette ggf. durch -O-, -S- oder Arylen, insbesondere Phenylen unterbrochen sein kann, wobei die Verknüpfung

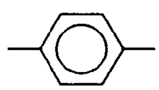 , 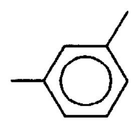 oder 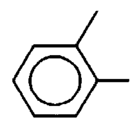

erfolgt und

die Kette insgesamt 2 bis 18, vorzugsweise 2 bis 12 Kettenglieder n enthält,

$L_1$, $L_2$, $L_3$ gleich oder verschieden sind und die Bedeutung von L haben, sowie

q 0 bis 5,

r 0 oder 1 und

s 0 oder 1

t 0 oder 1

bedeuten,

G2 eine Verbindung der allgemeinen Formel IV bedeutet

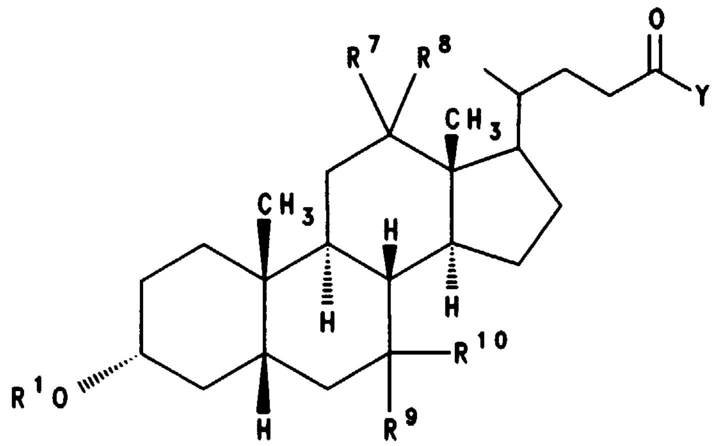

wobei

Y und $R^1$ die unter G1 angegebene Bedeutung haben sowie

$R^7$ bis $R^{10}$, wobei $R^7$ und $R^8$ bzw. $R^9$ und $R^{10}$ jeweils gemeinsam der Sauerstoff einer Carbonylgruppe oder einzeln und jeweils unabhängig voneinander H,

OT, -ST, -NHT,

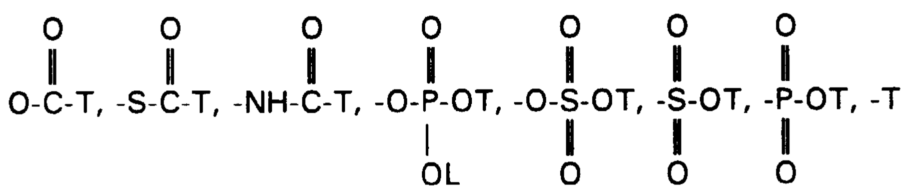

bedeutet, wobei L und T die unter G1 angegebene Bedeutung haben, ebenfalls mit der Beschränkung, daß insgesamt von G2 nur eine freie Valenz zur Bindung der Gruppe X ausgeht, wobei Gallensäurederivate der folgenden Formeln ausgeschlossen sind:

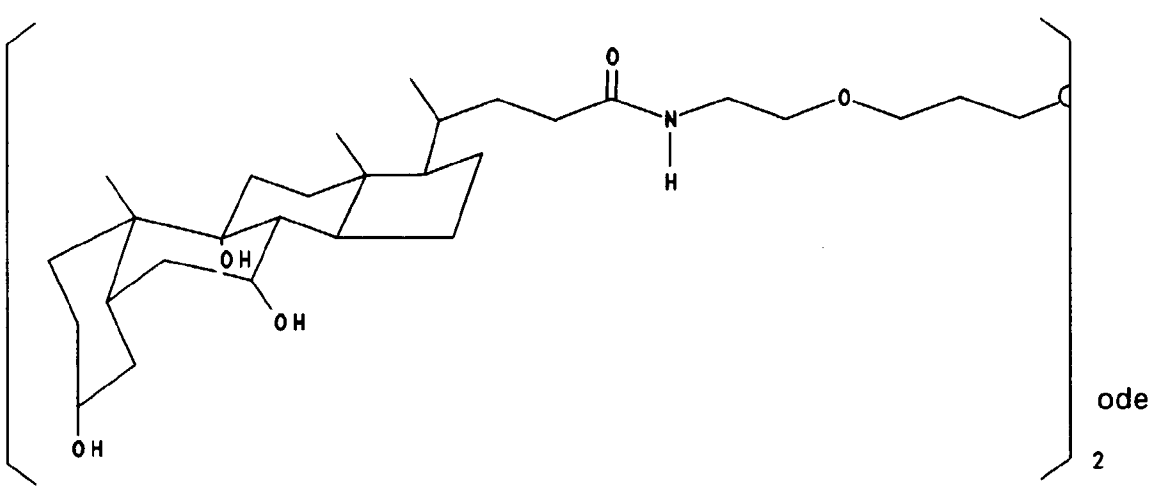

oder

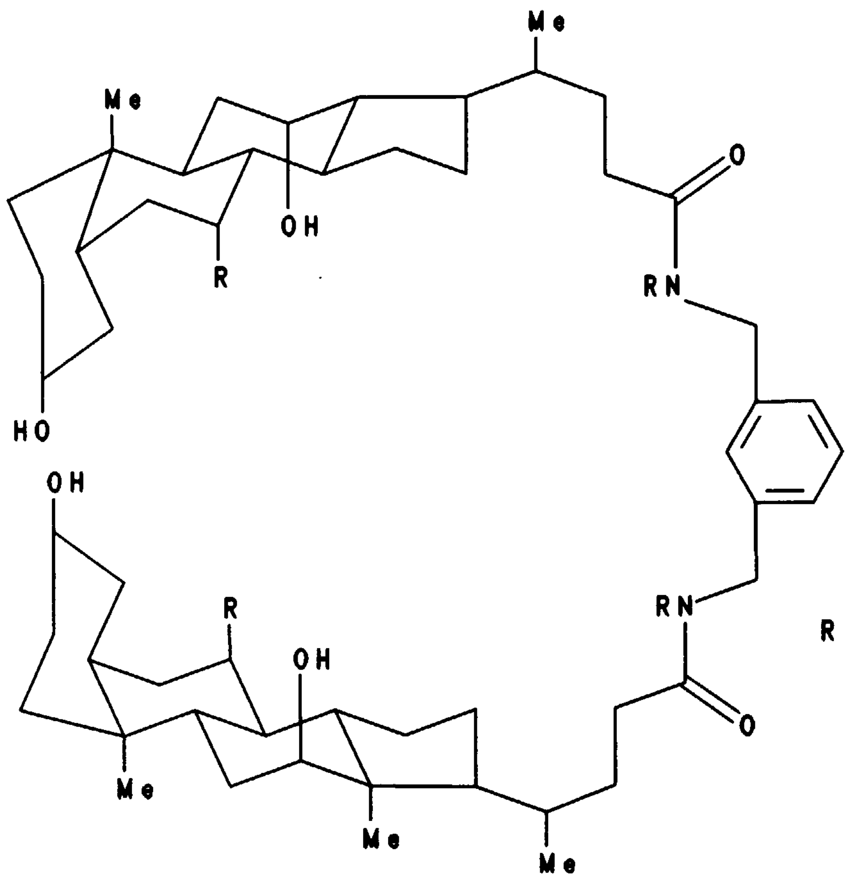

R = H oder OH

**2.** Verfahren nach Anspruch 1, dadurch gekennzeichnet, daß die Verknüpfung der Reste G1 und G2 nicht symmetrisch, d.h. nicht über die gleichen Ringe erfolgt.

**3.** Verfahren nach den Ansprüchen 1 bis 2, dadurch gekennzeichnet, daß die Verknüpfung des Restes G1 durch C-24 (Ring D) über X an eine der Positionen C-3 (Ring A), C-7 (Ring B) oder C-12 (Ring G) des Restes G2 erfolgt.

**4.** Verfahren zur Herstellung einer Verbindung gemäß einem oder mehreren der Ansprüche 1 bis 3, dadurch gekennzeichnet, daß man

a) im Falle von X = Einfachbindung geeignete reaktionsfähige Formen von G1 und G2 nach im Prinzip bekannten Verfahren miteinander zur Reaktion bringt oder

b) im Falle von X = Brückengruppe

α) reaktionsfähige Formen von G1-X mit G2 bzw.
β) reaktionsfähige Formen von G2-X mit G1

nach im Prinzip bekannten Verfahren miteinander zur Reaktion bringt oder

c) aus G1-X1 nach bekannten Verfahren Verbindungen der allgemeinen Formel I (G1-X-G2) herstellt, wobei X aus X1 und X2 entsteht durch Ausbildung einer kovalenten Bindung, insbesondere innerhalb einer Kondensations- oder Substitutionsreaktion.

**5.** Verwendung der Verbindung gemäß den Ansprüchen 1 bis 3 zur Herstellung eines Heilmittels.

**6.** Verbindung gemäß den Ansprüchen 1 bis 3 als Heilmittel.

**7.** Pharmazeutische Zubereitung, enthaltend Verbindungen gemäß den Ansprüchen 1 bis 3.

## Claims

**Claims for the following Contracting States : AT, BE, CH, LI, DE, DK, FR, GB, IT, LU, NL, SE**

**1.** A dimeric bile acid derivative of the formula I

$$G1\text{-}X\text{-}G2 \qquad (I)$$

in which G1 is a compound of the formula II

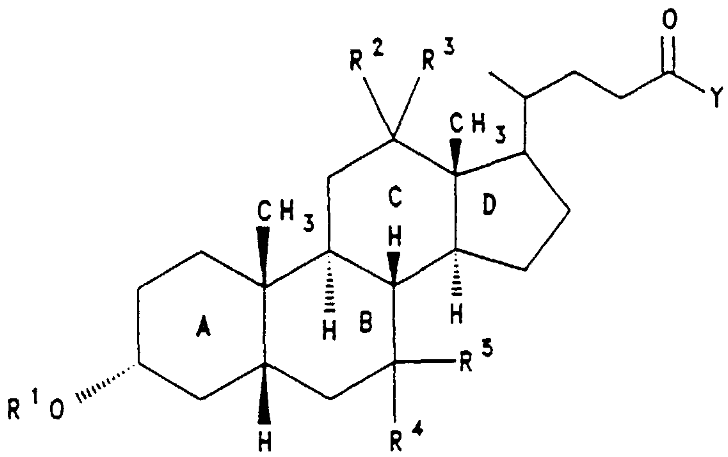

in which

Y is a free valency for bonding the group X or has the following meanings -OL, -NHL, $-NL_2$, an amino acid or aminosulfonic acid bonded via the amino group, such as, for example,

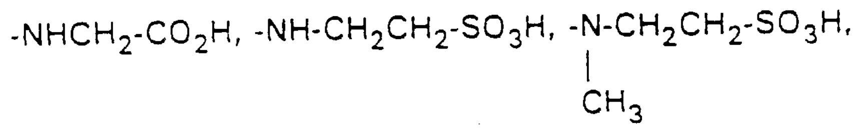

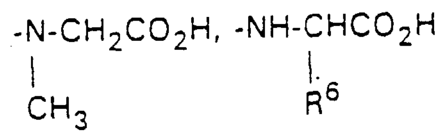

and their $(C_1\text{-}C_4)$-alkyl esters and alkali metal and alkaline earth metal salts, -OKa, where Ka is a cation, such as, for example, an alkali metal or alkaline earth metal ion or alternatively a quaternary ammonium ion and
where L
is H, a saturated or unsaturated alkyl radical having 1-10 carbon atoms, which is branched or unbranched,
a cycloalkyl radical having 3-8 carbon atoms, a phenyl radical which is unsubstituted or monosubstituted to trisubstituted by F, Cl, Br, $(C_1\text{-}C_4)$-alkyl or $(C_1\text{-}C_4)$-alkoxy,
a benzyl radical which is unsubstituted or monosubstituted to trisubstituted by F, Cl, Br, $(C_1\text{-}C_4)$ -alkyl or $(C_1\text{-}C_4)$ -alkoxy,

and $R^6$
is methyl, isopropyl, isobutyl, 2-butyl, benzyl, 4-hydroxybenzyl, hydroxymethyl, 1-hydroxyethyl, $H_3CSCH_2CH_2$-, $HO_2CCH_2$- or $HO_2CCH_2CH_2$-,

$R^1$ is a free valency for bonding the group X or H, a saturated or unsaturated alkyl radical having 1-10 carbon atoms, which is branched or unbranched,

a cycloalkyl radical having 3-8 carbon atoms,
a phenyl radical which is unsubstituted or monosubstituted to trisubstituted by F, Cl, Br, ($C_1$-$C_4$) -alkyl or ($C_1$-$C_4$) -alkoxy, $-N^{\oplus}H_3$, $-OPO_3^{\ominus}$,
a benzyl radical which is unsubstituted in the ring or monosubstituted to trisubstituted by F, Cl, Br, ($C_1$-$C_4$) -alkyl, ($C_1$-$C_4$) -alkoxy, $-N^{\oplus}H_3$, $-OPO_3^{\ominus}$ or phenyl, which can in turn be monosubstituted to trisubstituted by F, Cl, Br, ($C_1$-$C_4$)-alkyl or ($C_1$-$C_4$)-alkoxy, $-N^{\oplus}H_3$, $-OPO_3^{\ominus}$,
a biphenylmethyl radical which is unsubstituted or monosubstituted to trisubstituted by F, Cl, Br, ($C_1$-$C_4$) -alkyl or ($C_1$-$C_4$) -alkoxy, $-N^{\oplus}H_3$, $-OPO_3^{\ominus}$,
a triphenylmethyl radical which is unsubstituted or monosubstituted to trisubstituted by F, Cl, Br, ($C_1$-$C_4$)-alkyl or ($C_1$-$C_4$)-alkoxy, $-N^{\oplus}H_3$, $-OPO_3^{\ominus}$,
a 1- or 2-naphthylmethyl radical which is unsubstituted or monosubstituted to trisubstituted by F, Cl, Br, ($C_1$-$C_4$) -alkyl or ($C_1$-$C_4$)-alkoxy, $-N^{\oplus}H_3$, $-OPO_3^{\ominus}$,
a 9-fluorenyl radical which is unsubstituted or monosubstituted to trisubstituted by F, Cl, Br, ($C_1$-$C_4$) -alkyl or ($C_1$-$C_4$) -alkoxy, $-N^{\oplus}H_3$, $-OPO_3^{\ominus}$ ,
a 2-, 3- or 4-pyridyl radical,
a radical

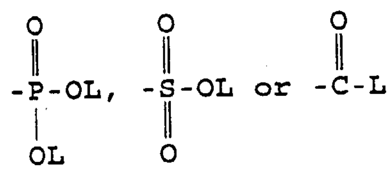

where L has the abovementioned meaning

$R^2$ to $R^5$, where $R^2$ and $R^3$ or $R^4$ and $R^5$ in each case together are the oxygen of a carbonyl group, or individually and in each case independently of one another are

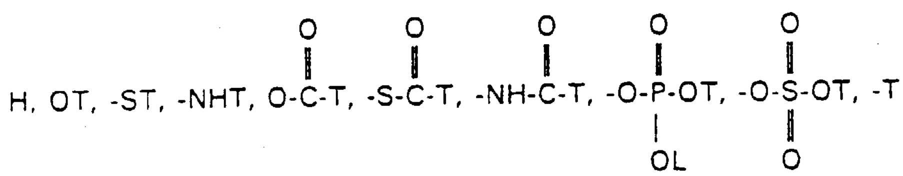

where L has the abovementioned meaning and T has the meaning of L or a free valency for bonding the group X,
=

with the restriction that altogether only one free valency for bonding the group X starts from G1,

X is a single bond or a group of the formula III

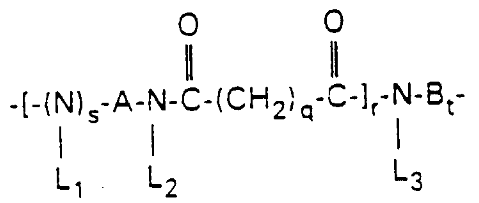

(III)

where

A is an alkylene chain which is branched or unbranched, saturated or unsaturated and can be optionally interrupted in the chain by -O-, -S- or arylene, in particular phenylene, where the linkage

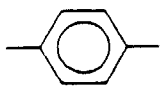 , 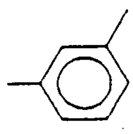 or 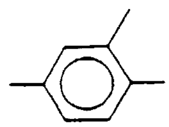

takes place and the chain includes altogether 2 to 12, preferably 2 to 6 chain members p,

B is an alkylene chain which is branched or unbranched, saturated or unsaturated and can be optionally interrupted in the chain by -O-, -S- or arylene, in particular phenylene, where the linkage

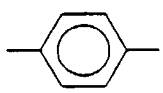 , 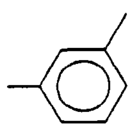 or 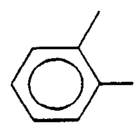

takes place and the chain contains altogether 2 to 18, preferably 2 to 12 chain members n,

$L_1$, $L_2$ and $L_3$ are identical or different and have the meaning of L, and

q is 0 - 5,

r is 0 or 1 and

s is 0 or 1

t is 0 or 1,

G2 is a compound of the formula IV

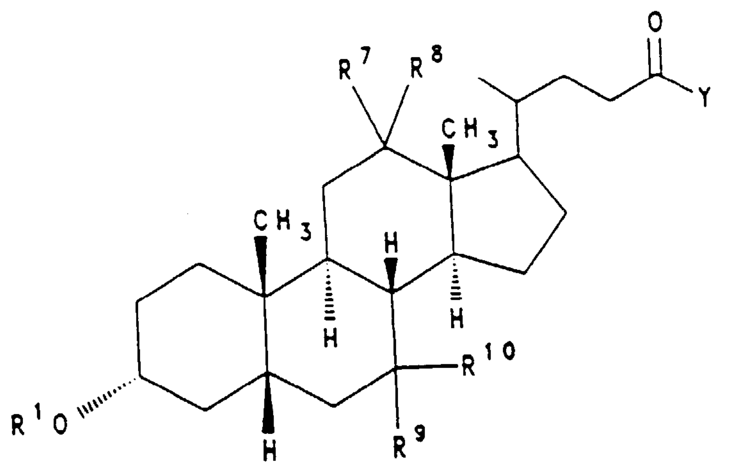

where

Y and $R^1$have the meaning indicated under G1 and

$R^7$ to $R^{10}$,where $R^7$ and $R^8$ or $R^9$ and $R^{10}$ in each case together are the oxygen of a carbonyl group or individually and in each case independently of one another are H, OT, -ST, -NHT,

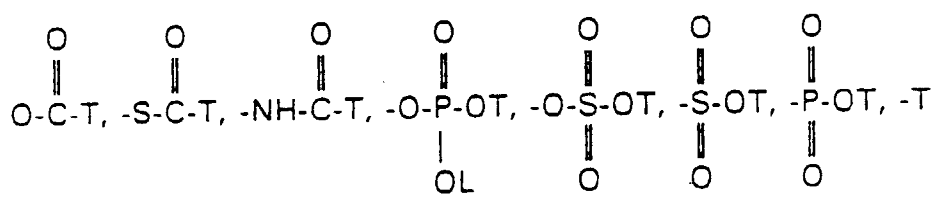

where L and T have the meaning indicated under G1, likewise with the restriction that altogether only one free valency for bonding the group X starts from G2,
bile acid derivatives of the following formulae being excluded:

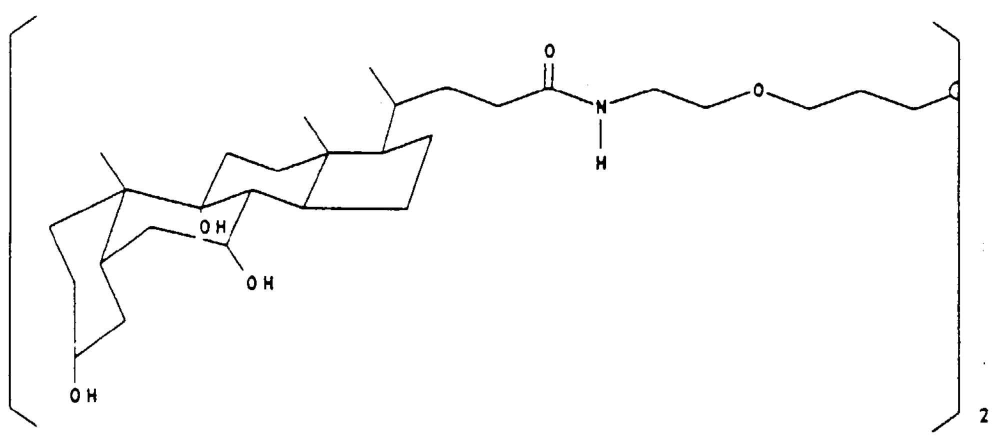

or

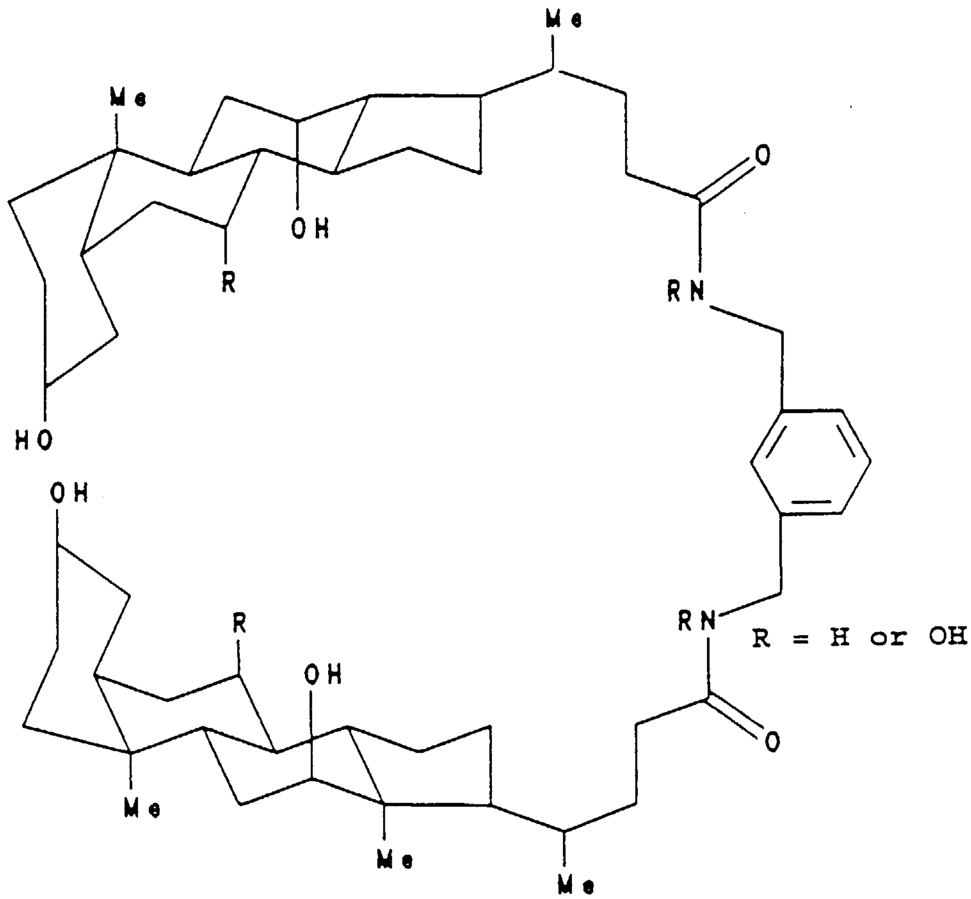

R = H or OH

2. A compound as claimed in claim 1, wherein the linkage of the radicals G1 and G2 is unsymmetrical, i.e. does not take place via the same rings.

3. A compound as claimed in claims 1 to 2, wherein the linkage of the radical G1 takes place through C24 (ring D) via X to one of the positions C3 (ring A), C7 (ring B) or C12 (ring C) of the radical G2.

4. A process for the preparation of a compound as claimed in one or more of claims 1 to 3, which comprises

a) if X = a single bond, bringing suitable reactive forms of G1 and G2 to reaction with one another by processes known in principle or

b) if X = a bridging group

α) bringing reactive forms of G1-X to reaction with G2 or
β) reactive forms of G2-X to reaction with G1 by processes known in principle or

c) compounds of the formula I (G1-X-G2) being prepared from G1-X1 by known methods, X being formed from X1 and X2 by construction of a covalent bond, in particular in the course of a condensation or substitution reaction.

5. The use of the compound as claimed in claims 1 to 3 for the production of a medicament.

6. The compound as claimed in claims 1 to 3 as a medicament.

7. A pharmaceutical preparation containing compounds as claimed in claims 1 to 3.

**Claims for the following Contracting States : ES, GR**

**1.** A process for the preparation of a dimeric bile acid derivative of the formula I

G1-X-G2 (I)

in which G1 is a compound of the formula II

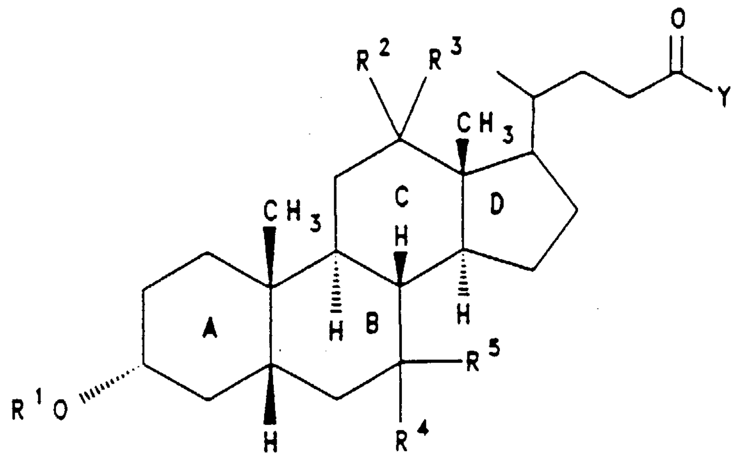

in which

Y is a free valency for bonding the group X or has the following meanings -OL, -NHL, $-NL_2$, an amino acid or aminosulfonic acid bonded via the amino group, such as, for example,

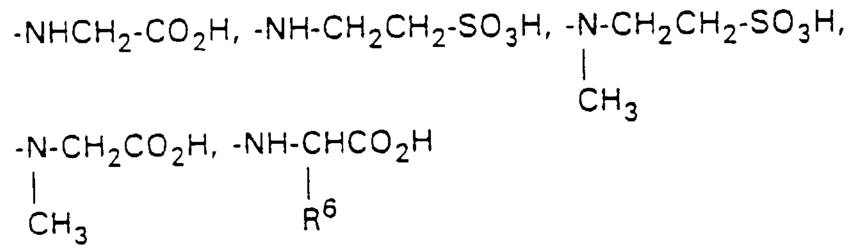

and their $(C_1-C_4)$-alkyl esters and alkali metal and alkaline earth metal salts, -OKa, where Ka is a cation, such as, for example, an alkali metal or alkaline earth metal ion or alternatively a quaternary ammonium ion and
where L
is H, a saturated or unsaturated alkyl radical having 1-10 carbon atoms, which is branched or unbranched,
a cycloalkyl radical having 3-8 carbon atoms,
a phenyl radical which is unsubstituted or monosubstituted to trisubstituted by F, Cl, Br, $(C_1-C_4)$-alkyl or $(C_1-C_4)$-alkoxy,
a benzyl radical which is unsubstituted or monosubstituted to trisubstituted by F, Cl, Br, $(C_1-C_4)$ -alkyl or $(C_1-C_4)$ -alkoxy,
and $R^6$
is methyl, isopropyl, isobutyl, 2-butyl, benzyl, 4-hydroxybenzyl, hydroxymethyl, 1-hydroxyethyl, $H_3CSCH_2CH_2$-, $HO_2CCH_2$- or $HO_2CCH_2CH_2$-,

$R^1$ is a free valency for bonding the group X or H, a saturated or unsaturated alkyl radical having 1-10

carbon atoms, which is branched or unbranched,

a cycloalkyl radical having 3-8 carbon atoms,
a phenyl radical which is unsubstituted or monosubstituted to trisubstituted by F, Cl, Br, $(C_1\text{-}C_4)$ -alkyl or $(C_1\text{-}C_4)$ -alkoxy, $-N^{\oplus}H_3$, $-OPO_3^{\ominus}$,
a benzyl radical which is unsubstituted in the ring or monosubstituted to trisubstituted by F, Cl, Br, $(C_1\text{-}C_4)$-alkyl, $(C_1\text{-}C_4)$-alkoxy, $-N^{\oplus}H_3$, $-OPO_3^{\ominus}$ or phenyl, which can in turn be monosubstituted to trisubstituted by F, Cl, Br, $(C_1\text{-}C_4)$-alkyl or $(C_1\text{-}C_4)$ -alkoxy, $N^{\oplus}H_3$, $-OPO_3^{\ominus}$,
a biphenylmethyl radical which is unsubstituted or monosubstituted to trisubstituted by F, Cl, Br, $(C_1\text{-}C_4)$ -alkyl or $(C_1\text{-}C_4)$-alkoxy, $-N^{\oplus}H_3$, $-OPO_3^{\ominus}$,
a triphenylmethyl radical which is unsubstituted or monosubstituted to trisubstituted by F, Cl, Br, $(C_1\text{-}C_4)$-alkyl or $(C_1\text{-}C_4)$ -alkoxy, $-N^{\oplus}H_3$, $-OPO_3^{\ominus}$,
a 1- or 2-naphthylmethyl radical which is unsubstituted or monosubstituted to trisubstituted by F, Cl, Br, $(C_1\text{-}C_4)$-alkyl or $(C_1\text{-}C_4)$-alkoxy, $-N^{\oplus}H_3$, $-OPO_3^{\ominus}$,
a 9-fluorenyl radical which is unsubstituted or monosubstituted to trisubstituted by F, Cl, Br, $(C_1\text{-}C_4)$ -alkyl or $(C_1\text{-}C_4)$ -alkoxy, $-N^{\oplus}H_3$, $-OPO_3^{\ominus}$ ,
a 2-, 3- or 4-pyridyl radical,
a radical

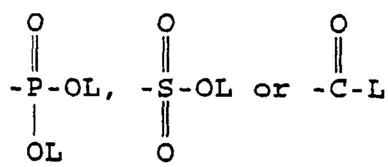

where L has the abovementioned meaning

R² to R⁵, where $R^2$ and $R^3$ or $R^4$ and $R^5$ in each case together are the oxygen of a carbonyl group, or individually and in each case independently of one another are

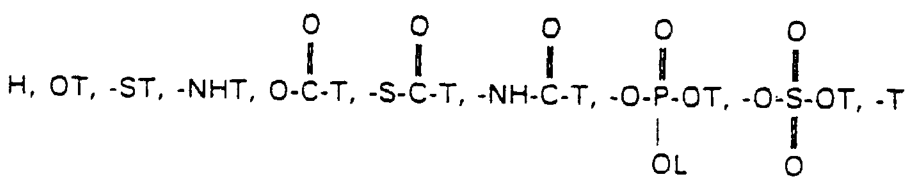

where L has the abovementioned meaning and T has the meaning of L or a free valency for bonding the group X, with the restriction that altogether only one free valency for bonding the group X starts from G1,

X is a single bond or a group of the formula III

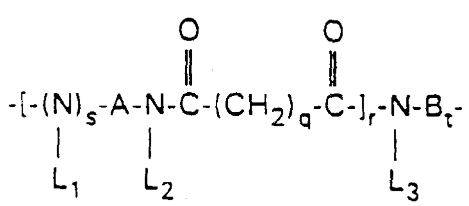

(III)

where

A is an alkylene chain which is branched or unbranched, saturated or unsaturated and can be optionally interrupted in the chain by -O-, -S- or arylene, in particular phenylene, where the linkage

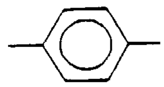 , 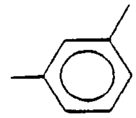 or 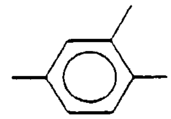

takes place and the chain includes altogether 2 to 12, preferably 2 to 6 chain members p,

B is an alkylene chain which is branched or unbranched, saturated or unsaturated and can be optionally interrupted in the chain by -O-, -S- or arylene, in particular phenylene, where the linkage

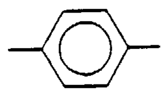 , 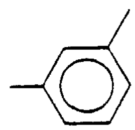 or 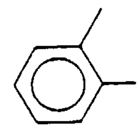

takes place and the chain contains altogether 2 to 18, preferably 2 to 12 chain members n,

$L_1$, $L_2$ and $L_3$ are identical or different and have the meaning of L, and

q is 0 - 5,

r is 0 or 1 and

s is 0 or 1

t is 0 or 1,

G2 is a compound of the formula IV

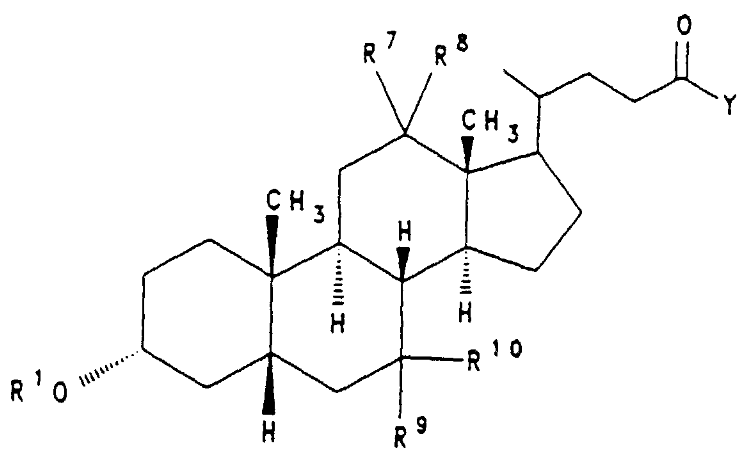

where

Y and $R^1$ have the meaning indicated under G1 and

$R^7$ to $R^{10}$, where $R^7$ and $R^8$ or $R^9$ and $R^{10}$ in each case together are the oxygen of a carbonyl group or individually and in each case independently of one another are H, OT, -ST, -NHT,

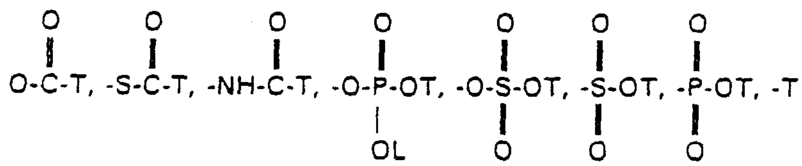

where L and T have the meaning indicated under G1, likewise with the restriction that altogether only one free valency for bonding the group X starts from G2, bile acid derivatives of the following formulae being excluded:

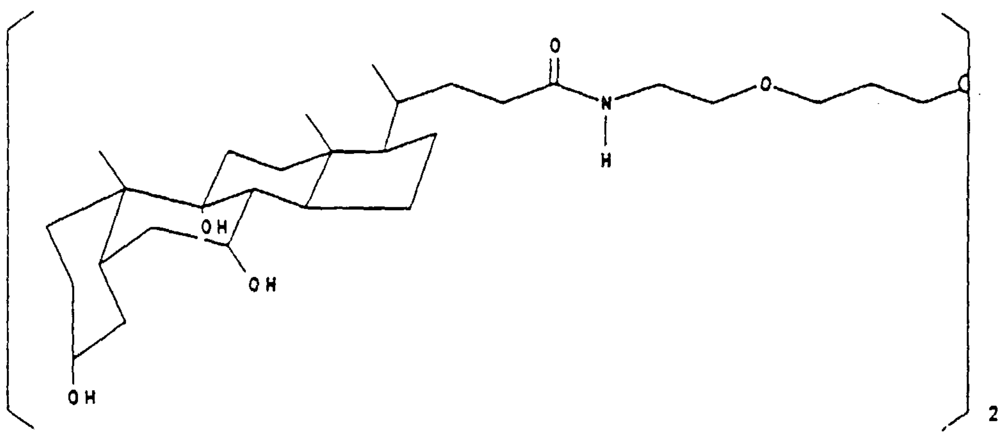

or

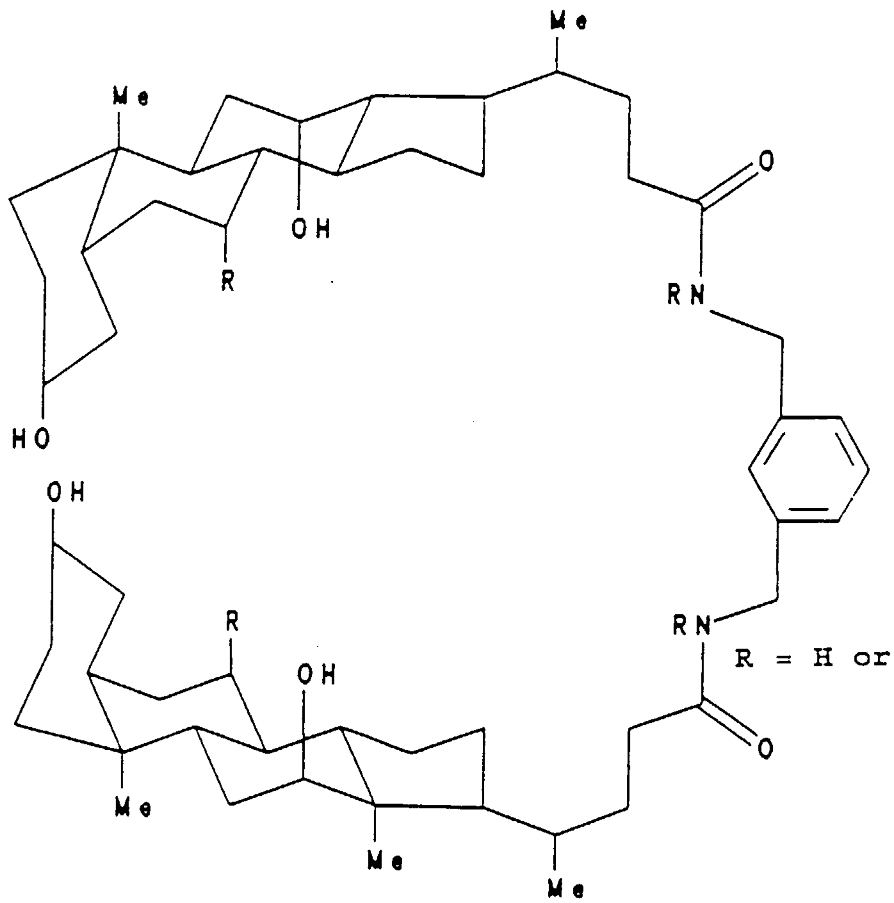

R = H or OH

2. The process as claimed in claim 1, wherein the linkage of the radicals G1 and G2 is unsymmetrical, i.e. does not take place via the same rings.

3. The process as claimed in claims 1 to 2, wherein the linkage of the radical G1 takes place through C24 (ring D) via X to one of the positions C3 (ring A), C7 (ring B) or C12 (ring C) of the radical G2.

4. A process for the preparation of a compound as claimed in one or more of claims 1 to 3, which comprises

a) if X = a single bond, bringing suitable reactive forms of G1 and G2 to reaction with one another by processes known in principle or

b) if X = a bridging group

α) bringing reactive forms of G1-X to reaction with $G^2$ or
β) reactive forms of G2-X to reaction with G1 by processes known in principle or

c) compounds of the formula I (G1-X-G2) being prepared from G1-X1 by known methods, X being formed from X1 and X2 by construction of a covalent bond, in particular in the course of a condensation or substitution reaction.

5. The use of the compound as claimed in claims 1 to 3 for the production of a medicament.

6. The compound as claimed in claims 1 to as a medicament.

7. A pharmaceutical preparation containing compounds as claimed in claims 1 to 3.

## Revendications

**Revendications pour les Etats Contractants suivants : AT, BE, CH, LI, DE, DK, FR, GB, IT, LU, NL, SE**

**1.** Dérivés dimères d'acides biliaires de formule I

G1-X-G2 (I)

dans laquelle G1 représente un composé de formule générale II

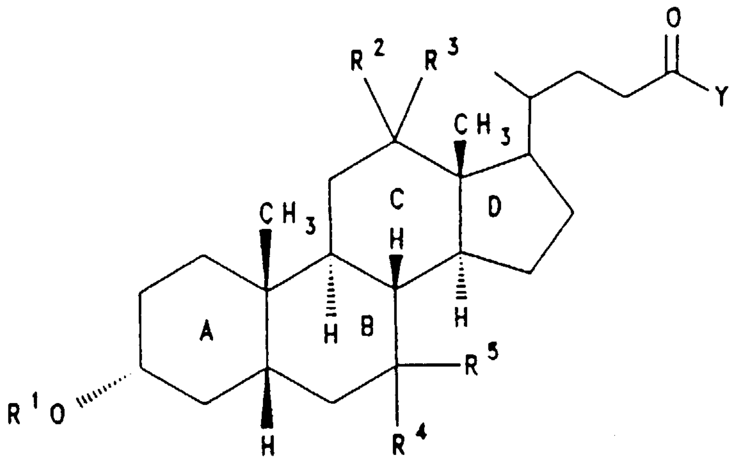

dans laquelle

Y représente une valence libre pour la liaison du groupe X ou a les significations suivantes: -OL, -NHL, $-NL_2$,

un aminoacide ou acide aminosulfonique lié par le groupe amino, comme par exemple $-NHCH_2-CO_2H$, $-NH-CH_2CH_2-SO_3H$,

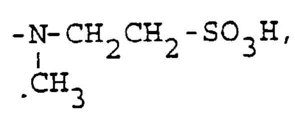 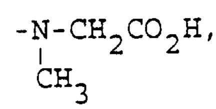 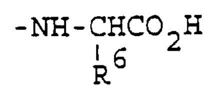

et ses esters alkyliques en $C_1$-$C_4$ et sels alcalins et alcalino-terreux, -OKa, Ka représentant un cation, comme par exemple un ion alcalin ou alcalino-terreux ou bien un ammonium quaternaire et
L représentant
H, un radical alkyle saturé ou insaturé ayant de 1 à 10 atomes de carbone, qui est ramifié ou non ramifié, un radical cycloalkyle ayant de 3 à 8 atomes de carbone, un radical phényle qui est non substitué ou 1 à 3 fois substitué par un ou des atomes de F, Cl, Br ou groupes alkyle en $C_1$-$C_4$ ou alcoxy en $C_1$-$C_4$,
un radical benzyle qui est non substitué ou 1 à 3 fois substitué par un ou des atomes de F, Cl, Br ou groupes alkyle en $C_1$-$C_4$ ou alcoxy en $C_1$-$C_4$,
et $R^6$ représentant le groupe méthyle, isopropyle, isobutyle, 2-butyle, benzyle, 4-hydroxybenzyle, hydroxy-méthyle, 1-hydroxyéthyle, $H_3CSCH_2CH_2$-, $HO_2CCH_2$-, $HO_2CCH_2CH_2$-,

$R^1$ représente une valence libre pour la liaison du groupe X ou H,

un radical alkyle saturé ou insaturé ayant de 1 à 10 atomes de carbone, qui est ramifié ou non

ramifié,
un radical cycloalkyle ayant de 3 à 8 atomes de carbone,
un radical phényle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical benzyle qui est non substitué sur le noyau ou 1 à 3 fois substitué sur le noyau par un ou des atomes de F, Cl, Br ou groupes alkyle en $C_1$-$C_4$, alcoxy en $C_1$-$C_4$, $-N^{\oplus}H_3$, $-OPO_3^{\ominus}$ ou phényle qui, pour sa part, peut être 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical biphénylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical triphénylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical 1- ou 2-naphtylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical 9-fluorényle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,
un radical 2-, 3- ou 4-pyridyle, un radical

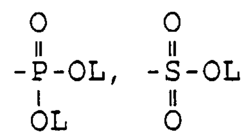

ou

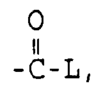

L ayant la signification indiquée plus haut,
$R^2$ à $R^5$ représentent, en ce qui concerne $R^2$ et $R^3$ ou $R^4$ et $R^5$, respectivement, conjointement l'atome d'oxygène d'un groupe carbonyle ou, individuellement et chacun indépendamment les uns des autres,

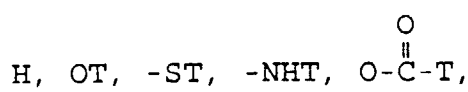

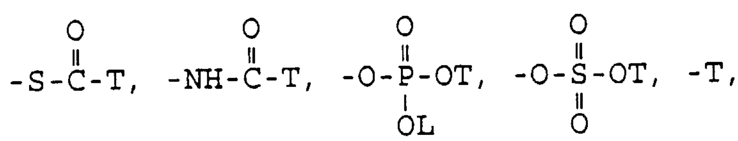

L ayant la signification donnée plus haut et T ayant la signification de L,
ou une valence libre pour la liaison du groupe X,
avec la restriction qu'au total une seule valence libre serve à la liaison du groupe X,

X représente une liaison simple ou un groupe de formule générale III

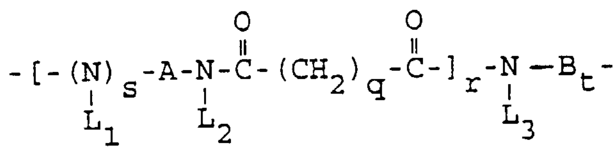

(III)

dans laquelle

A représente une chaîne alkylène qui est ramifiée ou non ramifiée, saturée ou non saturée, et peut éventuellement être interrompue dans la chaîne par -O-, -S- ou par un groupe arylène, en particulier phénylène, la liaison s'effectuant de la façon suivante

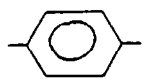 , 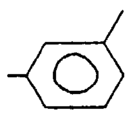 ou 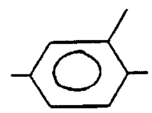

et la chaîne contenant au total de 2 à 12, de préférence 2 à 6 chaînons p,

B représente une chaîne alkylène qui est ramifiée ou non ramifiée, saturée ou insaturée, et peut éventuellement être interrompue dans la chaîne par -O-, -S- ou par un groupe arylène, en particulier phénylène, la liaison s'effectuant de la façon suivante

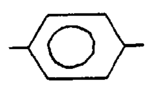 , 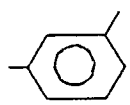 ou 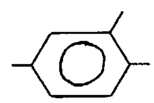

et la chaîne contenant au total de 2 à 18, de préférence 2 à 12 chaînons n,

$L_1$, $L_2$, $L_3$ sont identiques ou différents et ont la signification de L, et

q = 0 à 5,

r = 0 ou 1,

s = 0 ou 1 et

t = 0 ou 1,

G2 représente un composé de formule générale IV

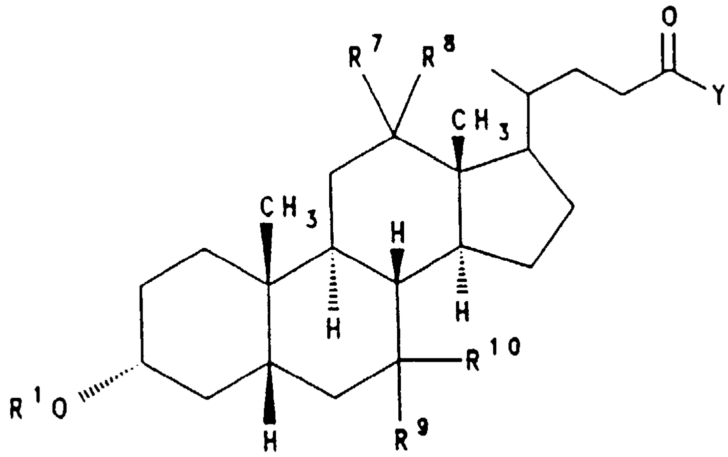

dans laquelle

Y et $R^1$ ont les significations indiquées à propos de G1, et
$R^7$ à $R^{10}$ représentent, en ce qui concerne $R^7$ et $R^8$ ou respectivement $R^9$ et $R^{10}$, conjointement l'atome d'oxygène d'un groupe carbonyle ou, individuellement et chacun indépendamment les uns des autres, H, OT, -ST, -NHT,

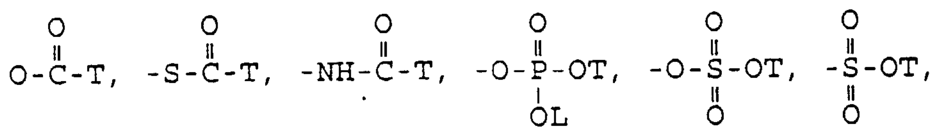

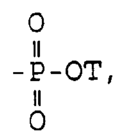

-T, L et T ayant les significations données à propos de G1, également avec la restriction qu'au total une seule valence libre de G2 serve à la liaison du groupe X,
à l'exclusion des dérivés d'acides biliaires de formules générales:

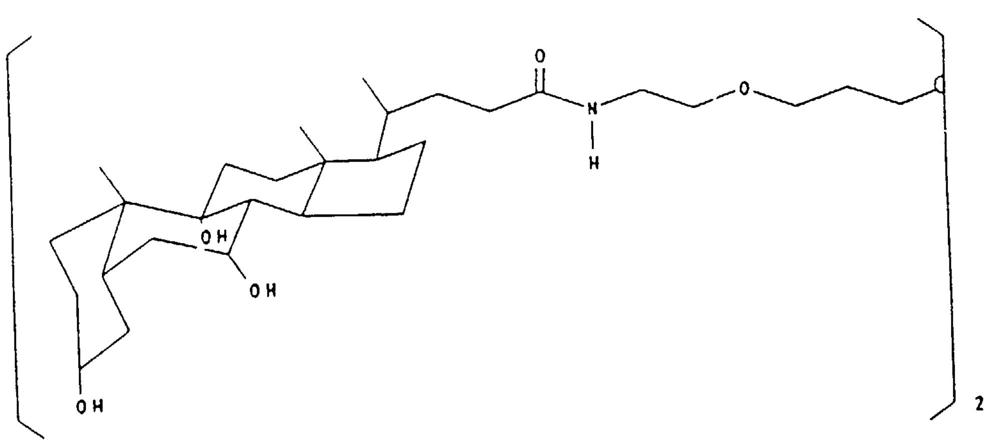

ou

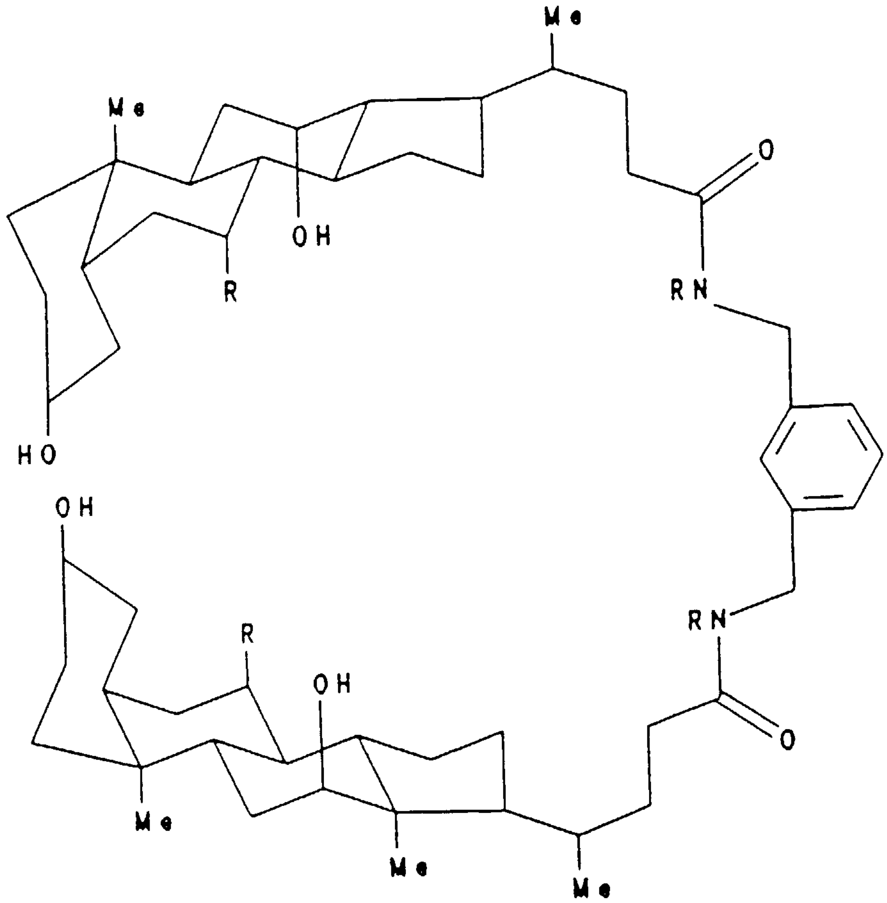

R = H ou OH

**2.** Composé selon la revendication 1, caractérisé en ce que la liaison des radicaux G1 et G2 n'est pas symétrique, c'est-à-dire qu'elle ne s'effectue pas par les mêmes cycles.

**3.** Composé selon les revendications 1 et 2, caractérisé en ce que la liaison du radical G1 s'effectue par C-24 (cycle D) par l'intermédiaire de X en l'une des positions C-3 (cycle A) , C-7 (cycle B) ou C-12 (cycle G) du radical G2.

**4.** Procédé pour la préparation d'un composé selon une ou plusieurs des revendications 1 à 3, caractérisé en ce que

a) lorsque X représente une liaison simple, on fait réagir l'une avec l'autre, selon des procédés en principe connus, des formes réactives appropriées de G1 et G2, ou
b) lorsque X représente un groupe pontant, on fait réagir l'une avec l'autre, selon des procédés en principe connus,

α) des formes réactives de G1-X avec G2 ou
β) des formes réactives de G2-X avec G1,

ou
c) à partir de G1-$X_1$, on prépare, selon des procédés connus, des composés de formule générale I (G1-X-G2), X étant formé à partir de $X_1$ et $X_2$, par formation d'une liaison covalente, en particulier dans une réaction de condensation ou de substitution.

**5.** Utilisation du composé selon les revendications 1 à 3, pour la fabrication d'un médicament.

**6.** Composé selon les revendications 1 à 3, en tant que médicament.

**7.** Composition pharmaceutique contenant des composés selon les revendications 1 à 3.

**Revendications pour les Etats Contractants suivants : ES, GR**

**1.** Procédé pour la préparation de dérivés dimères d'acides biliaires de formule I

G1-X-G2 (I)

dans laquelle G1 représente un composé de formule générale II

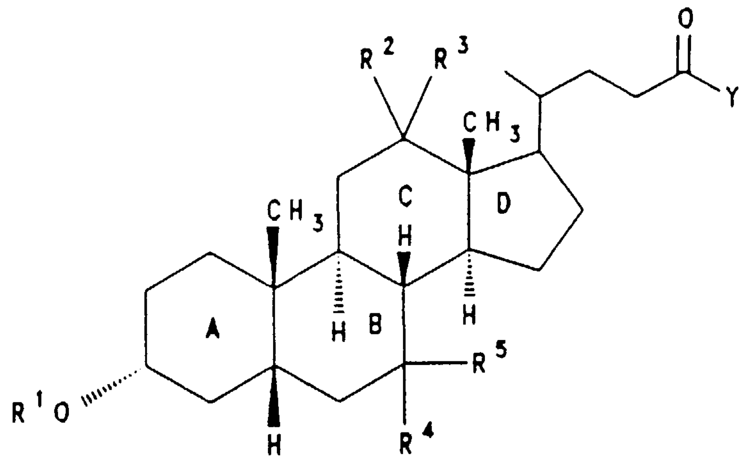

dans laquelle

Y représente une valence libre pour la liaison du groupe X ou a les significations suivantes: -OL, -NHL, $-NL_2$,

un aminoacide ou acide aminosulfonique lié par le groupe amino, comme par exemple $-NHCH_2-CO_2H$, $-NH-CH_2CH_2-SO_3H$,

$-N(CH_3)-CH_2CH_2-SO_3H$, $-N(CH_3)-CH_2CO_2H$, $-NH-CH(R^6)CO_2H$

et ses esters alkyliques en $C_1$-$C_4$ et sels alcalins et alcalino-terreux, -OKa, Ka représentant un cation, comme par exemple un ion alcalin ou alcalino-terreux ou bien un ammonium quaternaire et
L représentant
H, un radical alkyle saturé ou insaturé ayant de 1 à 10 atomes de carbone, qui est ramifié ou non ramifié,
un radical cycloalkyle ayant de 3 à 8 atomes de carbone,
un radical phényle qui est non substitué ou 1 à 3 fois substitué par un ou des atomes de F, Cl, Br ou groupes alkyle en $C_1$-$C_4$ ou alcoxy en $C_1$-$C_4$,
un radical benzyle qui est non substitué ou 1 à 3 fois substitué par un ou des atomes de F, Cl, Br ou groupes alkyle en $C_1$-$C_4$ ou alcoxy en $C_1$-$C_4$,
et $R^6$ représentant le groupe méthyle, isopropyle, isobutyle, 2-butyle, benzyle, 4-hydroxybenzyle, hydroxyméthyle, 1-hydroxyméthyle, $H_3CSCH_2CH_2$-, $HO_2CCH_2$-, $HO_2CCH_2CH_2$-,

$R^1$ représente une valence libre pour la liaison du groupe X ou H,

un radical alkyle saturé ou insaturé ayant de 1 à 10 atomes de carbone, qui est ramifié ou non ramifié, un radical cycloalkyle ayant de 3 à 8 atomes de carbone, un radical phényle qui est non substitué ou 1

à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,

un radical benzyle qui est non substitué sur le noyau ou 1 à 3 fois substitué sur le noyau par un ou des atomes de F, Cl, Br ou groupes alkyle en $C_1$-$C_4$, alcoxy en $C_1$-$C_4$, $-N^{\oplus}H_3$, $-OPO_3^{\ominus}$ ou phényle qui, pour sa part, peut être 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,

un radical biphénylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,

un radical triphénylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,

un radical 1- ou 2-naphtylméthyle qui est non substitué ou 1 à 3 fois substitué par un ou des atomes de F, Cl, Br ou groupes alkyle en $C_1$-$C_4$ ou alcoxy en $C_1$-$C_4$, $-N^{\oplus}H_3$, $-OPO_3^{\ominus}$,

un radical 9-fluorényle qui est non substitué ou 1 à 3 fois substitué par un ou des atomes de F, Cl, Br ou groupes alkyle en $C_1$-$C_4$ ou alcoxy en $C_1$-$C_4$, $N^{\oplus}H_3$, $-OPO_3^{\ominus}$,

un radical 2-, 3- ou 4-pyridyle, un radical

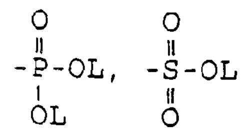

ou

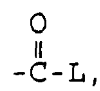

L ayant la signification indiquée plus haut,

$R^2$ à $R^5$ représentent, en ce qui concerne $R^2$ et $R^3$ ou $R^4$ et $R^5$, respectivement, conjointement l'atome d'oxygène d'un groupe carbonyle ou, individuellement et chacun indépendamment les uns des autres,

H, OT, -ST, -NHT, 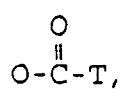

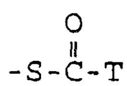 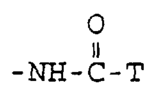 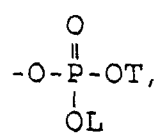 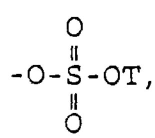 -T,

L ayant la signification donnée plus haut et T ayant la signification de L,

ou une valence libre pour la liaison du groupe X,

avec la restriction qu'au total une seule valence libre serve à la liaison du groupe X,

X représente une liaison simple ou un groupe de formule générale III

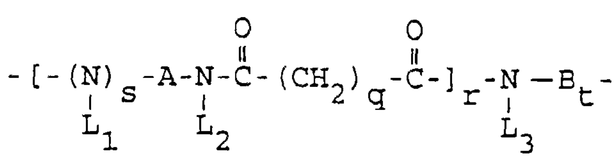 (III)

dans laquelle

A représente une chaîne alkylène qui est ramifiée ou non ramifiée, saturée ou non saturée, et peut éventuellement être interrompue dans la chaîne par -O-, -S- ou par un groupe arylène, en particulier phénylène, la liaison s'effectuant de la façon suivante

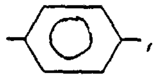, 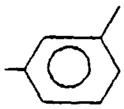 ou 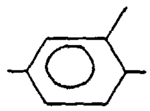

et la chaîne contenant au total de 2 à 12, de préférence 2 à 6 chaînons p,

B représente une chaîne alkylène qui est ramifiée ou non ramifiée, saturée ou insaturée, et peut éventuellement être interrompue dans la chaîne par -O-, -S- ou par un groupe arylène, en particulier phénylène, la liaison s'effectuant de la façon suivante

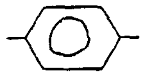, 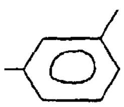 ou 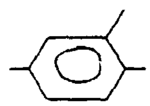

et la chaîne contenant au total de 2 à 18, de préférence 2 à 12 chaînons n,

$L_1$, $L_2$, $L_3$ sont identiques ou différents et ont la signification de L, et

q = 0 à 5,
r = 0 ou 1,
s = 0 ou 1 et
t = 0 ou 1,
G2 représente un composé de formule générale IV

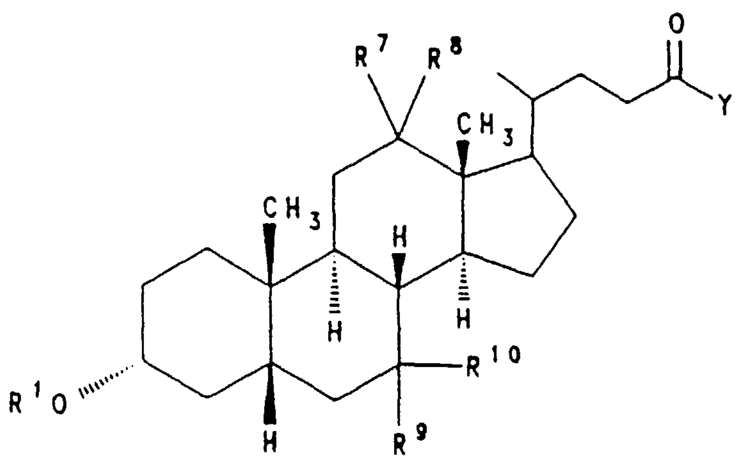

dans laquelle

Y et $R^1$ ont les significations indiquées à propos de G1, et
$R^7$ à $R^{10}$ représentent, en ce qui concerne $R^7$ et $R^8$ ou respectivement $R^9$ et $R^{10}$, conjointement l'atome d'oxygène d'un groupe carbonyle ou, individuellement et chacun indépendamment les uns des autres, H, OT, -ST,

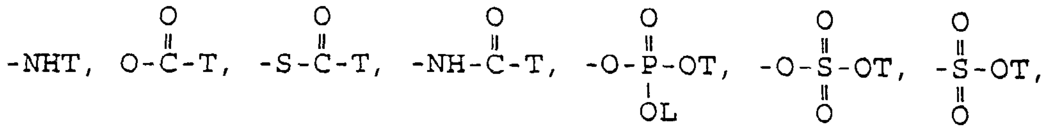

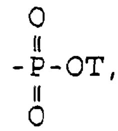

-T, L et T ayant les significations données à propos de G1, également avec la restriction qu'au total une seule valence libre de G2 serve à la liaison du groupe X,

à l'exclusion des dérivés d'acides biliaires de formules générales:

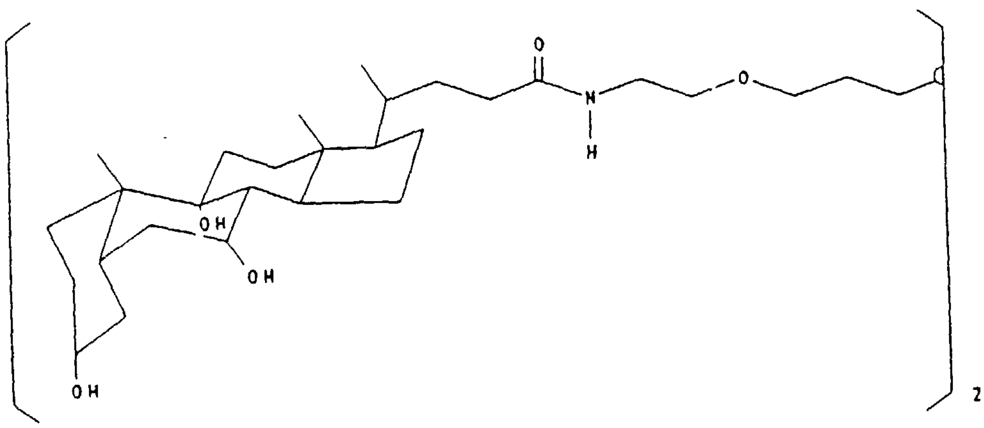

ou

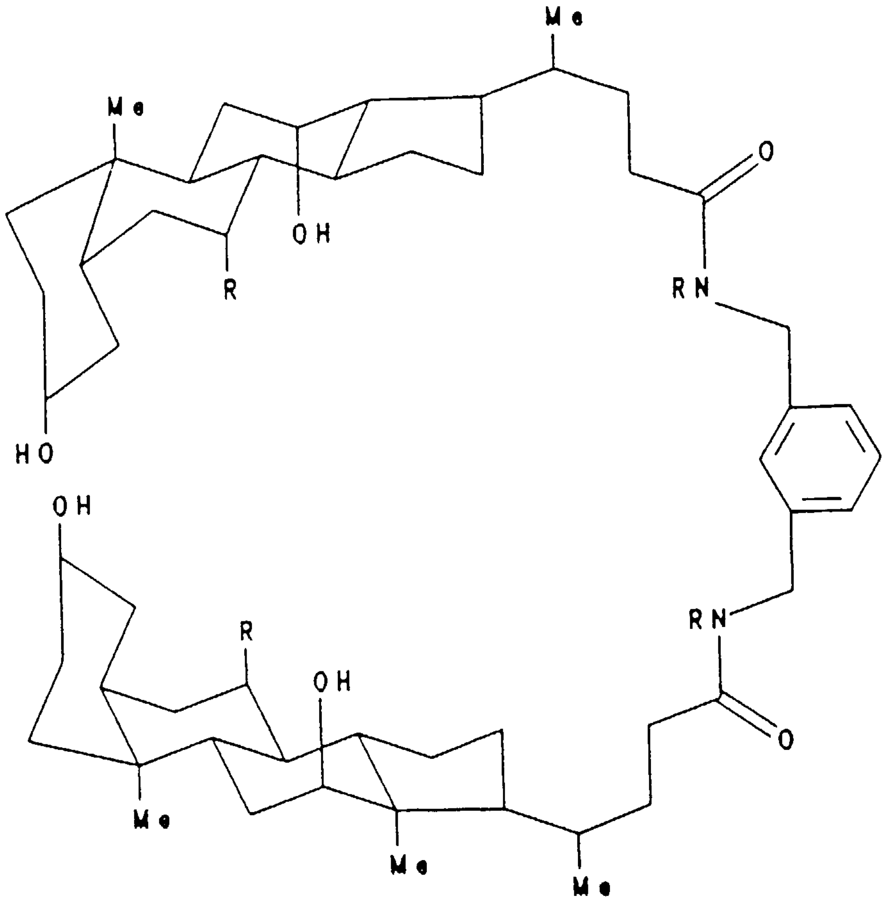

R = H ou OH

2. Procédé selon la revendication 1, caractérisé en ce que la liaison des radicaux G1 et G2 n'est pas symétrique, c'est-à-dire qu'elle ne s'effectue pas par les mêmes cycles.

3. Procédé selon les revendications 1 et 2, caractérisé en ce que la liaison du radical G1 s'effectue par C-24 (cycle D) par l'intermédiaire de X en l'une des positions C-3 (cycle A), C-7 (cycle B) ou C-12 (cycle G) du radical G2.

4. Procédé pour la préparation d'un composé selon une ou plusieurs des revendications 1 à 3, caractérisé en ce que

a) lorsque X représente une liaison simple, on fait réagir l'une avec l'autre, selon des procédés en principe connus, des formes réactives appropriées de G1 et G2, ou
b) lorsque X représente un groupe pontant, on fait réagir l'une avec l'autre, selon des procédés en principe connus,

α) des formes réactives de G1-X avec G2 ou
β) des formes réactives de G2-X avec G1,

ou
c) à partir de G1-$X_1$, on prépare, selon des procédés connus, des composés de formule générale I (G1-X-G2), X étant formé à partir de $X_1$ et $X_2$, par formation d'une liaison covalente, en particulier dans une réaction de condensation ou de substitution.

5. Utilisation du composé selon les revendications 1 à 3, pour la fabrication d'un médicament.

6. Composé selon les revendications 1 à 3, en tant que médicament.

7. Composition pharmaceutique contenant des composés selon les revendications 1 à 3.